# EUROPEAN PATENT APPLICATION

(11) **EP 2 455 397 A1**
(43) Date of publication of application: **23.05.2012**
(21) Application number: 10799831.2
(22) Date of filing: 13.07.2010
(51) Int. Cl.: C07K 14/82, C12N 15/09, C30B 7/04, C30B 29/58, G01N 33/15, G01N 33/50

(54) **MUTANT RAS POLYPEPTIDE CRYSTAL**

(30) Priority: 14.07.2009 JP 2009165717
(71) Applicant: National University Corporation Kobe University, Kobe-shi, Hyogo 657-8501 (JP); Japan Synchrotron Radiation Research Institute, Hyogo 679-5198 (JP)
(72) Inventor: KATAOKA, Tohru, Kobe-shi Hyogo 657-8501 (JP); SHIMA, Fumi, Kobe-shi Hyogo 657-8501 (JP); TAMURA, Atsuo, Kobe-shi Hyogo 657-8501 (JP); KUMASAKA, Takashi, Sayo-gun Hyogo 679-5198 (JP)
(74) Representative: Krauss, Jan
(86) International application number: PCT/JP2010/061821
(87) International publication number: WO 2011/007773

(57) **Abstract**

An object of the present invention is to provide a co-crystal of a Ras polypeptide which adopts a conformation having a pocket on the molecular surface of Ras and GTP or a GTP analog, a production method for the crystal, and a screening method for a Ras function inhibitor based on information about the conformation obtained by X-ray crystallographic analysis using the crystal. The object is achieved by focusing on a mutation which adopts a conformation having a pocket on the molecular surface of Ras, acquiring a mutant Ras polypeptide having introduced therein such mutation, producing a co-crystal of the mutant Ras polypeptide and a GTP analog, and further subjecting the co-crystal to X-ray crystallographic analysis to acquire structural information about the conformation including information about the structure surrounding the pocket.

SEQUENCE LISTING

<110>  National University Corporation KOBE University
       Japan Synchrotron Radiation Research Institute

<120>  Crystal structure of Ras polypeptide with mutation

<130>  GP10-1019PCT

<150>  2009-165717
<151>  2009-07-14

<160>  10

<170>  PatentIn version 3.5

<210>  1
<211>  166
<212>  PRT
<213>  Homo sapiens

<400>  1

<210>  2
<211>  178
<212>  PRT
<213>  Mus musculus

<400>  2

Met Ala Thr Ser Ala Val Pro Ser Glu Asn Leu Pro Thr Tyr Lys Leu
1               5                   10                  15

Val Val Val Gly Asp Gly Gly Val Gly Lys Ser Ala Leu Thr Ile Gln
            20                  25                  30

Phe Phe Gln Lys Ile Phe Val Pro Asp Tyr Asp Pro Thr Ile Glu Asp
        35                  40                  45

Ser Tyr Leu Lys His Thr Glu Ile Asp Asn Gln Trp Ala Ile Leu Asp
    50                  55                  60

Val Leu Asp Thr Ala Gly Gln Glu Glu Phe Ser Ala Met Arg Glu Gln
65                  70                  75                  80

Tyr Met Arg Thr Gly Asp Gly Phe Leu Ile Val Tyr Ser Val Thr Asp
                85                  90                  95

Lys Ala Ser Phe Glu His Val Asp Arg Phe His Gln Leu Ile Leu Arg
            100                 105                 110

Val Lys Asp Arg Glu Ser Phe Pro Met Ile Leu Val Ala Asn Lys Val
        115                 120                 125

Asp Leu Met His Leu Arg Lys Val Thr Arg Asp Gln Gly Lys Glu Met
    130                 135                 140

Ala Thr Lys Tyr Asn Ile Pro Tyr Ile Glu Thr Ser Ala Lys Asp Pro
145                 150                 155                 160

Pro Leu Asn Val Asp Lys Thr Phe His Asp Leu Val Arg Val Ile Arg
                165                 170                 175

Gln Gln


<210>  3
<211>  7
<212>  PRT
<213>  Artificial Sequence

<220>
<223>  Partial amino acid sequence of Ras

<400>  3

Tyr Asp Pro Thr Ile Glu Asp
1               5


<210>  4
<211>  17
<212>  PRT
<213>  Artificial Sequence

<220>
<223>  Partial amino acid sequence of Ras


<220>
<221>  MISC_FEATURE
<222>  (1)..(5)
<223>  Xaa can be any naturally or unnaturally occurring amino acid

<220>
<221>  MISC_FEATURE
<222>  (13)..(17)
<223>  Xaa can be any naturally or unnaturally occurring amino acid

<400>  4

Xaa Xaa Xaa Xaa Xaa Tyr Asp Pro Thr Ile Glu Asp Xaa Xaa Xaa Xaa
1               5                   10                  15


Xaa



<210>  5
<211>  17
<212>  PRT
<213>  Artificial Sequence

<220>
<223>  Partial amino acid sequence of H-Ras

<400>  5

His Phe Val Asp Glu Tyr Asp Pro Thr Ile Glu Asp Ser Tyr Arg Lys
1               5                   10                  15


Gln



<210>  6
<211>  17
<212>  PRT
<213>  Artificial Sequence

<220>
<223>  Partial amino acid sequence of M-Ras

<400>  6

Ile Phe Val Pro Asp Tyr Asp Pro Thr Ile Glu Asp Ser Tyr Leu Lys
1               5                   10                  15

His

<210>  7
<211>  30
<212>  DNA
<213>  Artificial Sequence

<220>
<223>  Primer for amplifying H-Ras gene

<400>  7
cgggatccga tatgaccgaa tacaaactgg                                        30

<210>  8
<211>  36
<212>  DNA
<213>  Artificial Sequence

<220>
<223>  Primer for amplifying H-Ras gene

<400>  8
gcgggatccc tactagtgct gacggatttc acgaac                                 36

<210>  9
<211>  28
<212>  DNA
<213>  Artificial Sequence

<220>
<223>  Primer for amplifying M-Ras gene

<400>  9
gaagatcttt gtggatgact acgacccc                                          28

<210>  10
<211>  28
<212>  DNA
<213>  Artificial Sequence

<220>
<223>  Primer for amplifying M-Ras gene

<400>  10
ggggtcgtag tcatccacaa agatcttc                                          28

## Description

### Technical Field

The present invention relates to a crystal of a mutant Ras polypeptide having introduced therein a mutation which adopts a conformation having a pocket on the molecular surface of Ras, a production method for the crystal, and a screeningmethod for a Ras function inhibitor utilizing information about the conformation obtained from the crystal.

The present application claims priority of Japanese Patent Application No. 2009-165717 which is incorporated herein by reference.

### Background Art

Ras, a product of the ras proto-oncogene, is a low molecular weightGprotein. Ras has three isoforms, i.e., H-, N-, K-Ras inmammals. Ras also has homologs having similar amino acid sequences, such as M-Ras and Rap. Members including those isoforms and homologs form the Ras family. In human cancers, Ras activation through a mutation of any one of H-, N-, and K-Ras is observed at a high frequency. Hence, Ras is considered as an ideal molecular target in the development of an anti-cancer agent.

Ras controls signaling while cycling between its GTP-bound form as an active form (Ras-GTP) and its GDP-bound form as an inactive form (Ras-GDP). When a ligand (extracellular signal) such as a growth factor binds to a cell membrane receptor, Ras-GDP is activated through the conversion of GDP included therein to GTP, and Ras-GTP binds to a target molecule to induce cell growth signaling. After that, Ras-GTP returns to Ras-GDP through the action of a factor for stimulating the GTP-hydrolyzing (GTPase) activity inherent in Ras (GTPase-activating protein: GAP), and is present in an inactive state until the next signal arrives. In human cancers, it is estimated that a mutation (mutation found in the active form) of Ras reduces the GTP-hydrolyzing ability of Ras and increases the percentage of Ras in the GTP-bound form in cells, resulting in sustained cell growth signaling leading to the development of cancers.

In addition, it has hitherto been pointed out that Ras-GTP, the GTP-bound form, exists in two kinds of interconverting conformations (state 1 and state 2) based on ³¹P-nuclear magnetic resonance spectrometry (NMR) . State 2 is a bona fide active form capable of binding to a target protein to induce signaling, whereas state 1 is an inactive formincapable of binding to a target protein. The conformation of state 2 has been elucidated by X-ray crystallographic and NMR analyses.

The elucidation of the conformation of state 1 of H-Ras-GTP has been attempted, but the conformation has not yet been elucidated completely. An H-Ras mutant H-Ras T35S (amino acid residues 1 to 189) was subj ected to X-ray crystallographic analysis (Non Patent Literature 1). However, it cannot be said that the conformation of state 1 disclosed inNon Patent Literature 1 is a complete conformation because the electron density of the main chain of several amino acid residues is missing. Further, such electron density-missing regions correspond to a part of the two switch regions (switches I and II) important for Ras to recognize and activate a target protein.

Focused on M-Ras, a member of the Ras family, state 1 of wild-type M-Ras-GTP was subjected to X-ray crystallographic analysis (PDB ID: 1X1S) (Non Patent Literature 2). The results revealed that state 1 of M-Ras-GTP had a pocket, which was not observed anywhere in Ras-GDP or state 2 of Ras-GTP, in its molecular surface, and such pocket was surrounded by the switch I and switch II regions. However, the conformation of state 1 of M-Ras-GTP disclosed in Non Patent Literature 2 was incomplete in that there was a partial deletion in electron density around the pocket.

A Ras function inhibitor is a promising candidate for an anti-cancer agent. Hence, there is a strong demand for the structural information on state 1 of Ras-GTP, which is so complete as to be able to be used for computer docking simulation for the development of the Ras function inhibitor.

### Citation List

### Non Patent Literature

[NPL 1] Spoerner, M., et al., Proc Natl Acad Sci U S A. 2001 Apr 24; 98(9): 4944-9.
[NPL 2] Ye M., et al., J Biol Chem. 2005 Sep 2; 280(35): 31267-75.

### Summary of Invention

### Technical Problem

An object of the present invention is to provide a crystal of a Ras polypeptide which adopts a conformation (state 1) having a pocket on the molecular surface of Ras, a production method for the crystal, and a screening method for Ras function inhibitors based on information about the conformation obtained by X-ray crystallographic analysis using the crystal.

### Solution to Problem

The inventors of the present invention have made intensive studies in order to solve the above-mentioned problems, focused on a mutation which adopts a conformation (state 1) having a pocket on the molecular surface of Ras, acquired a mutant Ras polypeptide having introduced therein such a mutation, produced a co-crystal of the mutant Ras polypeptide and a GTP analog, and further subjected the co-crystal to X-ray crystallographic analysis. Thus, the inventors have succeeded in acquiring structural information about the conformation including the information about the structure surrounding the pocket and completed the present invention.

That is, the present invention includes the following items.
1. A co-crystal, including: a mutant Ras polypeptide; and GTP or a GTP analog, in which the mutant Ras polypeptide includes one or more substitutions of amino acid residues introduced in a site including a vicinity of the switch I region in a partial polypeptide of Ras, and adopts a conformation having a pocket on the molecular surface through the introduced mutation.
2. A co-crystal according to the item 1, in which the mutant Ras polypeptide includes the following polypeptide 1) or 2):
   1) a polypeptide in which the partial polypeptide includes a partial polypeptide of H-Ras formed of amino acid residues at positions 1 to 166 set forth in SEQ ID NO: 1, and the mutation includes a threonine-to-serine substitution at position 35 of an amino acid sequence set forth in SEQ ID NO: 1; or
   2) a polypeptide in which the partial polypeptide includes a partial polypeptide of M-Ras formed of amino acid residues at positions 1 to 178 set forth in SEQ ID NO: 2, and the mutation includes a proline-to-aspartic acid substitution at position 40 of an amino acid sequence set forth in SEQ ID NO: 2.
3. Aco-crystal according to the item 1 or 2, inwhich the co-crystal has a space group of R32, 1222, or C2 and lattice constants of a=30 to 100 Å, b=60 to 100 Å, c=70 to 125 Å, α=90°, β=90 to 100°, and γ=90 to 120°.
4. A co-crystal according to the item 2 or 3, in which the mutant Ras polypeptide includes the polypeptide according the item 1) and the co-crystal has a space group of R32 and lattice constants of a=b=91.81 to 95.20 Å, c=116.13 to 121.97 Å, α=β=900°, and γ=120°.
5. A co-crystal according to the item 4, in which the co-crystal has lattice constants of a=94.20 Å, b=94.20 Å, c=120.97 Å, α=β=90.000, and γ=120.00°.
6. A co-crystal according to the item 1 or 2, in which the mutant Ras polypeptide includes the polypeptide according the item 1) and the co-crystal has a space group of 1222 and lattice constants of a=34.28 to 34.88 Å, b=81.20 to 82.80 Å, c=120.80 to 123.20 Å, and α=β=γ=90°.
7. A co-crystal according to the item 1 or 2, in which the co-crystal has lattice constants of a=34.58 Å, b=82.00 Å, c=122.00 Å, and α=β=γ=90°.
8. A co-crystal according to the item 1 or 2, in which the mutant Ras polypeptide includes the polypeptide according the item 2) and the co-crystal has a space group of C2 and lattice constants of a=33.12 to 34.04 Å, b=64. 69 to 66.33 Å, c=72. 67 to 74.93 Å, α=γ=90°, and β=94.92 to 95.33°.
9. A co-crystal according to the item 8, in which the co-crystal has lattice constants of a=33.72 Å, b=65.70 Å, c=74.08 Å, α=γ=90.00°, and β=95.02°.
10. A production method for a co-crystal including: a mutant Ras polypeptide; and GTP or a GTP analog, in which the mutant Ras polypeptide includes one or more substitutions of amino acid residues introduced in a site including a vicinity of the switch I region in a partial polypeptide of Ras, and adopts a conformation having a pocket on the molecular surface through the introduced mutation.
11. A production method according to the item 10, in which the mutant Ras polypeptide includes the following polypeptide 1) or 2):
   1) a polypeptide in which the partial polypeptide includes a partial polypeptide of H-Ras formed of amino acid residues at positions 1 to 166 set forth in SEQ ID NO: 1, and the mutation includes a threonine-to-serine substitution at position 35 of an amino acid sequence set forth in SEQ ID NO: 1; or
   2) a polypeptide in which the partial polypeptide includes a partial polypeptide of M-Ras formed of amino acid residues at positions 1 to 178 set forth in SEQ ID NO: 2, and the mutation includes a proline-to-aspartic acid substitution at position 40 of an amino acid sequence set forth in SEQ ID NO: 2.
12. A production method according to the item 11, in which the mutant Ras polypeptide includes the polypeptide according to the item 1), and the method includes the step of crystallizing a solution including the Ras protein by a vapor diffusion method using a precipitant solution including ammonium sulfate or polyethylene glycol having a molecular weight of 2,000 to 5,000.
13. A production method according to the item 12, in which the mutant Ras polypeptide includes the polypeptide according to the item 2), and the method includes the step of crystallizing a solution including the Ras protein by the vapor diffusion method using a precipitant solution including polyethylene glycol having an average molecular weight of 1,000 to 2,000.
14. A screening method for a Ras function inhibitor, including the following steps of:
   (1) designing or selecting a candidate compound capable of binding to a pocket using information about a conformation obtained from the co-crystal according to any one of the items 1 to 9;
   (2) synthesizing or acquiring the designed or selected candidate compound; and
   (3) bringing the candidate compound into contact with Ras to examine a Ras function-inhibiting activity of the candidate compound.
15. A screening method for a Ras function inhibitor, including the following steps of:
   (1) producing a co-crystal including a mutant Ras polypeptide and GTP or a GTP analog by the method according to any one of the items 10 to 13;
   (2) elucidating a structure of the crystallized polypeptide by X-ray crystallographic analysis to obtain information about a conformation of the polypeptide;
   (3) designing or selecting a candidate compound capable of binding to a pocket using the obtained information about the conformation;
   (4) synthesizing or acquiring the designed or selected candidate compound; and
   (5) bringing the candidate compound into contact with Ras to examine a Ras function-inhibiting activity of the candidate compound.

### Advantageous Effects of Invention

In the present invention, it is possible to obtain the co-crystal of a mutant polypeptide which adopts a conformation (state 1) having a pocket on the molecular surface of Ras, and a GTP analog, and it is also possible to obtain the information about a conformation including the pocket of Ras by the X-ray crystallographic analysis of the crystal. Further, such information about the conformation can be used for designing and screening a Ras function inhibitor. The Ras function inhibitor is expected to act as an anti-cancer agent.

### Brief Description of Drawings

[FIG. 1] FIG. 1 is a photograph showing a crystal of H-Ras T35S (Example 2-1).
[FIG. 2] FIG. 2 is a photograph showing a crystal of H-Ras T35S (Example 2-2).
[FIG. 3] FIG. 3 is a photograph showing a crystal of M-Ras P40D (Example 2-3) .
[FIG. 4] FIG. 4 is a view illustrating an electron density map of H-Ras T35S from a specific angle (Example 3).
[FIG. 5] FIG. 5 is a view illustrating an electron density map of H-Ras T35S from a specific angle (Example 3).
[FIG. 6] FIG. 6 is a view illustrating an electron density map of M-Ras P40D from a specific angle (Example 3).
[FIG. 7] FIG. 7 is a view illustrating conformations of H-Ras T35S and M-Ras P40D (Example 3).
[FIG. 8] FIG. 8 is a view showing NMR spectra of M-Ras P40D (Reference Example 1).
[FIG. 9] FIG. 9 is a view illustrating a fluctuation in conformation of switch I of H-Ras T35S.
[FIG. 10] FIG. 10 is a view illustrating a fluctuation in conformation of switch II of H-Ras T35S.
[FIG. 11] FIG. 11 is a view illustrating a fluctuation in conformation of switch I of M-Ras P40D.
[FIG. 12] FIG. 12 is a view illustrating a fluctuation in conformation of switch II of M-Ras P40D.

### Description of Embodiments

The present invention is based on the elucidation of information about a conformation including a pocket of state 1 of Ras-GTP by producing a mutant Ras polypeptide to provide a co-crystal of the mutant Ras polypeptide and GTP or a GTP analog, and subjecting the co-crystal to X-ray crystallographic analysis.

### (Mutant Ras polypeptide)

In the present invention, Ras has only to be included in the Ras family, and encompasses three Ras isoforms, i.e., H-, N-, andK-Ras, and homologs such as M-Ras and Rap. An Ras protein is not particularly limited as long as it is derived from mammals, and examples thereof include ones of human, bovine, and porcine origins. Ras is preferably H-Ras or M-Ras.

The mutant Ras polypeptide of the present invention is obtained by introducing, in a partial polypeptide of Ras, a mutation which adopts a conformation having a pocket on the molecular surface.

The partial polypeptide of Ras is derived from the above-mentioned Ras family and includes an amino acid sequence including a region which constitutes a pocket. Examples thereof include polypeptides formed of an amino acid sequence set forth in SEQ ID NO: 1 (at positions 1 to 166 of H-Ras) and an amino acid sequence set forth in SEQ ID NO: 2 (at positions 1 to 178 of M-Ras) shown below.
SEQ ID NO: 1: H-Ras (1-166aa):
SEQ ID NO: 2: M-Ras (1-178aa):

MATSAVPSENLPTYKLVVVGDGGVGKSALTIQFFQKIFVPDYDPTIEDSYLKHTEIDNQWAILDVL

DTAGQEEFSAMREQYMRTGDGFLIVYSVTDKASFEHVDRFHQLILRVKDRESFPMILVANKVDLMH

LRKVTRDQGKEMATKYNIPYIETSAKDPPLNVDKTFHDLVRVIRQQ

It is known that the pocket on the molecular surface in the present invention is not present on the molecular surface of Ras-GDP or state 2 of Ras-GTP, and is present in state 1 of Ras-GTP. The pocket is surrounded by regions called the switch regions (including the switch I region and the switch II region) in an amino acid sequence of Ras. The switch regions are defined as two regions which undergo drastic conformational changes between the GDP-bound form and the GTP-bound form and are regions important for Ras to recognize and activate a target molecule, and GTP binds to the vicinity of the regions.

The switch I region has an amino acid sequence represented by YDPTIED (SEQ ID NO: 3). Amino acid sequences in the vicinity of the switch I region are different among members in the Ras family. Here, the vicinity of the switch I region means a region formed of three to five amino acids preceding the switch I region and a region formed of three to five amino acids following the switch I region. The "site including a vicinity of a switch I region" is a site formed of the switch I region and the vicinity of the switch I region, and can have an amino acid sequence represented by X₁X₂X₃X₄X₅YDPTIEDX₆X₇X₈X₉X₁₀ (SEQ ID NO: 4) (the switch I region is underlined). The "site including a vicinity of a switch I region" can have an amino acid sequence represented by HFVDEYDPTIEDSYRKQ (SEQ ID NO: 5) in H-Ras, and can have an amino acid sequence represented by IFVPDYDPTIEDSYLKH (SEQ ID NO: 6) in M-Ras.

It is thought that state 2 of Ras-GTP does not have a pocket owing to the presence of a hydrogen bond between threonine (T) located at position 4 of the switch I region and the γ-phosphate group in GTP. The mutant polypeptide of the present invention is estimated to have a pocket on the molecular surface because there is no hydrogen bond between threonine (T) located at position 4 of the switch I region and the GTP-γ phosphate group and there is no hydrogen bond between an amino acid corresponding to each of X₃ and X₄ and ribose in GTP. X₃ and X₄ correspond to amino acids at positions 29 and 30 in H-Ras and correspond to amino acids at positions 39 and 40 in M-Ras. Depending on whether or not an Ras polypeptide having introduced therein a mutation has the above-mentioned hydrogen bond, whether or not the mutation is a mutation which adopts a conformation having a pocket on the molecular surface of a Ras protein can be predicted. This may be confirmed using molecular modeling with computer simulation or the like known per se.

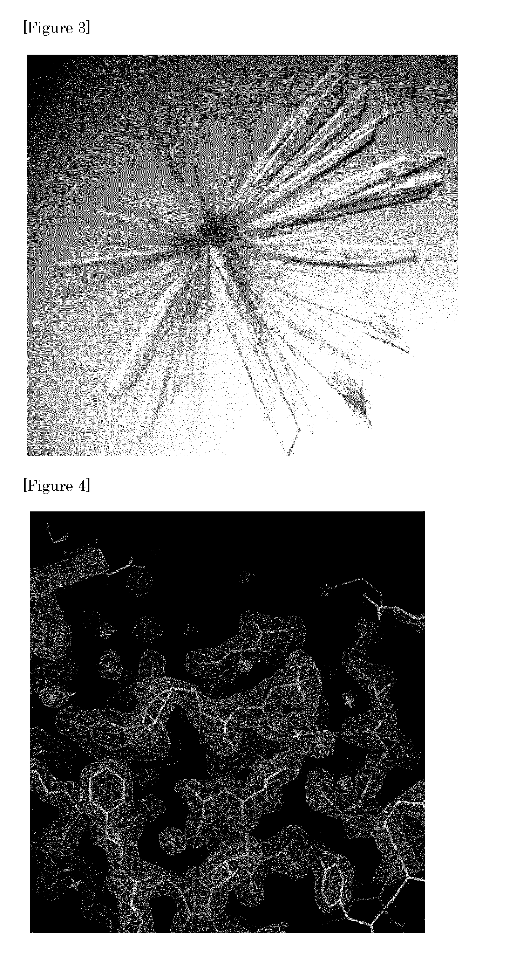

In addition, ³¹P-NMR analysis shows that the mutant polypeptide of the present invention corresponds to state 1, and a chemical shift attributed to a phosphorus atom of the γ-phosphate group in GTP is present in the lower magnetic field side. The presence of a target protein induces the transition of Ras-GTP to state 2. In the case where the mutant polypeptide of the present invention and a target protein (e.g., Raf) coexist, a chemical shift attributed to a phosphorus atom of the γ-phosphate group in GTP shifts to the higher magnetic field side, or a peak in the higher magnetic field side becomes larger, as compared to the case where the mutant polypeptide and the target protein do not coexist. Thus, it is possible to confirm whether or not the mutation introduced in Ras is a mutation which adopts a conformation having a pocket on the molecular surface of the mutant Ras polypeptide. For example, the ³¹P-NMR analysis of H-Ras T35S is shown in Fig. 4 of Spoerner M, Herrmann C, Vetter IR, Kalbitzer HR, Wittinghofer A. Proc Natl Acad Sci USA. 2001 Apr 24; 98(9): 4944-9. PMID: 11320243.

When the partial polypeptide of Ras is a partial polypeptide of H-Ras set forth in SEQ ID NO: 1, the mutation is substitutions of one or more amino acids in a region formed of amino acids at positions 27 to 43, preferably a region formed of amino acids at positions 30 to 41. The mutation is preferably amino acid substitutions at positions 31 and/or 35, particularly preferably a threonine substitution at position 35. The threonine at position 35 is preferably substituted by serine (hereinafter, this mutant Ras polypeptide is referred to as "H-Ras T35S").

When the partial polypeptide of Ras is a partial polypeptide of M-Ras set forth in SEQ ID NO: 2, the mutation is substitutions of one or more amino acids in a region formed of amino acids at positions 37 to 53, preferably a region formed of amino acids at positions 40 to 51. The mutation is preferably amino acid substitutions at positions 40, 41 and/or 45, particularly preferably a proline substitution at position 40. The proline at position 40 is preferably substituted by aspartic acid (hereinafter, this mutant Ras polypeptide is referred to as "M-Ras P40D").

The mutant Ras polypeptide of the present invention also includes a polypeptide including an amino acid sequence having substitutions, deletions, additions, or insertions of one to five amino acids in addition to the mutation which adopts a conformation having a pocket in a molecular surface in the above-mentioned amino acid sequences set forth in SEQ ID NOS: 1 and 2, and having substantially the same biological function as that of H-Ras or M-Ras. The phrase "having substantially the same biological function as that of H-Ras or M-Ras" means that state 2 of Ras-GTP adopts a conformation free of a pocket and is capable of binding to a target molecule.

An amino acid mutation in a polypeptide may be introduced by a site-directed mutagenesis method well-known to a person skilled in the art, and may be introduced using a kit (for example, a QuikChange^{™}Site-Directed Mutagenesis Kit (STRATAGENE)). Alternatively, a DNA fragment encoding an amino acid mutation may be introduced or substituted using a genetic engineering technique well-known to a person skilled in the art. For example, a mutant Ras polypeptide can be produced by using a PCR reaction, a restriction enzyme reaction, a ligation reaction, and the like in combination.

Specifically, a method described in Examples may be employed. pGEX-6p-1-H-Ras T35S or pGEX-6p-1-M-Ras P40D may be produced by a PCR method using a plasmid pGEX-6P-1-H-Ras (1-166aa) or pGEX-6p-1-M-Ras (1-178aa) as a template and using sense/antisense primers for mutation introduction. Whether or not a mutation is introduced has only to be confirmed by DNA sequencing employing a dye terminator method.
Alternatively, H-Ras T35S may be produced by amplifying a DNA fragment corresponding to 1-166aa by ordinary PCR using, as a template, H-Ras T35S (pBR322H-Ras T35S (1-189aa)) subcloned into a vector pBR322, and cloning the amplified fragment into pGEX-6p-1. Further, M-Ras P40D may be produced by carrying out PCR for mutation introduction using pGEX-6p-M-Ras (1-178aa) as a template, followed by the so-called site-directed mutagenesis method.

In order to facilitate the purification of the expressed polypeptide, a fused polypeptide having added thereto a tag for purification may be produced. When the fused polypeptide is produced as a protein having an affinity to a specific ligand or a fused polypeptide with a peptide sequence (tag for affinity purification), the fused polypeptide may be efficiently purified by affinity chromatography or the like using the ligand as a carrier. For example, as a tag for purification, there are given a 6X histidine tag, an FLAG tag, a glutathione S-transferase (GST) tag, a maltose-binding protein, and protein A. Further, the preliminary insertion of an amino acid sequence which is recognized and cleaved by a specific protease between a polypeptide of interest and a tag for purification can cleave the fused polypeptide to collect only the polypeptide of interest. For example, PreScission protease, Factor Xa, thrombin, enterokinase, collagenase, or the like may be used as the specific protease. After the cleavage with such protease, several amino acids may remain at the end of the polypeptide of interest, but the polypeptide of interest may be directly used for the production of a crystal of the present invention as long as the amino acids do not affect an activity.

In order to produce a crystal using the mutant Ras polypeptide of the present invention and obtain information about a conformation including a pocket from such crystal, Ras in the GTP-bound form needs to be used as the mutant Ras polypeptide. Ras in the GTP-bound form means the mutant Ras polypeptide bound to GTP or bound to a GTP analog. The GTP analog includes one having a GTP-like backbone subjected to chemical modification or salt formation, and is capable of binding to a GTP-binding site. Examples of the GTP analog include guanosine 5'-(γ-thio)-triphosphate (GTPγS) and (guanosine 5'-[β,γ-imido]triphosphate (Gpp(NH)p). Of those, Gpp(NH)p is preferred. Gpp(NH)p manufactured by Sigma may be purchased and used.

In order to convert the resultant mutant Ras polypeptide into one in a GTP-bound form, the binding of GTP or a GTP analog to the mutant Ras polypeptide is carried out. A stable GTP analog-bound form may be produced by adding a GTP analog (Gpp (NH) p or GTPγS) to a Ras polypeptide in the GDP-boundform (0.5mM), which has been subjected to mass expression and purification in *Escherichia coli,* under a low magnesium environment (10 mM EDTA, 5 mM MgCl₂) in the presence of alkaline phosphatase (5 units with respect to 1 mg of the Ras polypeptide), and placing the resultant under a high magnesium environment (20 mM MgCl₂) again.

### (Production method for co-crystal)

A co-crystal of the present invention is a co-crystal of a complex of a mutant polypeptide bound to GTP or a GTP analog.

First, a solution of a mutant Ras polypeptide (GTP-bound form) is produced. The mutant Ras polypeptide (GTP-bound form) is allowed to exist in a solution formed of a buffer, a salt, a reducing agent, and the like. Such solution preferably includes GTP or a GTP analog. Any buffer, salt, and reducing agent may be used as long as the conformation of the mutant Ras polypeptide is not affected. Examples of the buffer include 1 to 500 mM Na-HEPES, sodium phosphate, potassium phosphate, and Tris-HCl. Examples of the salt include 1 mM to 1 M sodium chloride, lithium chloride, and magnesium chloride. Examples of the reducing agent include 0.1 to 10 mM β-mercaptoethanol and dithiothreitol (DTT). Further, the solution of the mutant Ras polypeptide may include dimethylsulfoxide (DMSO). The solution including the mutant Ras polypeptide has a pH of 4 to 11, preferably a pH of 6 to 9. Such solution of the mutant polypeptide may be used for crystallization without any further treatment, or as necessary, a preservative, a stabilizer, a surfactant, or the like is further added to the solution, and the resultant solution may be used for crystallization.

As a crystallization method for a protein (polypeptide), a general technique for protein crystallization such as a vapor diffusion method, a batch method, or a dialysis method may be employed. Further, in the crystallization of a protein, it is important to determine physical and chemical factors such as the concentration of the protein, the concentration of a salt, a pH, the kind of a precipitant, and a temperature.

The vapor diffusion method refers to a method involving placing a droplet of a protein solution including a precipitant in a container including a buffer (external solution) including the precipitant at a higher concentration, sealing the container, and then leaving the resultant to stand still. The vapor diffusion method is classified into a hanging drop method and a sitting drop method depending on how to place the droplet, and any of the methods may be adopted in the present invention. The hanging drop method is a method involving placing a small droplet of a protein solution on a cover glass, inversing the cover glass in a reservoir, and sealing the reservoir. On the other hand, the sitting drop method is a method involving installing an appropriate droplet stage in a reservoir, placing a droplet of a protein solution on the droplet stage, and sealing the reservoir with a cover glass or the like. In any of the methods, a precipitant is incorporated into the solution in the reservoir (reservoir solution). As appropriate, a small amount of the precipitant may be incorporated into a protein small droplet.

The reservoir solution to be used in the vapor diffusion method is a solution formed of a buffer, a precipitant, a salt, and the like. Any buffer, precipitant, and salt may be used as long as a crystal can be efficiently produced. Examples of the buffer include 1 to 500 mM Na-HEPES, sodium phosphate, potassium phosphate, Tris-HC1, sodium acetate, and citric acid at a pH of 4 to 9. Examples of the precipitant include 1 to 50 vol% polyethylene glycol (PEG) having a molecular weight of 400 to 10,000 or 0.2 to 3 M ammonium sulfate, 1 to 20 vol% methylpentanediol (MPD), and 5 to 10 vol% isopropanol. Examples of the salt include 0.05 to 0.3 M sodium chloride, lithium chloride, and magnesium chloride. The components for the reservoir solution are not limited to those described above.

In the case of the mutant Ras polypeptide of the present invention, a crystal suitable for X-ray crystallographic analysis can be obtained in the case of employing a sitting drop method as the vapor diffusion method and using ammonium sulfate or polyethylene glycol (PEG) as the precipitant under the conditions of a pH of 4 to 9, a polypeptide (protein) concentration of 10 to 20 mg/mL, and a temperature of 4 to 25°C.

Further, a preferred condition for the production of a co-crystal of H-Ras T35S and GTP or a GTP analog is one using, as the precipitant, ammonium sulfate or PEG having a molecular weight of 2,000 to 5,000 (preferably 4,000 to 5,000, more preferably 5,000), and a solution obtained by dissolving a 10 to 20 mg/mL mutant polypeptide (GTP-bound form) (preferably 15 to 17 mg/mL). As a preferred precipitant, a solution including 2 M ammonium sulfate or 30% PEG5000 may be selected. Further, a temperature condition is preferably 15 to 20°C, more preferably 20°C.

Specifically, a co-crystal of H-Ras T35S and Gpp(NH)p may be produced as described below. A complex of H-Ras T35S and Gpp(NH)p is dissolved in a buffer (40 mM Tris-HCl, pH 7.4, 50 mM NaCl, 5 mM MgCl₂, 7% DMSO, 10 mM Gpp(NH)p). The co-crystal of H-Ras T35S-Gpp(NH)p is produced by employing a sitting drop vapor diffusion method using 1.5 µl of the polypeptide solution (16 mg/mL) and 1 µl of a reservoir solution (100 mM Tris-HCl, pH 8.5, 2 M (NH₄)₂SO₄) at 20°C.

As another aspect, the co-crystal of H-Ras T35S and Gpp(NH)p may also be produced as described below. A complex of H-Ras T35S and Gpp (NH) p is dissolved in a buffer (40 mM Tris-HCl, pH 7.4, 50 mM NaCl, 5 mM MgCl₂, 10 mM Gpp(NH)p). The co-crystal of H-Ras T35S-Gpp(NH)p is produced by employing a sitting drop vapor diffusion method using 1 µl of the polypeptide solution (16 mg/mL) and 1 µl of a reservoir solution (0.1 M MES, pH 6.5, 0.2 M (NH₄)₂SO₄, 30% PEG5000) at 20°C.

Further, a preferred condition for the production of a co-crystal of M-Ras P40D and GTP or a GTP analog is one using PEG having a molecular weight of 1,000 to 2,000 (preferably 1,500), and a solution obtained by dissolving a 10 to 20 mg/mL mutant protein (GTP-bound form) (preferably 15 to 18 mg/mL). As a preferred precipitant solution, a solution including 28% PEG1500 maybe selected. Further, a temperature condition is preferably 15 to 20°C, more preferably 20°C.

A crystal of M-Ras P40D may be produced as described below. A complex of M-Ras P40D and Gpp (NH)p is dissolved in a buffer (50 mM Tris-HCl, pH 7.4, 50 mM NaCl, 5 mM MgCl₂, 1 mM EDTA, 1 mM DTT) . The crystal is produced by employing a sitting drop vapor diffusion method using 2 µl of the polypeptide solution (18 mg/mL) and 2 µl of a reservoir solution (28% (w/v) polyethylene glycol (PEG) -1500, 150 mM MgSO₄, 100 mM sodium cacodylate, pH 6.5) at 20°C.

In the present invention, it is preferred to obtain a crystal with quality enough to provide at least a resolution of 10 Å or less, preferably a resolution of 4.0 Å or less, more preferably a resolution of 3.4 Å or less, still more preferably a resolution of 2.8 Å or less, particularly preferably a resolution of 2.2 Å or less when the crystal being subjected to X-ray crystallographic analysis ("Introduction to Protein Structure" Carl Brandon & JohnTooze, translated by Yukiteru Katsube et al., Kyoikusya, 1992, p. 276-277).

### (Co-crystal)

The co-crystal of the mutant Ras polypeptide (GTP-bound form) of the present invention is substantially free of impurities and has an activity even when redissolved. Examples of the impurities include GST, a mutant Ras polypeptide decomposition product, and a protein peculiar to *Escherichia coli.*

The crystal of the present invention has unit lattice constants of a=30 to 100 Å, b=60 to 100 Å, c=70 to 125 Å, α=90°, β=90 to 100°, and γ=90 to 120°, and belongs to the trigonal, orthorhombic, or monoclinic space group R32, I222, or C2. The crystal obtained according to the present invention has a resolution of about 1.0 Å to about 3.5 Å, and hence has sufficient quality and size for X-ray crystallographic analysis.

The crystal of H-Ras T35S belongs to the trigonal space group R32, has such a size that the lattice constants are a=b=91.81 to 95.20 Å, c=116.13 to 121.97 Å, α=β=90°, and γ=120°, and has a resolution of 2.60 to 1.70 Å. It is preferred that the crystal have such a size that the lattice constants are a=b=92.81 to 94.20 Å, c=117.13 to 120.97 Å, α=β=90°, and γ=120°, and have a resolution of 2.60 to 1.70 Å. It is more preferred that the crystal have such a size that the lattice constants are a=b=94.20 Å, c=120.97 Å, α=β=90.00°, and γ=120.00°, and have a resolution of 1.95 Å.

Alternatively, the crystal of H-Ras T35S belongs to the orthorhombic space group I222, has such a size that the lattice constants are a=34.28 to 34.88 Å, b=81.20 to 82.80 Å, c=120.80 to 123.20 Å, and α=β=γ=90°, and has a resolution of 2.20 to 2.09 Å. It is preferred that the crystal have such a size that the lattice constants are a=34.58 Å, b=82.00 Å, c=122.00 Å, and α=β=γ=90° (preferably α=β=γ=90.00°), and have a resolution of 2.10 Å.

The crystal of M-Ras P40D belongs to the monoclinic space group C2, has such a size that the lattice constants are a=33.12 to 34.04 Å, b=64.69 to 66.33 Å, c=72.67 to 74.93 Å, α=γ=90°, and β=94.92 to 95.33°, and has a resolution of 1.95 to 1.35 Å. It is preferred that the crystal have such a size that the lattice constants are a=33.42 to 33.74 Å, b=65.29 to 65.73 Å, c=73.37 to 74.23 Å, α=γ=90°, and β=94.92 to 95.33°, and have a resolution of 1.95 to 1.35 Å. It is more preferred that the crystal have such a size that the lattice constants are a=33.72 Å, b=65.70 Å, c=74.08 Å, α=γ=90.00°, and β=95.02°, and have a resolution of 1.35 Å.

### (X-ray crystallographic analysis)

X-ray crystallographic analysis is most commonly carried out as a technique for clarifying a conformation of a protein (polypeptide). The technique involves crystallizing a protein, applying monochromatic X-rays to the crystal, and clarifying information about a conformation in the protein based on the resultant X-ray diffraction image. The information about the conformation includes an electron density map and atomic coordinates, and the atomic coordinates may be analyzed and acquired by a method known in the art (D.E. McRee, Practical Protein Crystallography, Academic Press, San Diego (1993)).

The X-ray crystallographic analysis includes the steps of: acquiring diffraction data by applying X-rays to a crystal; acquiring an electron density of a protein (polypeptide) by analyzing the resultant diffraction data; and acquiring atomic coordinates of the protein (polypeptide) by analyzing the resultant electron density.

In the X-ray crystallographic analysis, by use of an X-ray diffractometer in a laboratory or a large radiation facility (e.g., ESRF, APS, SPring-8, PF, ALS, CHESS, SRS, LLNL, SSRL, or Brookhaven), diffraction data is collected by oscillation photography or the like with two-dimensional detector such as an imaging plate or a CCD camera, and an electron density can be obtained from the data to elucidate atomic coordinates. For example, it is recommended to generate a radiation from structural biology beamline I (BL41XU) of a large radiation facility SPring-8 and collect diffraction data using an imaging plate or a CCD camera.

A crystal of a protein often undergoes damage by applying X-rays, resulting in a deterioration in diffraction ability. Hence, it is preferred to carry out high-resolution X-ray diffraction through low-temperature measurement. The low-temperature measurement refers to a method involving rapidly cooling and freezing a crystal to about -173°C, and collecting diffraction data in the state. In general, in the freezing of a crystal of a protein, a contrivance such as treatment in a solution including a protectant (cryoprotectant) such as glycerol is made for the purpose of preventing the collapse of the crystal due to the freezing. A frozen crystal may be prepared, for example, by flash freezing involving directly immersing a crystal, which has been immersed in a preservative solution supplemented with a protectant, in liquid nitrogen.

A diffraction image collected by an X-ray diffraction experiment can be processed with data processing software to calculate diffraction intensities obtained by the indexing and integration of individual diffraction spots. Electron densities in a three-dimensional space are derived by carrying out inverse Fourier transform using the diffraction intensities and phase information of the diffraction spots. In a diffraction experiment, it is impossible in principle to measure phase information of each of the diffraction spots necessary for the calculation of the electron density. Hence, in order to obtain the electron density, the phase as lost information is estimated by a molecular replacement method, a heavy atom isomorphous replacement method, a multiwavelength anomalous dispersion method (MAD method), or a modified method thereof.

An electron density map is depicted based on the thus obtained electron density, and a three-dimensional model is constructed using software which operates in a graphics workstation in accordance with the electron density map. After the construction of the model, structural refinement is carried out by a least-squares method or the like to give final atomic coordinates (conformational coordinates) of a protein.

### (Information about conformation of mutant Ras polypeptide)

Electron density data is obtained by a technique for crystallographic analysis with X-ray diffraction using a crystal. An electron density map can be prepared based on the electron density data. It is conceivable that the electron density data with reproducibility is obtained by using a specificX-ray diffraction apparatus for a specific crystal.

As the electron density of the mutant polypeptide of the present invention, FIG. 4 to FIG. 6 show electron density maps around Gpp(NH)p of a crystal of H-Ras T35S (having a size of a=b=94.20+0.00 Å, c=120.97+0.00 Å, α=β=90.00±0.00°, and γ=120.00±0.00° and a resolution of 1.95 Å: or having a size of a=34.58 Å, b=82.00 Å, c=122.00 Å, and α=β=γ=90.00° and a resolution of 2.1 Å) and a crystal of a M-Ras P40D (having a size of a=33.72+0.00 Å, b=65.70+0.00 Å, c=74.08+0.00 Å, α=γ=90.00±0.00°, and β=95.02±0.00° and a resolution of 1.35 Å) obtained utilizing SPring-8.

Atomic coordinates (values showing a spatial positional relationship among the respective atoms) can be obtained from the electron density map. The atomic coordinates mean mathematical coordinates in which the positions of the atoms of the protein described above are expressed as three-dimensional coordinates. The atomic coordinates substantially mean a space configuration determined depending on a distance between the respective molecules (atoms) which construct a chemical structure. When the space configuration is processed as information on a computer, a relative configuration is converted into numerical information as specific coordinates in a certain coordinate system (referred to as conversion to coordinates). This is processing necessary for convenience in carrying out computer processing, and it should be understood that the nature of the atomic coordinates is a configuration determined depending on a distance between the respective molecules (atoms) as described above and is not a coordinate value specified temporarily at the time of computer processing. Further, the atomic coordinates as used herein mean coordinates of individual atoms which construct a substance (such as a protein or an amino acid).

As the atomic coordinates of the mutant polypeptide of the present invention, Tables 1 to 3 show atomic coordinates of a crystal of H-Ras T35S (having a size of a=b=94.20±0.00 Å, c=120.97±0.00 Å, α=β=90.00±0.00°, and γ=120.00±0.00° and a resolution of 1.95 Å: and having a size of a=34.58 Å, b=82.00 Å, c=122.00 Å, and a=β=γ=90.00° and a resolution of 2.1 Å) and a crystal of M-Ras P40D (having a size of a=33.72+0.00 Å, b=65.70+0.00 Å, c=74.08+0.00 Å, α=γ=90.00±0.00°, and β=95.02±0.00° and a resolution of 1.35 Å) obtained from the electron density data and electron density maps obtained utilizing SPring-8. Table 1 shows atomic coordinates (conformational coordinates) determined for the crystal of H-Ras T35S, Table 2 shows atomic coordinates (conformational coordinates) determined for the crystal of H-Ras T35S different from Table 1, and Table 3 shows atomic coordinates (conformational coordinates) determined for the crystal of M-Ras P40D. The data described in each of Tables 1 to 3 is in conformity with the **format of Protein Data Bank (PDB)** (http://www.wwpdb.org/documentation/format23/v2.3.html).

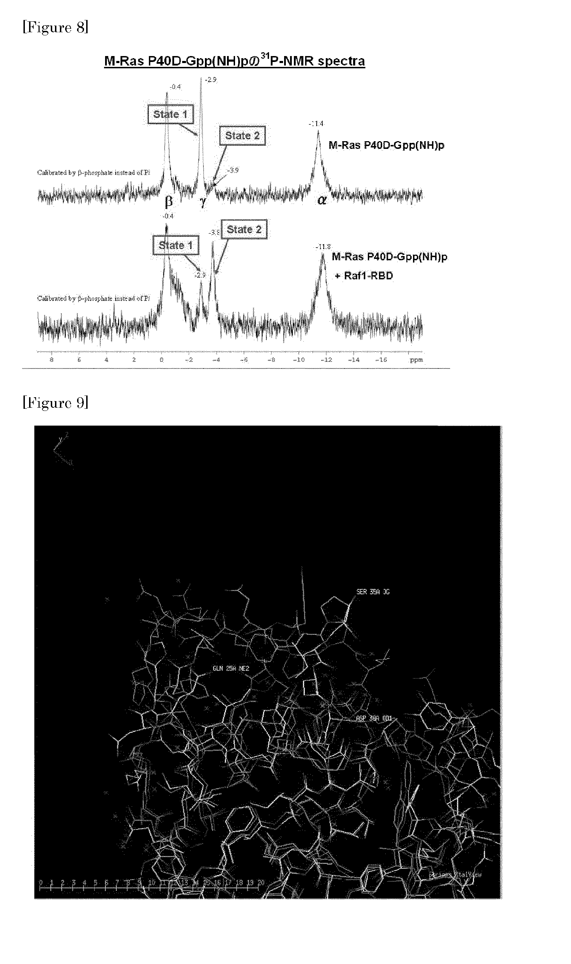

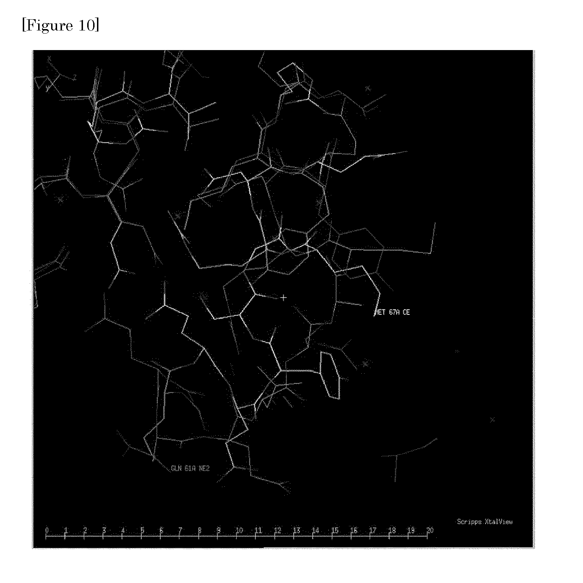

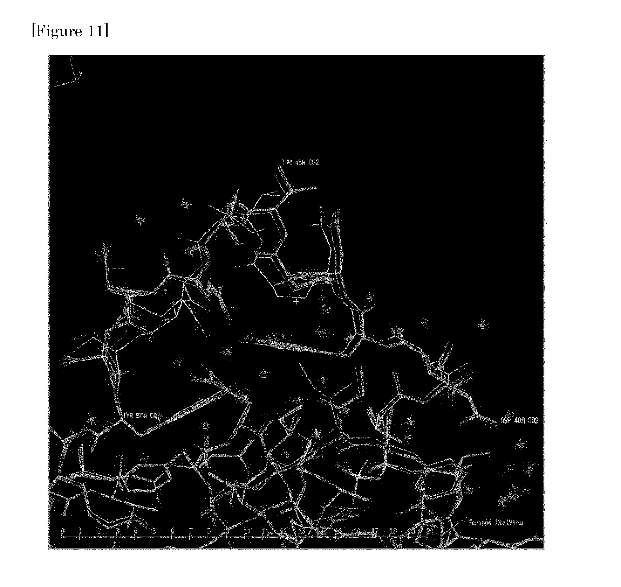

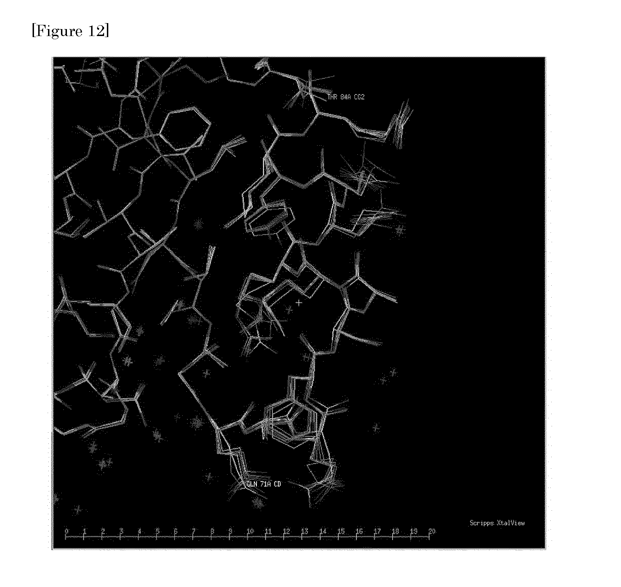

Further, it is thought that the switch I region and the switch II region of Ras are regions important for the opening and closing of a pocket, and undergo drastic conformational changes between state 1 and state 2. The present invention has revealed that, in a conformation of state 1 in a state in which a pocket is opened, there exist, for example, the shift and directional change of a main chain and the directional change of a side chain in the switch I region and the switch II region. Specifically, the shift of the main chain is found at positions 25 to 38 around switch I and at positions 61 to 67 of switch II in H-Ras T35S (see Table 1 and Table 2, and FIG. 9 and FIG. 10), whereas there are given the shift of the main chain at positions 40 to 50 around switch I, the directional change of the main chain and the side chain around position 45, and the directional change of the side chain at positions 71 to 84 around switch II in M-Ras P40D (see FIG. 11 and FIG. 12). The information about the conformation in the present invention also includes atomic coordinates in which the main chain and the side chain have undergone changes in the switch I region and the switch II region described above as compared to Tables 1 to 3.

Further, in the present invention, by use of the information about the conformation represented by the electron density and atomic coordinates described above, atomic coordinates obtained by homology modeling or the like on a computer may also be obtained as derivatives for a protein having 40% or more homology to the polypeptide of the present invention.

### (Screening method for a Ras function inhibitor)

The selection of a compound which may affect a pocket on the molecular surface of Ras or a compound which has a structure capable of filling the pocket by calculation allows a Ras-specific function inhibitor to be efficiently selected from numerous compounds.

The screening method of the present invention includes the following steps of:
(1) designing or selecting a candidate compound capable of binding to a pocket of Ras-GTP using information about a conformation obtained from a crystal of a mutant Ras polypeptide;
(2) synthesizing or acquiring the designed or selected candidate compound; and
(3) bringing the candidate compound into contact with Ras to examine an Ras function-inhibiting activity of the candidate compound.

In the step (1), the information about the conformation of the mutant Ras polypeptide is used for carrying out the matching of atomic coordinates, which represent information about a conformation having a pocket of Ras, with atomic coordinates, which represent information about a conformation of any candidate compound, on a computer. The matching state is converted into a numerical value, for example, by using an empirical scoring function as an indicator to evaluate an ability of the candidate compound to bind to the pocket of Ras. As atomic coordinates having the pocket of Ras, the whole atomic coordinates of the mutant Ras polypeptide, derivatives thereof including the pocket, and parts thereof may be utilized. Further, atomic coordinates of a pocket part appropriately altered on a computer so as to become suitable for screening may be utilized in the present invention.

A mode of a three-dimensional chemical interaction of Ras can be displayed in detail by inputting the atomic coordinates out of the information about the conformation of the mutant Ras polypeptide to a computer or a storage medium of the computer in which a computer program that displays atomic coordinates of a molecule operates. There are known a large number of commercially available computer programs that display atomic coordinates of a molecule. In general, those programs include means for inputting atomic coordinates of a molecule, means for visually displaying the coordinates on a computer screen, means for measuring a distance, a bond angle, and the like between the respective atoms in the displayed molecule, means for additionally correcting the coordinates, and the like. In addition, it is also possible to use a program including means for calculating structural energy of a molecule based on coordinates of the molecule and means for calculating free energy in consideration of a solvent molecule such as a water molecule. Computer programs InsightII and QUANTA commercially available from Accerlys are suitably used for the screening method of the present invention. However, computer programs to be used in the present invention are not limited to the above-mentioned programs.

The candidate compound may be any of known and novel compounds, and structures, origins, physical properties, and the like thereof are not particularly limited. Further, the candidate compound may be any of a natural compound, a synthetic compound, a high molecular weight compound, a low molecular weight compound, a peptide, and a nucleic acid analog. A known program has only to be used for the conversion of the conformation of the candidate compound into coordinates. For example, as a program that converts the conformation of the lowmolecular weight compound into coordinates, CORINA (http://www2.chemie.uni-erlangen.de/software/corina/index.html), Concord
(http://www.tripos.com/sciTech/inSilicoDisc/chemInfo/concord.html), Converter, or the like may be utilized.

The step of evaluating the matching state of the atomic coordinates of the candidate compound and the atomic coordinates having a pocket of Ras in the same coordinate system by overlapping both the coordinates can be carried out using the above-mentioned commercially available package software and a computer system capable of operating the software. The computer system appropriately includes various means necessary for operating software of interest, for example, storage means for storing a structural formula of a compound, means for converting a conformation of a compound into coordinates, storage means for storing atomic coordinates of a compound, storage means for storing atomic coordinates of Ras, storage means for storing evaluation results, means for displaying contents in each storage means, input means such as a keyboard, display means such as a display, and a central processing unit.

Any software for analysis may be used as long as the software can carry out an operation of docking a ligand to a protein on a computer, and for example, DOCK, FlexX (Tripos), LigandFit (Accerlys), Ludi (Accerlys), and the like may be used. In addition, the operation may be carried out interactively using molecular display software such as InsightII. In that case, as an indicator in evaluating the matching state using each of those programs, a free energy calculated value for the whole complex, an empirical scoring function, shape complementarity evaluation, and the like may be arbitrarily selected and used. The indicator allows whether the binding is good or bad to be objectively evaluated.

The design or selection of an Ras function inhibitor using atomic coordinates of Ras allows quick screening on a computer. Further, it is desired to experimentally evaluate a group of candidate compounds selected by screening utilizing a computer.

In order to experimentally evaluate an Ras function inhibitory action of a candidate compound, the candidate compound is synthesized or acquired in the step (2). The candidate compound has only to be synthesized using a known technique or acquired, for example, by purifying a substance derived from a living body.

In the step (3), the candidate compound is experimentally evaluated by subjecting the compound to a biochemical technique or a cell biological technique using Ras, which makes it possible to select a more effective Ras function inhibitor. In order to confirm whether or not the candidate compound exhibits an Ras function-inhibiting action, it is recommended to examine whether or not there is a difference in Ras function between the cases where the compound is added and is not added to a system in which the Ras function can be confirmed. The phrase "having an Ras function-inhibiting action" means that an Ras function measured value of a candidate compound addition group shows a lowered function value as compared to that of a candidate compound non-addition group.

The biochemical technique and the cell biological technique are exemplified by a system involving bringing a candidate compound into contact with Ras in the presence of a target protein of Ras and detecting the binding property of Ras to a target molecule. Examples of such system include a system involving subjecting Ras and a candidate compound to an in Vitro reaction, or culturing Ras-expressing cells in the presence of a candidate compound to detect the binding property of Ras to a target protein using an immunological technique or the like using an antibody or the like.

The Ras function inhibitor obtained by the present invention may be used as a drug. The Ras function inhibitor may be used as therapeutic drugs for various diseases, which may develop based on Ras function abnormality, such as therapeutic drugs for tumors including an anti-cancer agent.

The anti-tumor effect of the Ras function inhibitor may be evaluated by culturing cancer cells in the presence of the Ras function inhibitor to examine whether or not a cancer-specific trait is suppressed, and further using a cancer-bearing model animal. It should be noted that the confirmation of the Ras function-inhibiting activity in the above-mentioned step (3) may be performed by directly subjecting a candidate compound to the evaluation of an anti-tumor effect using cancer cells or a cancer-bearing model animal to bring the candidate compound into contact with Ras.

### Examples

Hereinafter, the present invention is further specifically described by way of examples of the present invention. However, the present invention is not limited thereto, and a variety of applications thereof are possible without departing the technological idea of the present invention.

### (Example 1) Expression and purification of mutant Ras polypeptide (1) Production of expression vector

A cDNA part encoding H-Ras T35S (1-166aa) was amplified by a PCR method using a plasmid pBR322-H-Ras T35S (1-189aa) having incorporated therein cDNA encoding human H-Ras T35S (1-189aa) (see Oncogene (1994), 9, 2153-2157 and Oncogene (1992), 7, 475-480: cDNA donated by RIKEN) as a template and using two kinds of primers (primers 1 and 2). 5'- and 3'-regions of cDNA were treated with a restriction enzyme and then incorporated into a pGEX-6p-1 vector to produce pGEX-6p-H-Ras T35S (1-166aa).
Primer 1: cgggatccgatatgaccgaatacaaactgg (SEQ ID NO: 7)
Primer 2: gcgggatccctactagtgctgacggatttcacgaac (SEQ ID NO: 8)

A vector for mutant polypeptide expression pGEX-6p-1-M-Ras P40D (1-178aa) was produced by the so-calledsite-directedmutagenesismethod using a plasmid pGEX-6p-1-M-Ras (1-178aa) having incorporated therein cDNA (GenBank Accession No.: AF031159) encoding mouse M-Ras (1-178aa) as a template and using two kinds of primers (primers 3 and 4) for mutation introduction. Specifically, PCR was performed using the above-mentioned two kinds of primers, and the PCR product was then treated with a DpnI (NEB) enzyme to digest and remove the template plasmid. The resultant DNA solution was transformed into an *Escherichia coli* JM109 strain to serve as a host, plasmid DNAwas purified from a plurality of *Escherichia coli* clones, and the DNA sequence was confirmed (DNA sequencing) to identify the vector.
Primer 3: gaagatctttgtggatgactacgacccc (SEQ ID NO: 9)
Primer 4: ggggtcgtagtcatccacaaagatcttc (SEQ ID NO: 10)

### (2) Expression and purification of mutant Ras polypeptide

The thus produced two kinds of plasmids pGEX-6p-1-H-Ras T35S (1-166aa) and pGEX-6p-1-M-Ras P40D (1-178aa) were each treated with a restriction enzyme. The fragments obtained by treatment with a restriction enzyme were each fused with a polynucleotide encoding GST and introduced into a pGEX-6p-1 vector (GE Healthcare Bioscience) . The fragments were each transformed into the vector and expressed in an *Escherichia coli* strain AG-1.

*Escherichia coli* was harvested by centrifugation and suspended in a lysis solution (50 to 100 mM Tris-HCl, pH 8.0, 100 to 150 mM NaCl, 5 mM MgCl₂, 1 mM DTT, 1 mM EDTA, 0 to 0.01% TritonX-100) to lyse cells. After that, mutant Ras polypeptides were purified using chromatography at 4°C. Glutathione Sepharose 4B (GE Healthcare Bioscience) was used as a resin for affinity chromatography, POROS HQ (Applied Biosystem) was used as a column for ion-exchange chromatography, and a solution including 50 to 100 mM Tris-HCl, pH 8.0, 50 to 1,000 mM NaCl, 5 mM MgCl₂, 1 mM DTT, and 1 mM EDTA was used as an eluting solution.

After that, GDP bound to each of the mutant polypeptides was hydrolyzed using an alkaline phosphatase-bound resin (Phosphatase, Alkaline-Agarose, SIGMA), and a nonhydrolyzable GTP analog Gpp(NH)p was added. Gpp(NH)p unbound to each of the mutant polypeptides was removed by buffer exchange (PD-10 Columns, GE Healthcare Bioscience), and the sample solutions were concentrated to 10 to 20 mg/mL (Amicon Ultra-15, MILLIPORE). After that, the solutions were analyzed by an SDS-polyacrylamide electrophoresis method (SDS-PAGE), and found to have polypeptides with a purity of 95% or more.

### (Example 2) Production of co-crystal of polypeptide andGTP analog

The resultant Gpp(NH)p-bound Ras mutant polypeptides were concentrated and then crystallized by a vapor diffusion method.

### (Example 2-1)

Gpp(NH)p-bound H-Ras T35S (hereinafter, also referred to as "H-Ras T35S-Gpp (NH)p") was dissolved in a buffer including 40 mM Tris-HCl, pH 7.4, 50 mM NaCl, 5 mM MgCl₂, 7% DMSO, and 10 mM Gpp(NH)p. 1.5 µl of the peptide solution at 16 mg/mL were mixed with 1.0 µl of a reservoir solution (0.1 M MES, pH 6.5, 0.2 M (NH₄)₂SO₄, 30% PEG5000) to produce a crystal of H-Ras T35S-Gpp (NH) p by a sitting drop vapor diffusionmethod at 20°C.

The crystal of H-Ras T35S-Gpp(NH)p had a size of 0.3×0.2×0.1 mm³, a space group of R32, and lattice constants of a=b=92.81 to 94.20 Å, c=117.13 to 120.97 Å, α=β=90°, and γ=120° (five data sets).

### (Example 2-2)

Further, H-Ras T35S was solubilized in 40 mM Tris-HCl, pH 7.4, 50mMNaCl, 5 mM MgCl₂, and 10 mM Gpp (NH) p. 1.0 µl of the peptide solution at 16 mg/mL was mixed as a different peptide solution with 1.0 µl of a reservoir solution (0.1 M MES, pH 6.5, 0.2 M (NH₄)₂SO₄, 30% PEG5000) to produce a crystal of H-Ras T35S-Gpp(NH)p by a sitting drop vapor diffusion method at 20°C.

The crystal of H-Ras T35S-Gpp(NH)p had a size of 0.3×0.01×0.01 mm³, a space group of I222, and lattice constants of a=34.58 Å, b=82.00 Å, c=122.00 Å, and α=β=γ=90.00° (one data set).

### (Example 2-3)

Gpp(NH)p-bound M-Ras P40D (hereinafter, also referred to as "M-Ras P40D-Gpp (NH)p") was dissolved in a buffer including 50 mM Tris-HCl, pH 7.4, 50 mM NaCl, 5 mM MgCl₂, 1 mM DTT, and 1 mM EDTA. 2 µl of the peptide solution at 18 mg/mL were mixed with 2 µl of a reservoir solution (150 mM MgSO₄, 100 mM Na cacodylate, pH 6.5, 28% (w/v) PEG-1500) to produce a crystal of M-Ras P40D-Gpp(NH)p by a sitting drop vapor diffusion method at 20°C.

The crystal of M-Ras P40D-Gpp(NH)p had a size of 0.2×0.1×0.01 mm³, a space group of C2, and lattice constants of a=33.42 to 33.74 Å, b=65.29 to 65.73 Å, c=73.37 to 74.23 Å, α=γ=90.00°, and β=94.92 to 95.33°Å (nine data sets).

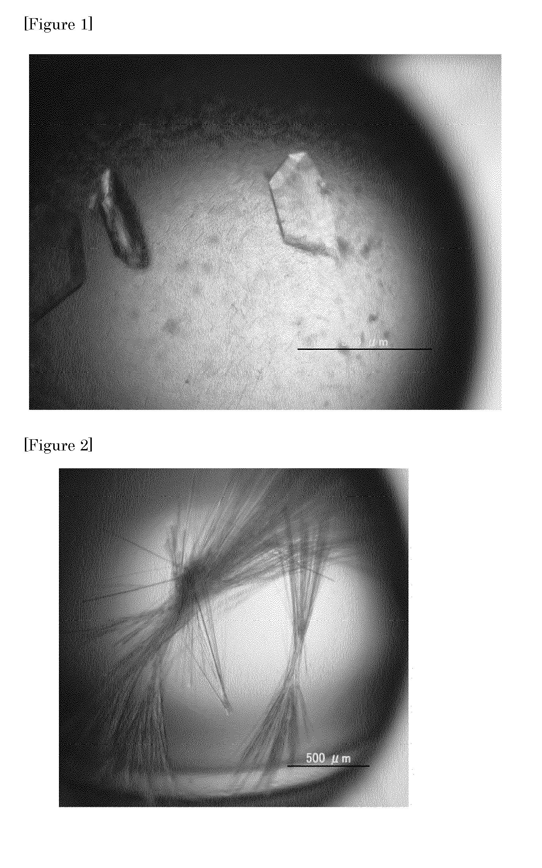

FIGS. 1 to 3 show typical photographs of the respective crystals obtained in Examples 2-1 to 2-3.

### (Example 3) Crystallographic analysis by X-ray diffraction

Monochromatic X-rays were applied to the resultant crystal, and information about a conformation of each of H-Ras T35S-Gpp(NH)p and M-Ras P40D-Gpp(NH)p was clarified based on the resultant X-ray diffraction image. The diffraction intensity was measured at beamlines BL38B1 and BL41XU of SPring-8 (Harima Science Garden City) usingaJupiter 210 detector (Rigaku/MSC Corporation) and a Quantum 315 CCD detector (ADSC). Further, the measurement was carried out at a low temperature of 100 K.

X-ray diffraction data was collected to carry out the indexing of individual diffraction spots and the calculation of diffraction intensities. Phase angles were determined from the resultant diffraction intensities and search models by a molecular replacement method. Electron density maps were derived by inverse Fourier transform based on the diffraction intensities of the diffraction spots and the phase angles described above. Atomic coordinates were constructed based on the resultant electron density maps.

Specifically, the data obtained for H-Ras T35S-Gpp(NH)p in Example 2-1 was processed using HKL2000 and scaled with SCALEPA CK. H-Ras (PDB ID: 5P21) was used as a search model to determine the crystal structure of H-Ras T35S-Gpp(NH)p by a molecular replacement method. The resultant model was refined at 1.95 Å using CNS and REFMAC. After each refinement, the resultant model was corrected with an electron density map 2Fo-Fc map using COOT (FIG. 4). Water molecules were picked by Find Waters of COOT.

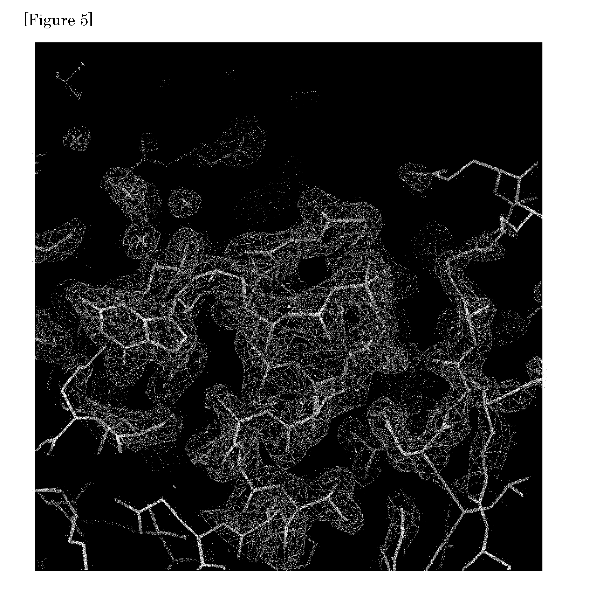

Further, the data obtained for H-Ras T35S-Gpp(NH)p in Example 2-2 was refined at 2.10 Å by the same method as described above (FIG. 5) .

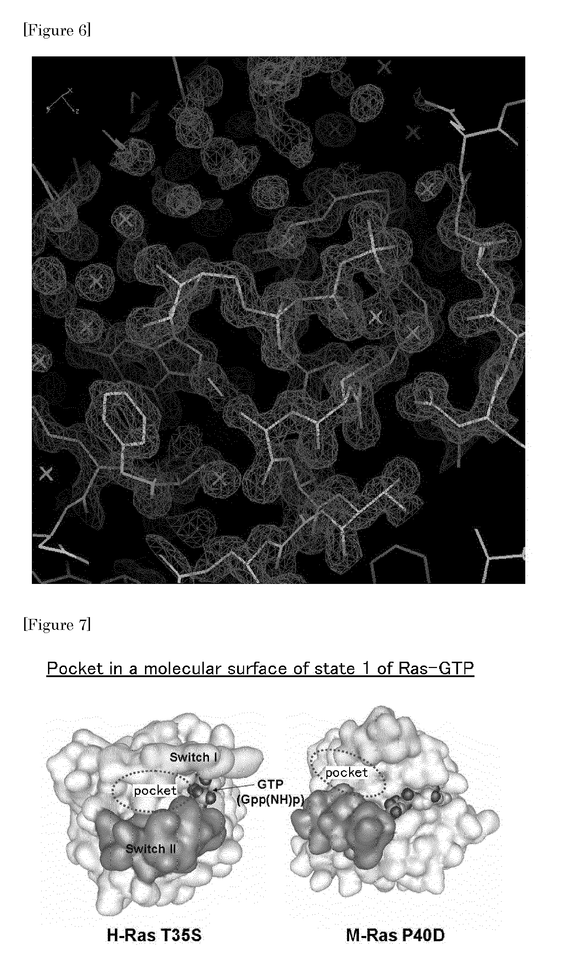

The data obtained for M-Ras P40D-Gpp(NH)p in Example 2-3 was processed using MOSFLM and scaled with SCALA. M-Ras (PDB ID: 1X1S) was used as a search model to determine the crystal structure of M-Ras P40D-Gpp(NH)p by a molecular replacement method. The resultant model was refined at 1.35 Å using CNS and REFMAC. After the refinement, the resultant model was corrected with an electron density map 2Fo-Fc map using COOT (FIG. 6). Water molecules were picked by Find Waters of COOT.

Tables 1 to 3 below show data of atomic coordinates of H-Ras T35S-Gpp(NH)p and M-Ras P40D-Gpp(NH)p. Table 1 shows atomic coordinates obtained from the crystal of H-Ras T35S-Gpp(NH)p obtained in Example 2-1 (having a size of 0.3×0.2×0.1 mm³, a space group of R32, and lattice constants of a=b=94.20+0.00 Å, c=120.97+0.00 Å, α=β=90.00+0.00°, and γ=120.00±0.00°), Table 2 shows atomic coordinates obtained from the crystal of H-Ras T35S-Gpp(NH)p obtained in Example 2-2 (having a size of 0.3×0.01×0.01 mm³, a space group of I222, and lattice constants of a=34.58 Å, b=82.00 Å, c=122.00 Å, and α=β=γ=90.00°), and Table 3 shows atomic coordinates obtained from the crystal of M-Ras P40D-Gpp(NH)p (having a size of 0.2×0.1×0.01 mm³, a space group of C2, and lattice constants of a=33.72±0.00 Å, b=65.70±0.00 Å, c=74.08±0.00 Å, α=γ=90.00±0.00°, and β=95.02±0.00°). Further, FIG. 7 illustrates a conformation constructed from data of the atomic coordinates.

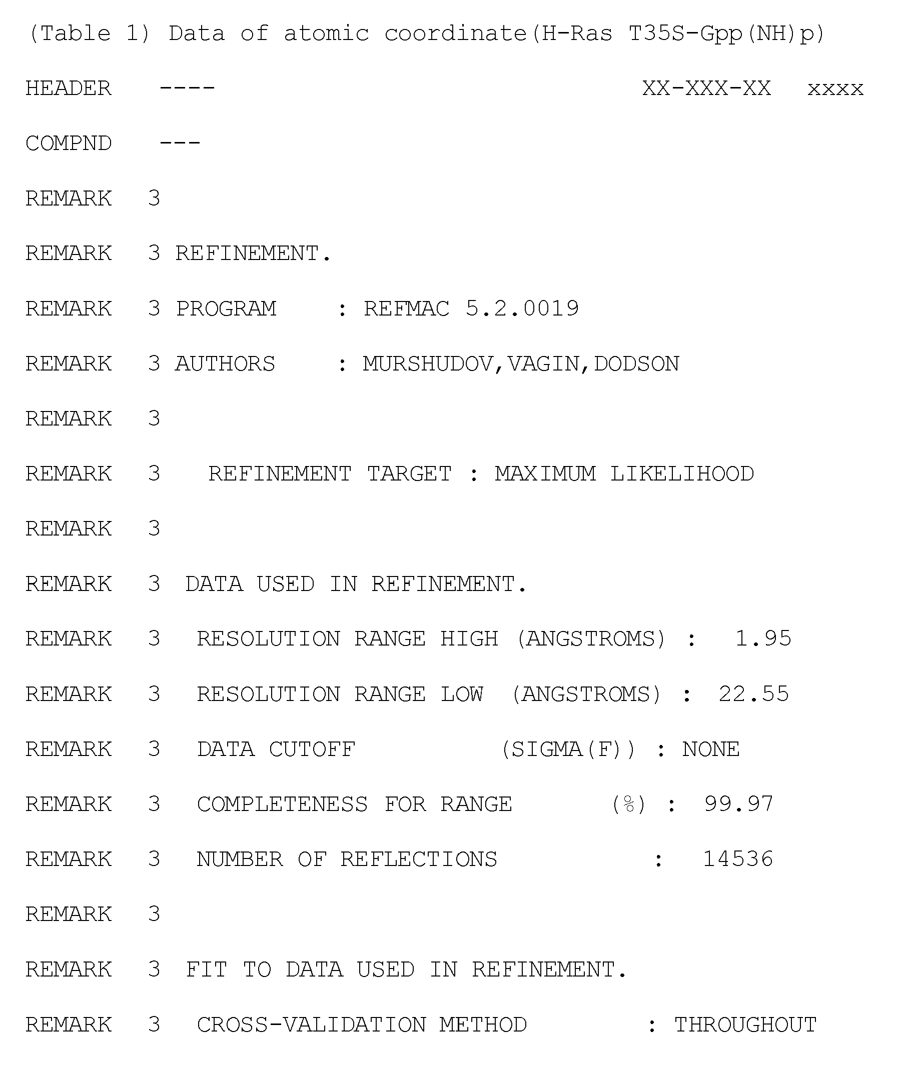

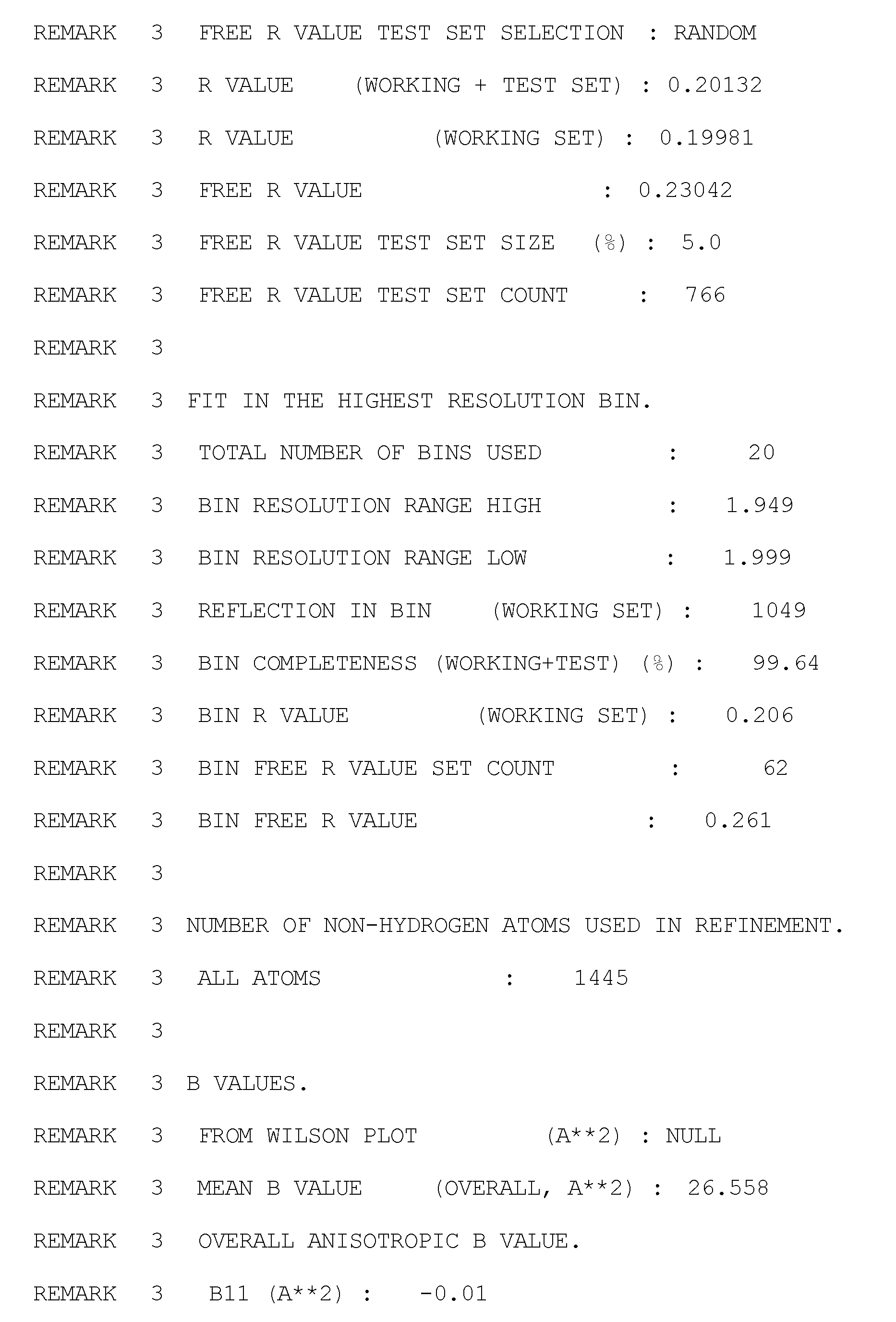

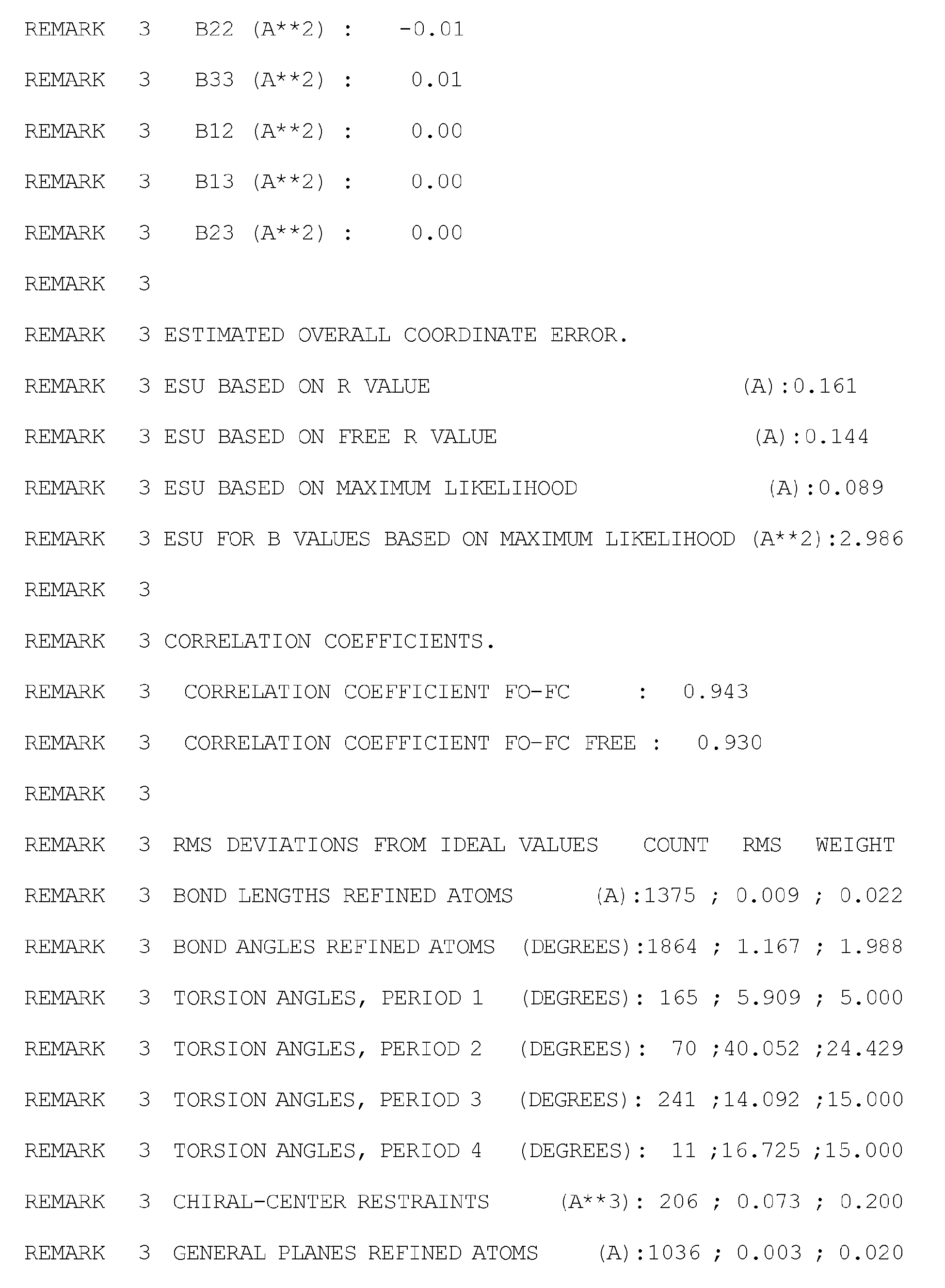

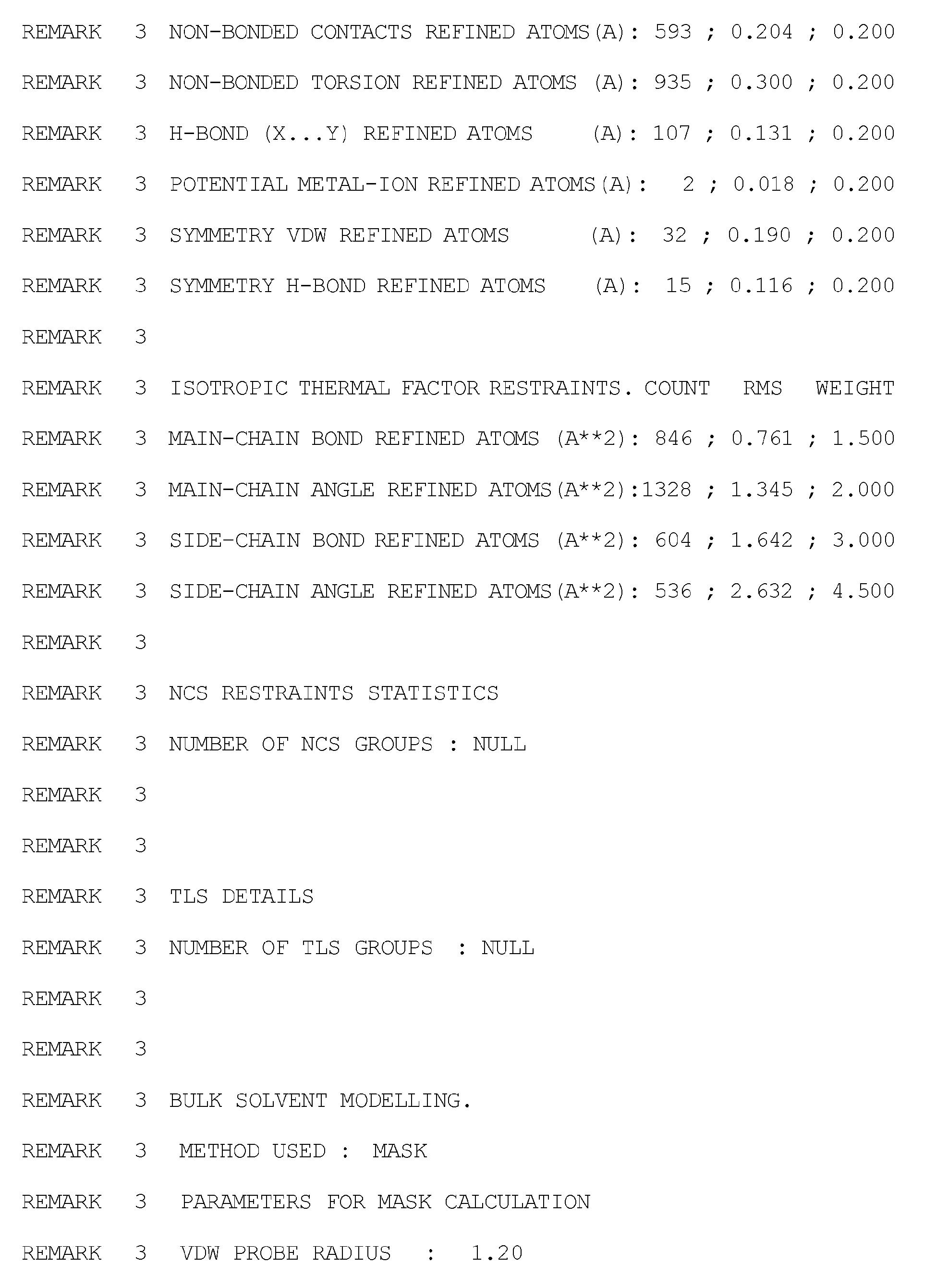

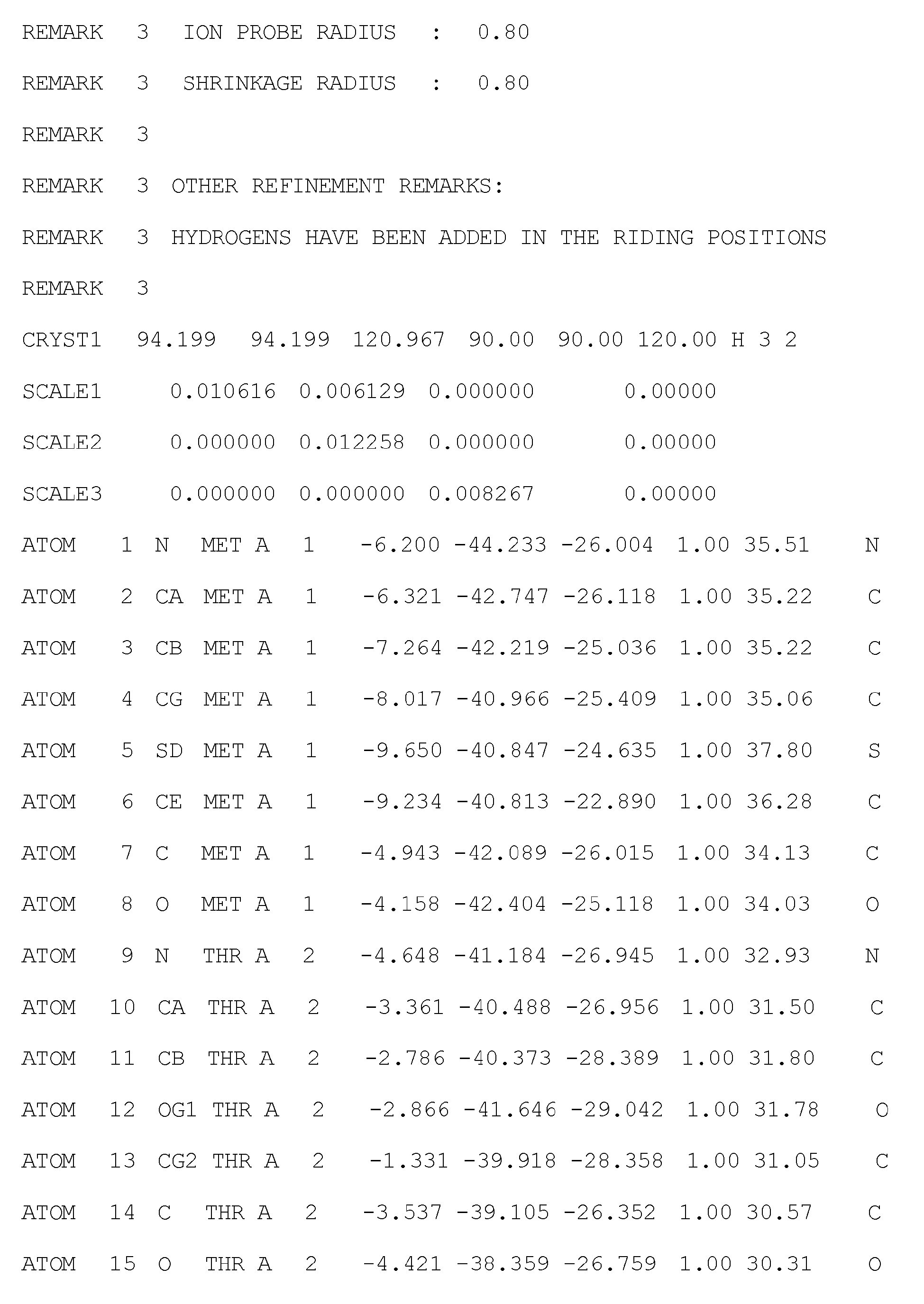

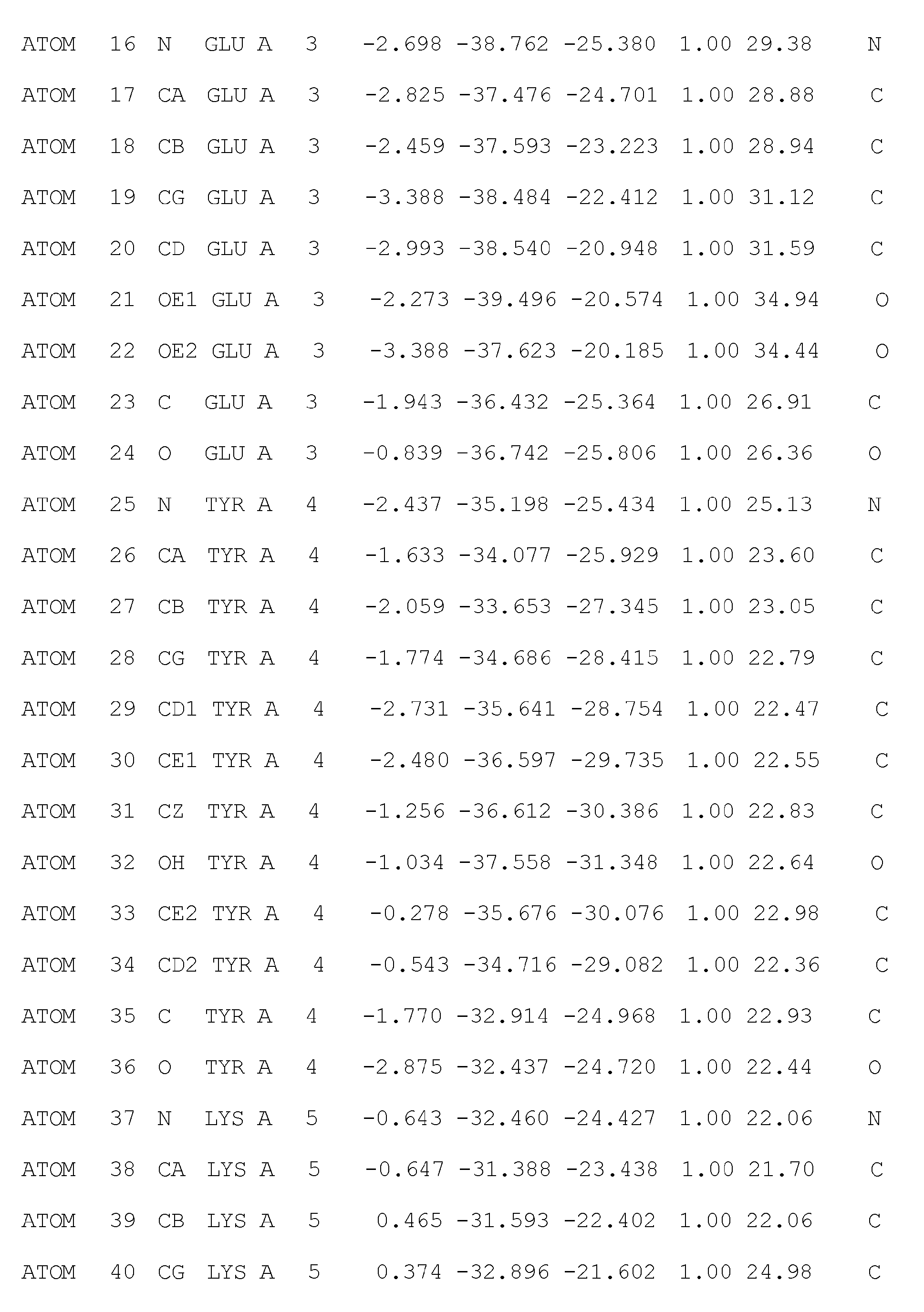

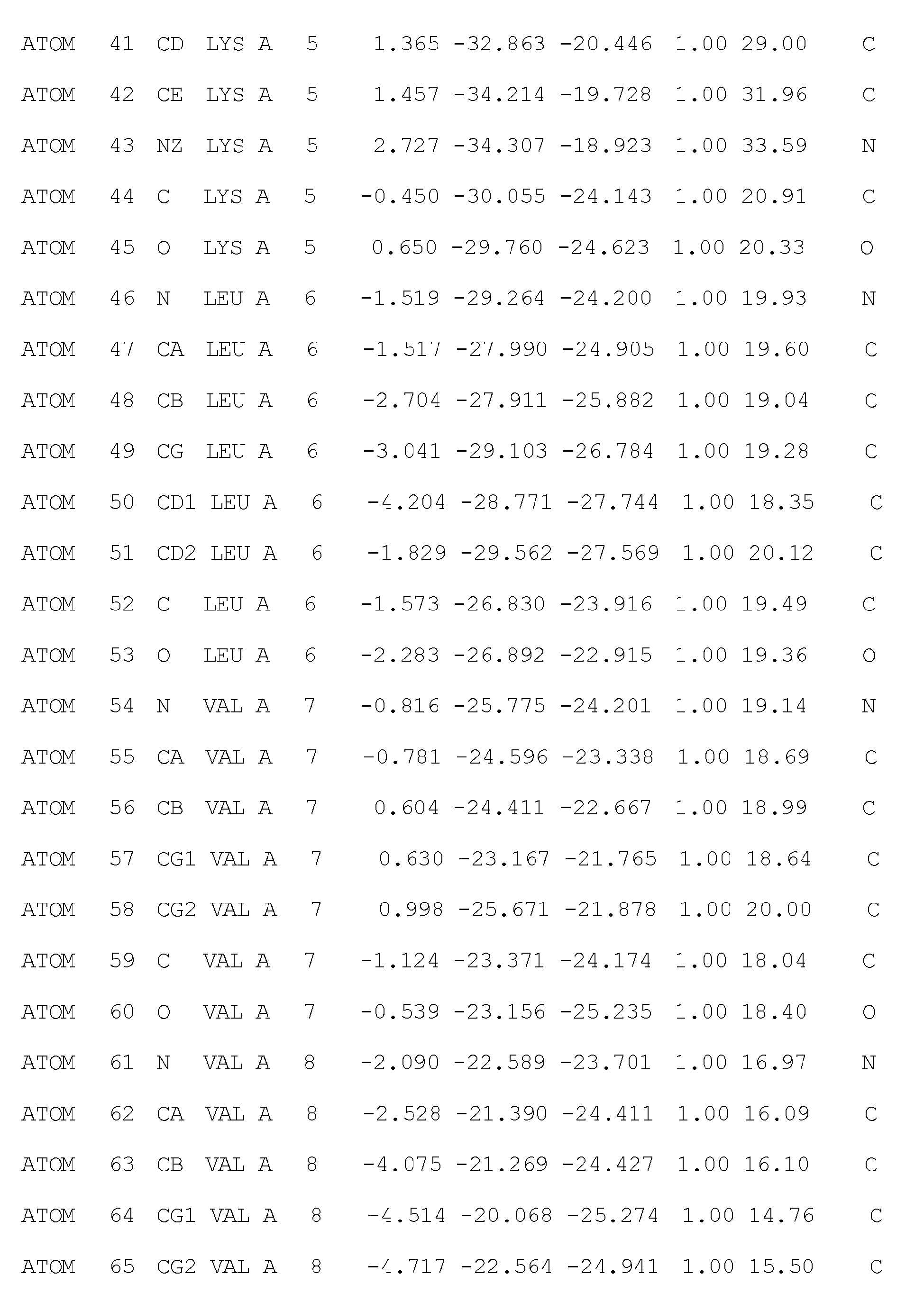

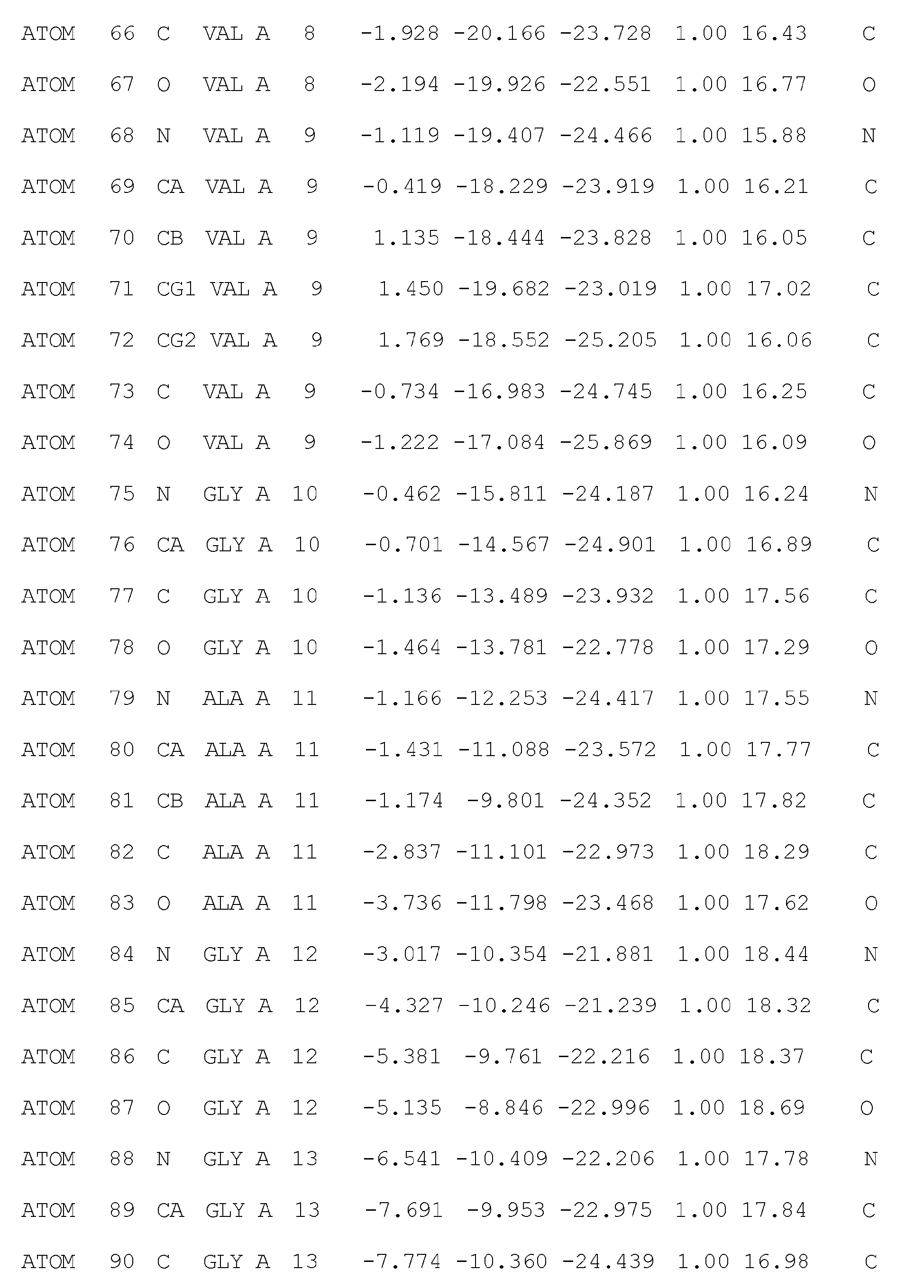

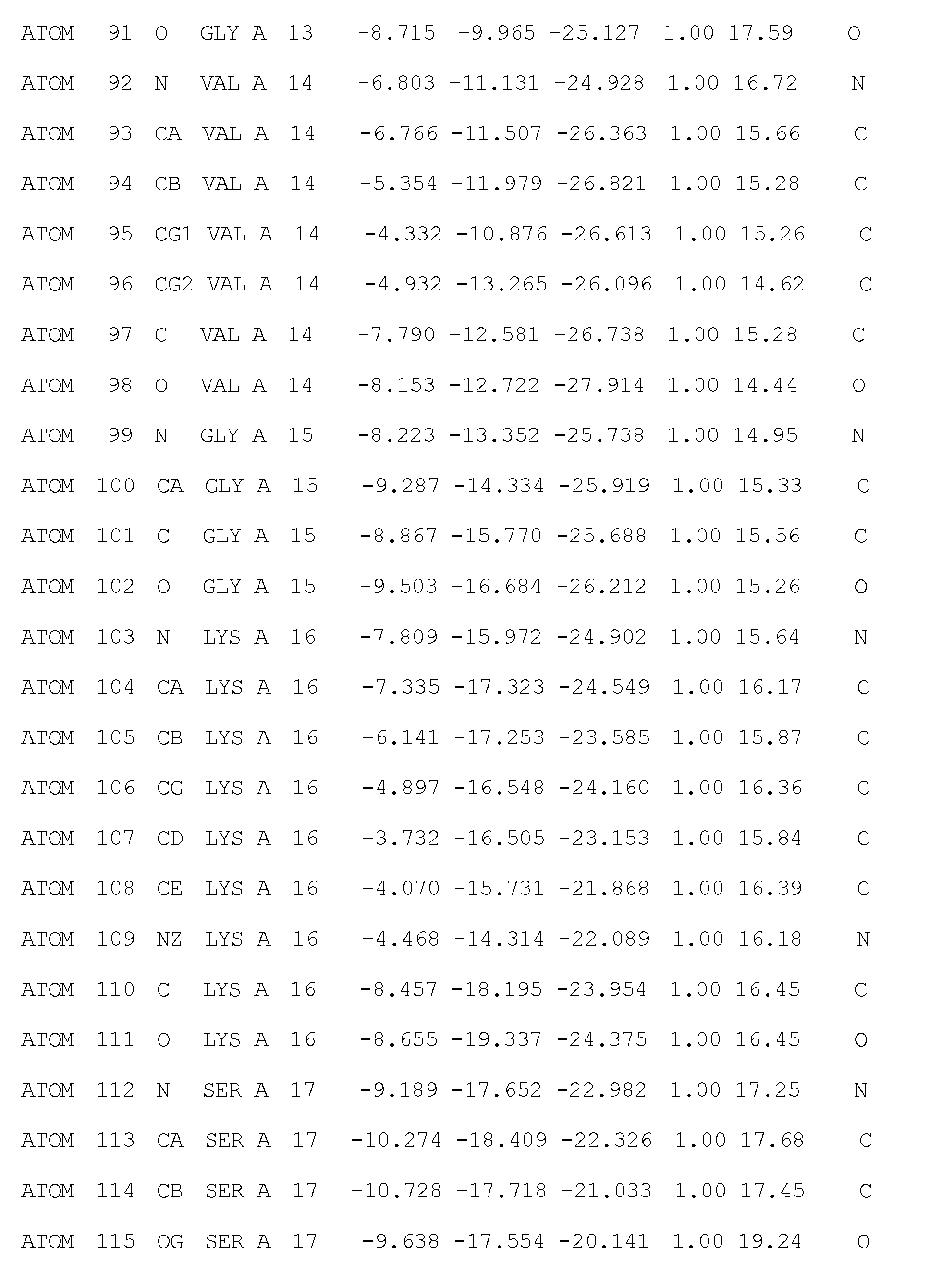

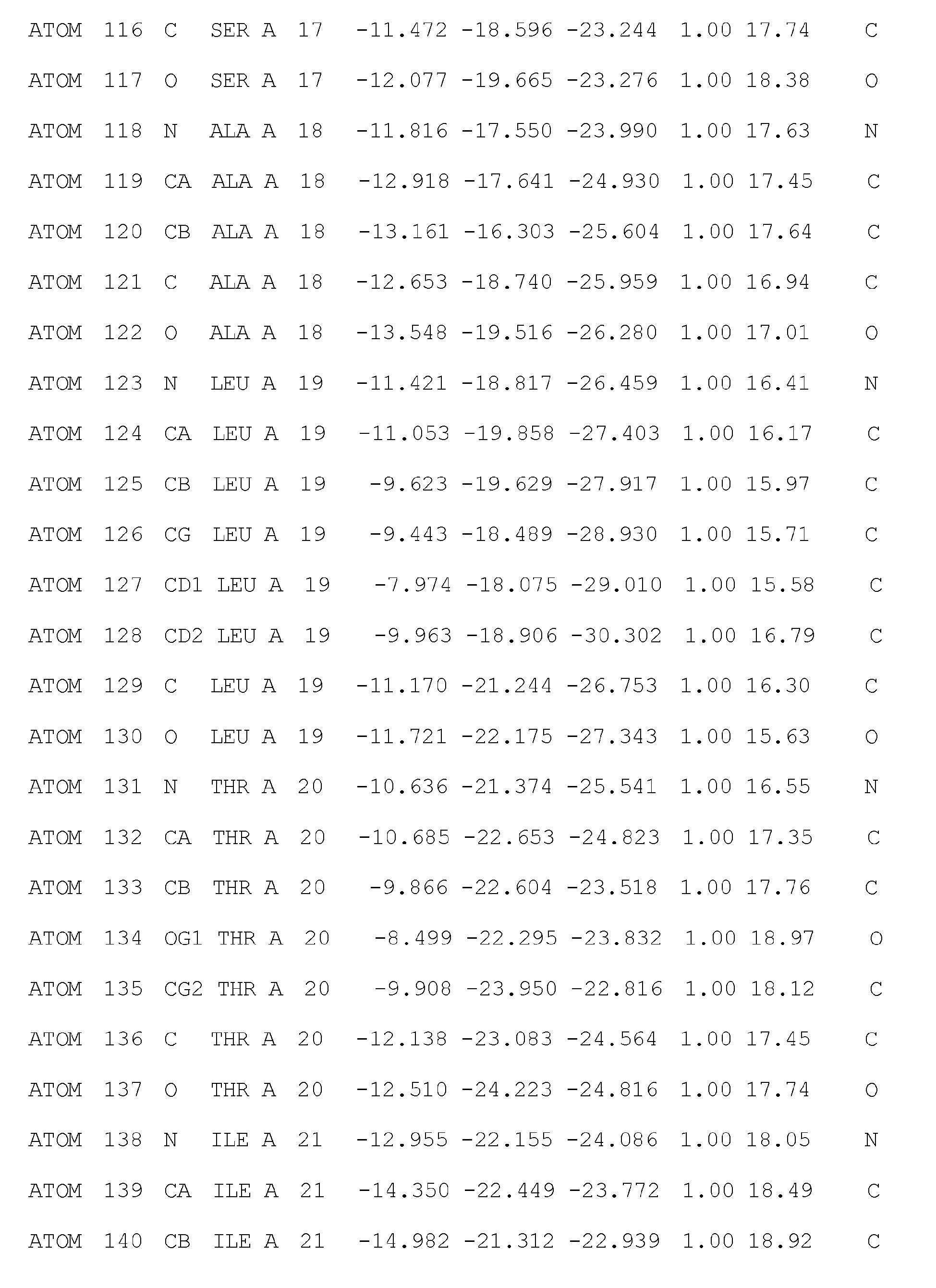

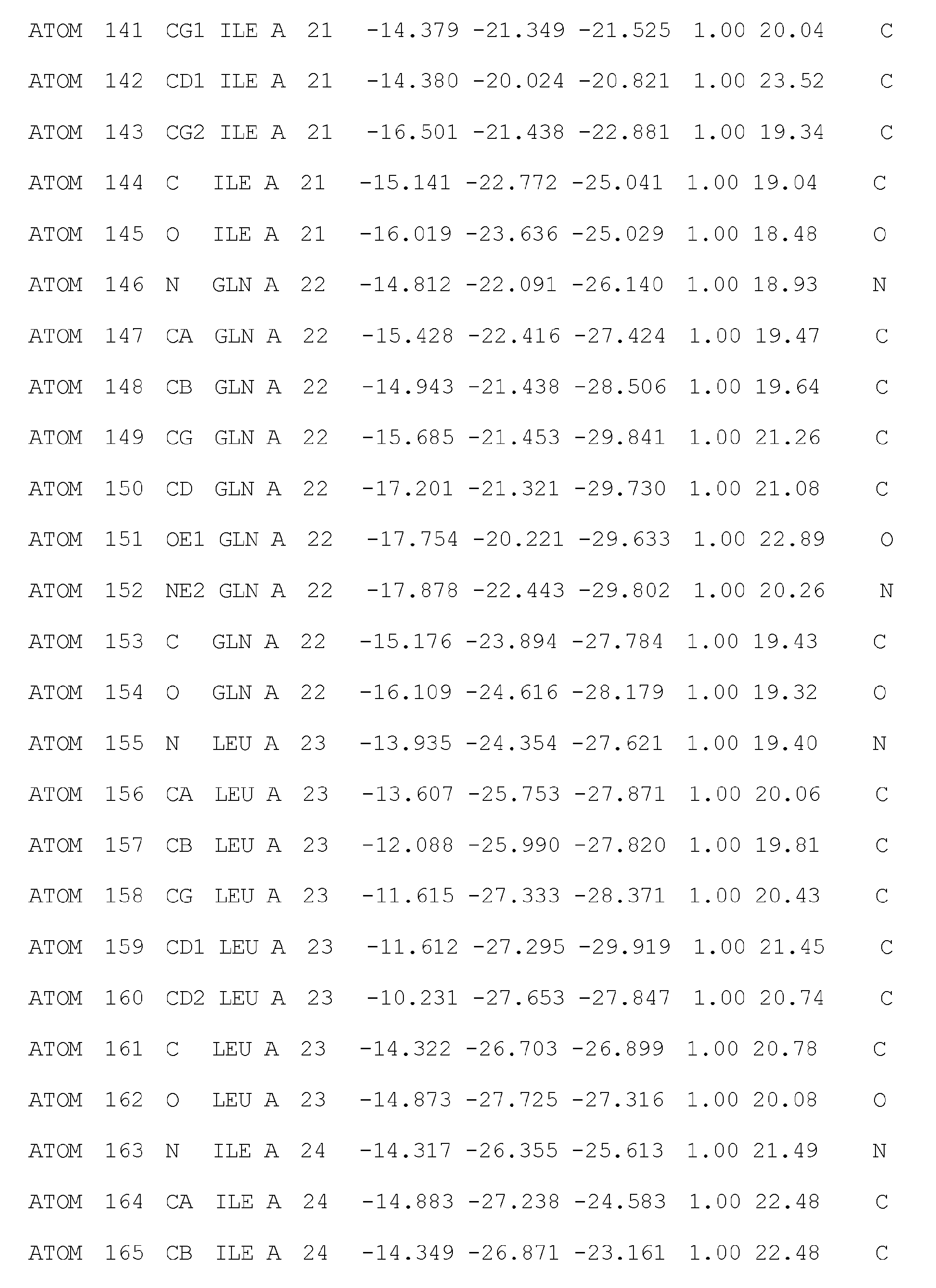

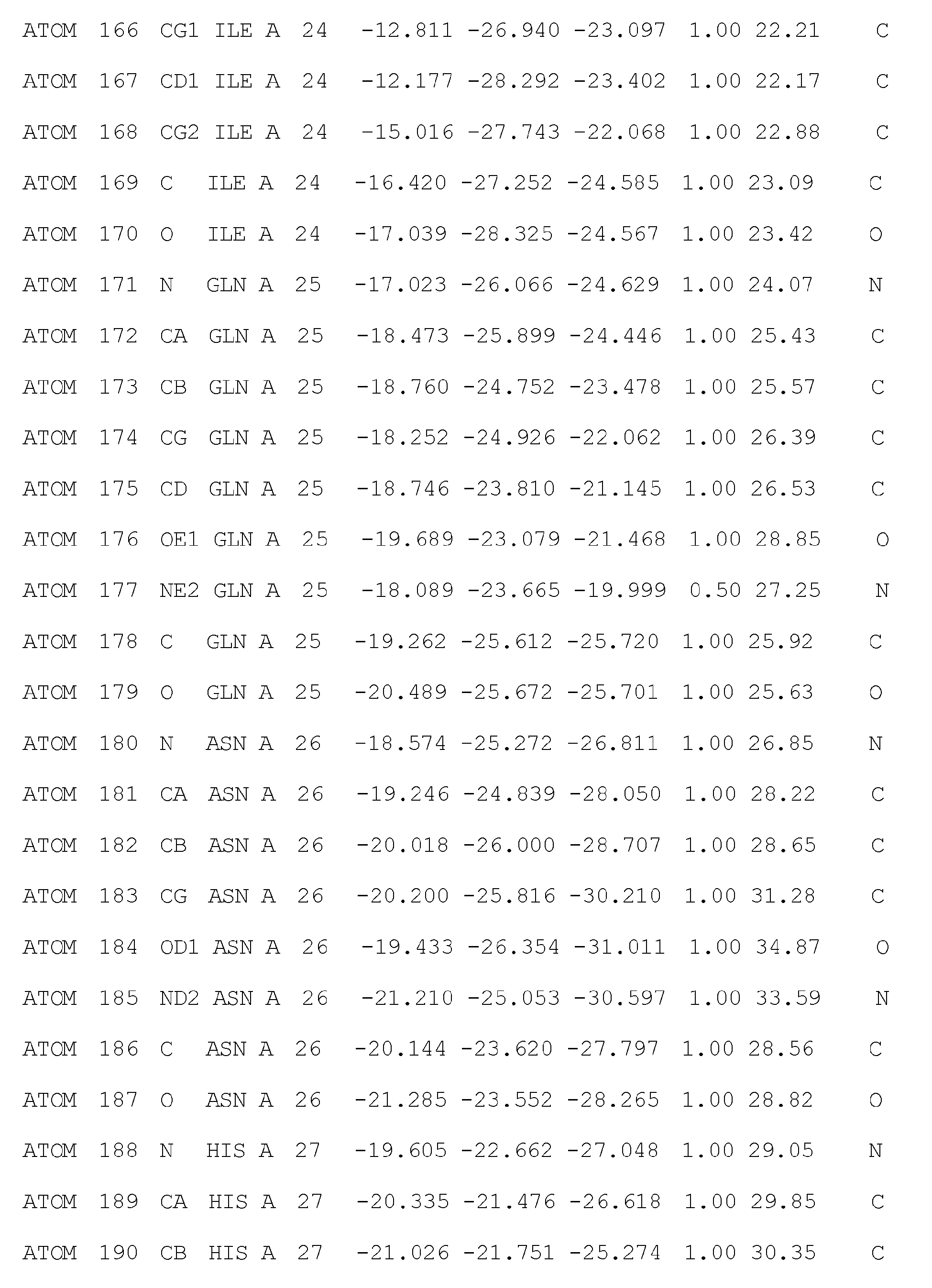

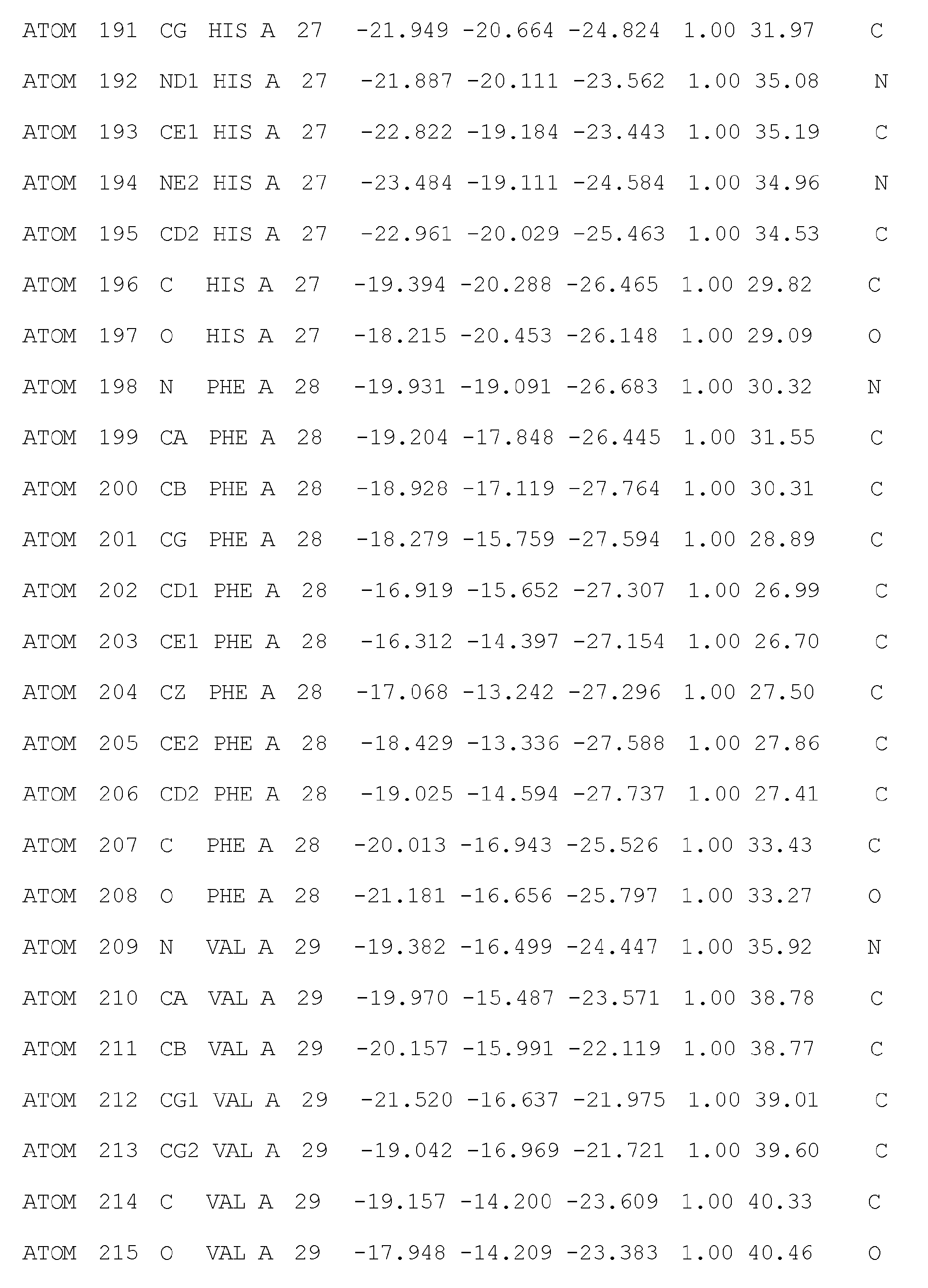

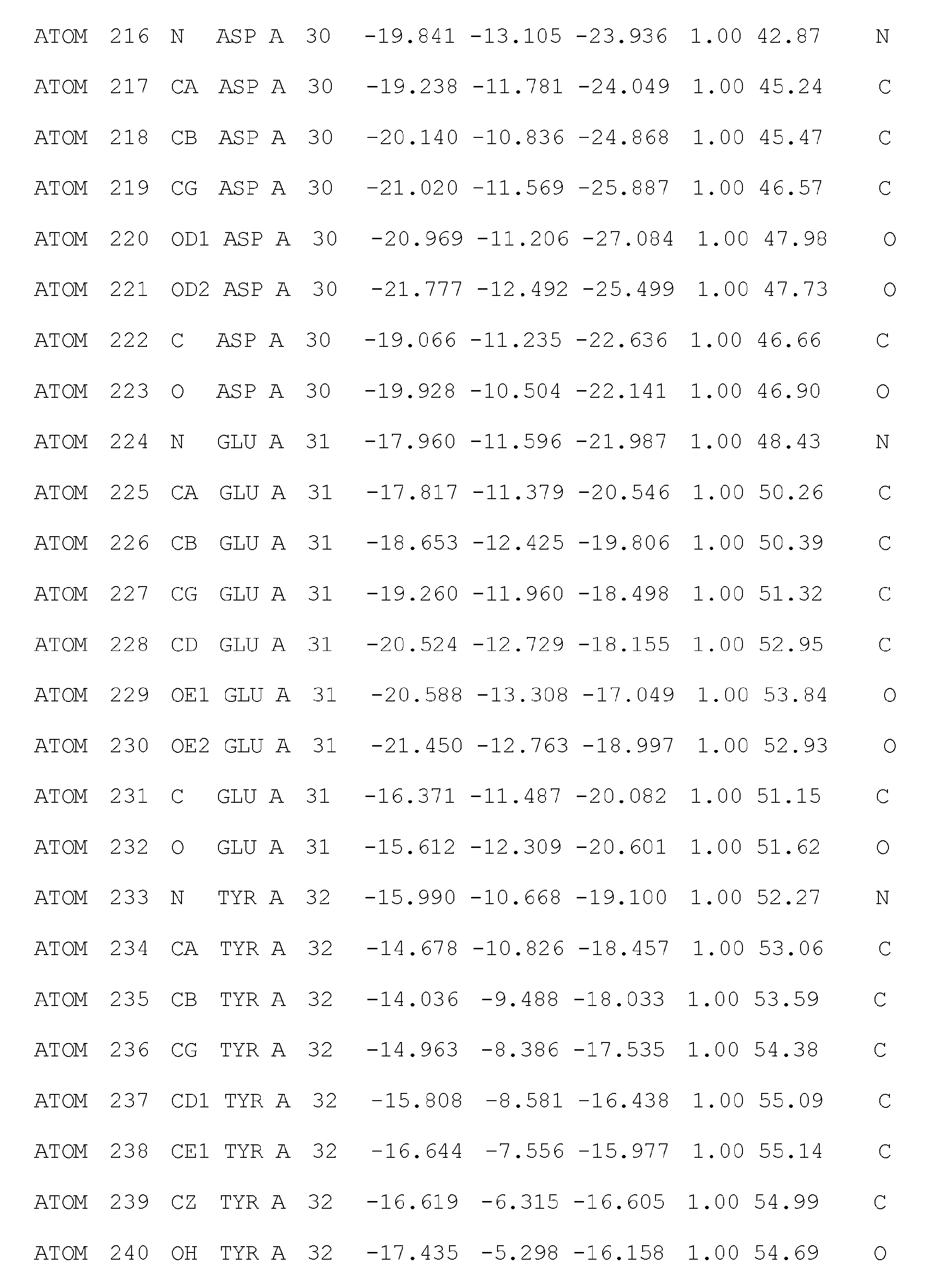

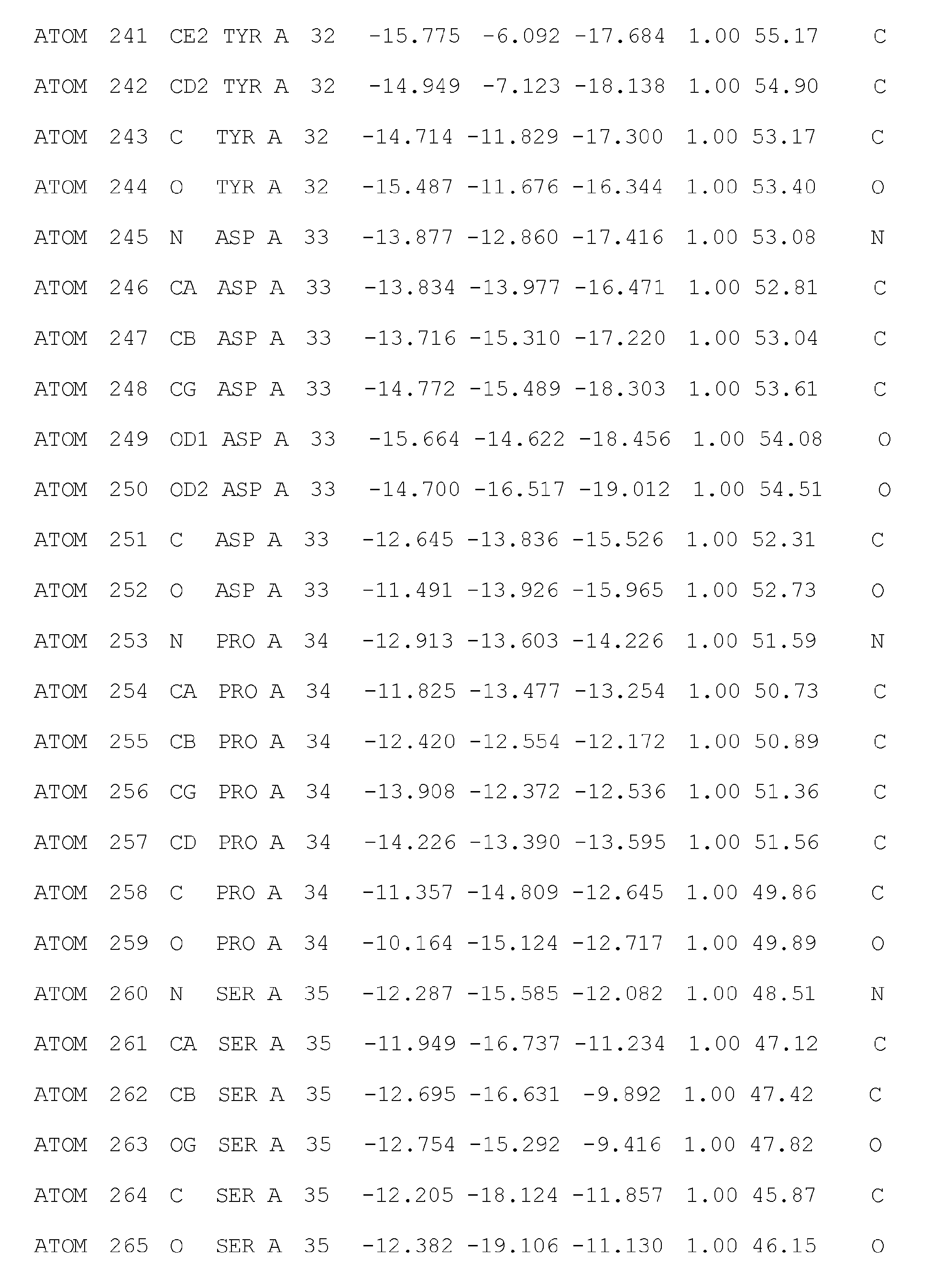

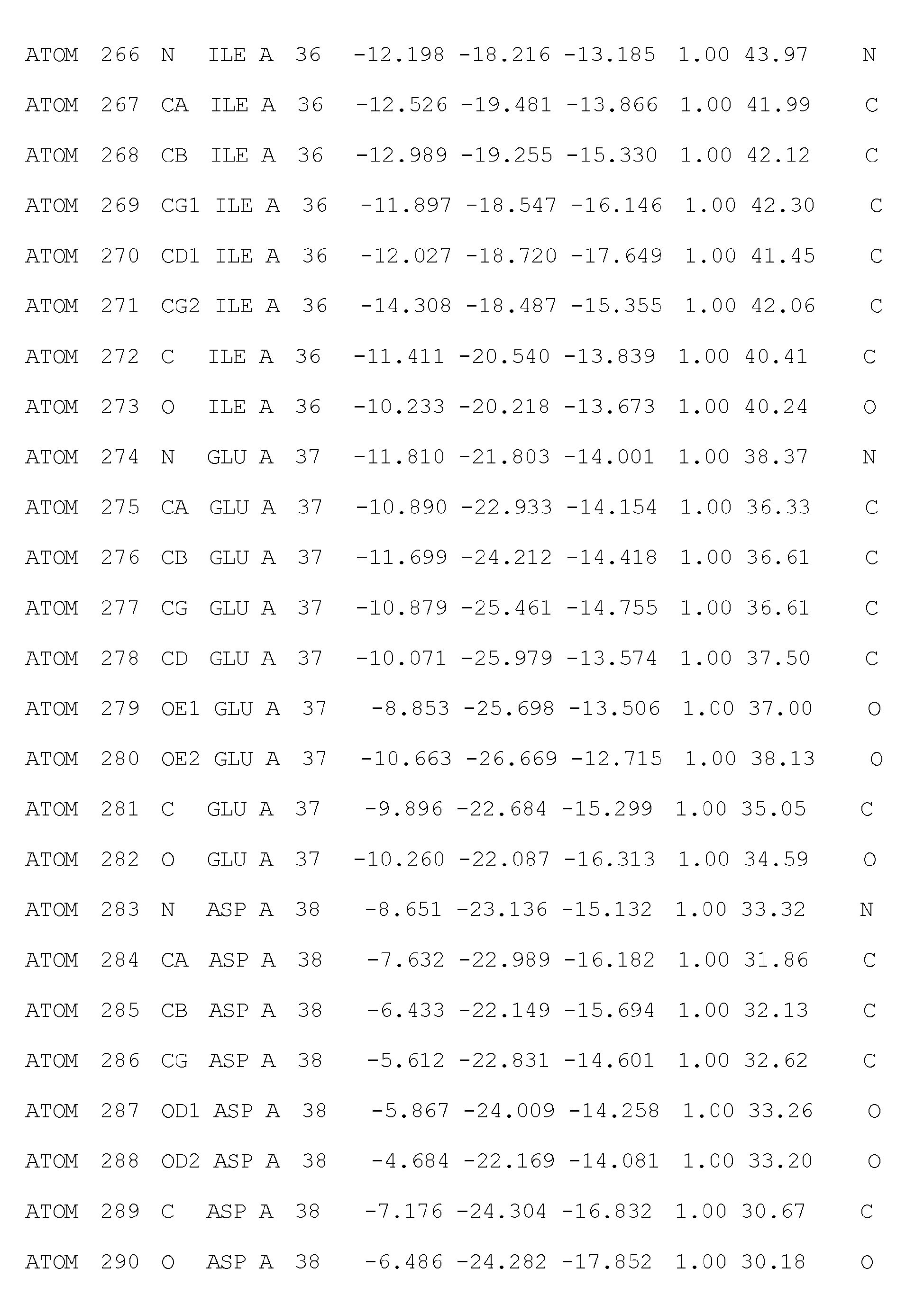

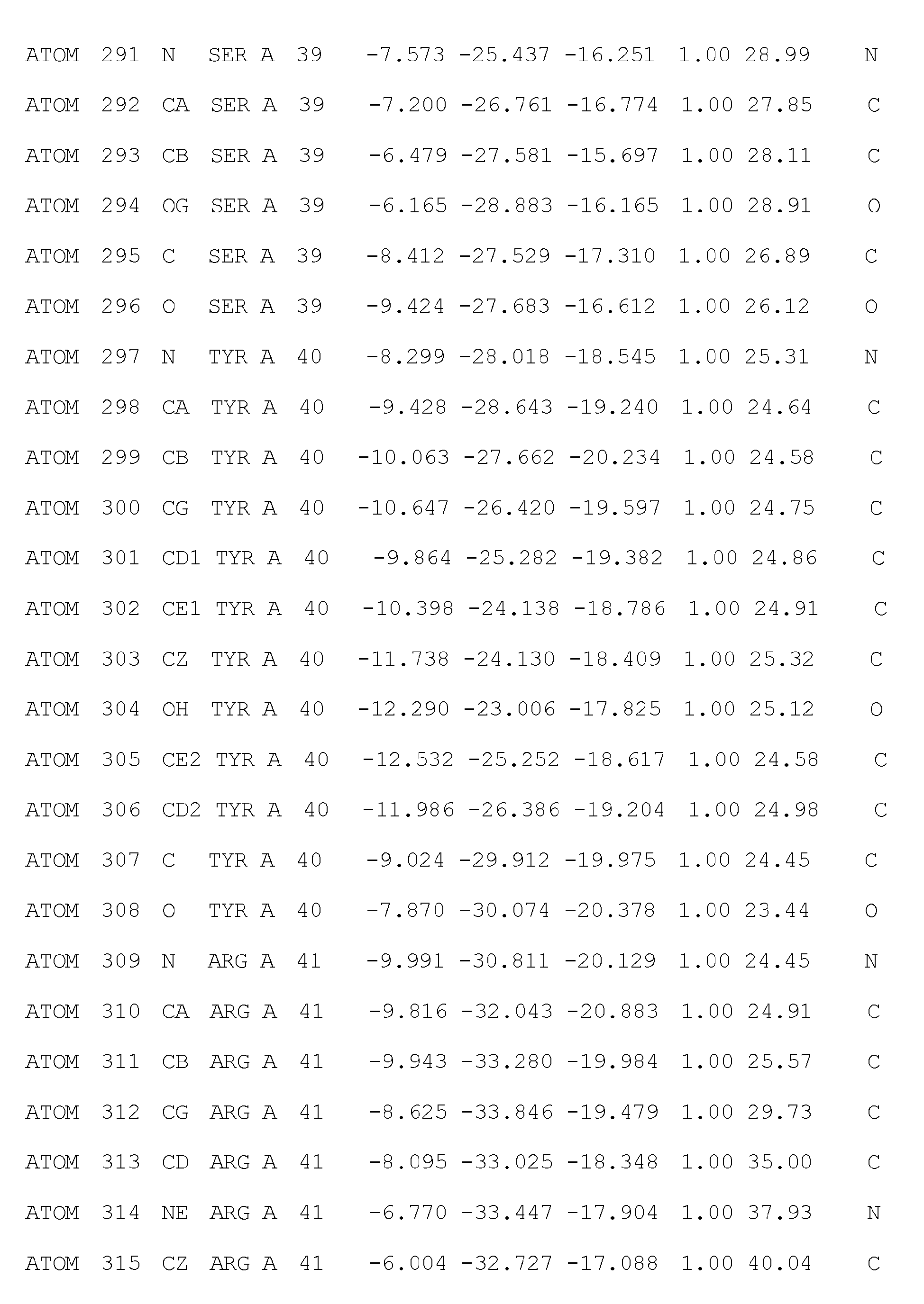

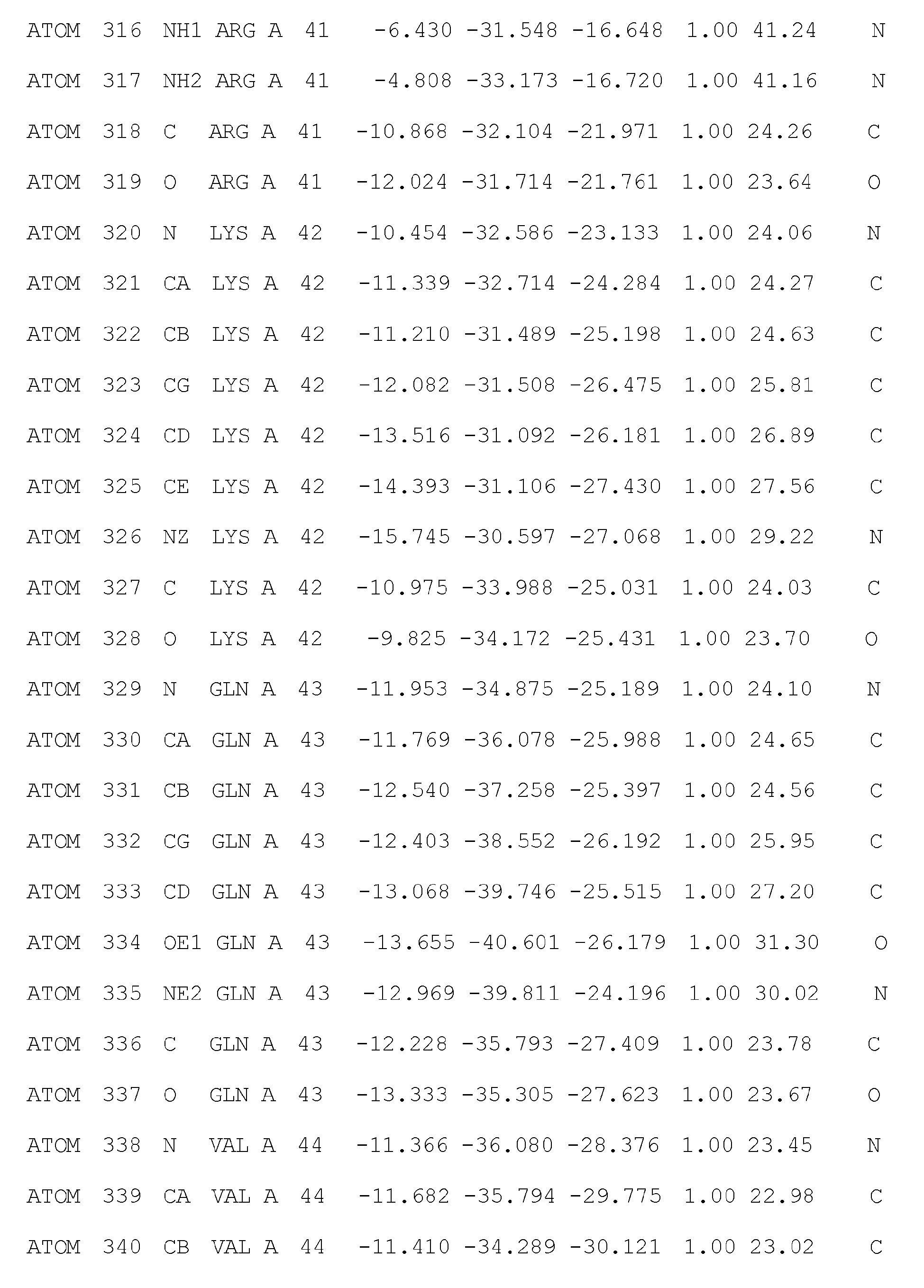

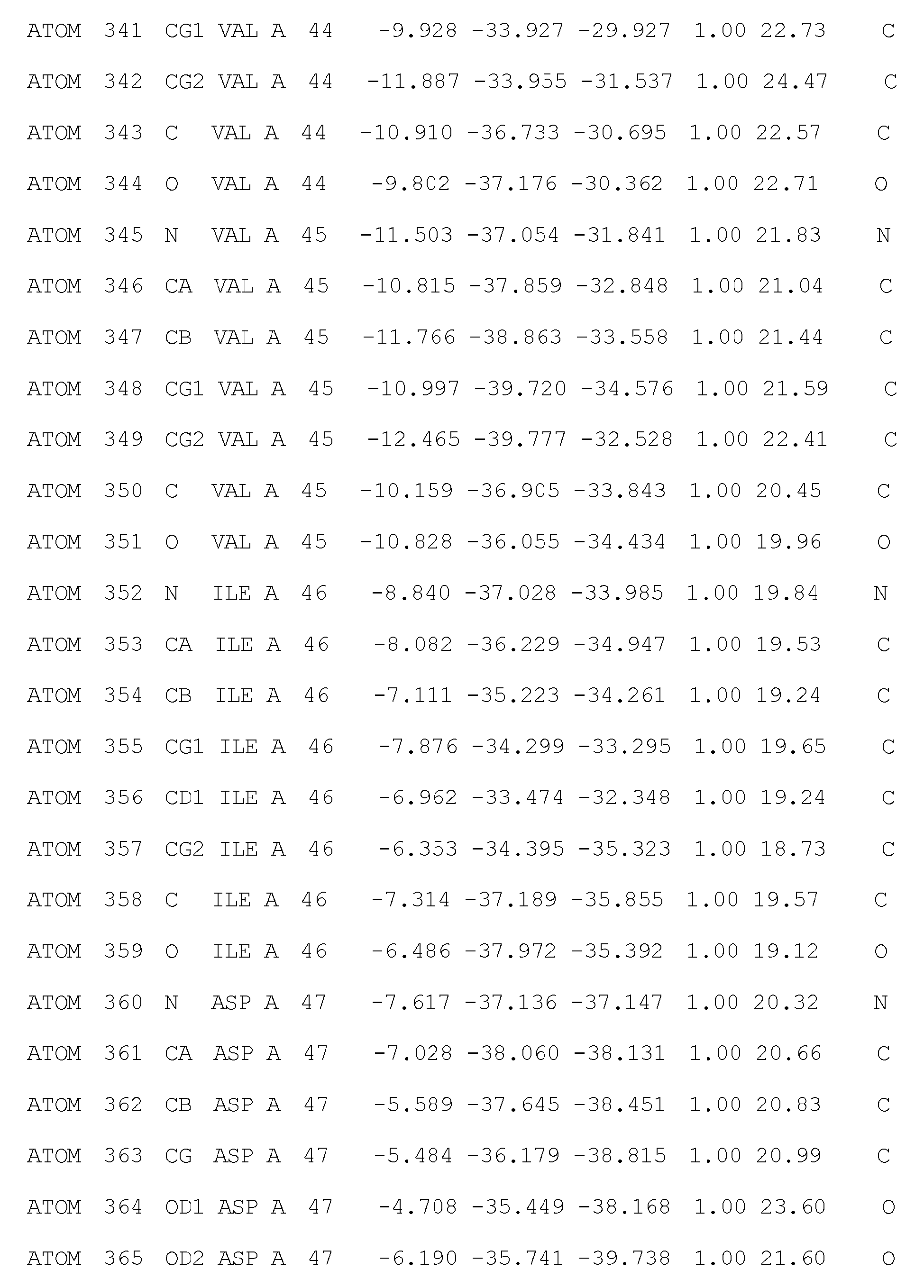

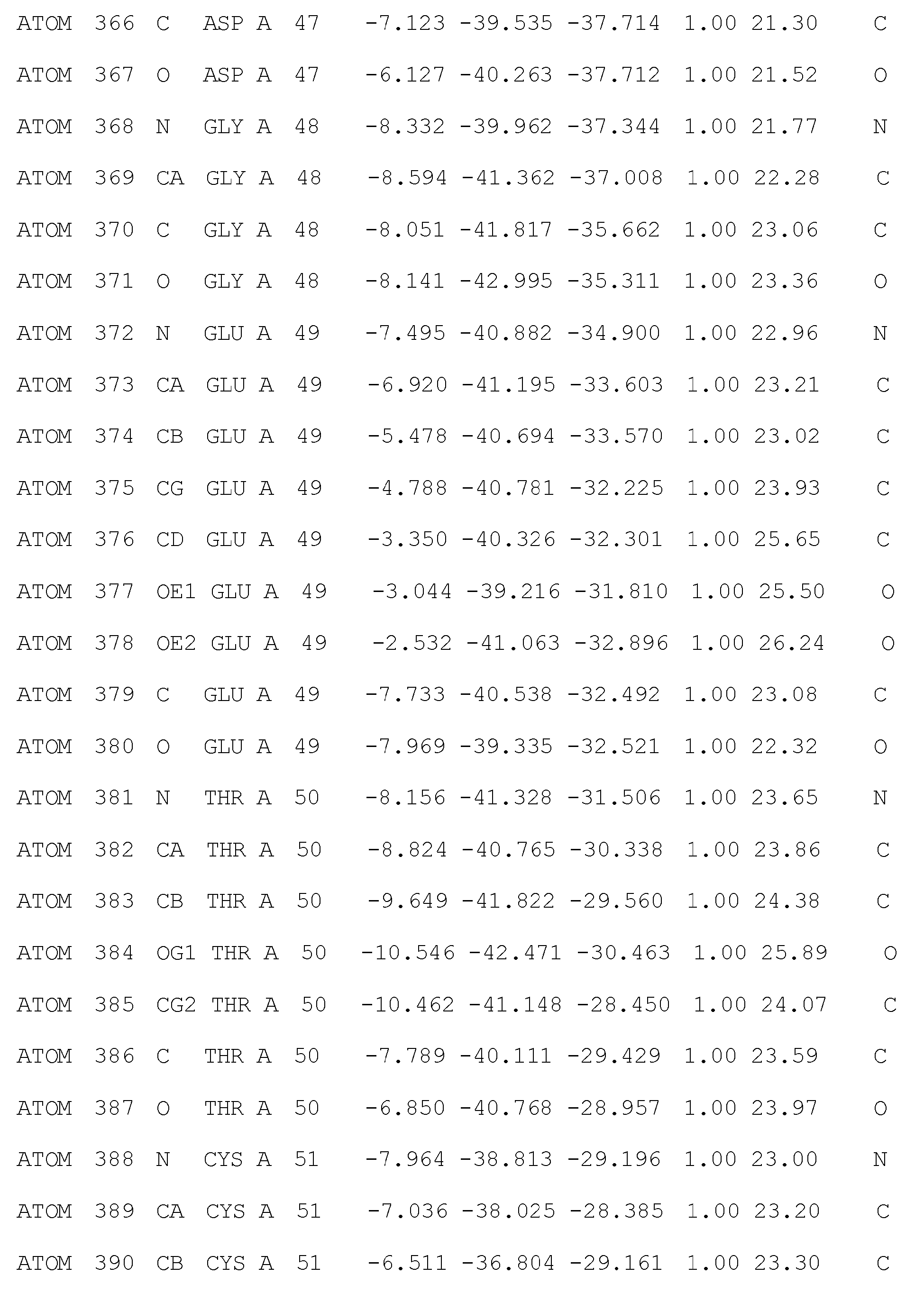

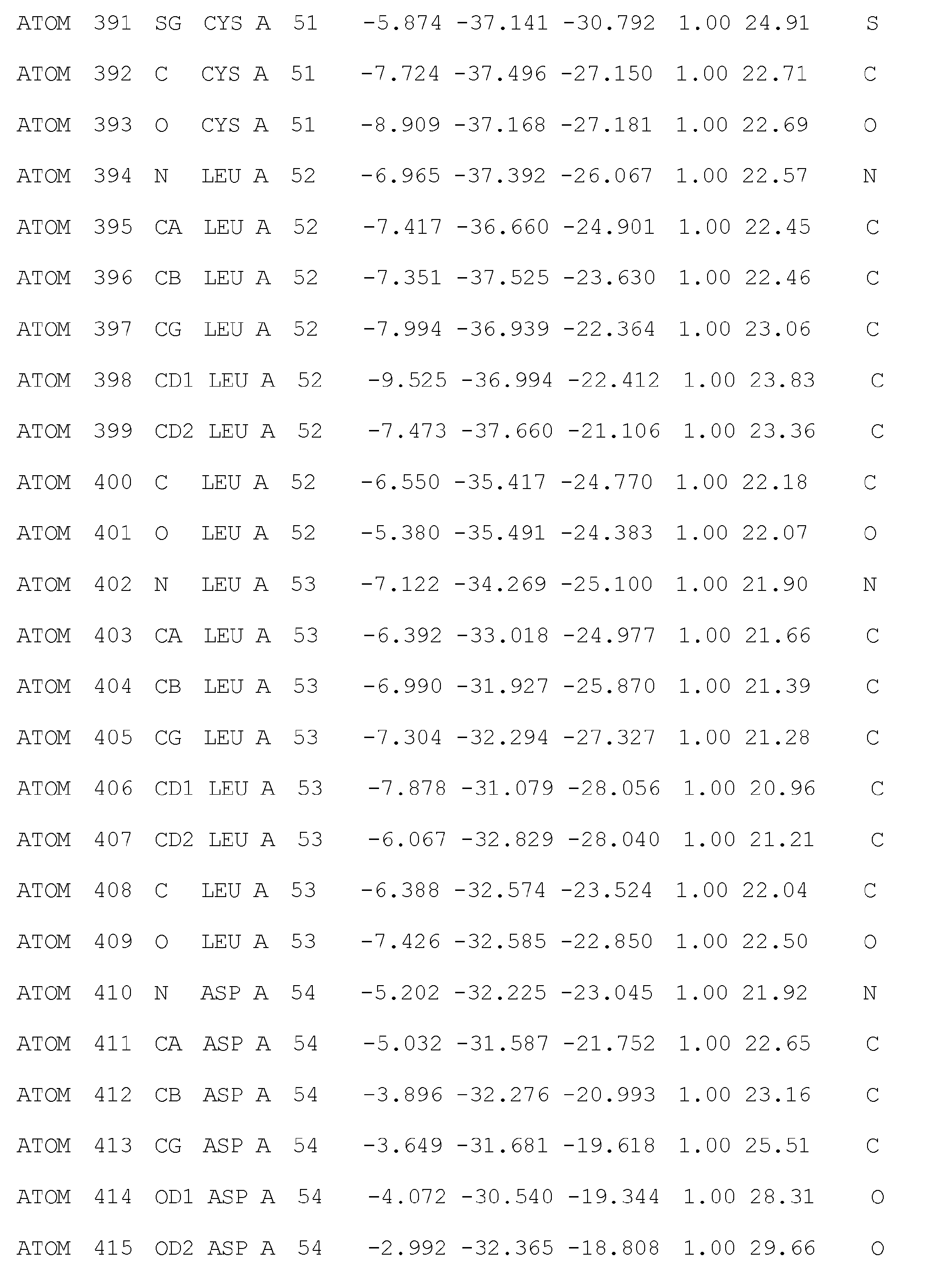

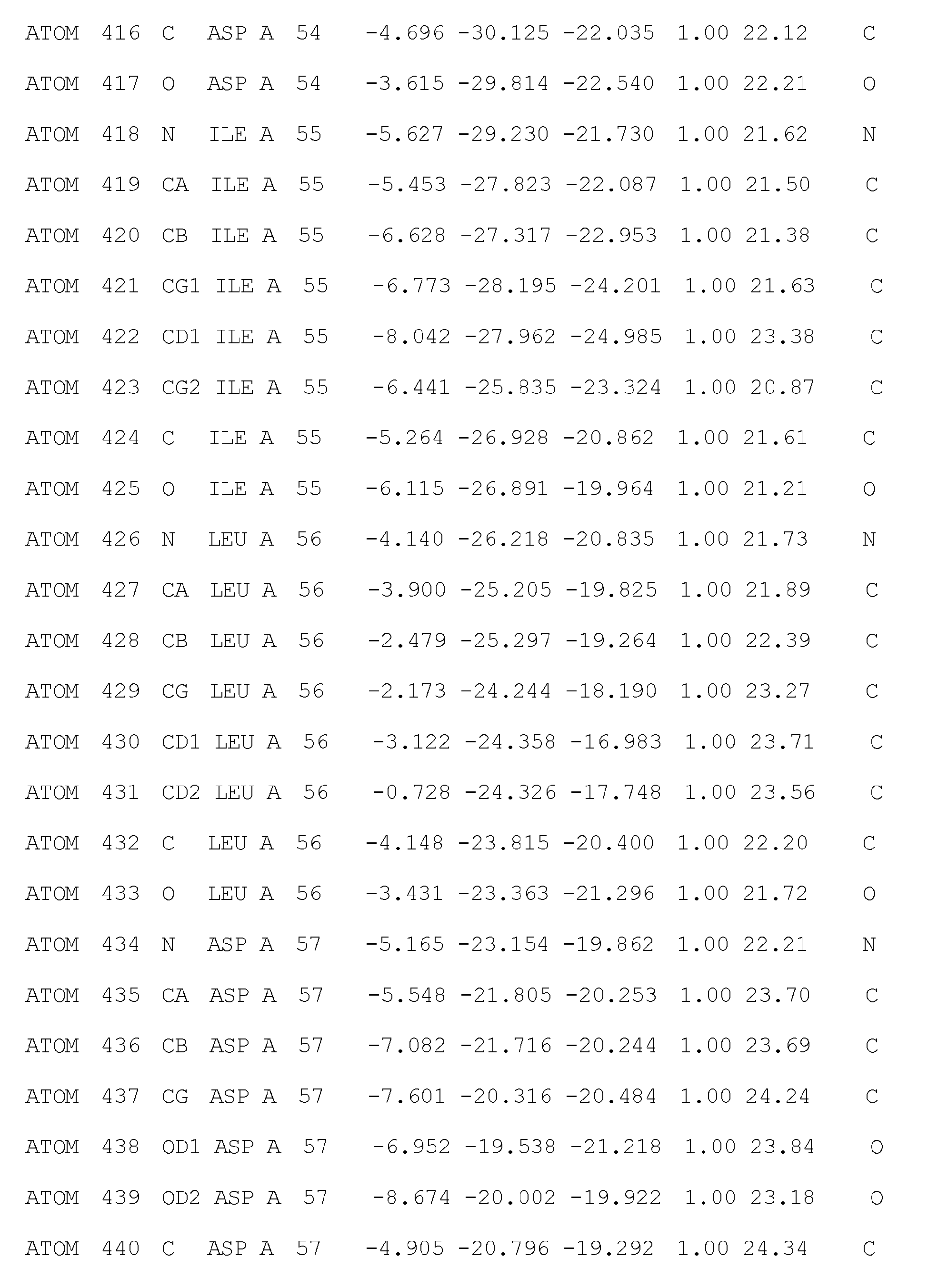

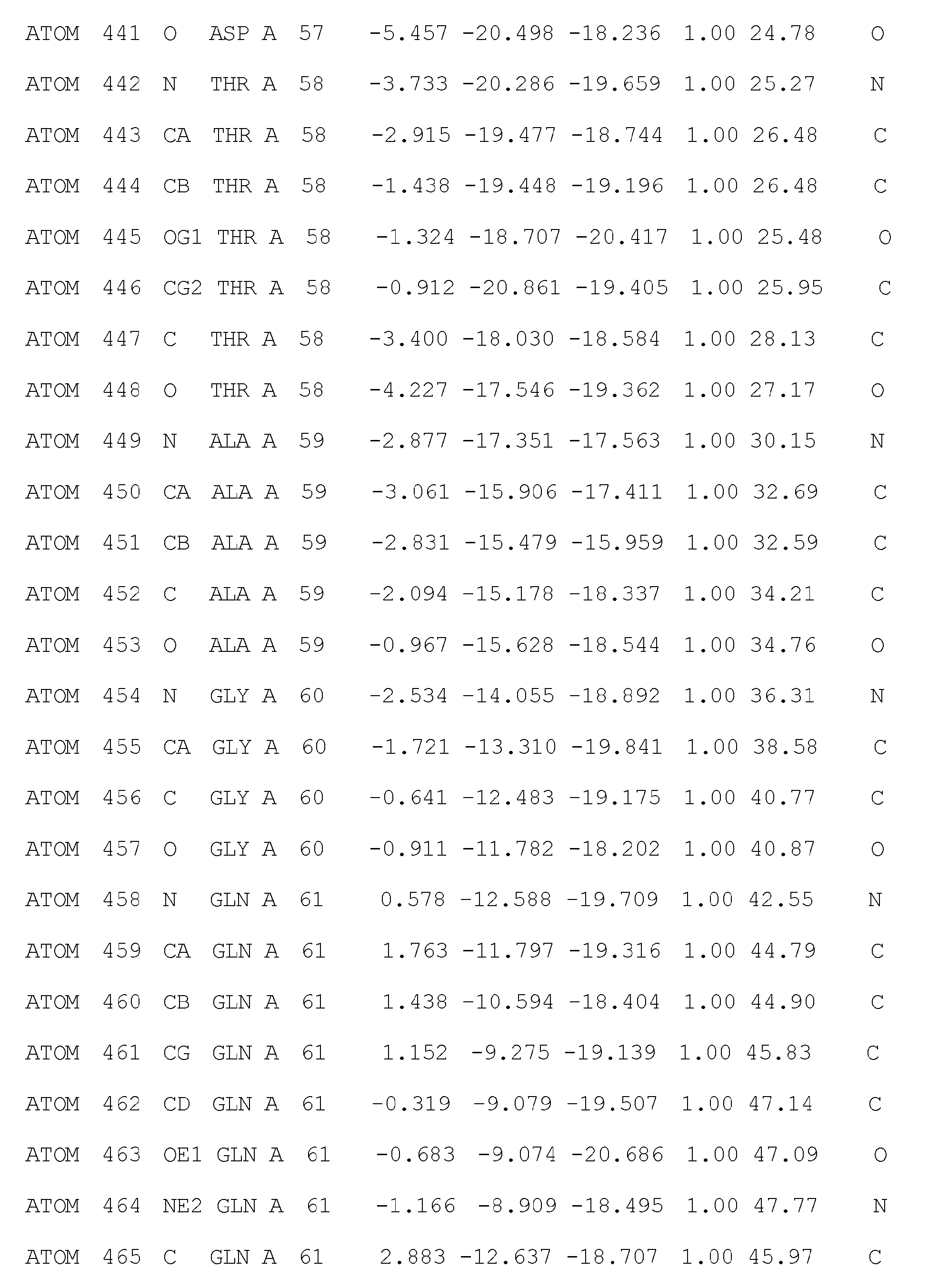

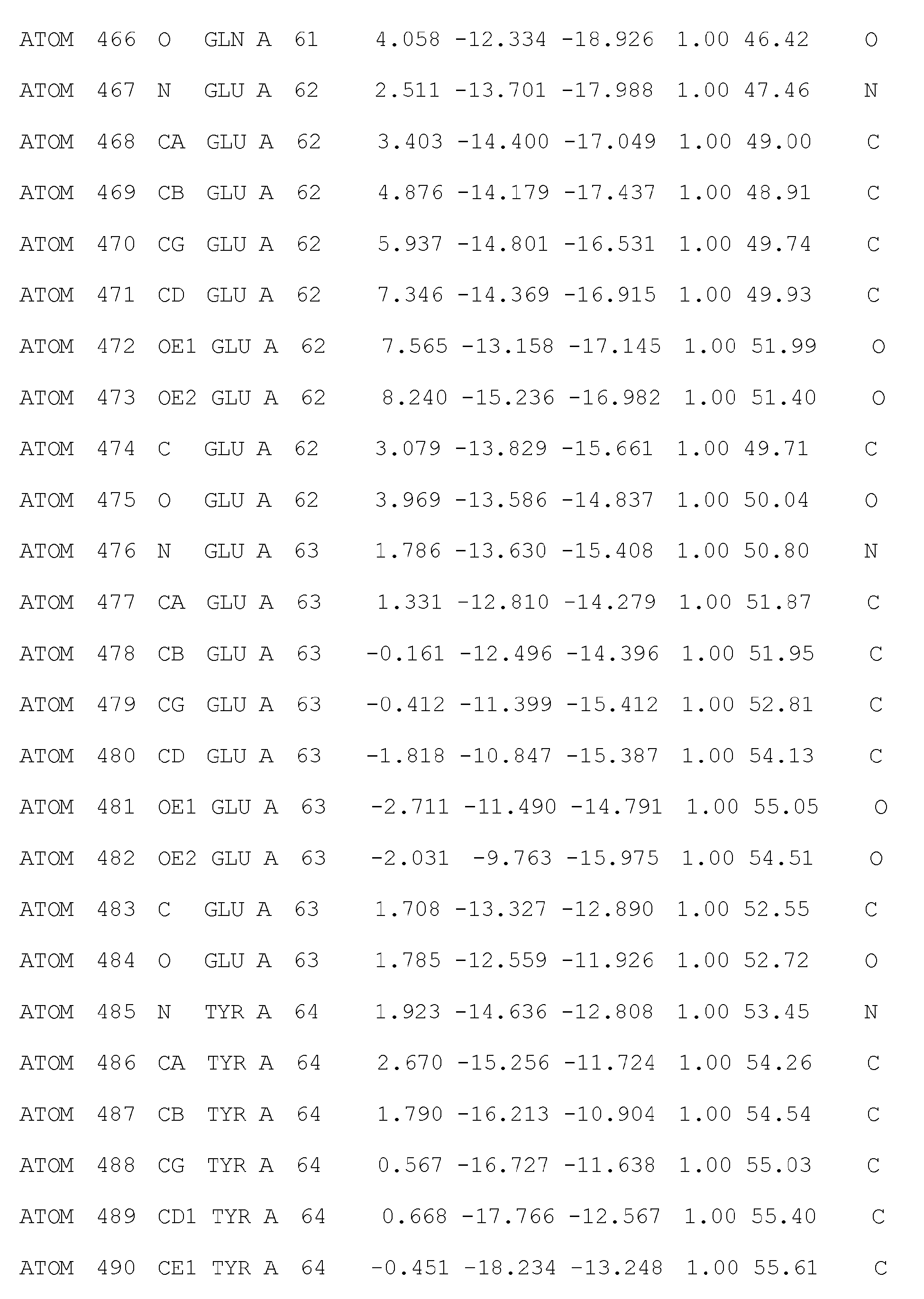

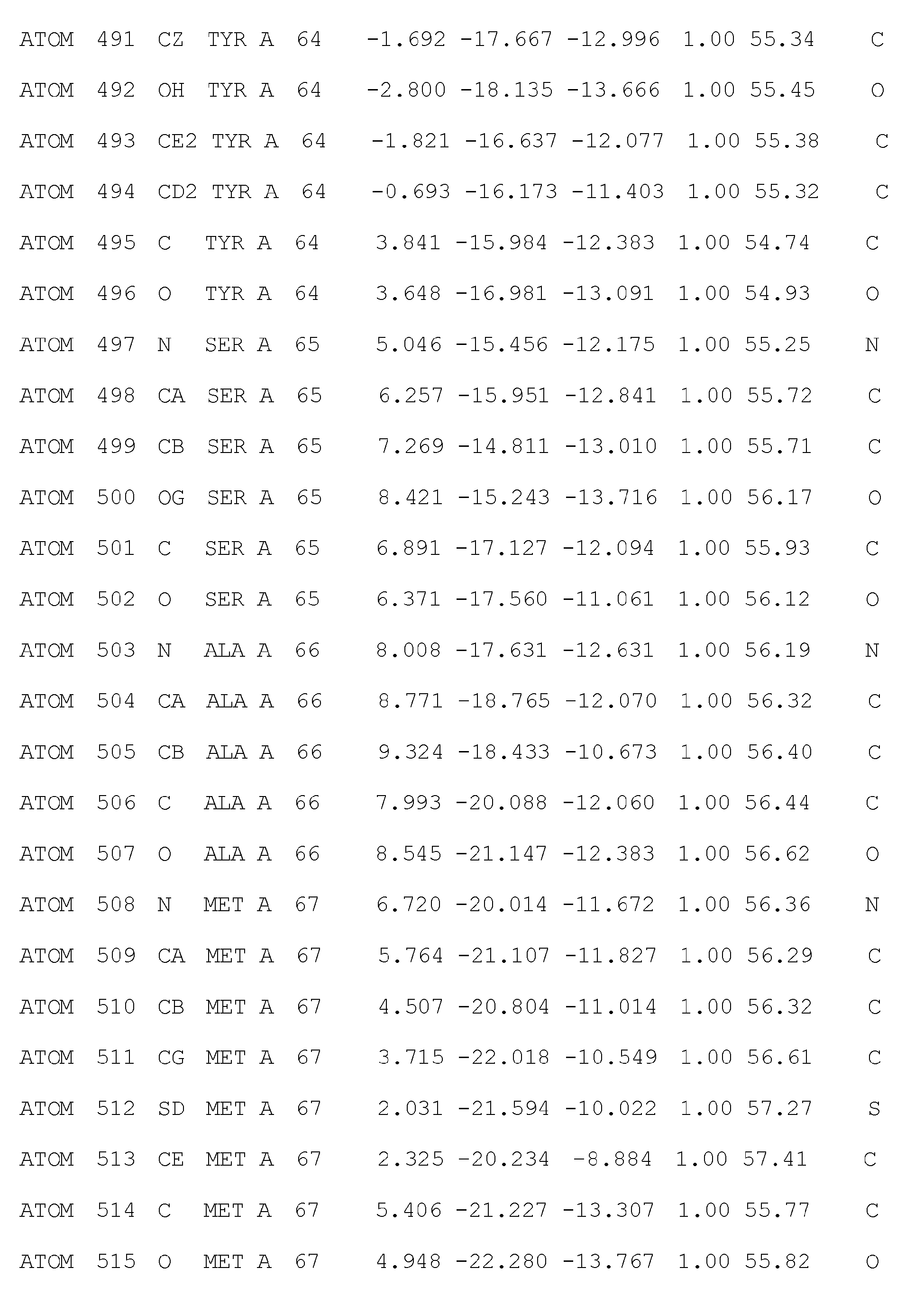

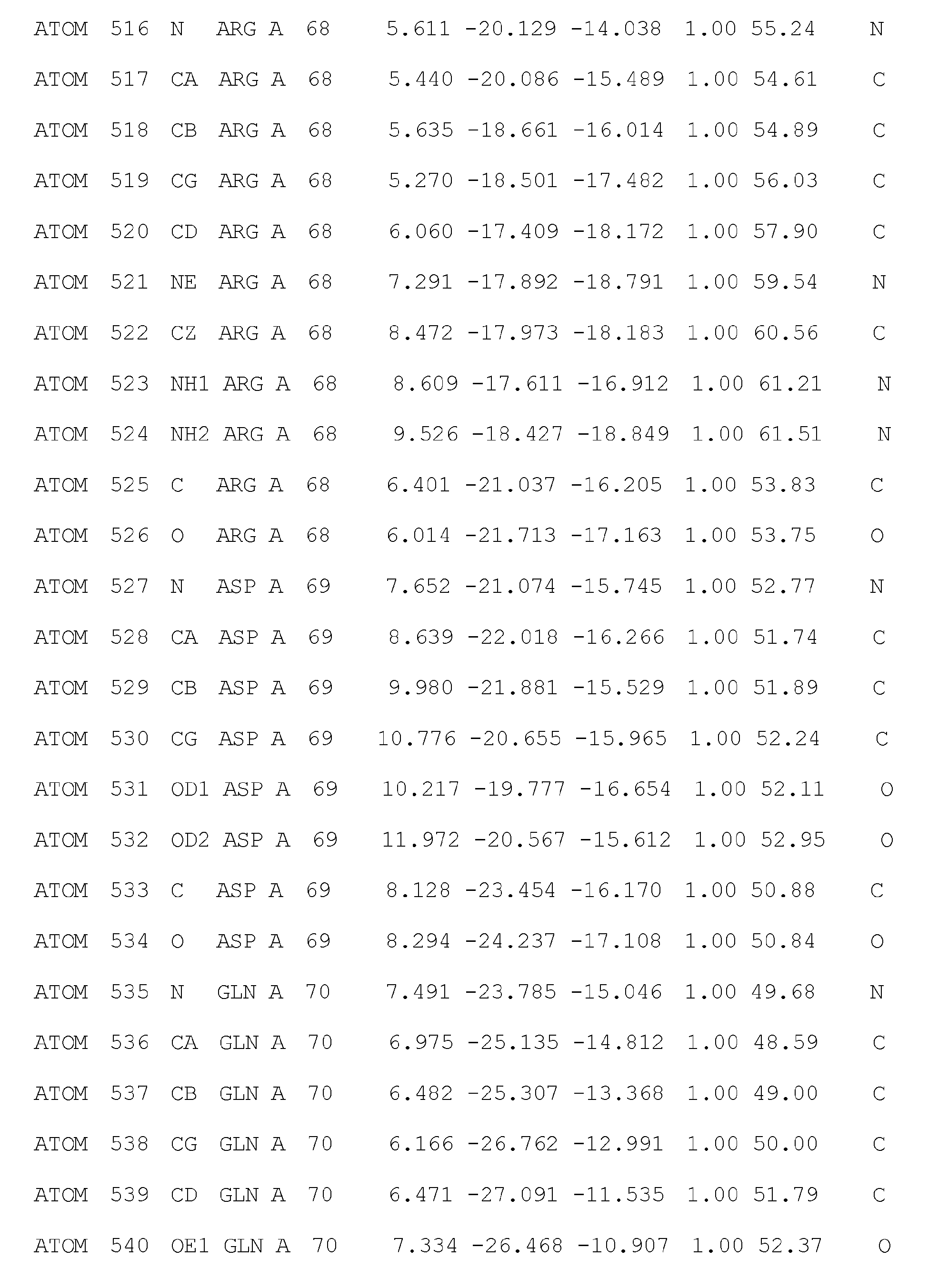

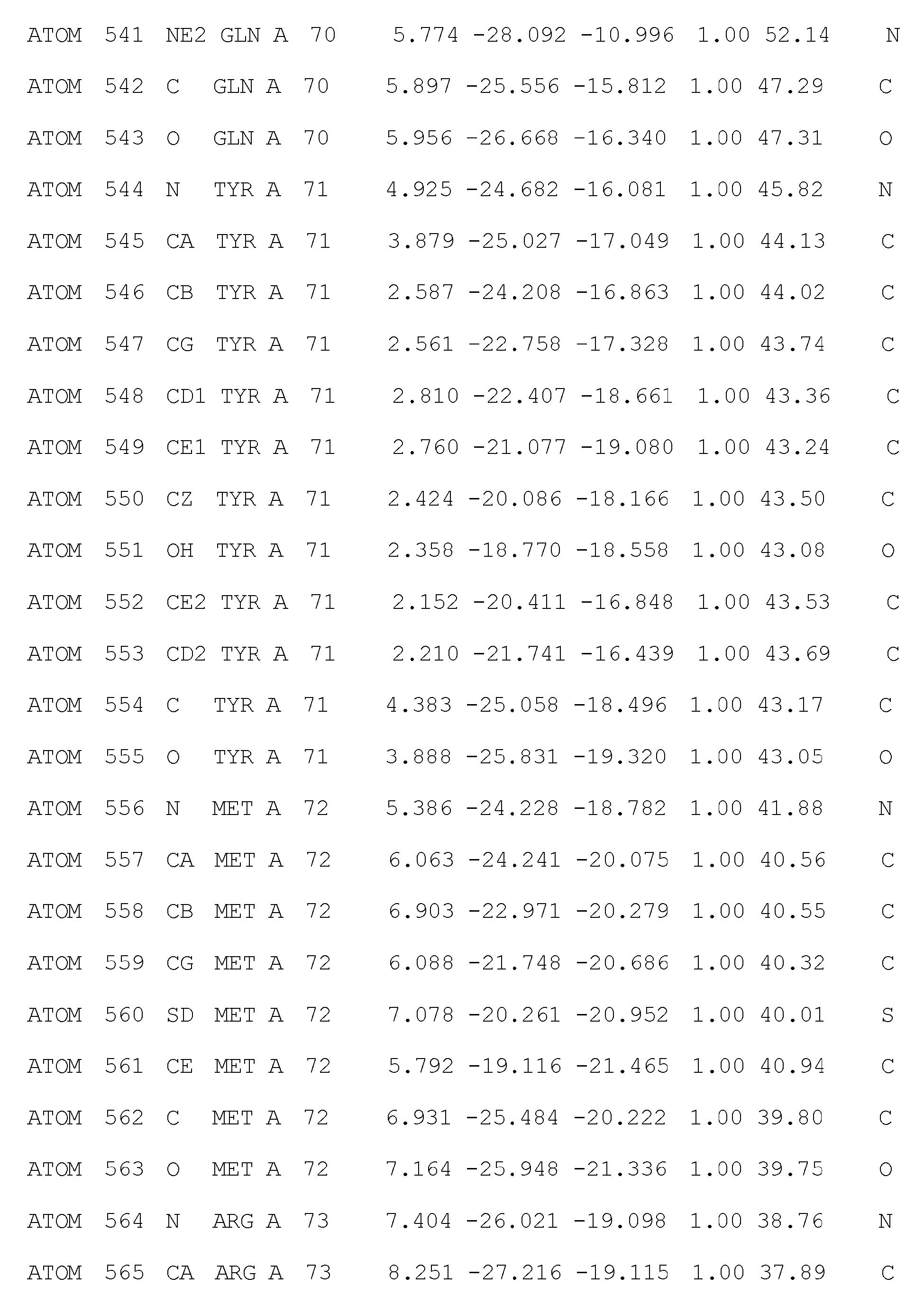

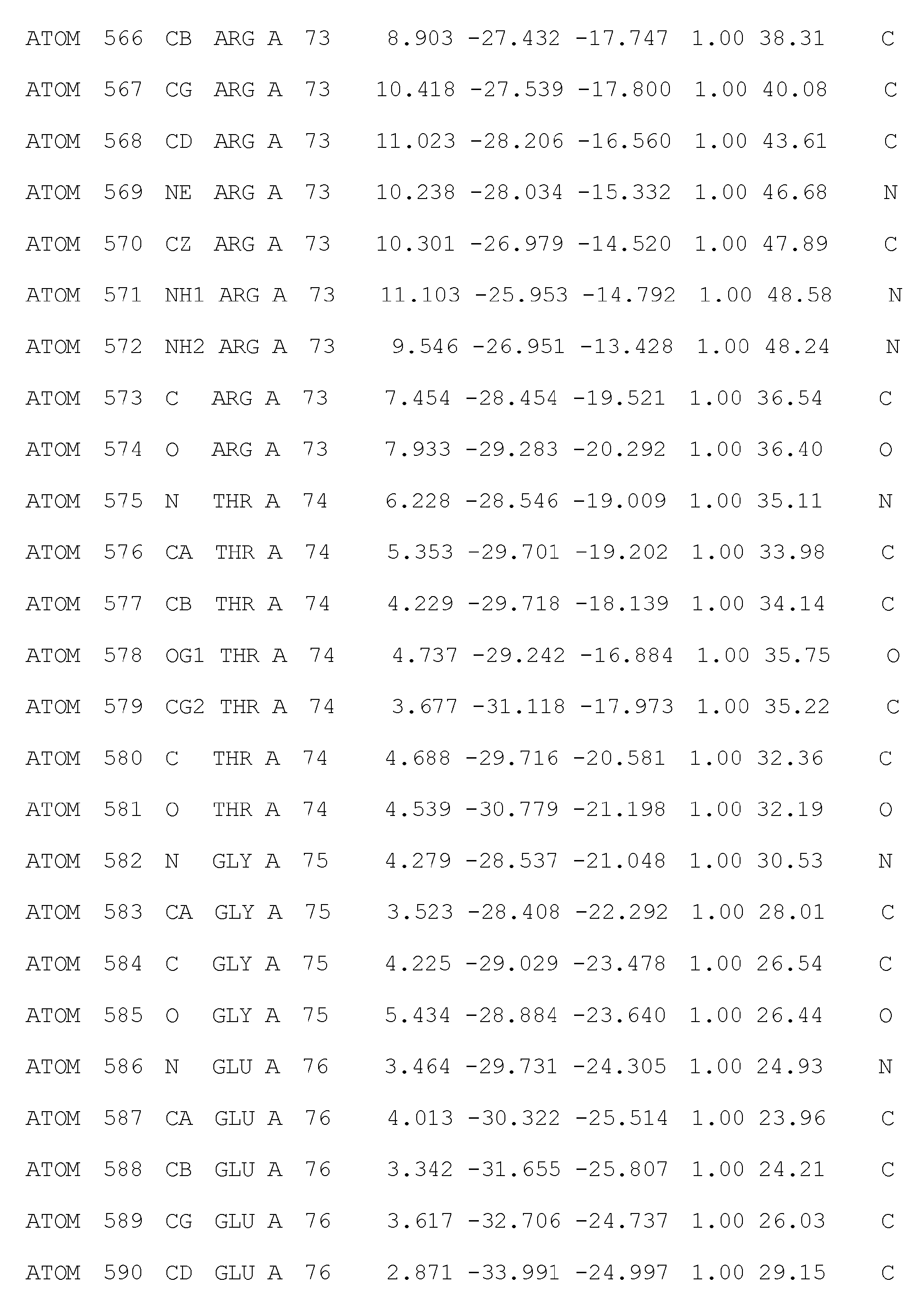

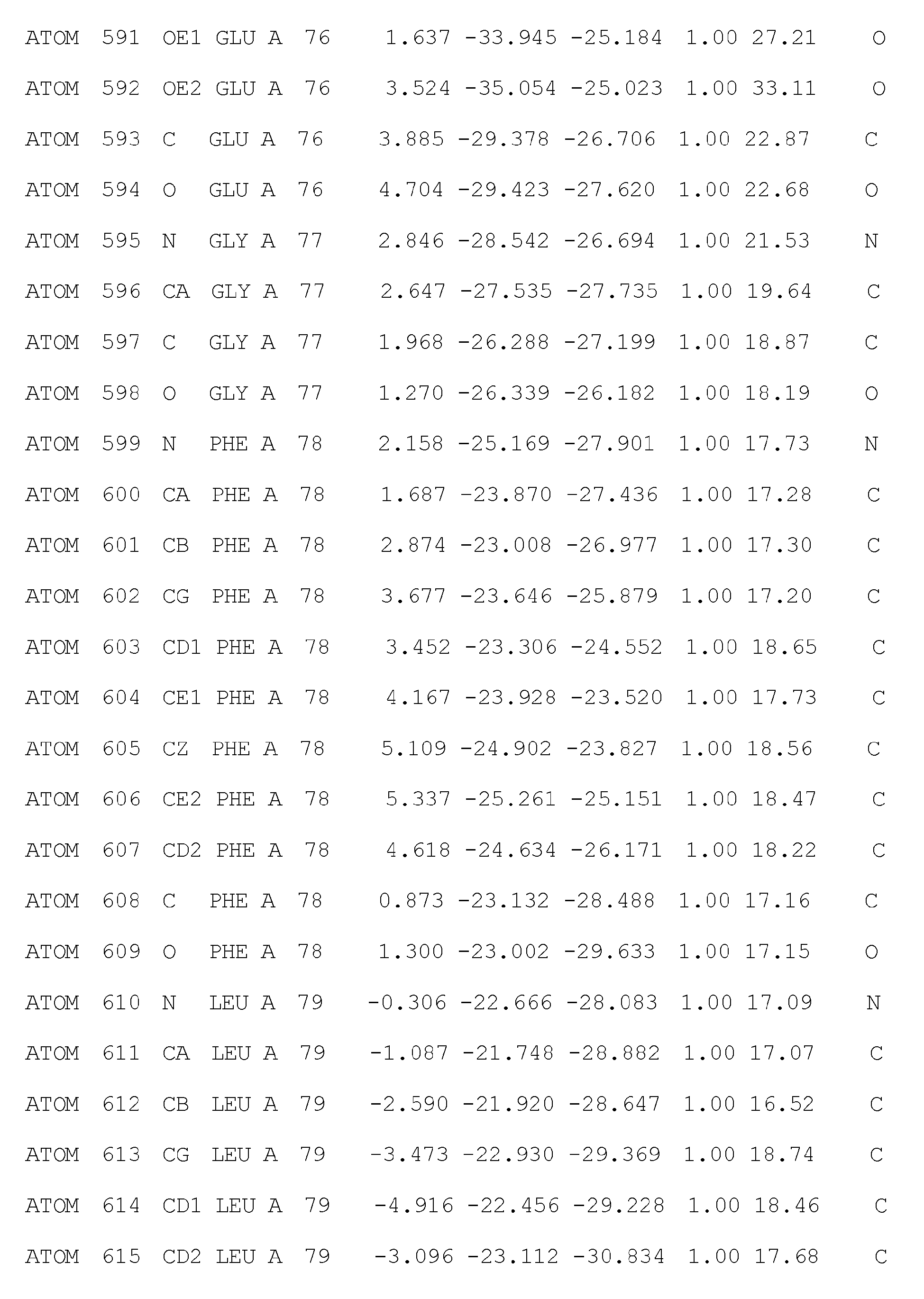

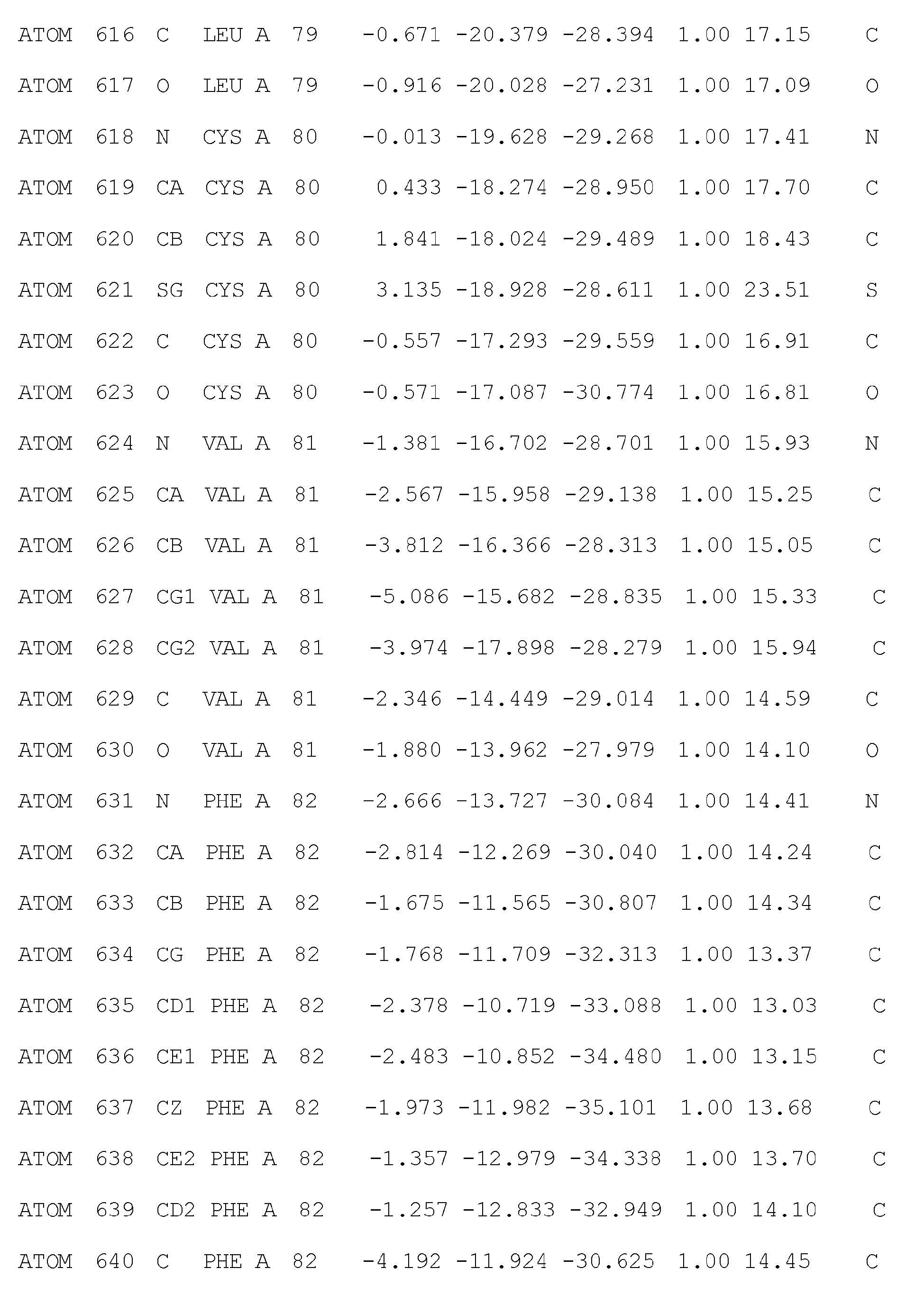

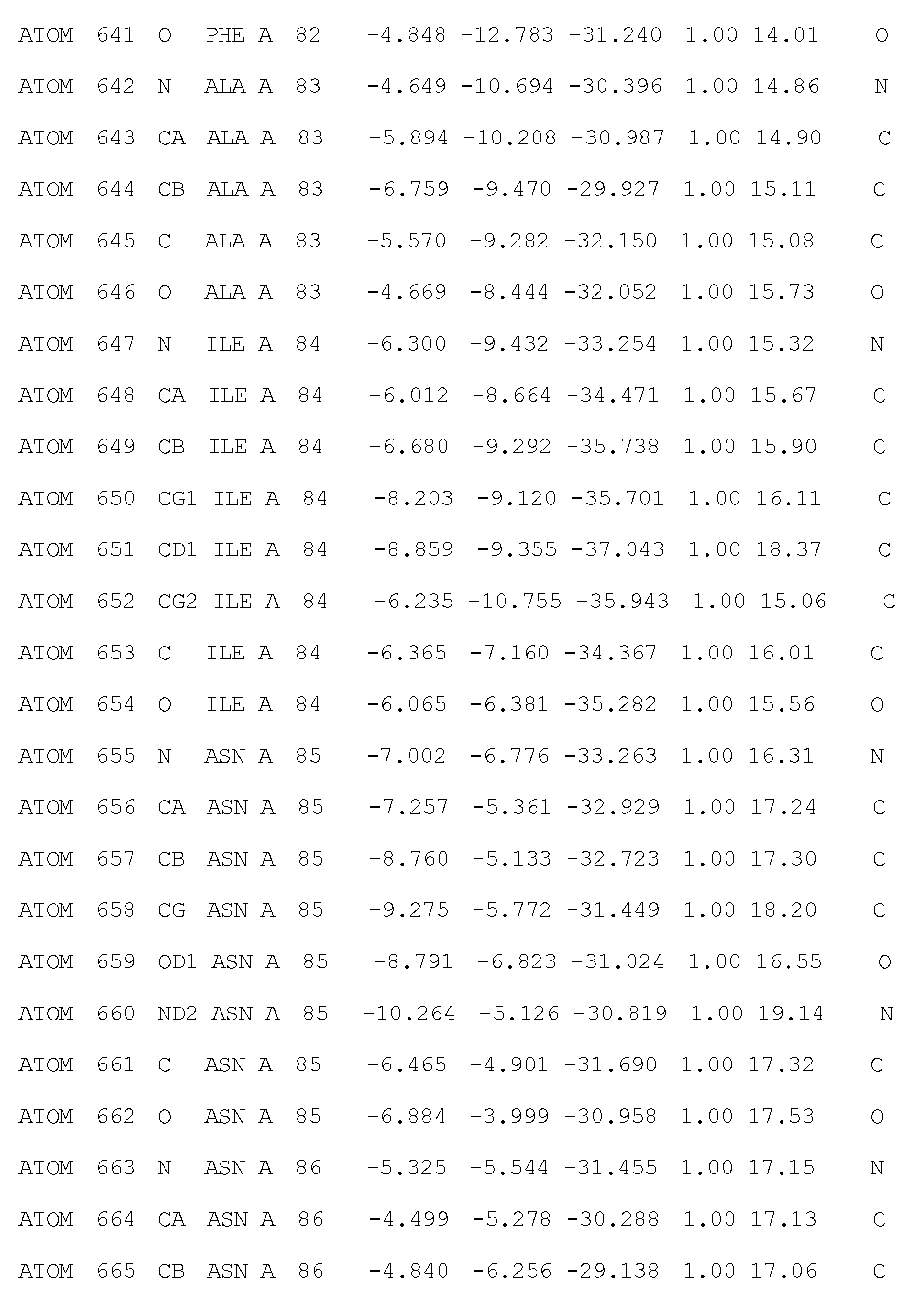

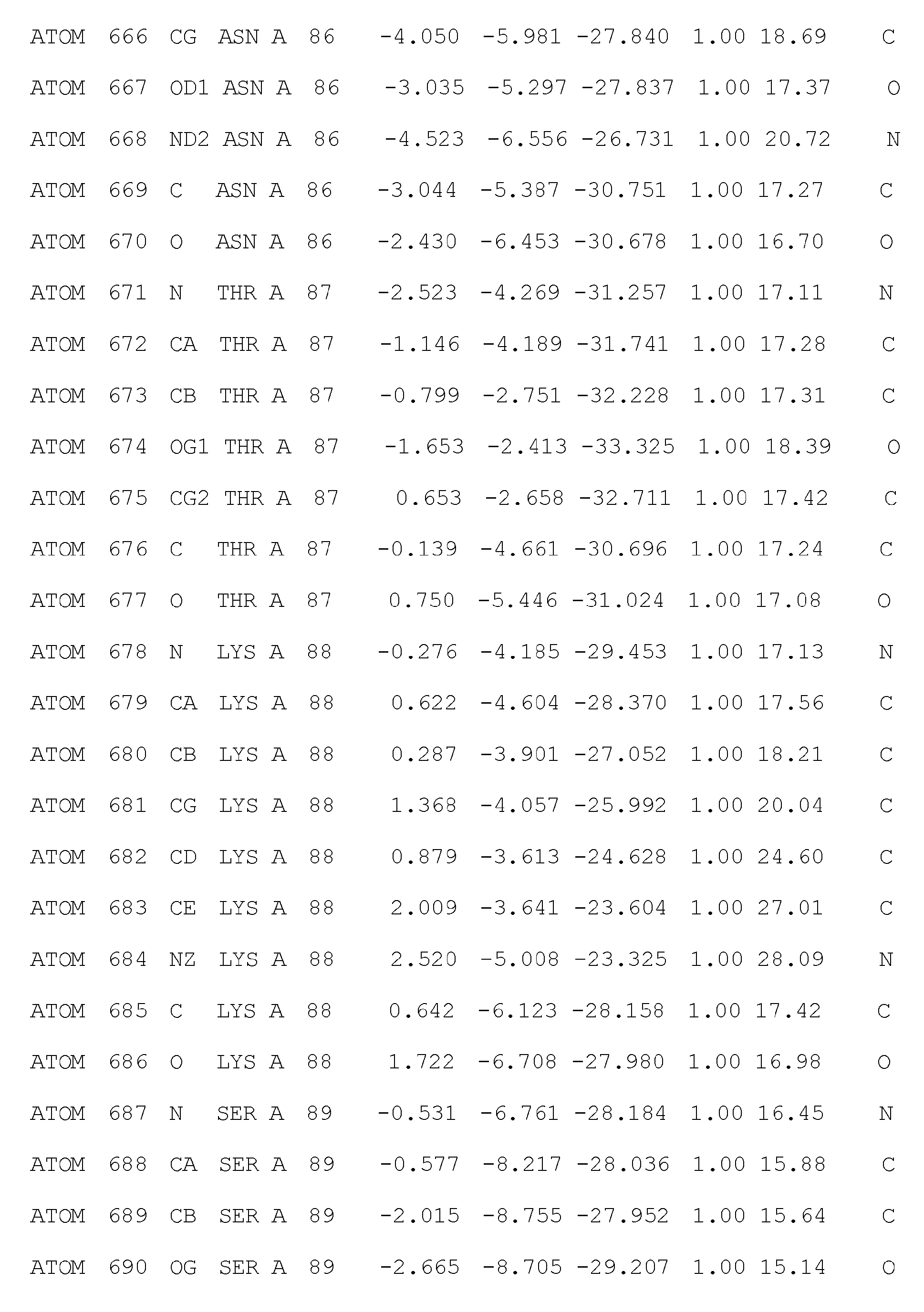

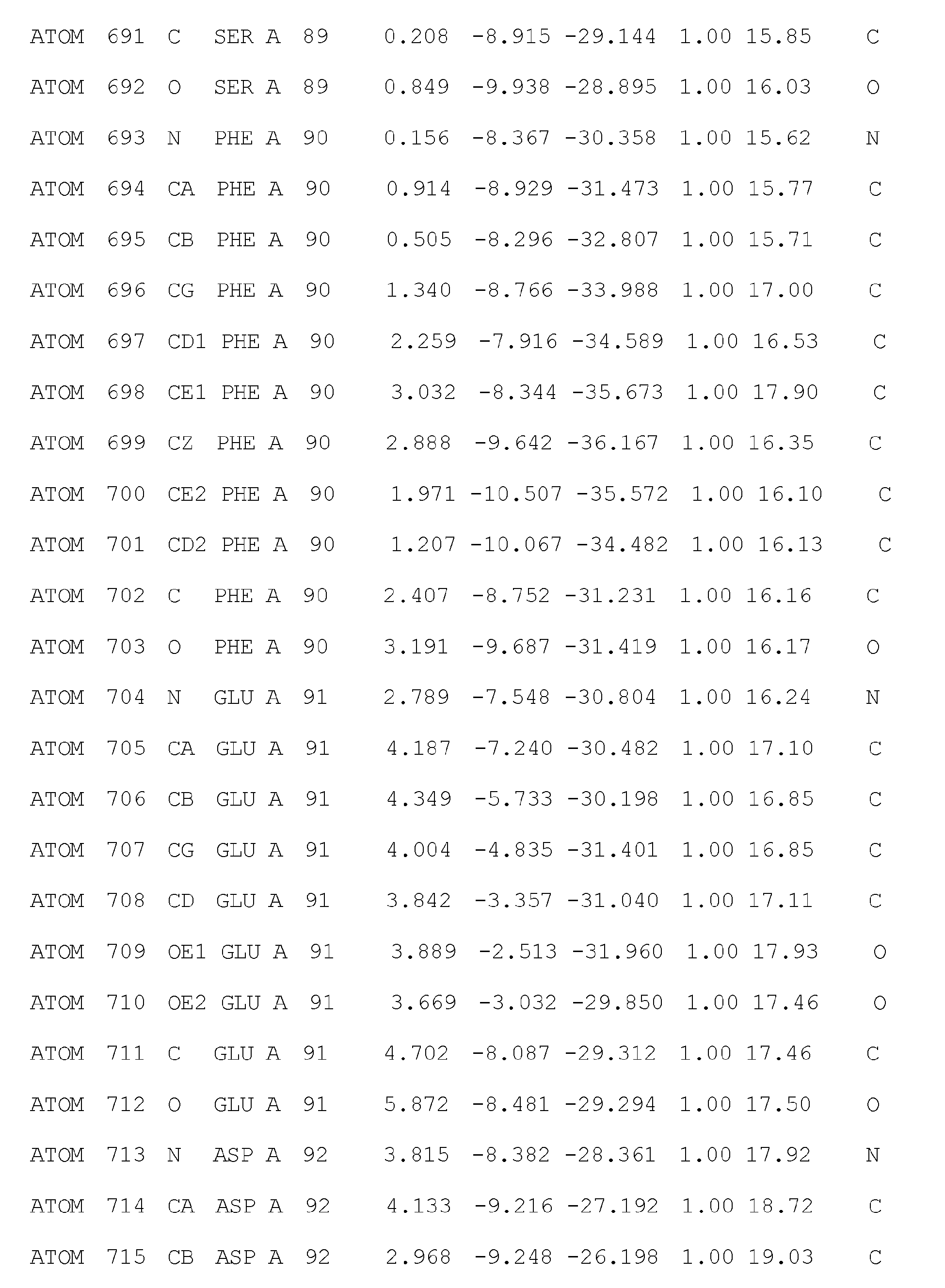

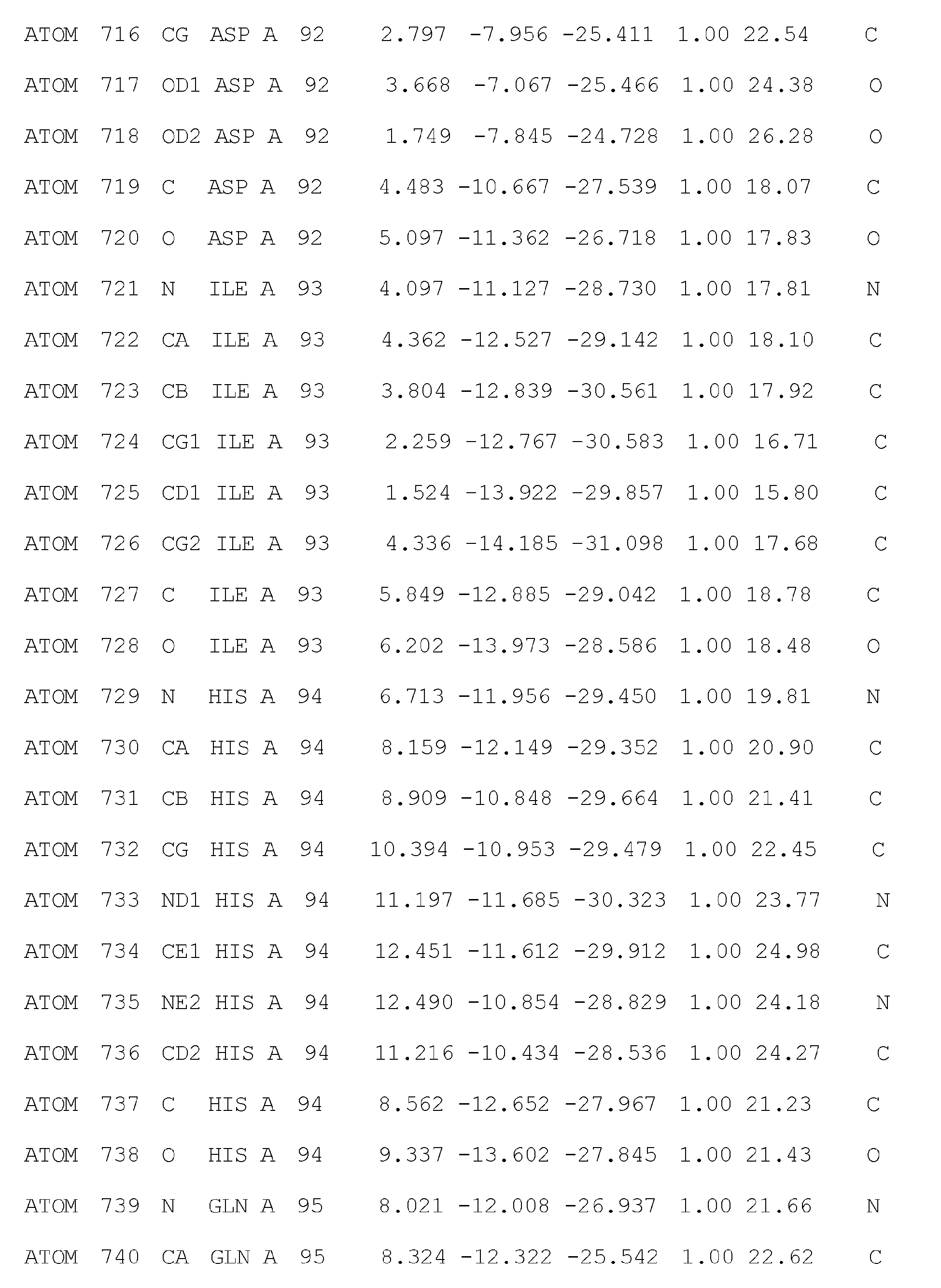

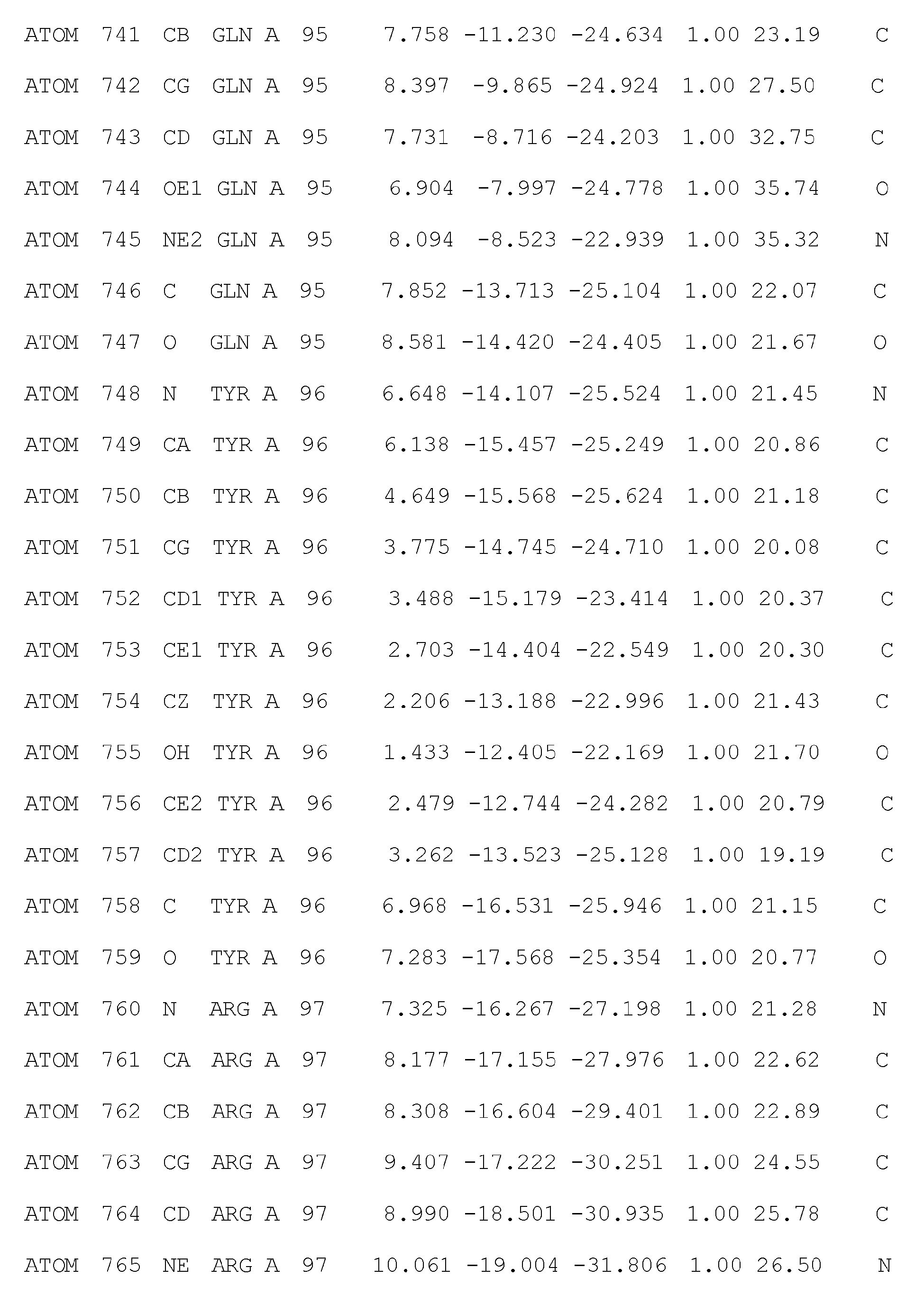

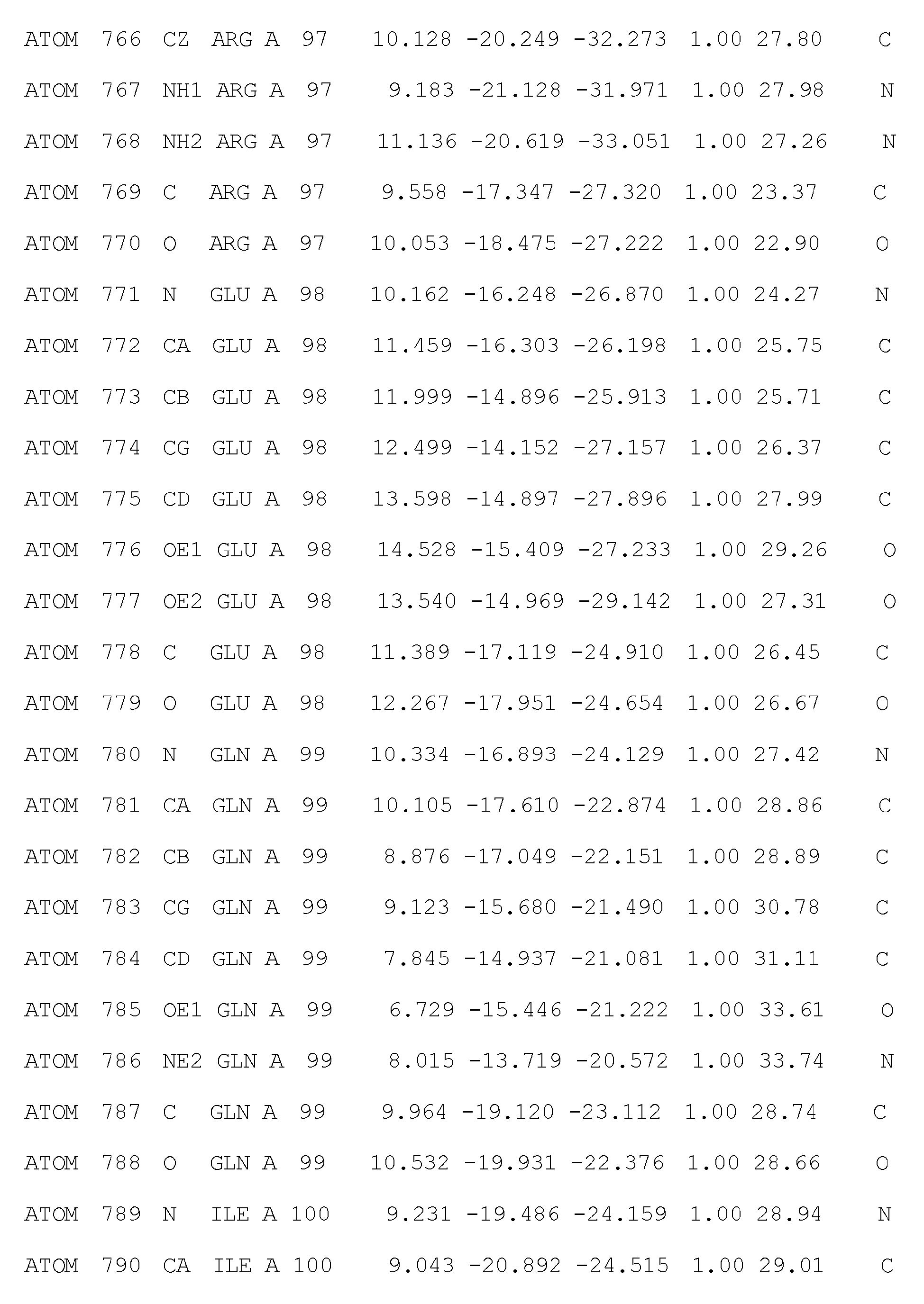

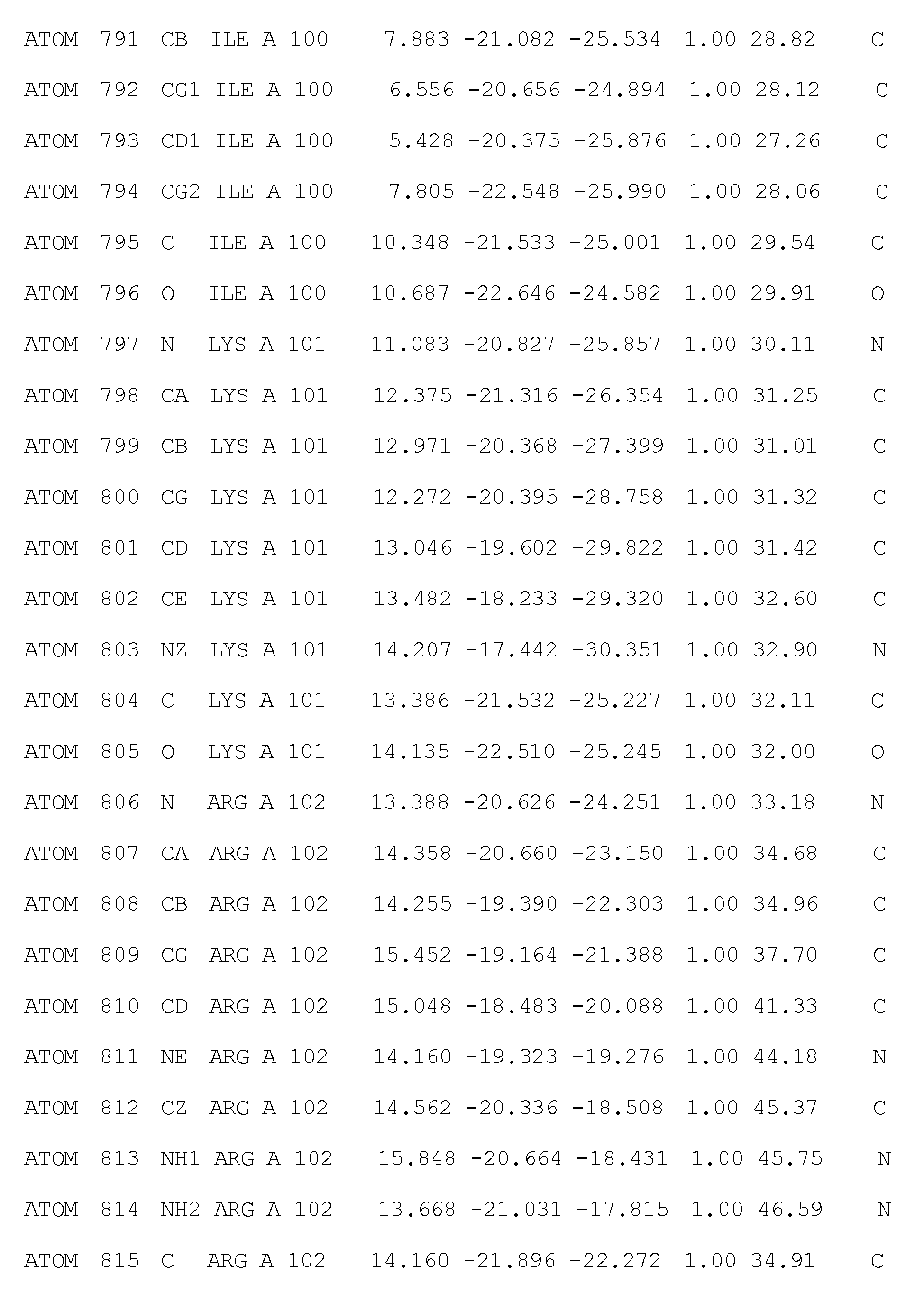

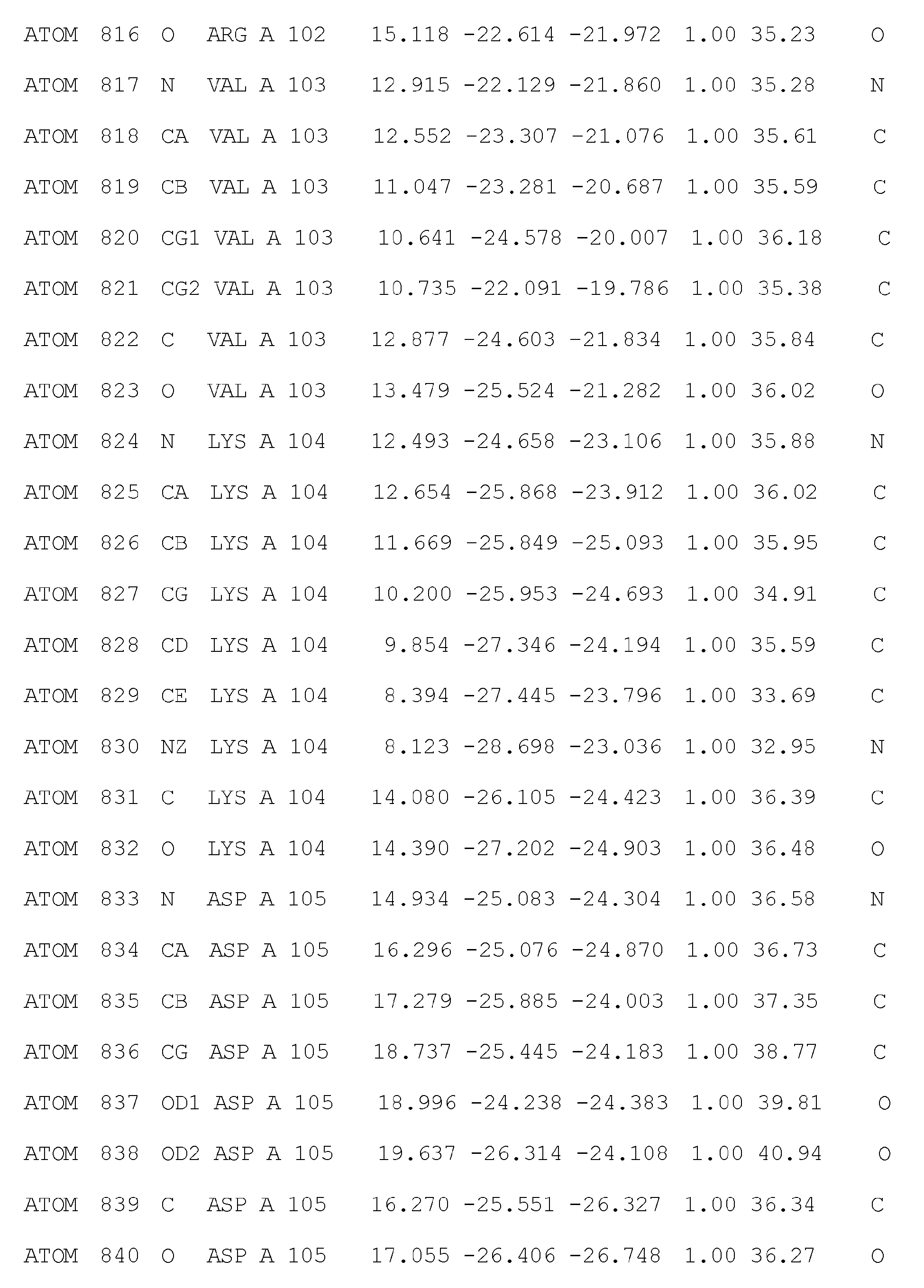

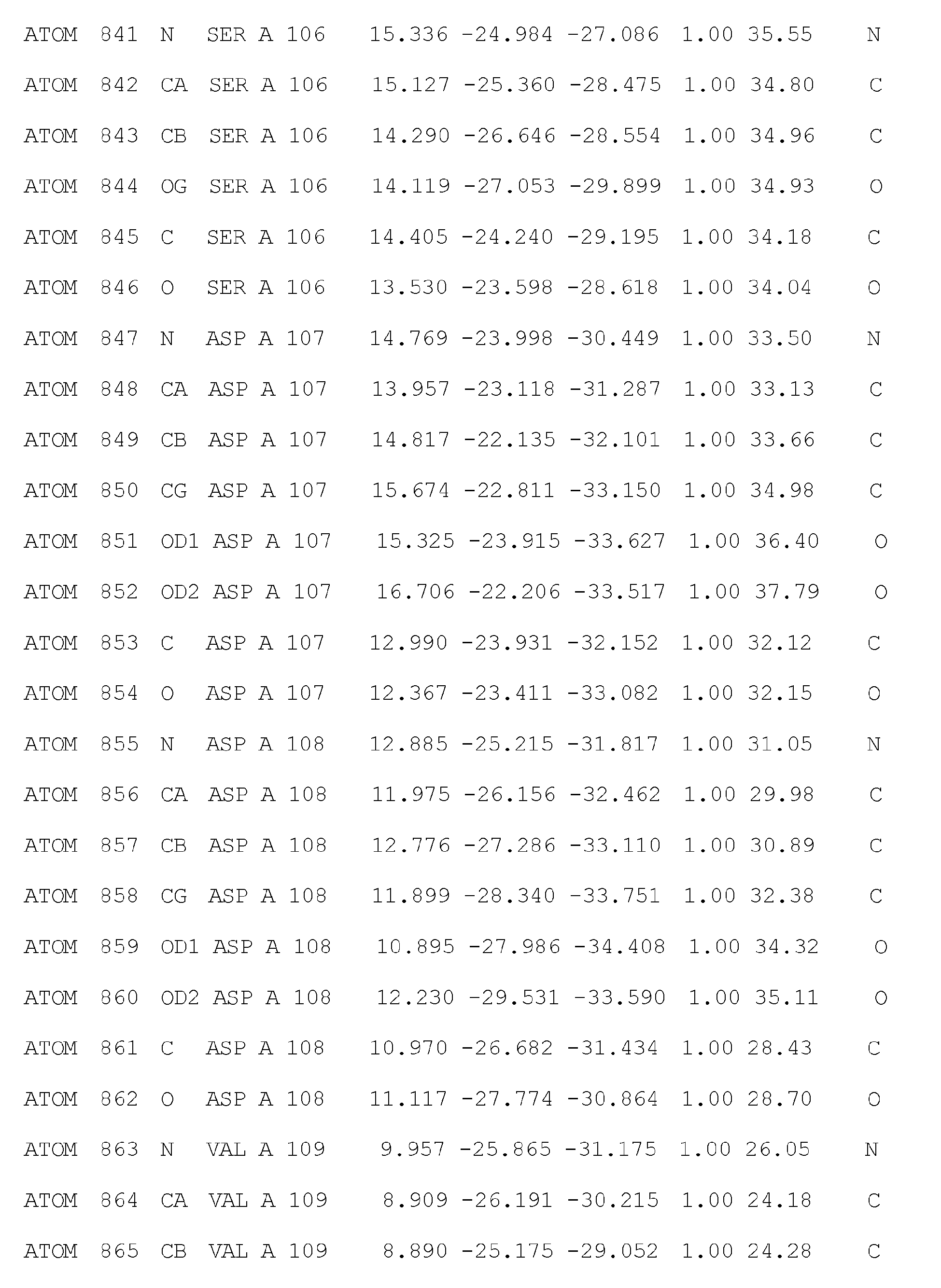

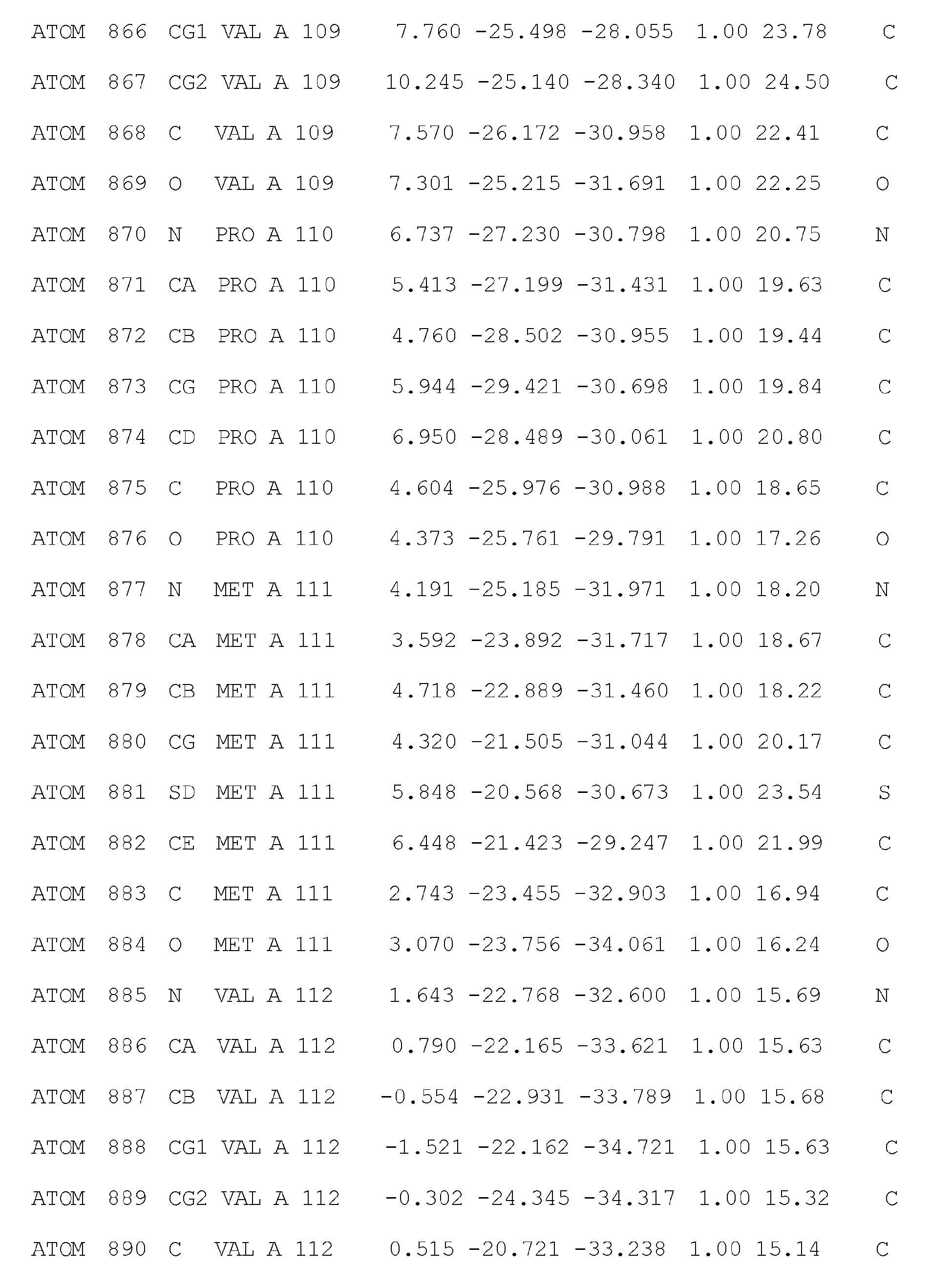

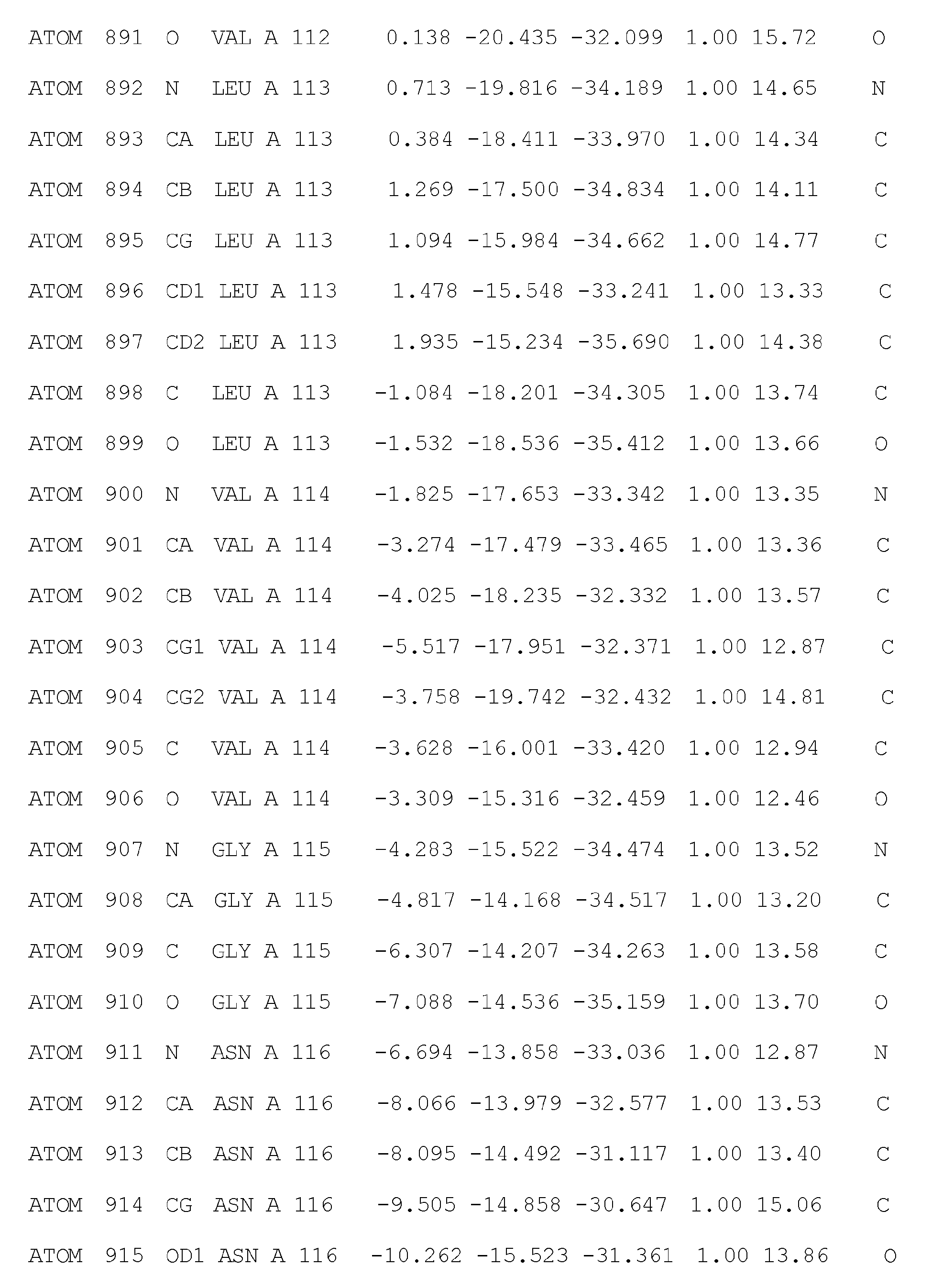

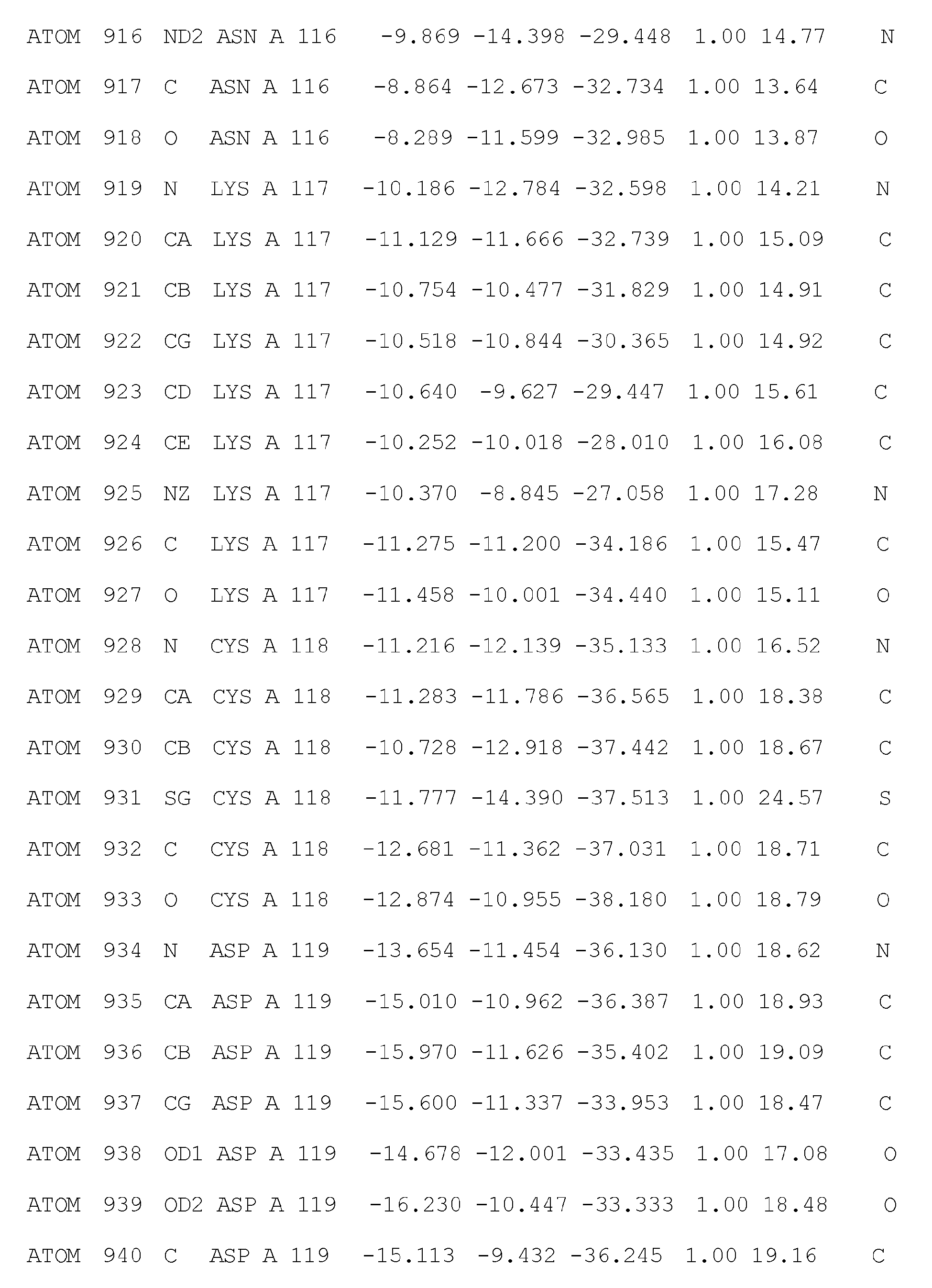

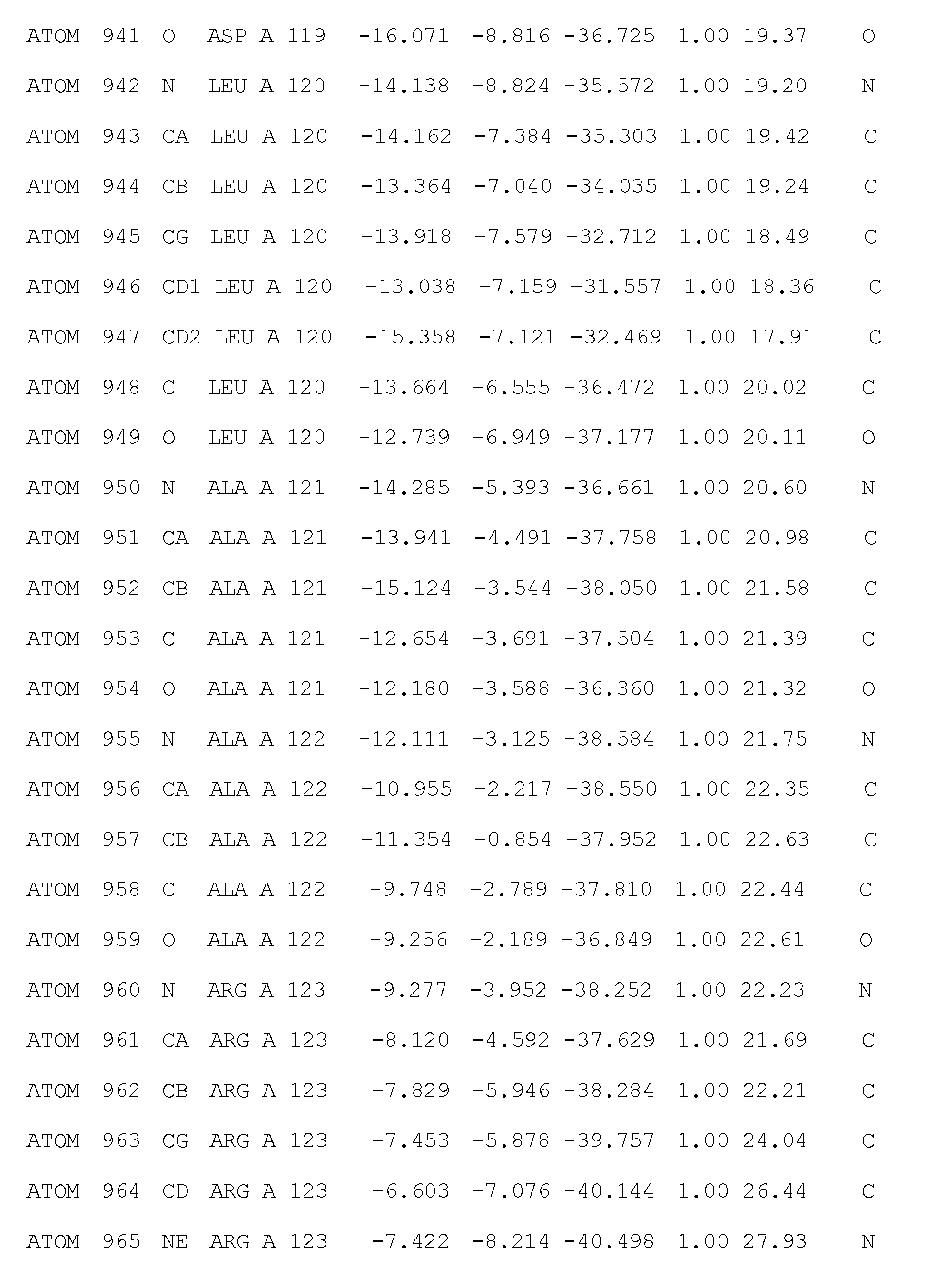

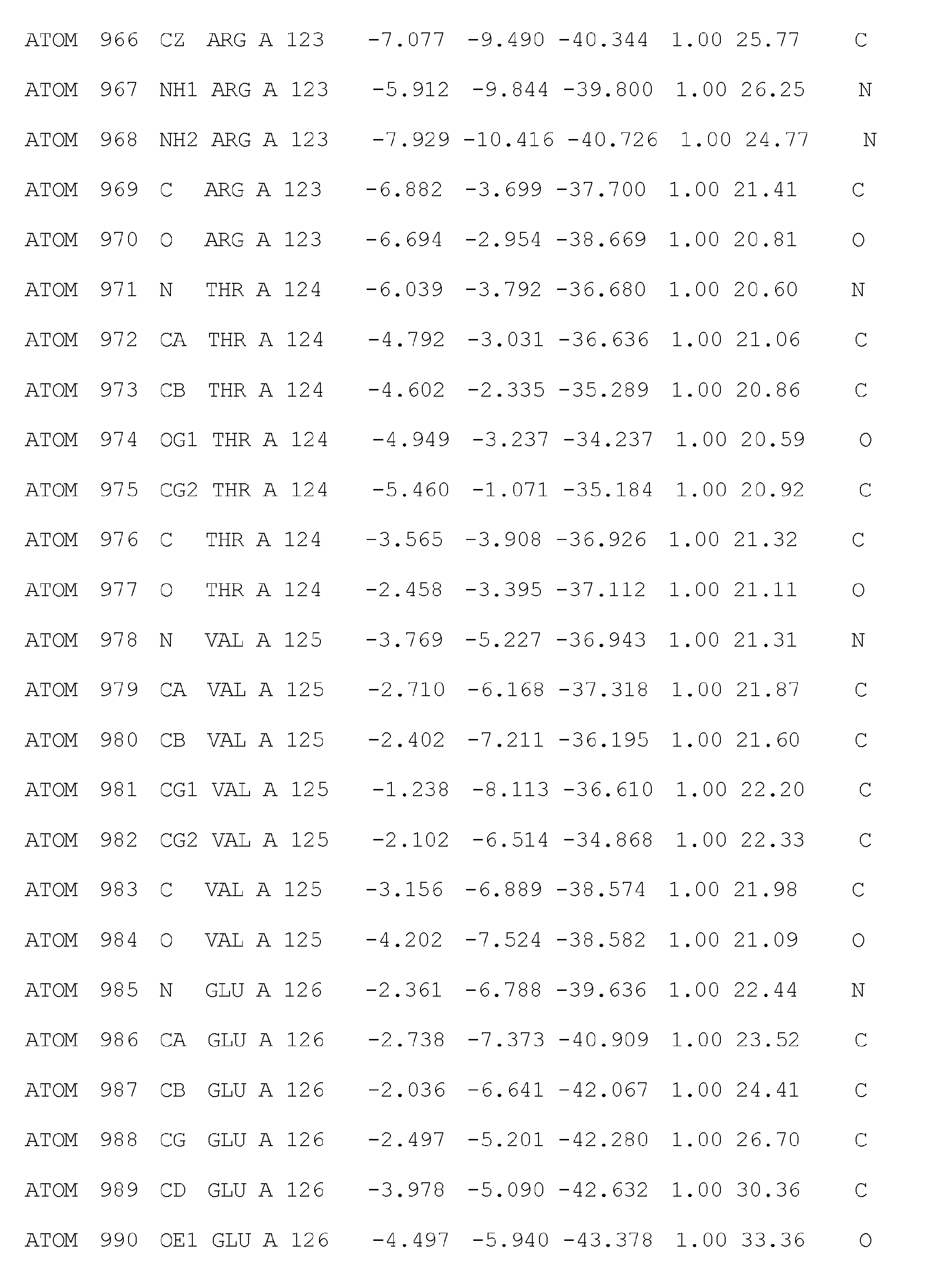

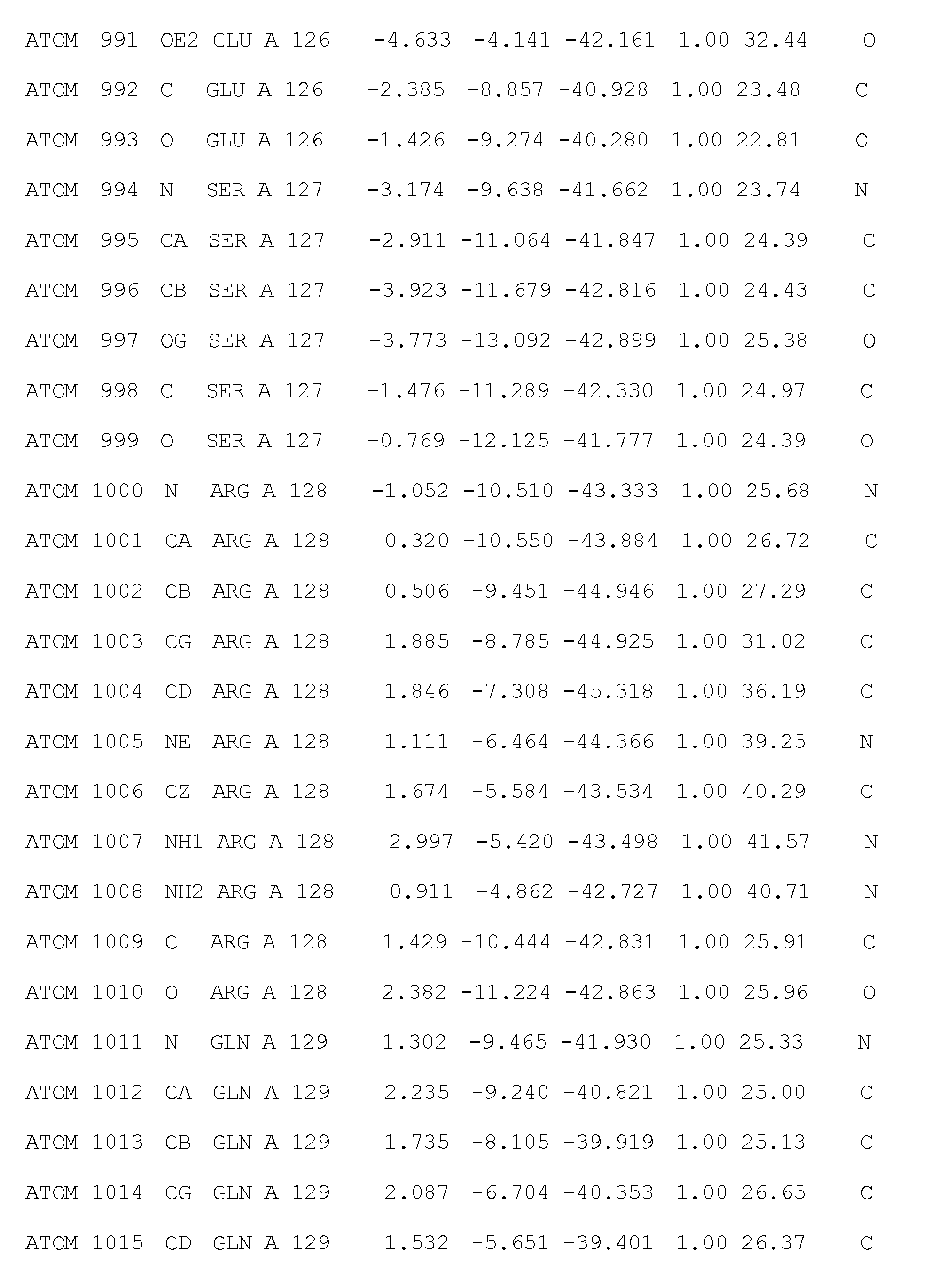

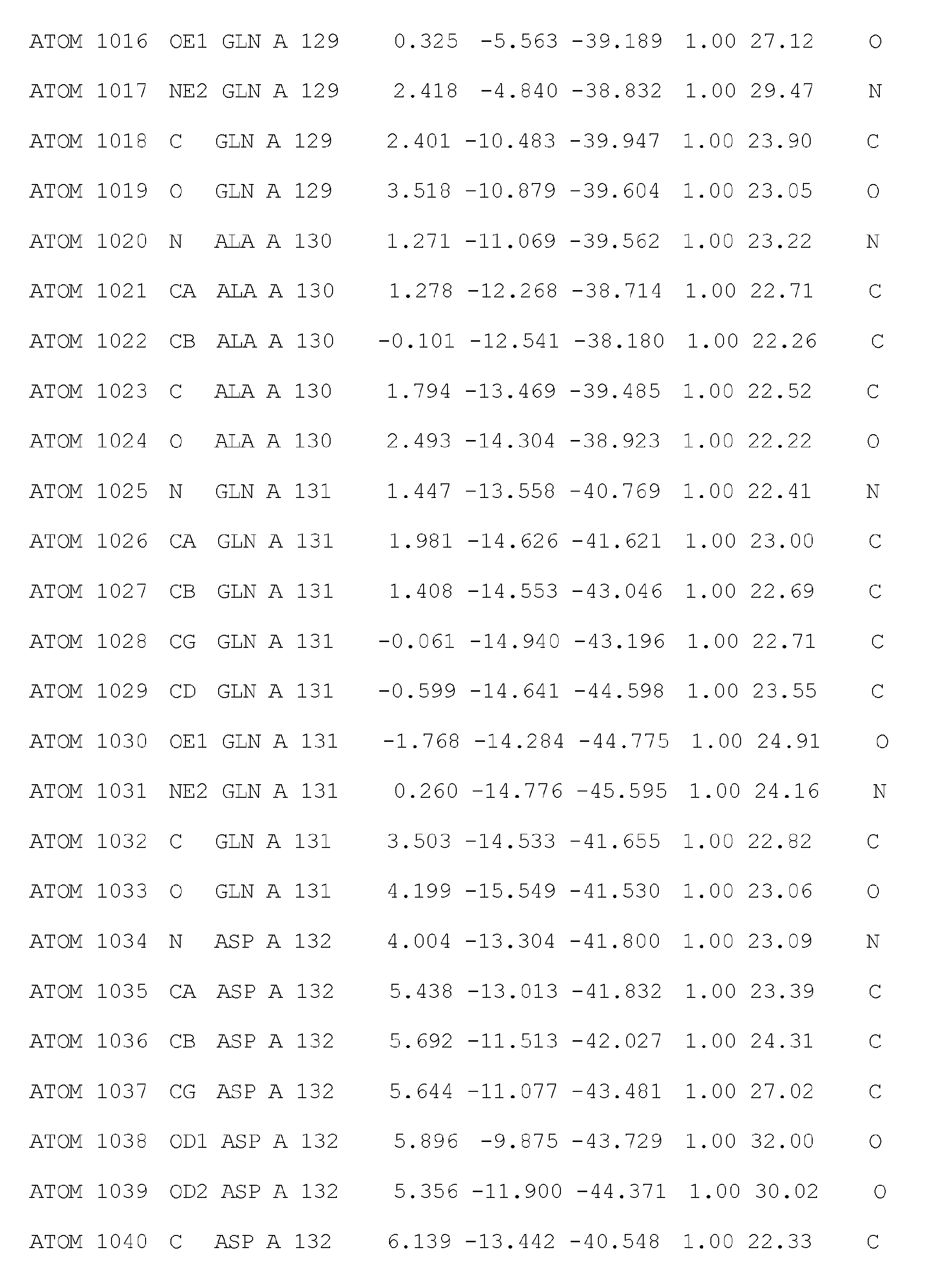

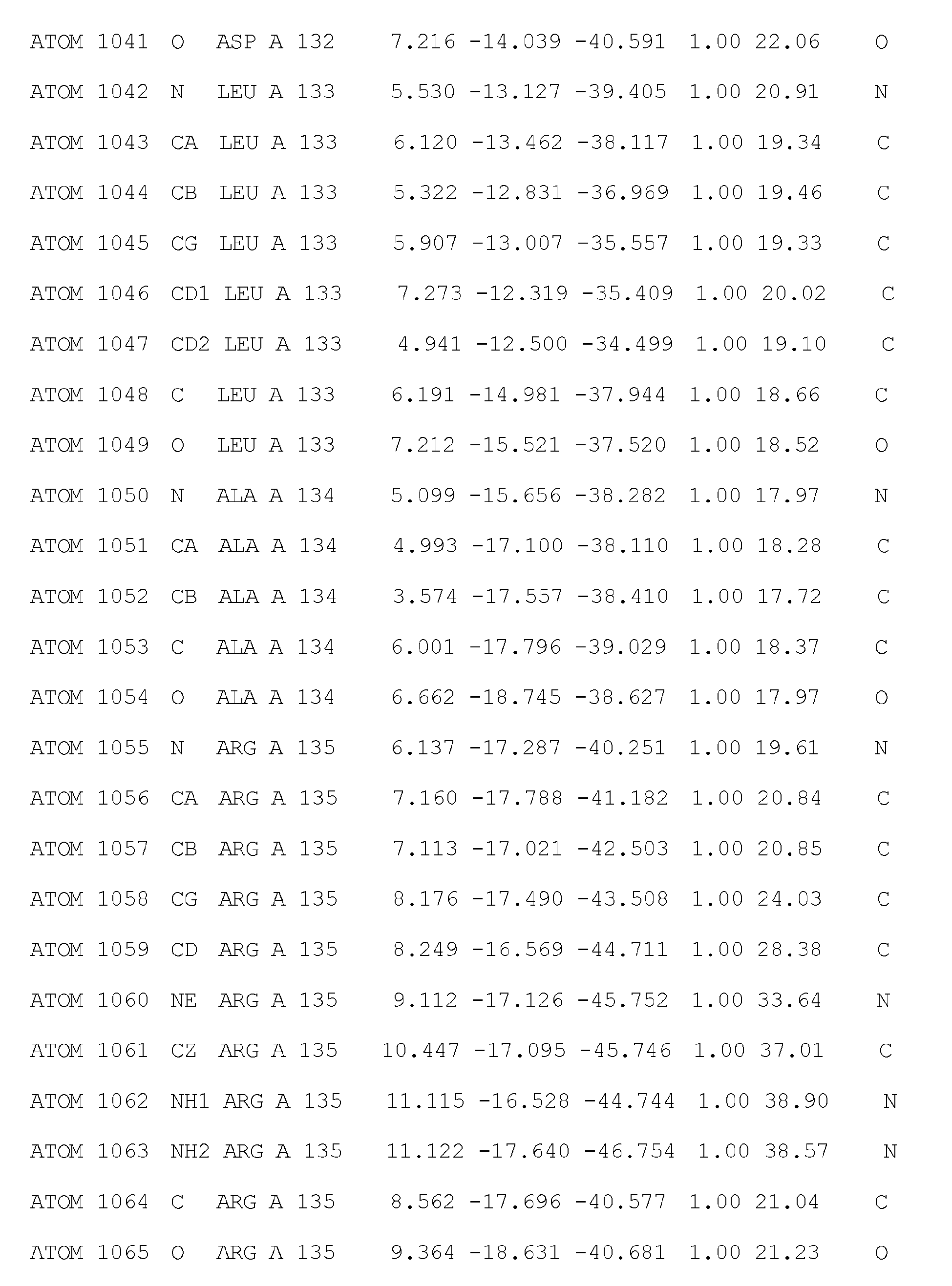

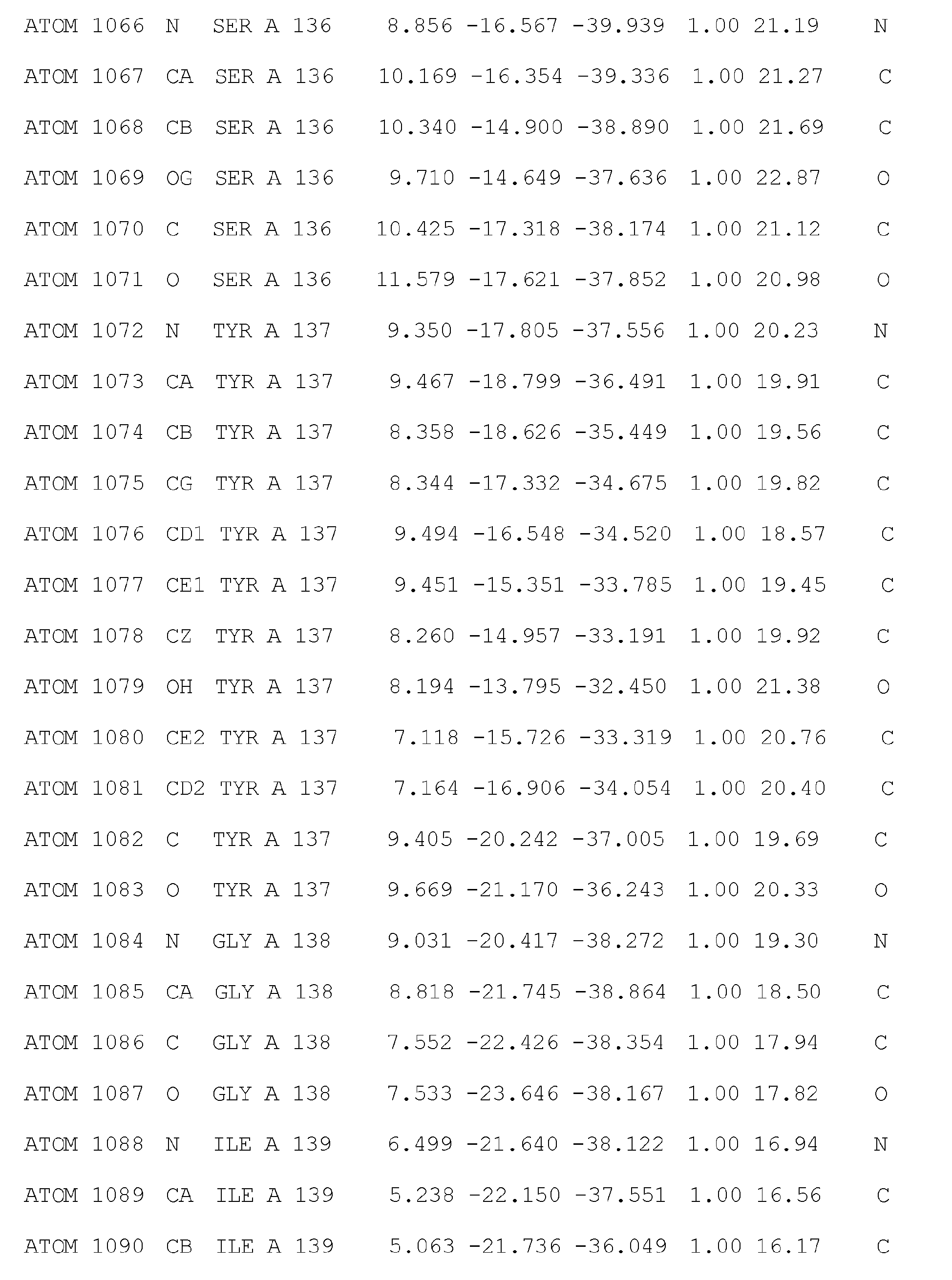

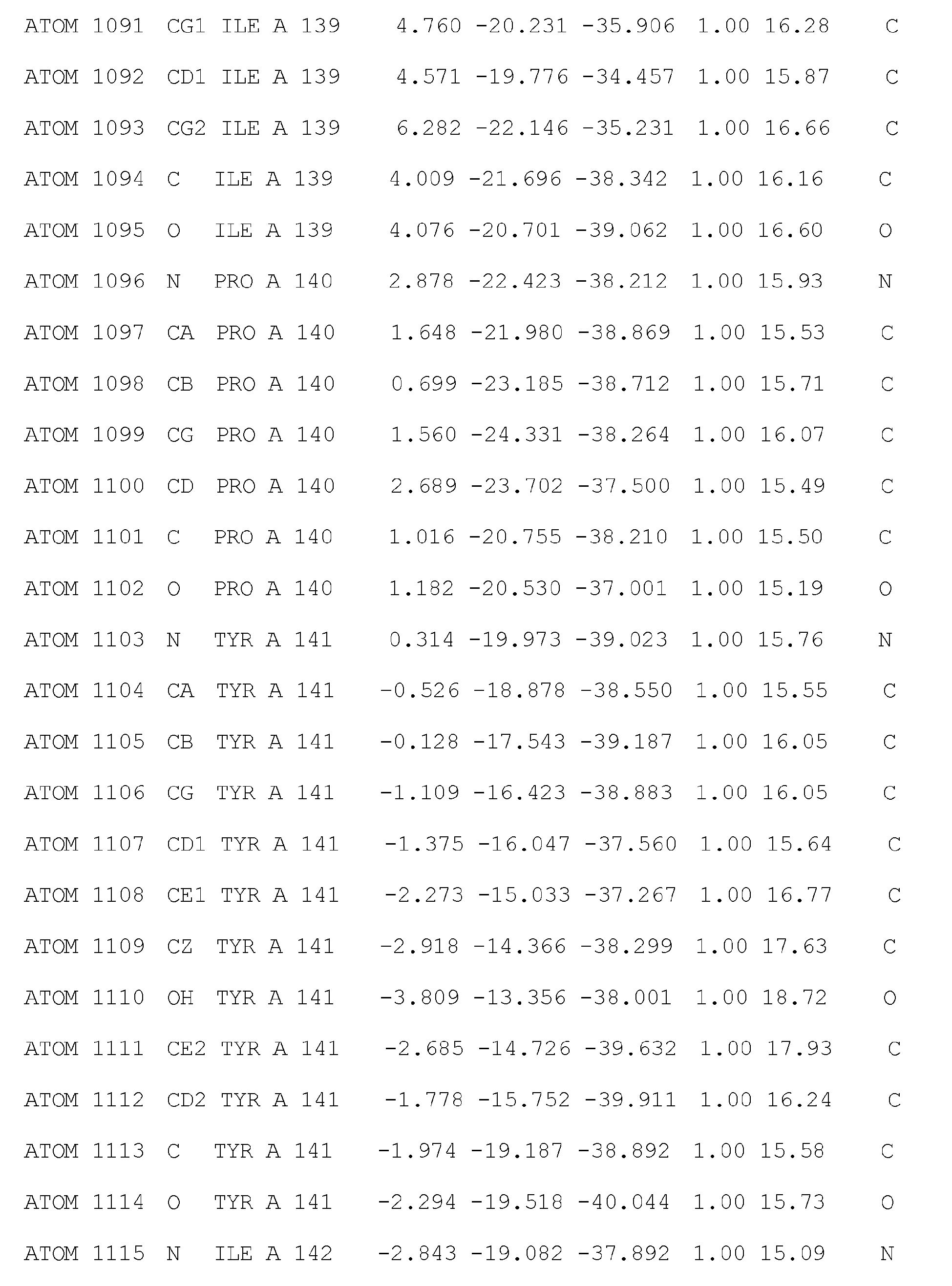

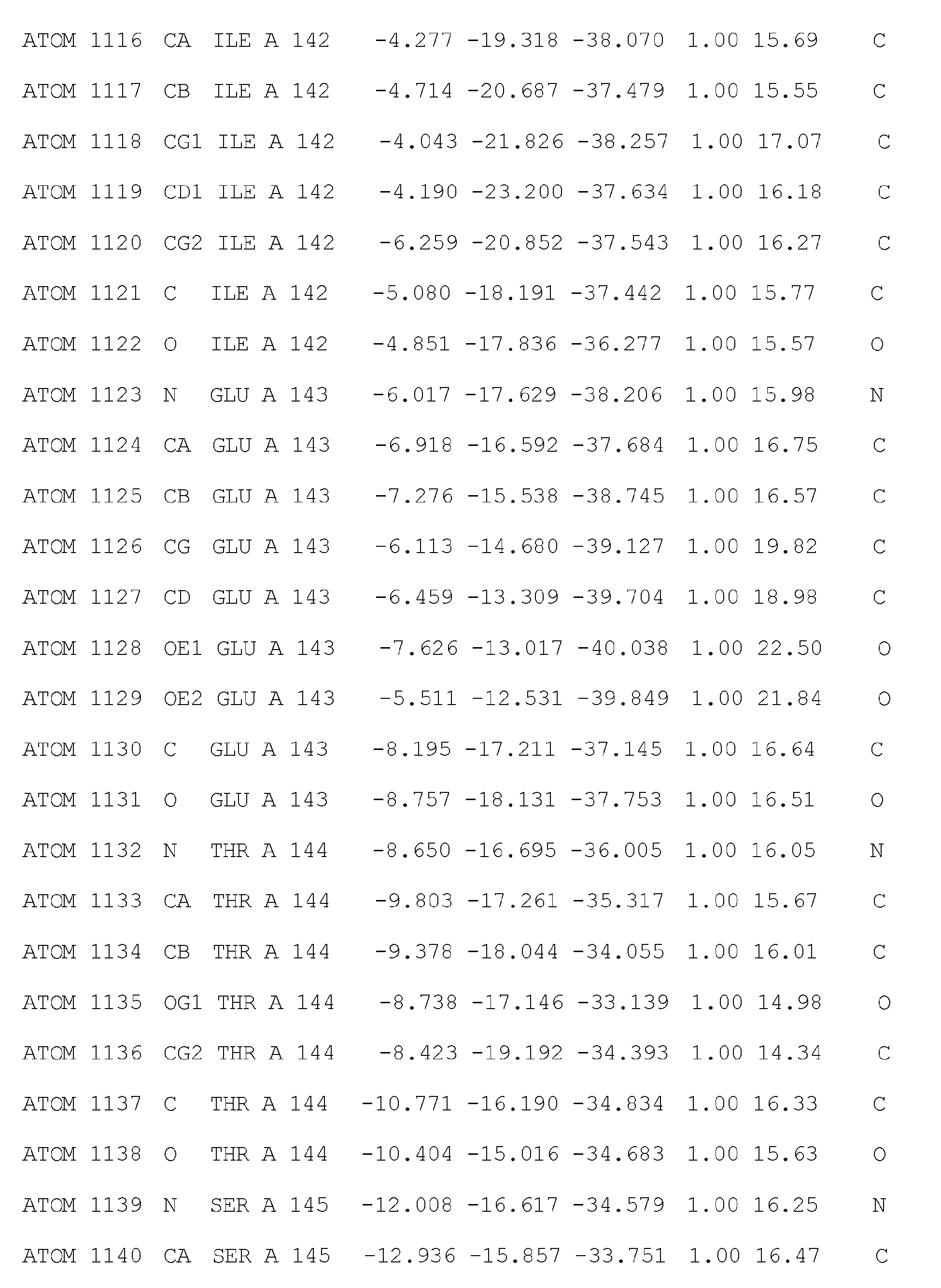

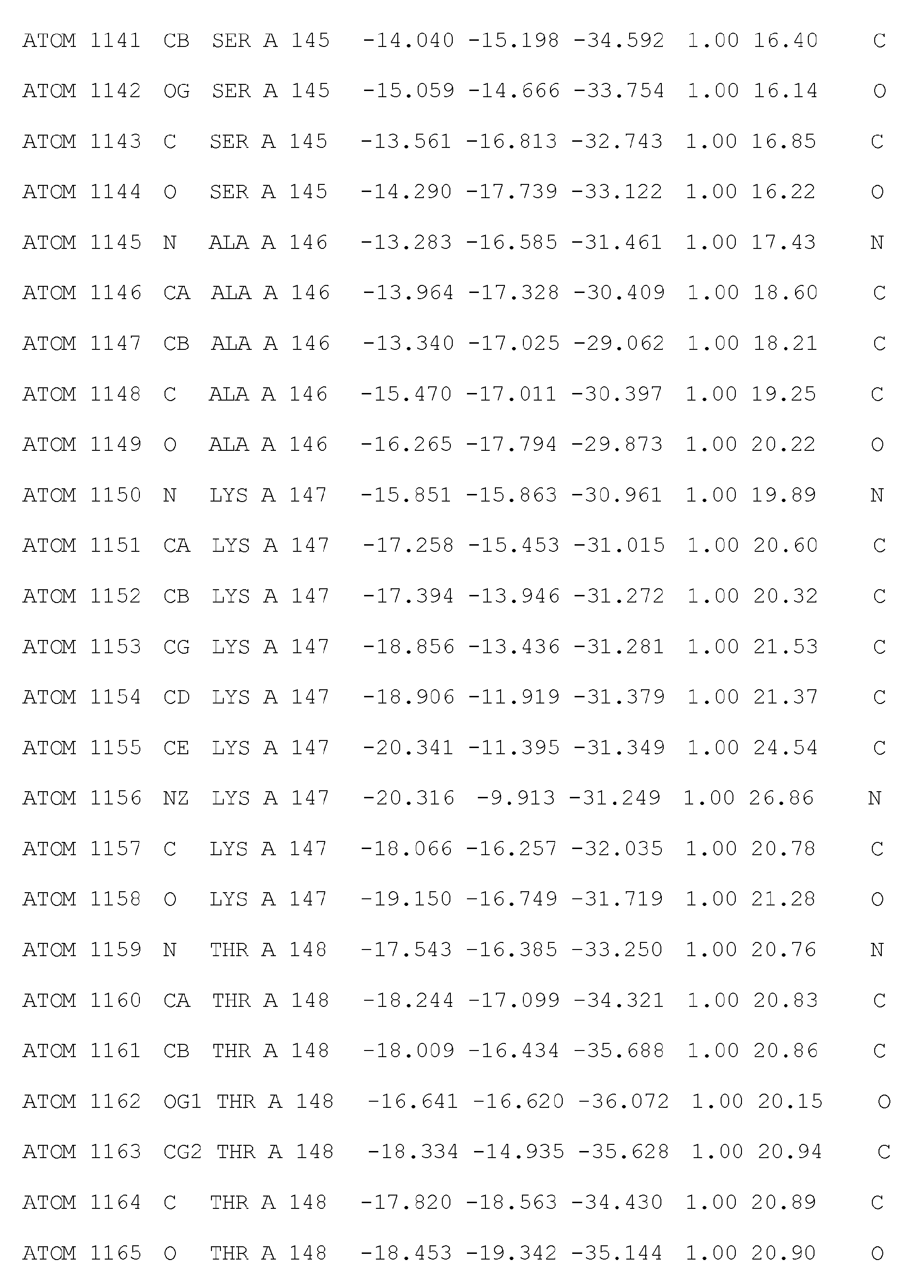

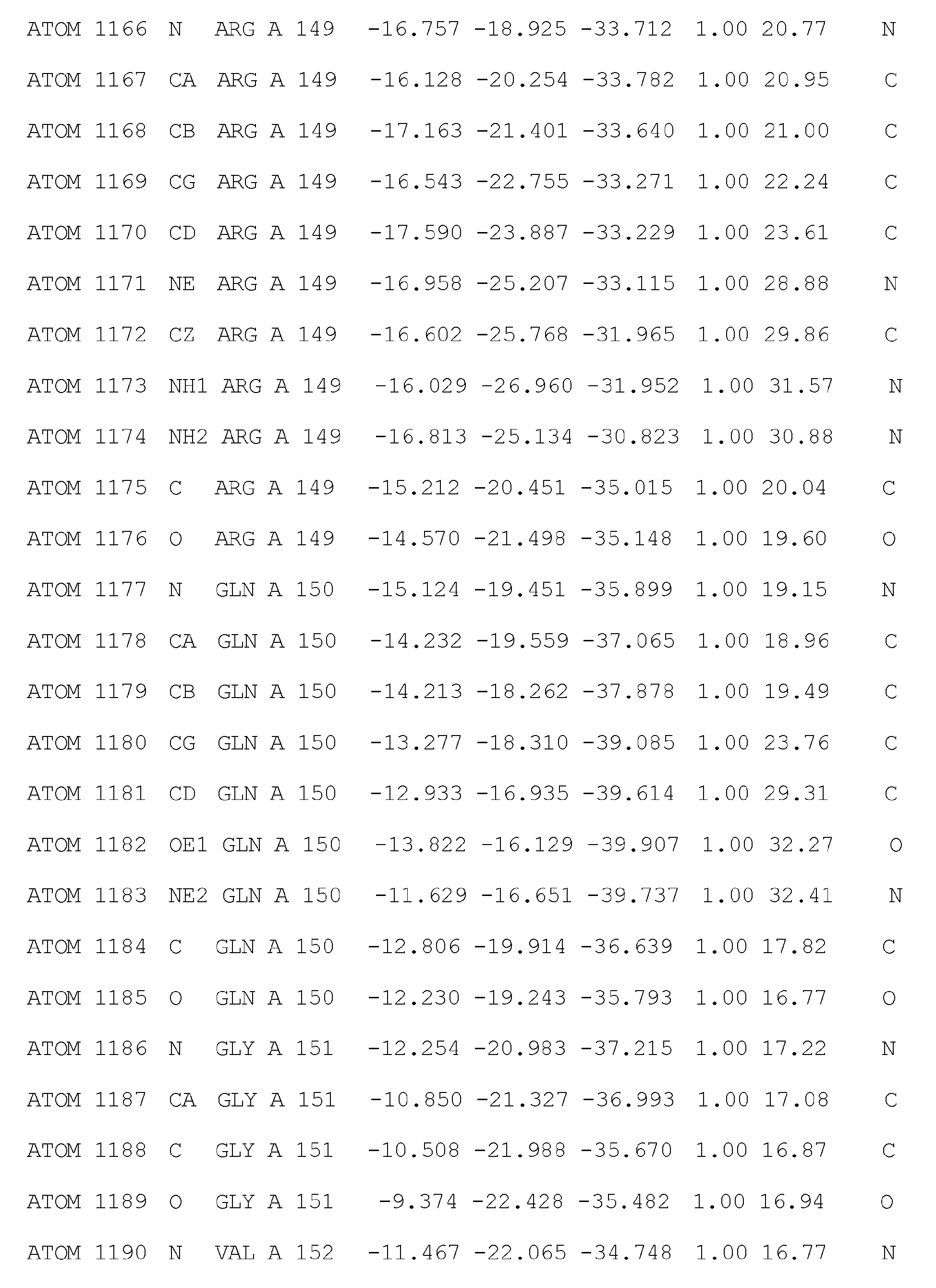

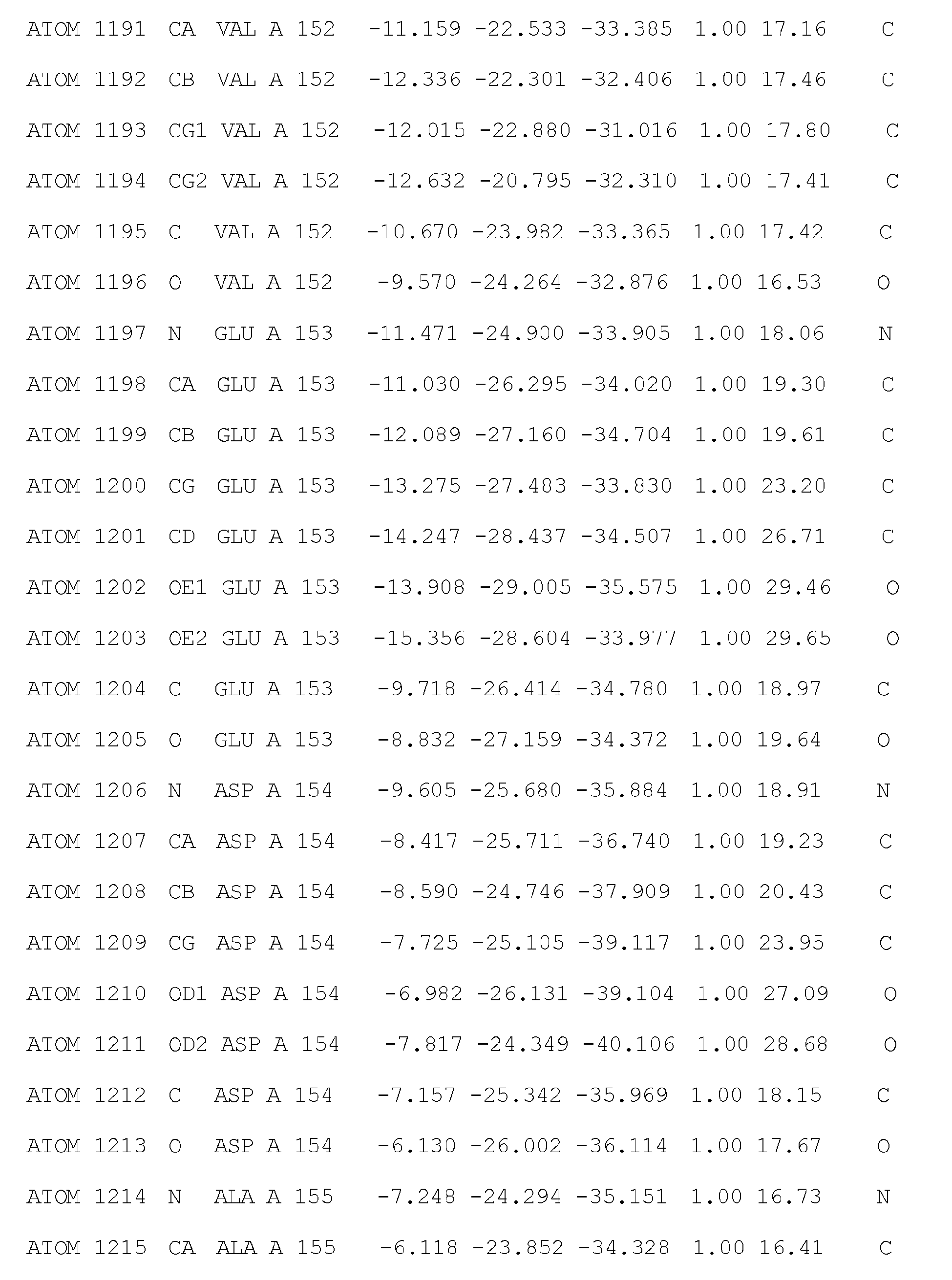

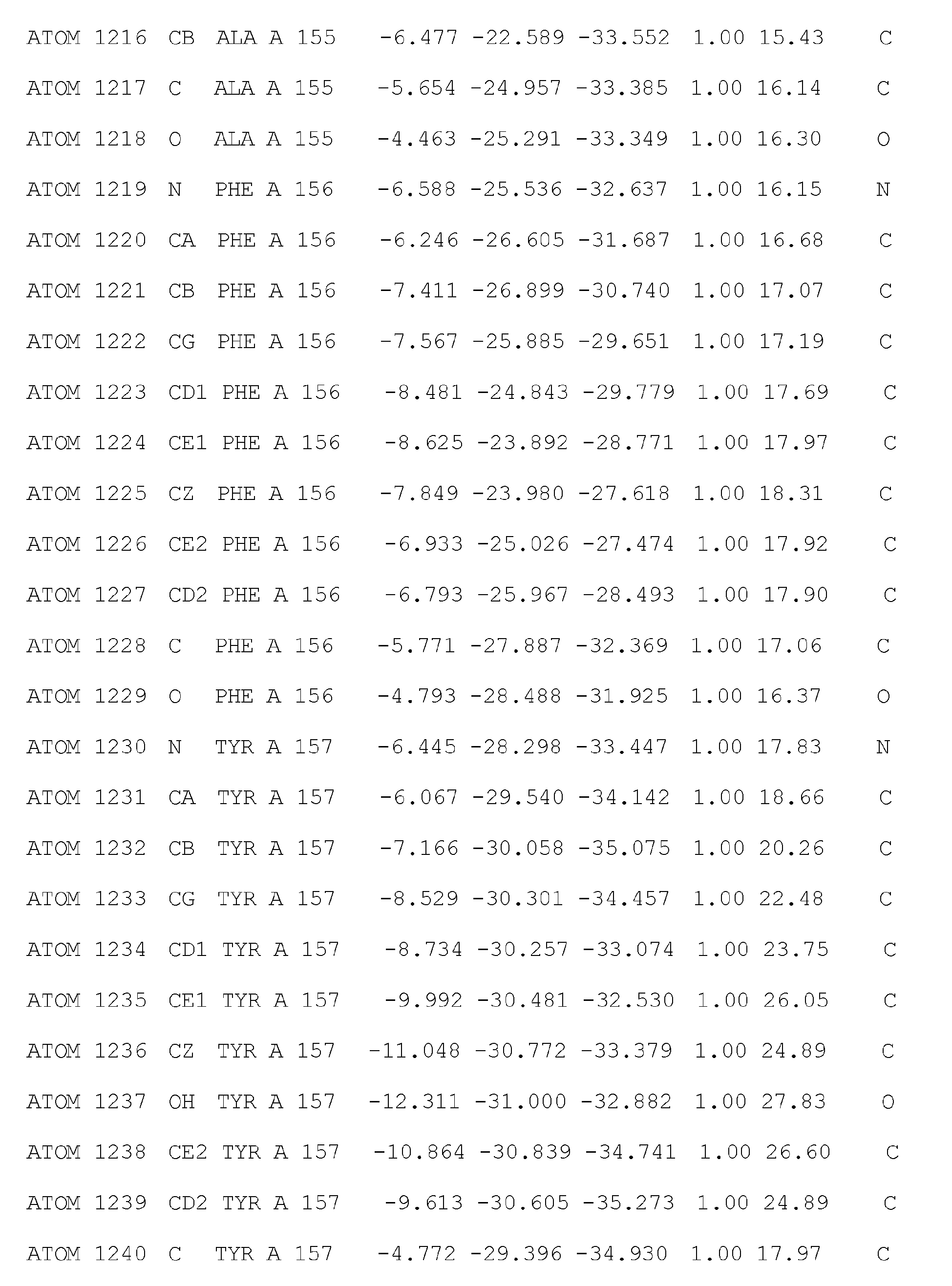

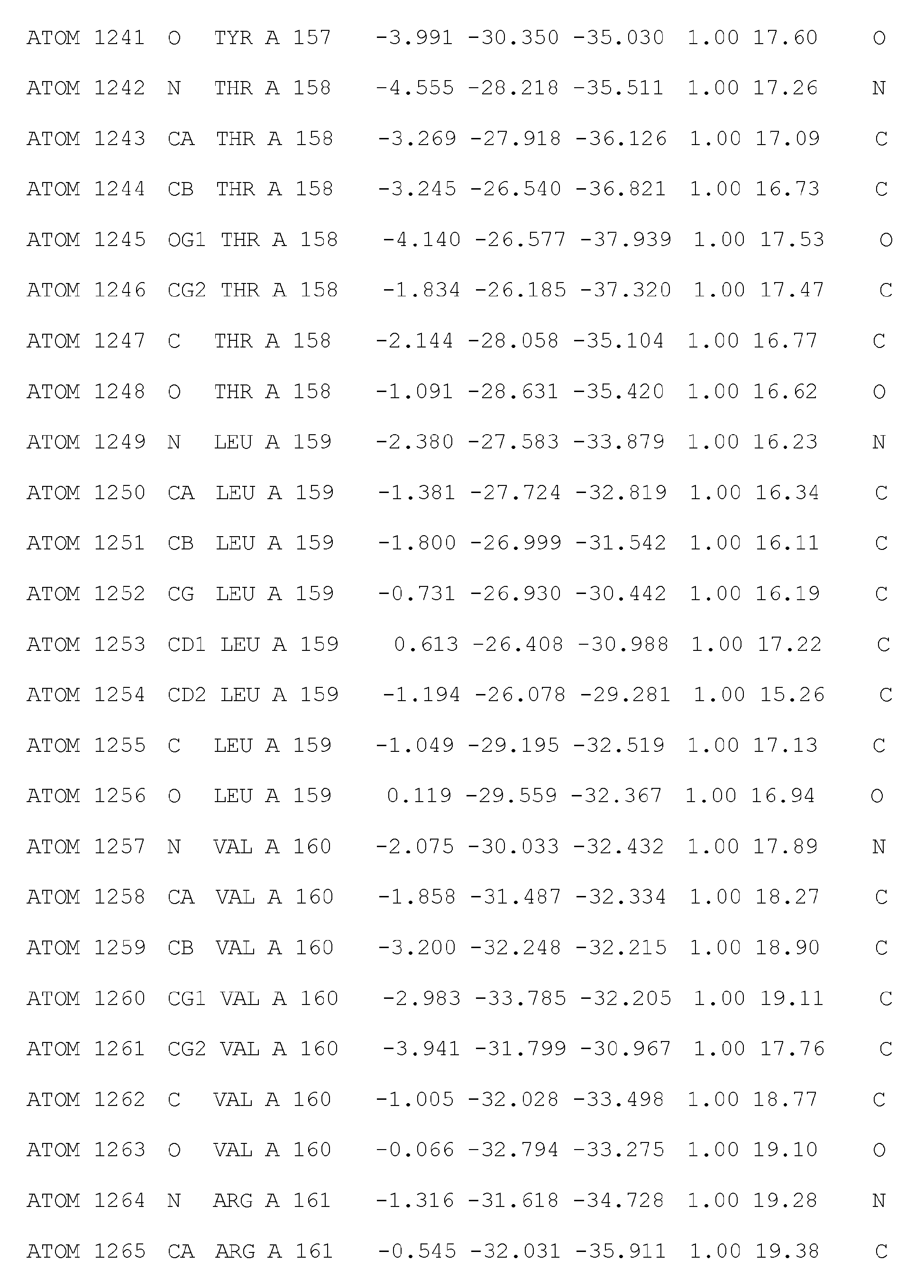

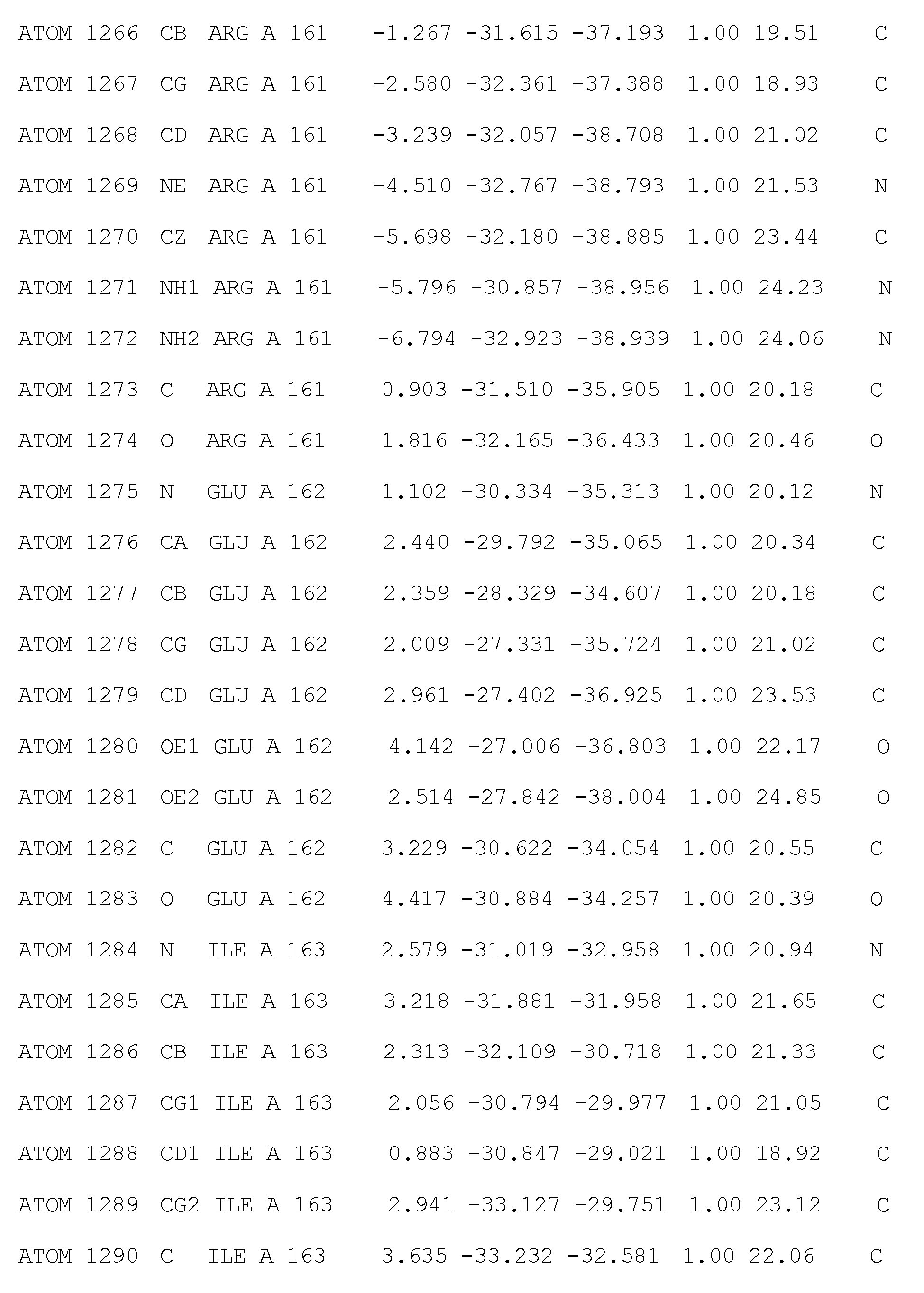

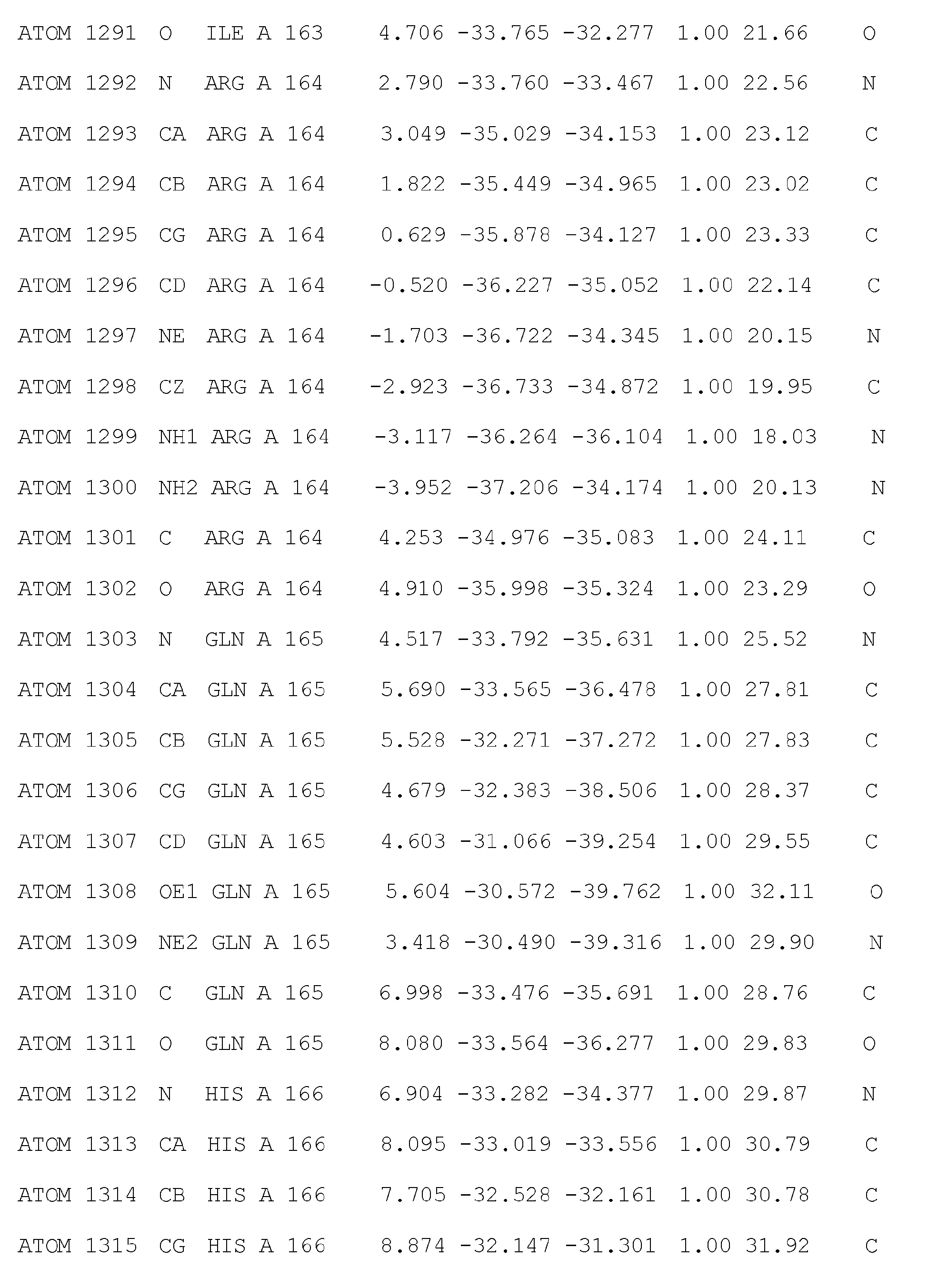

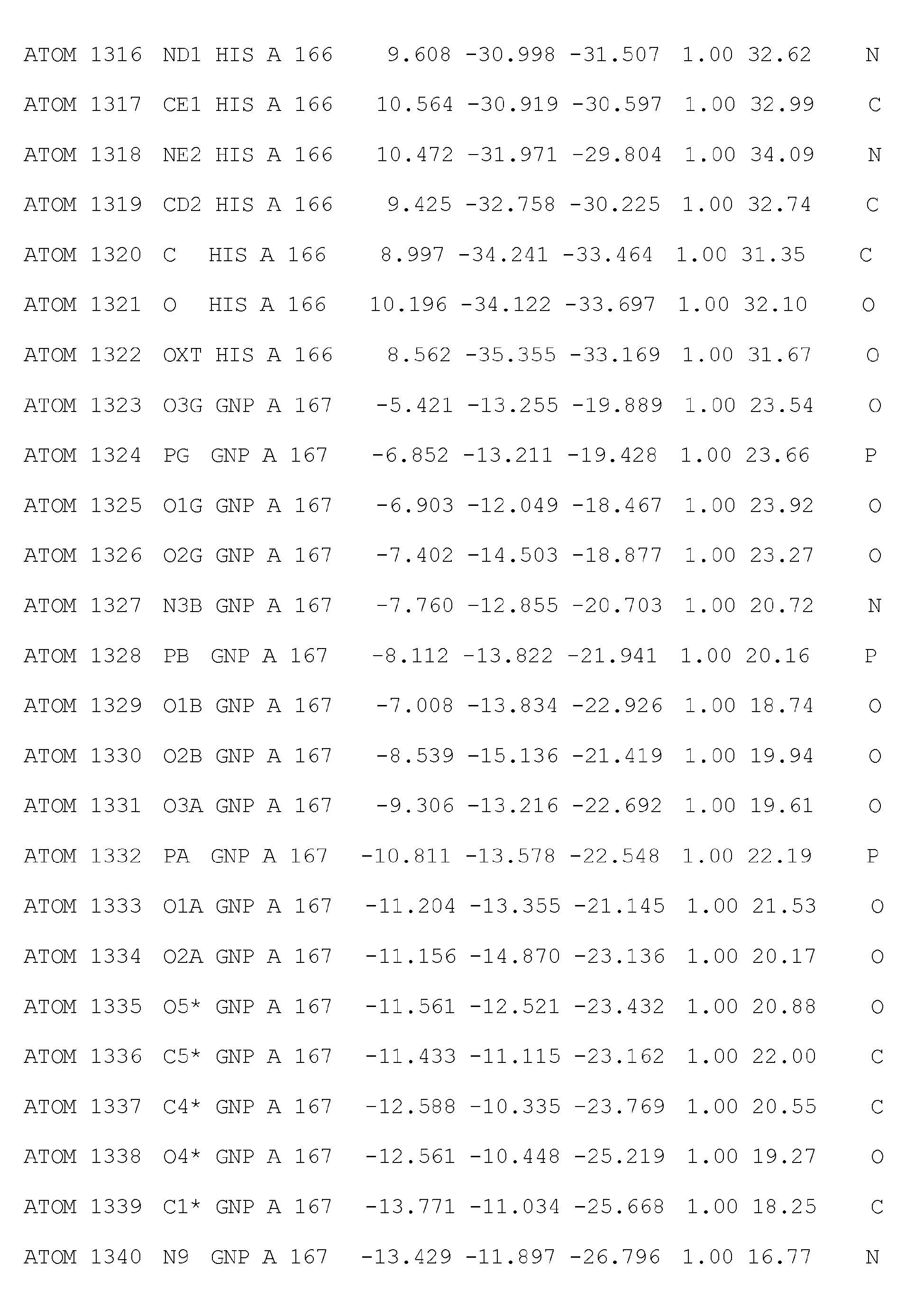

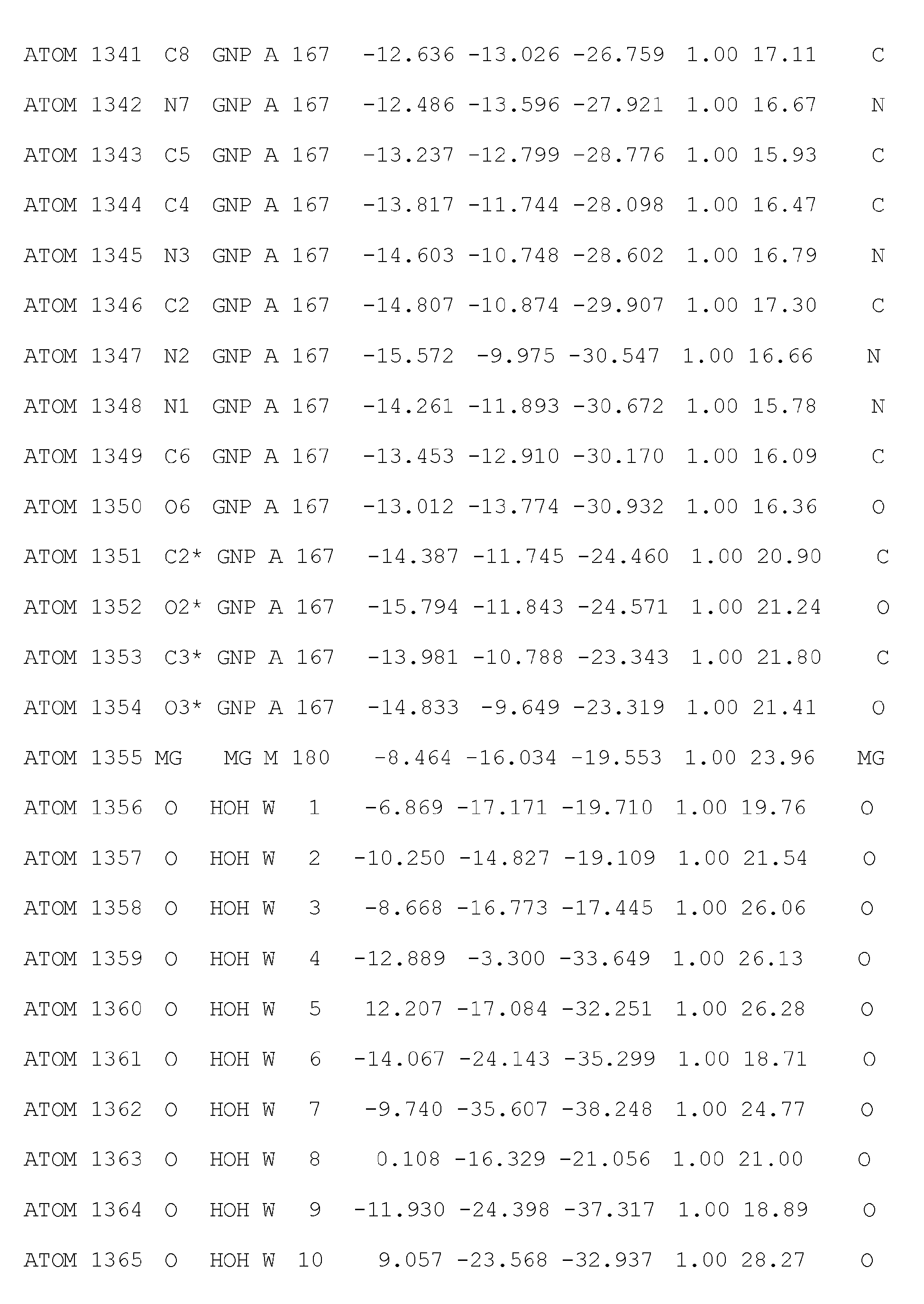

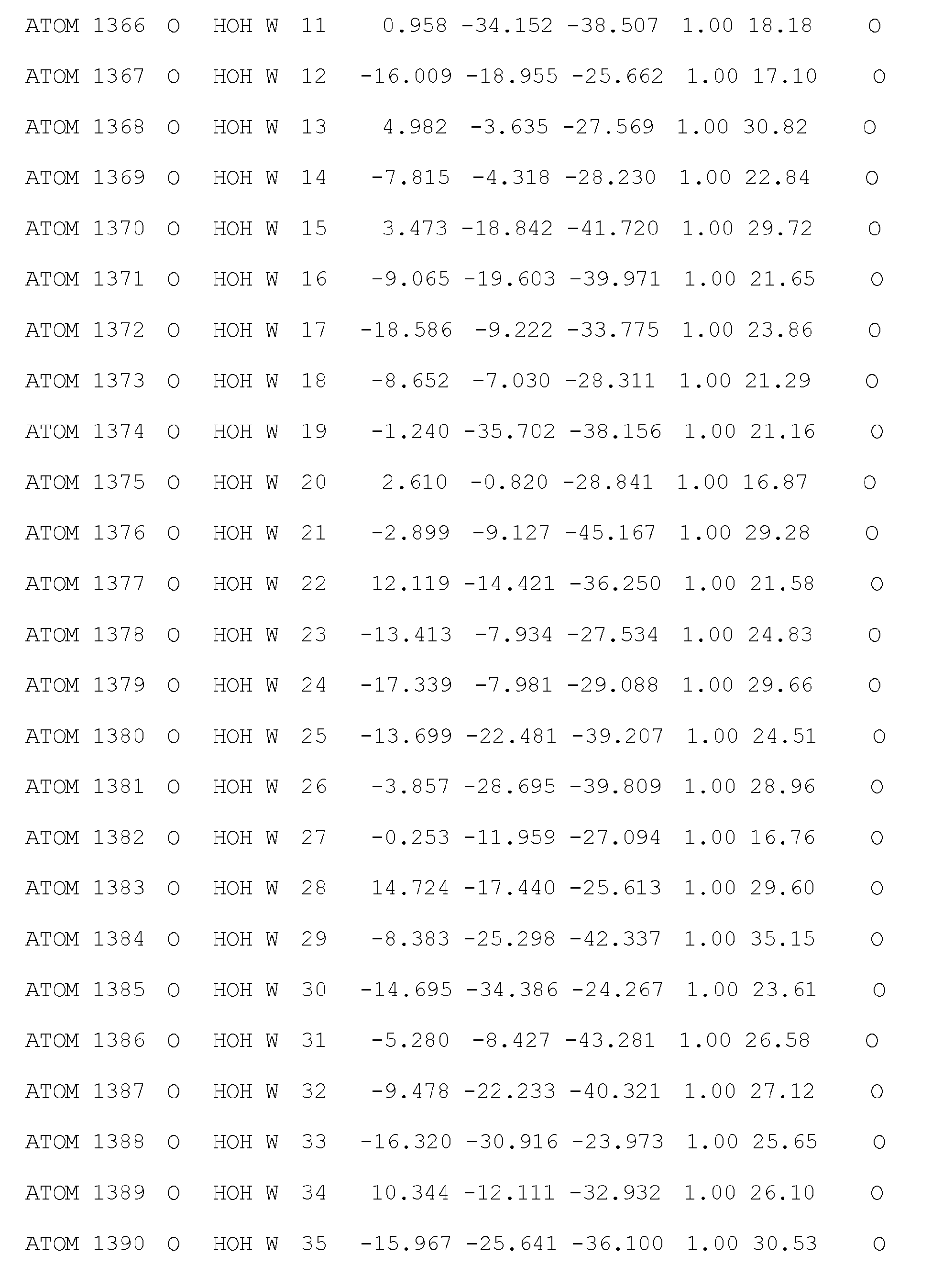

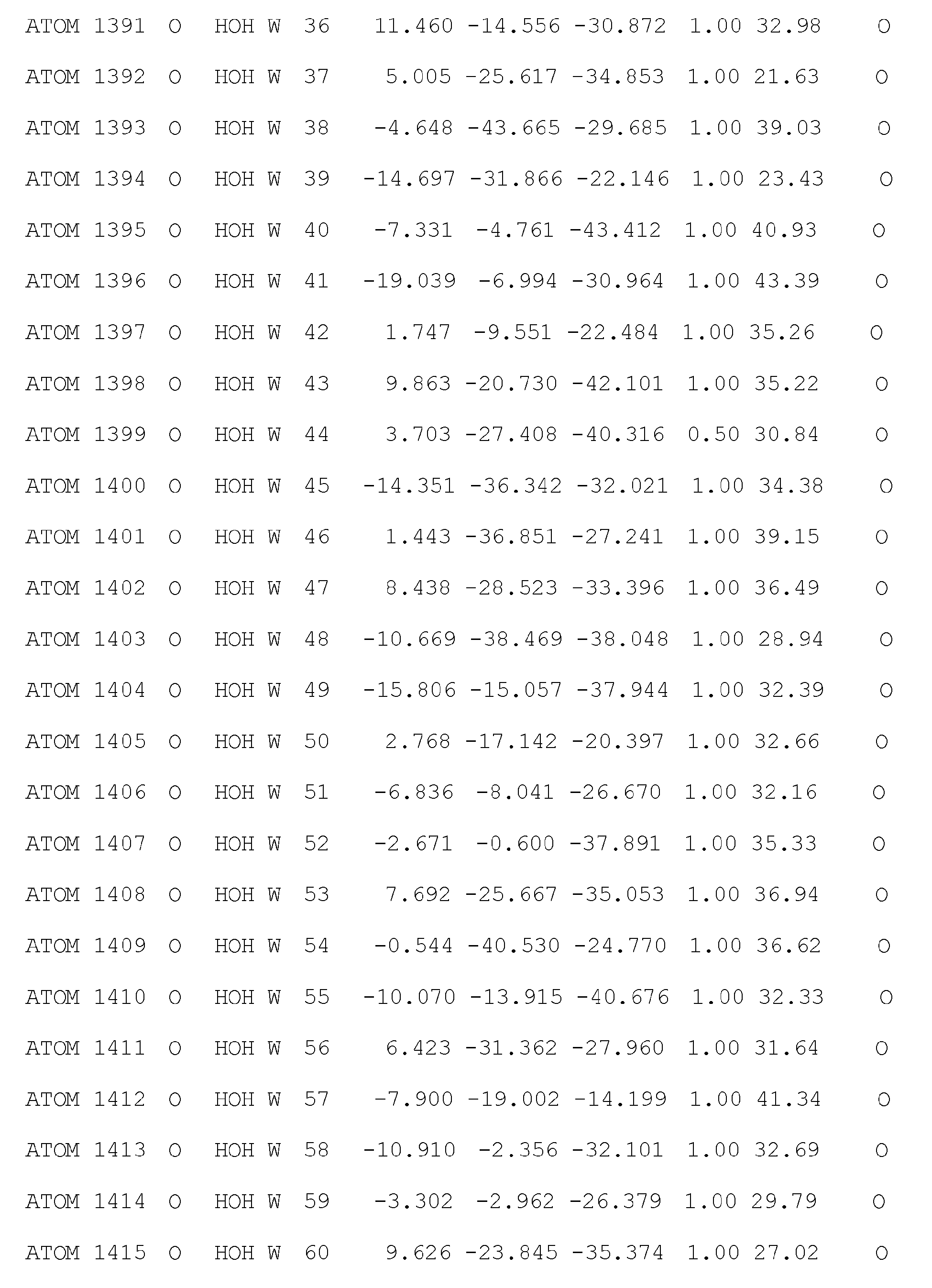

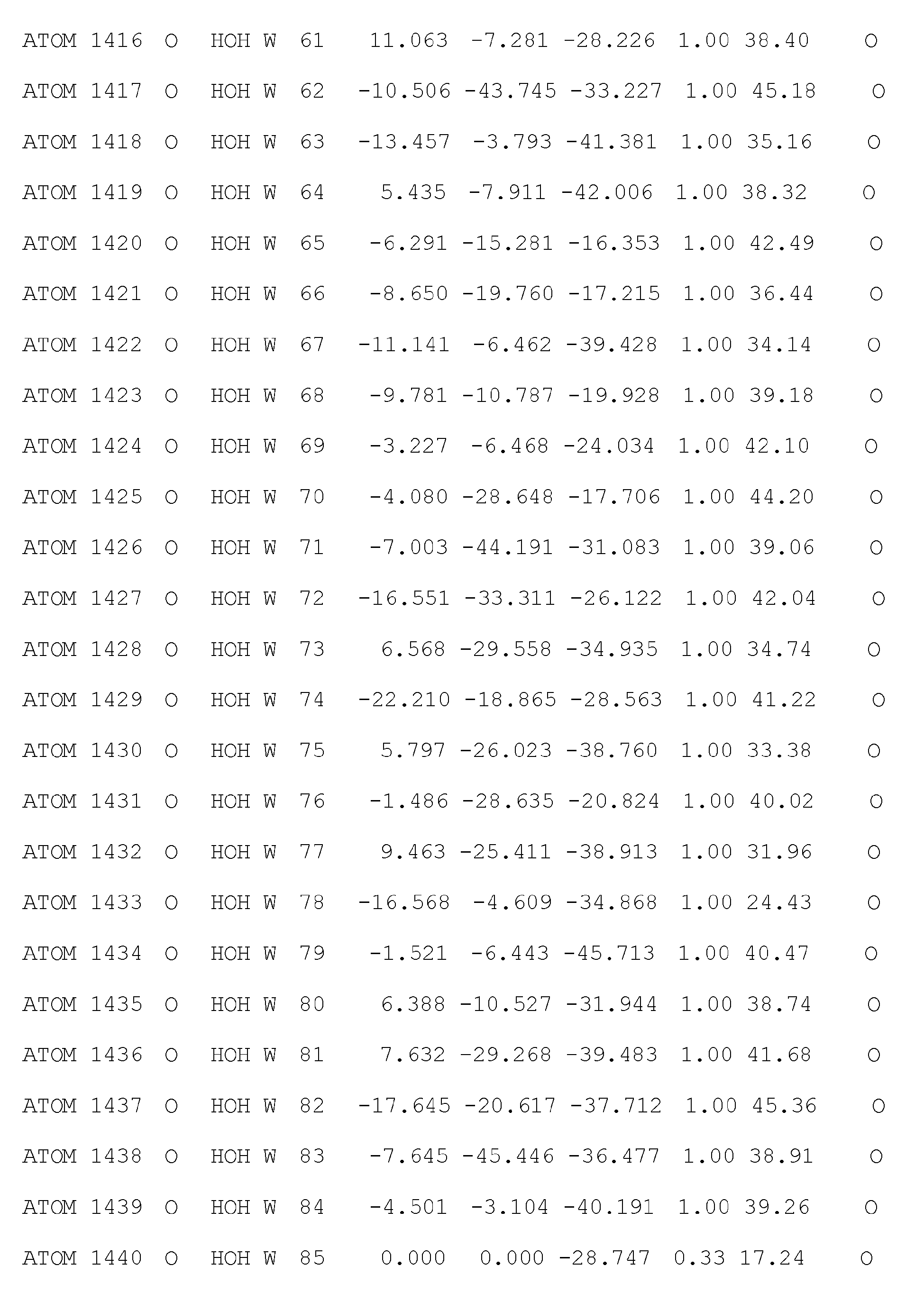

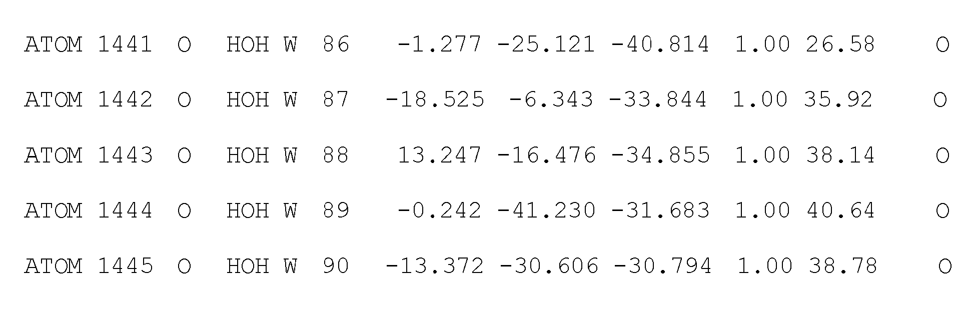

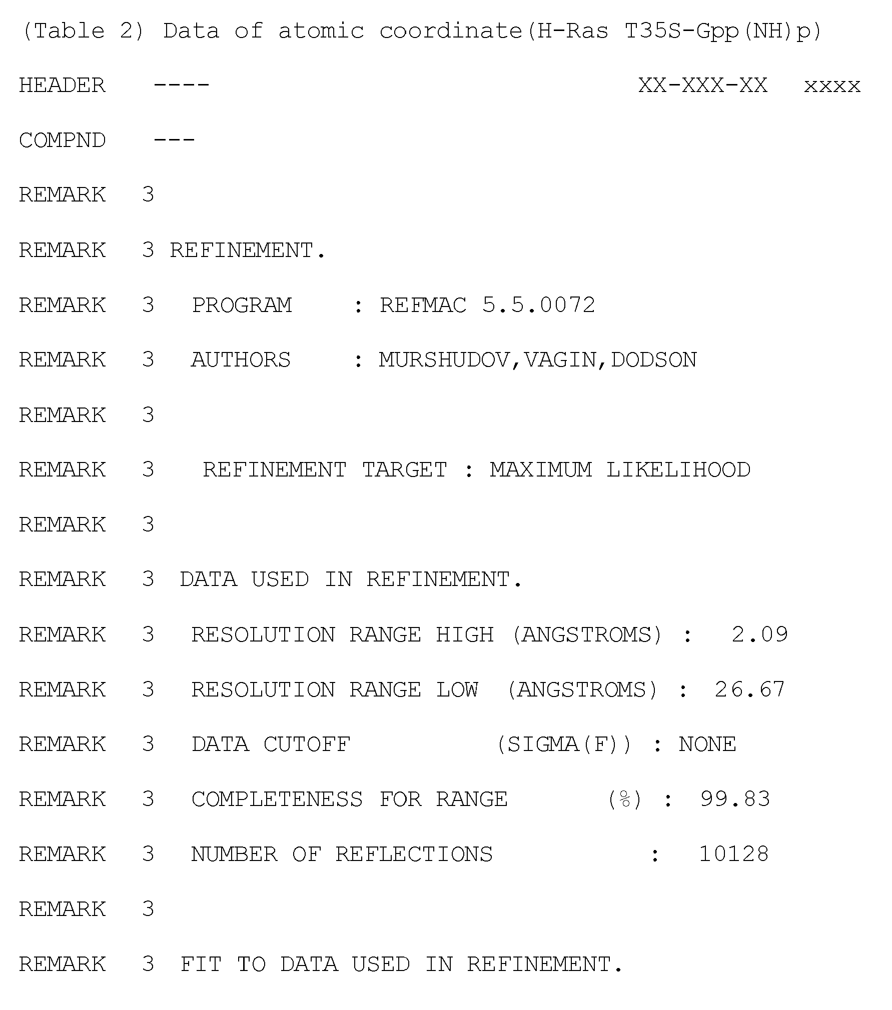

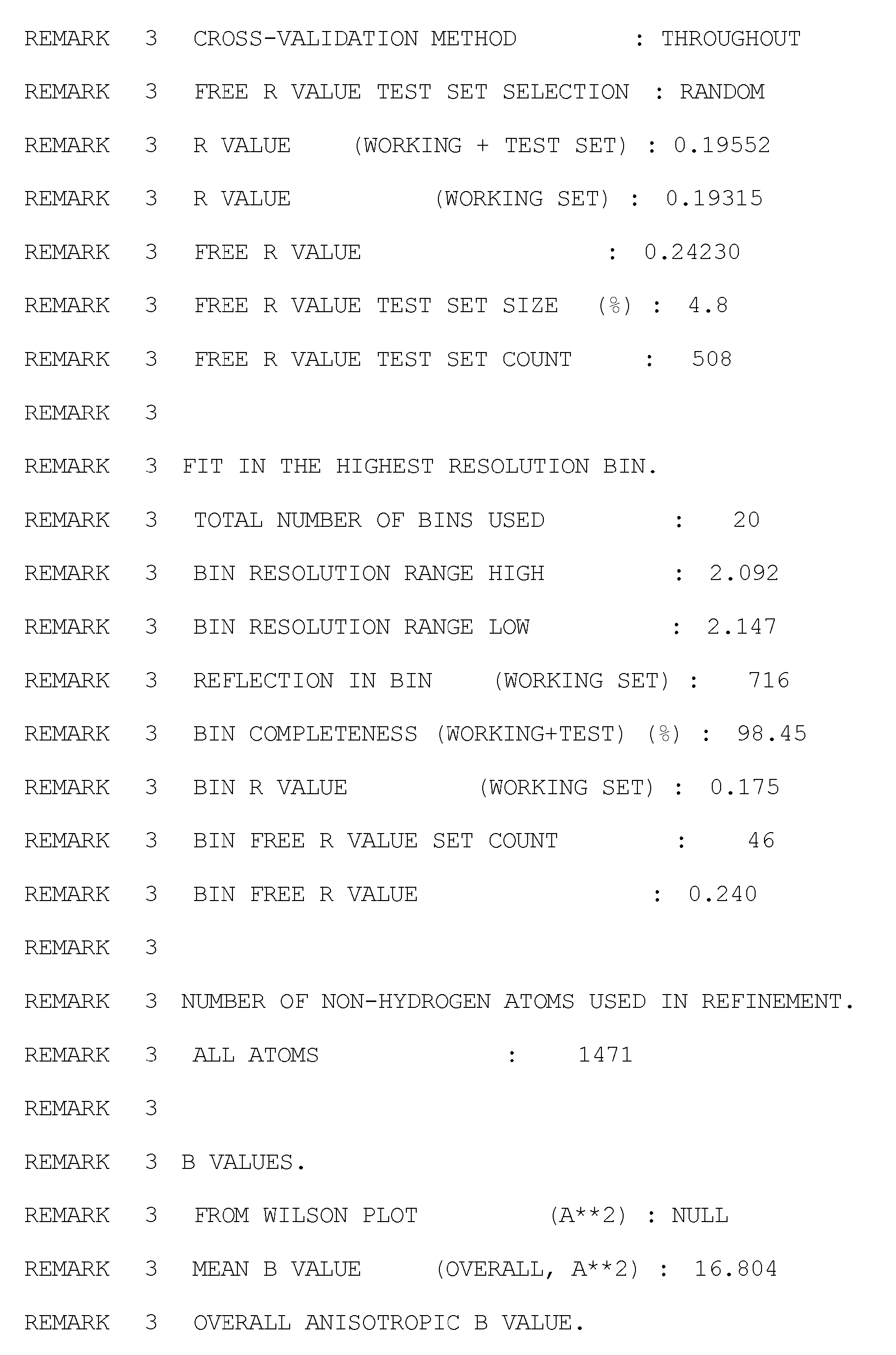

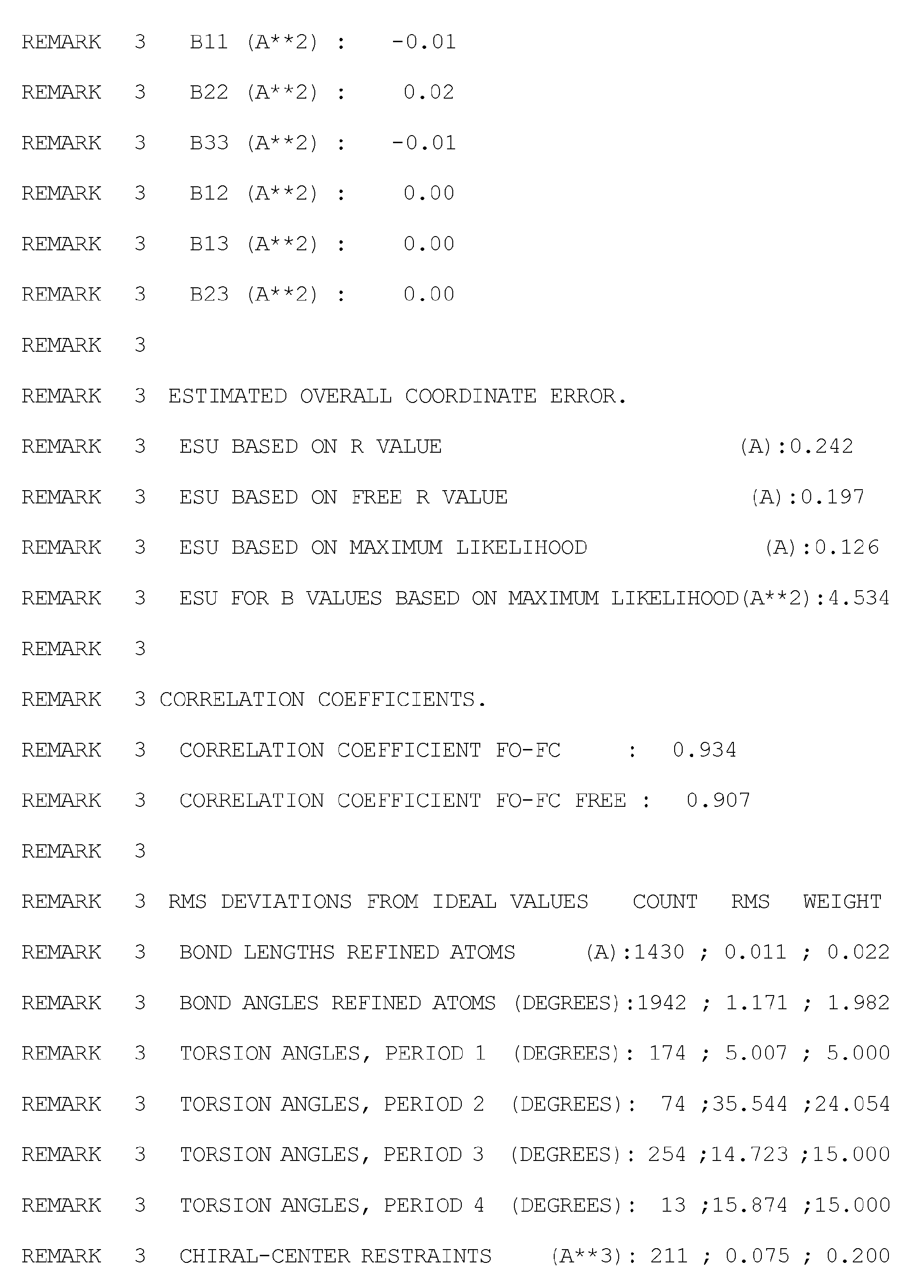

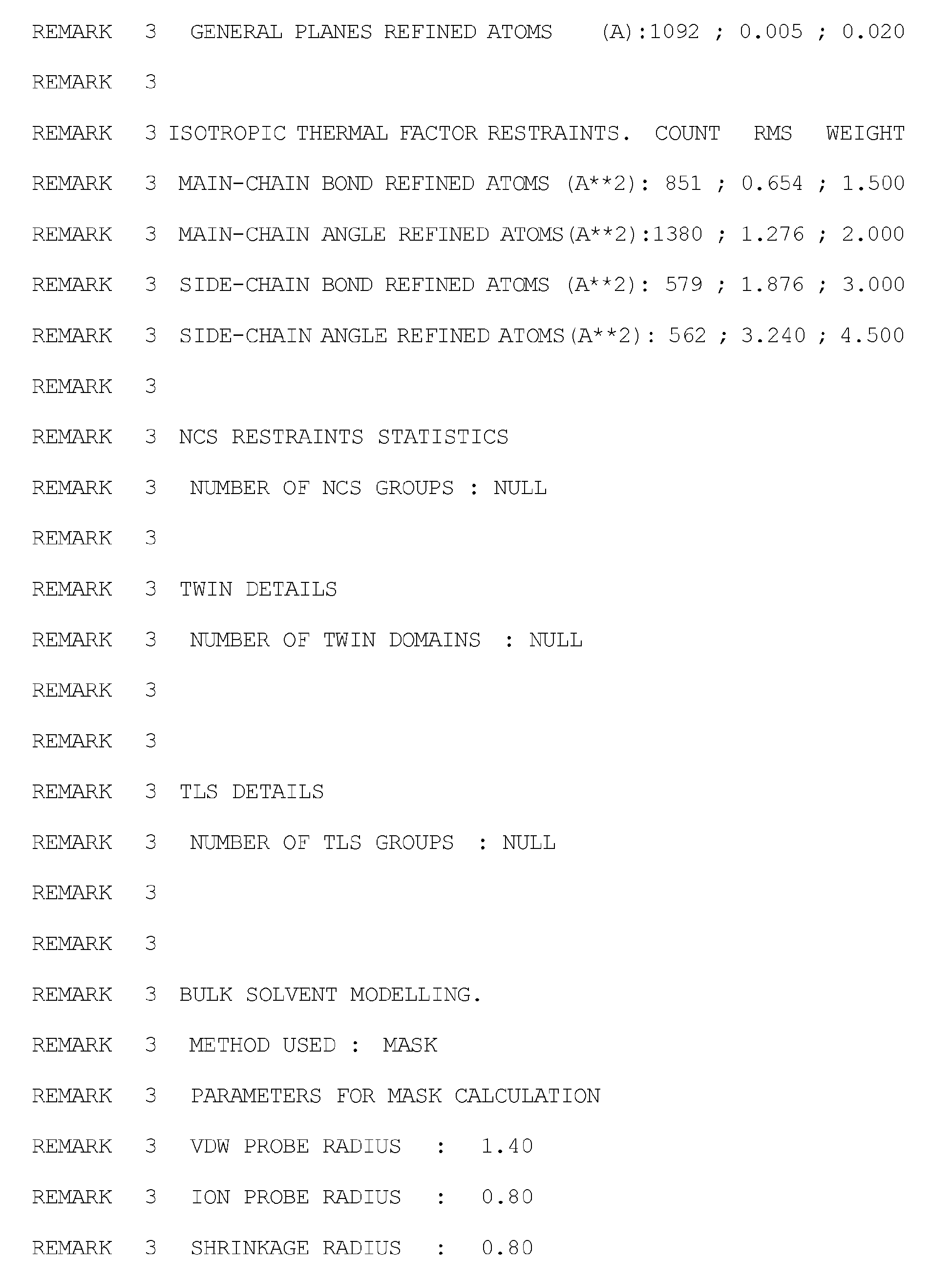

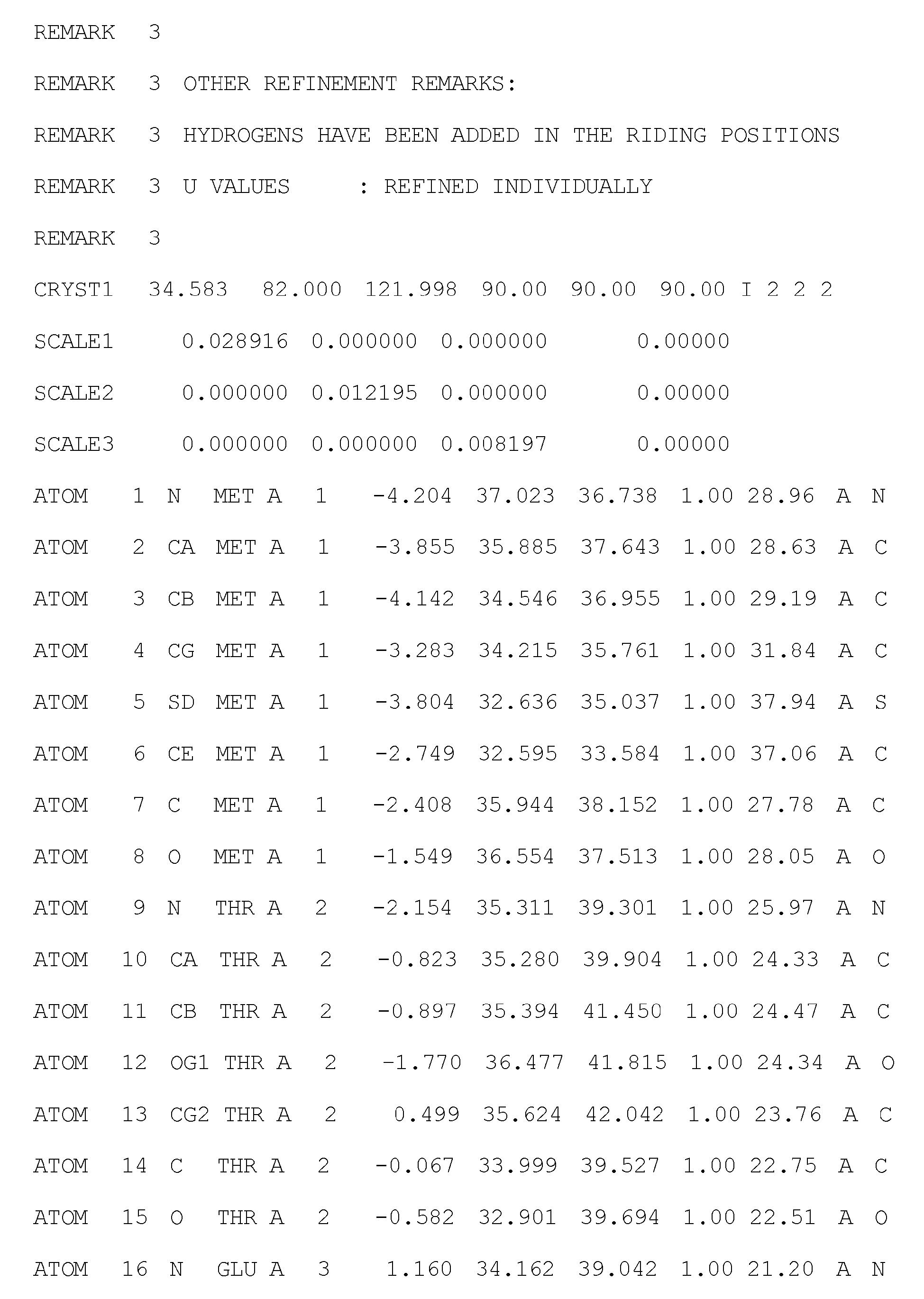

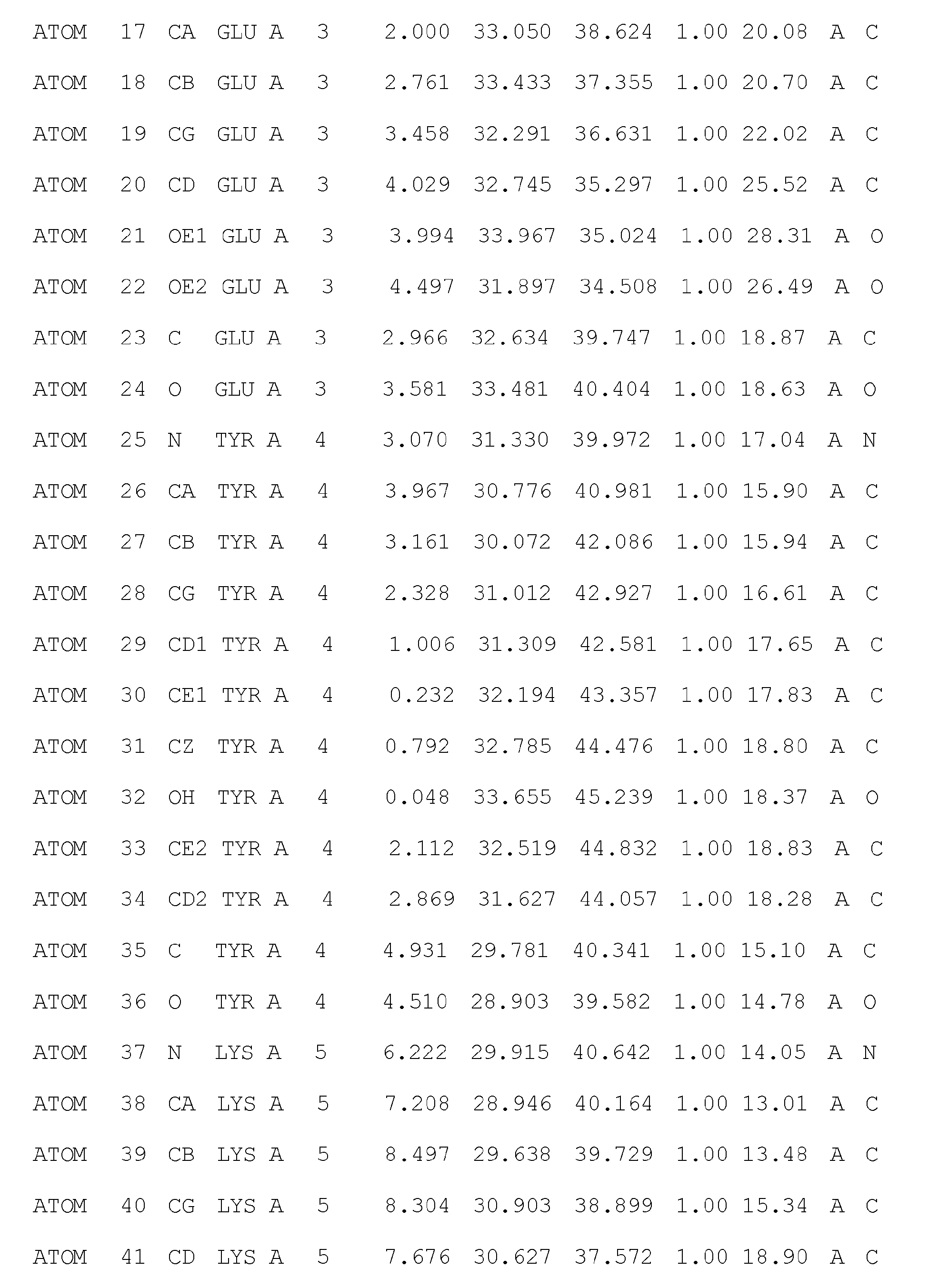

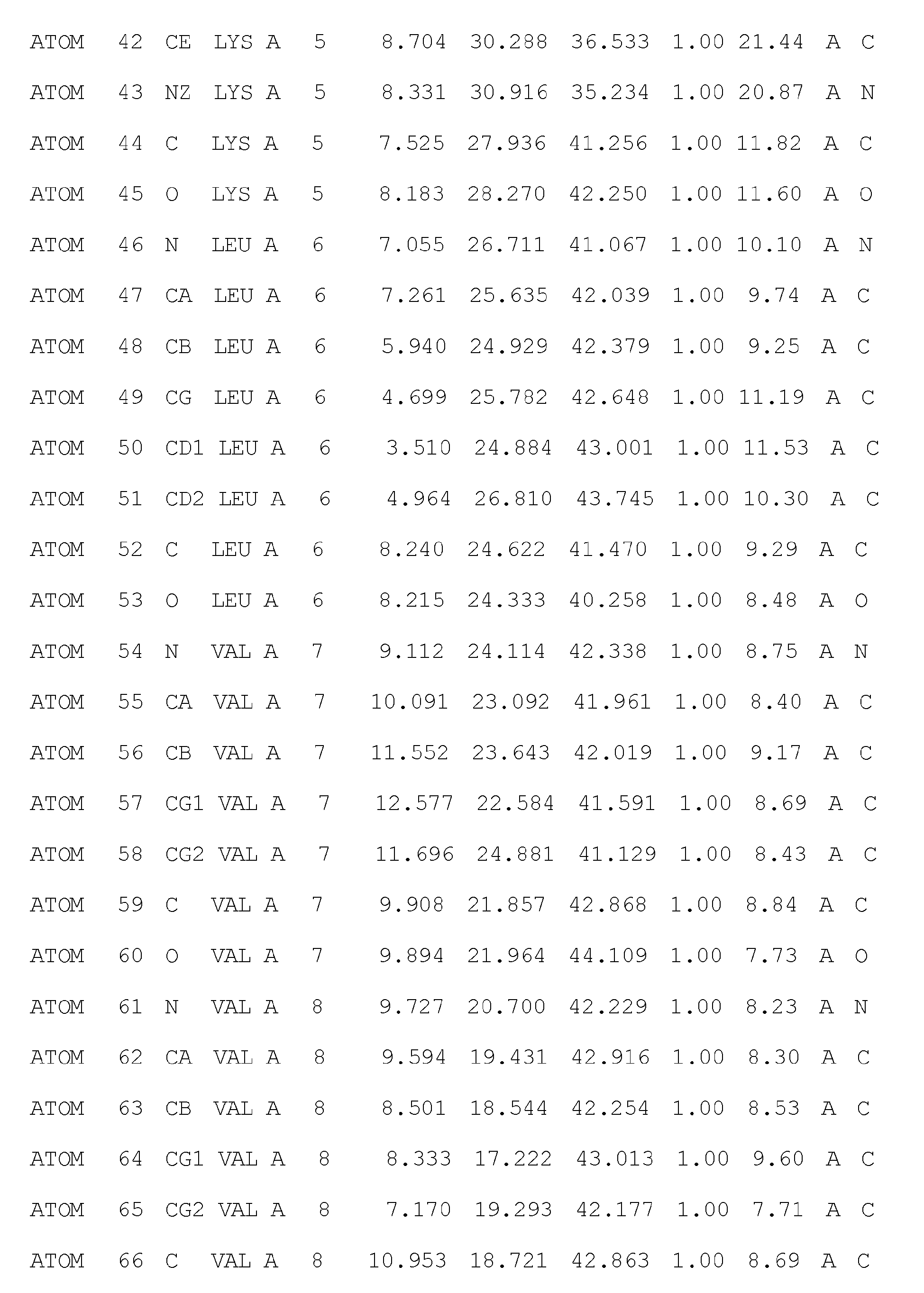

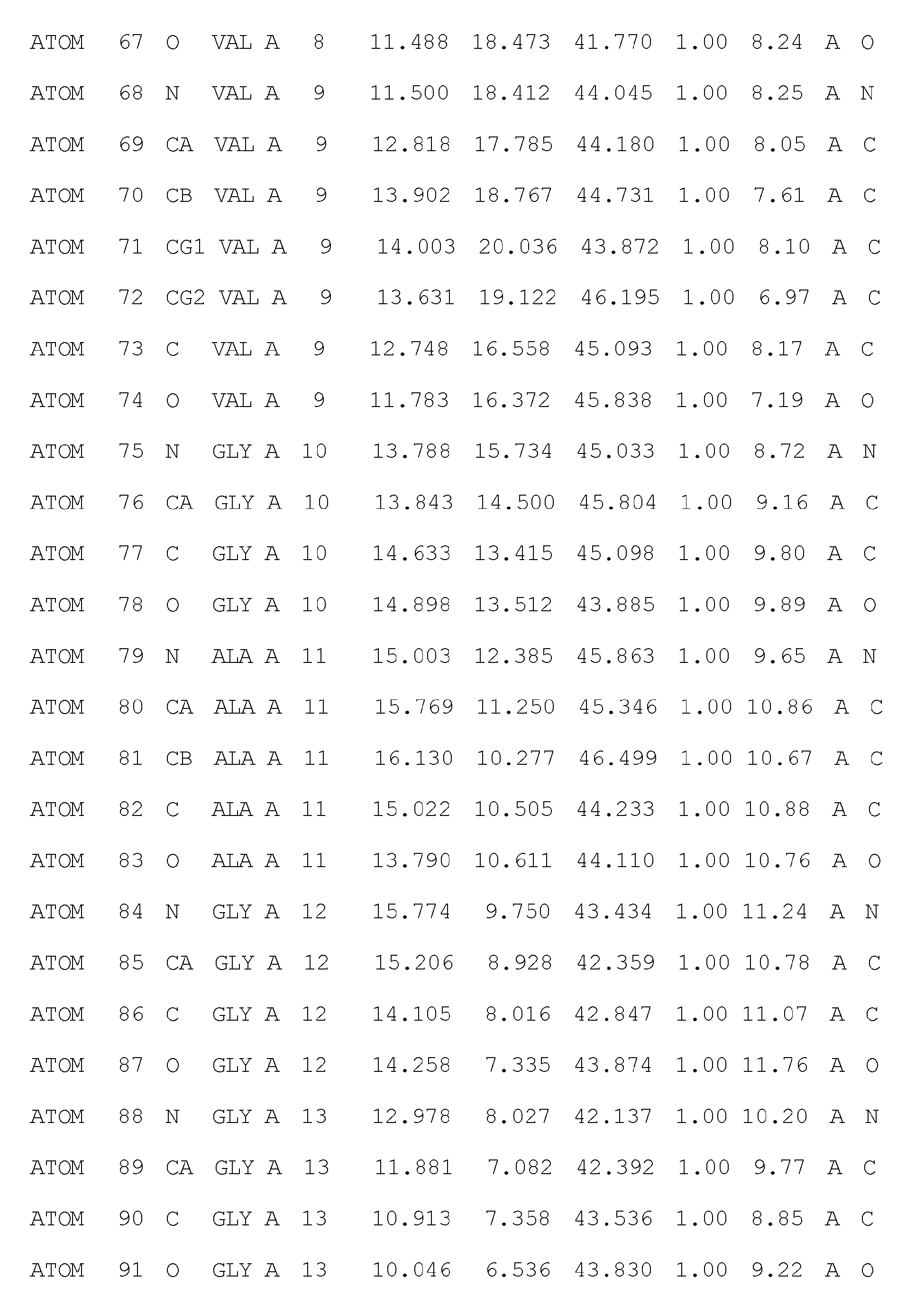

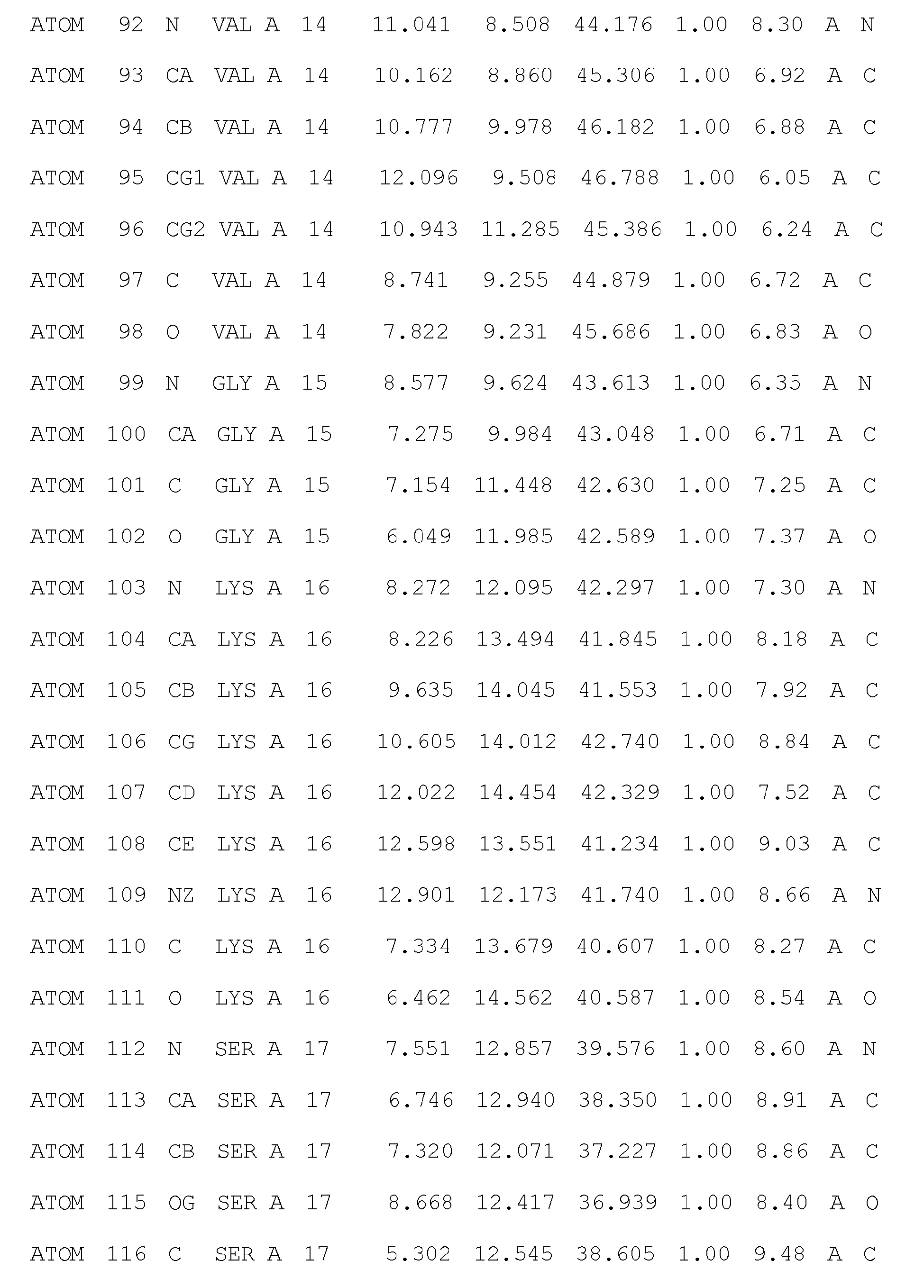

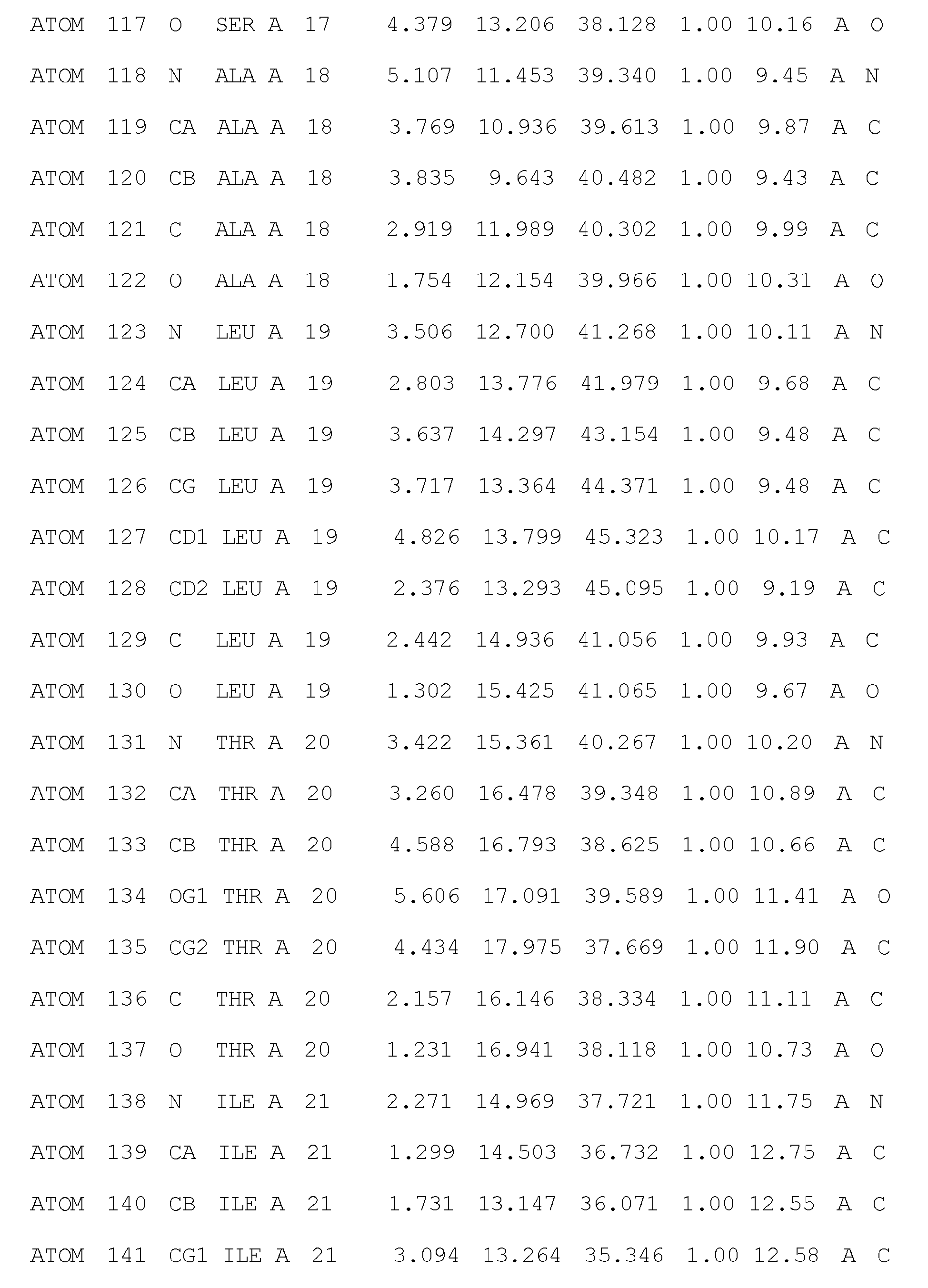

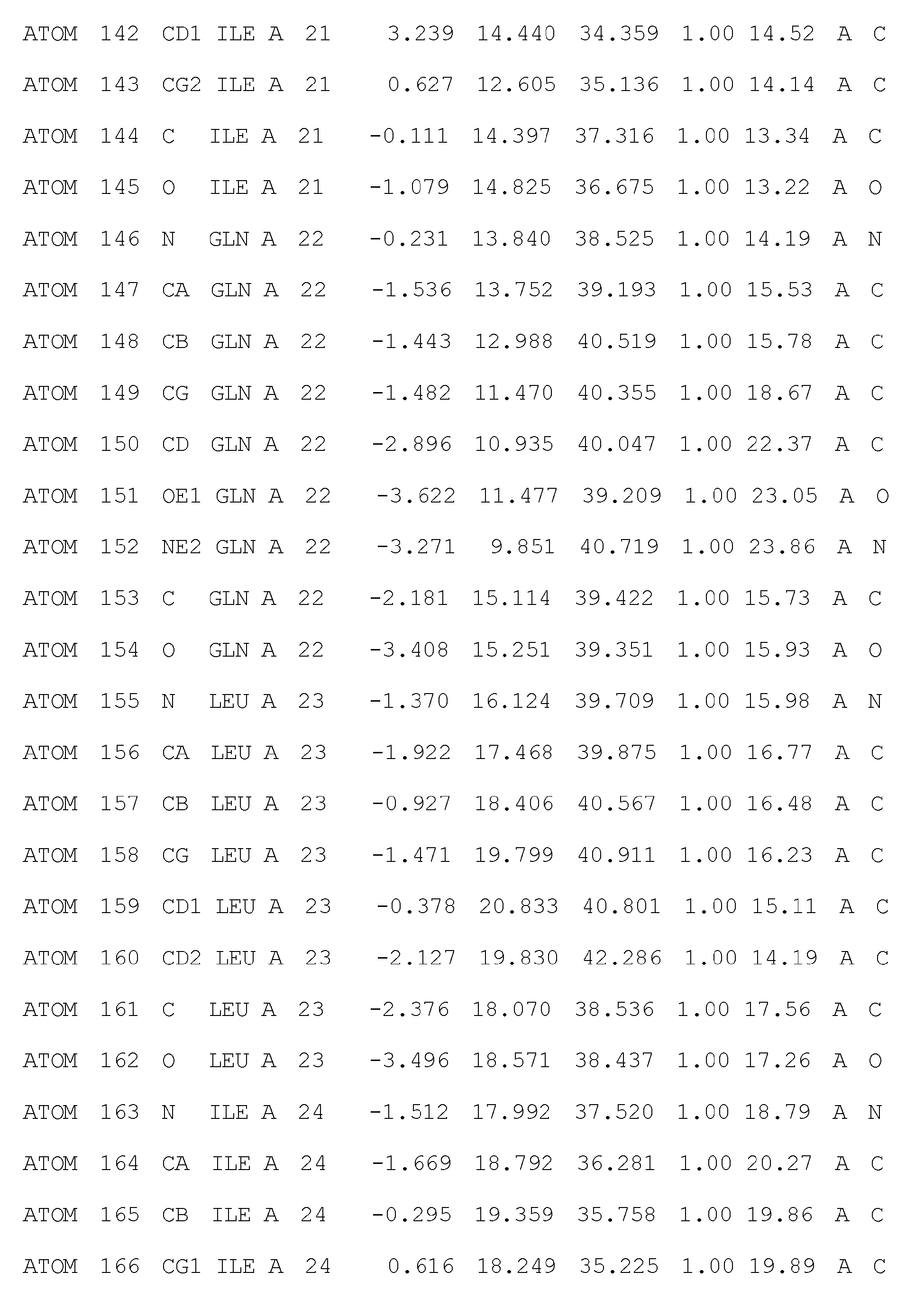

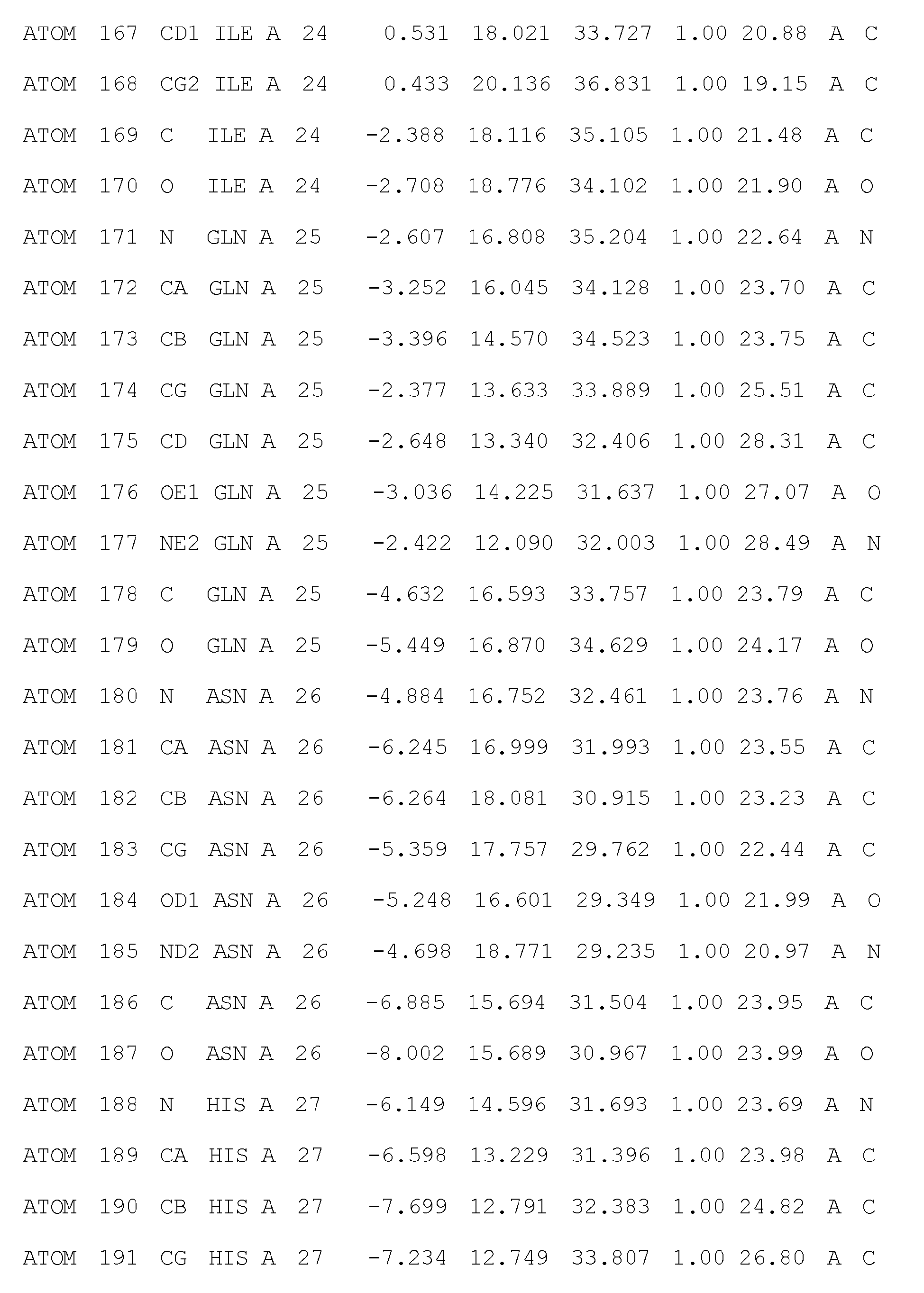

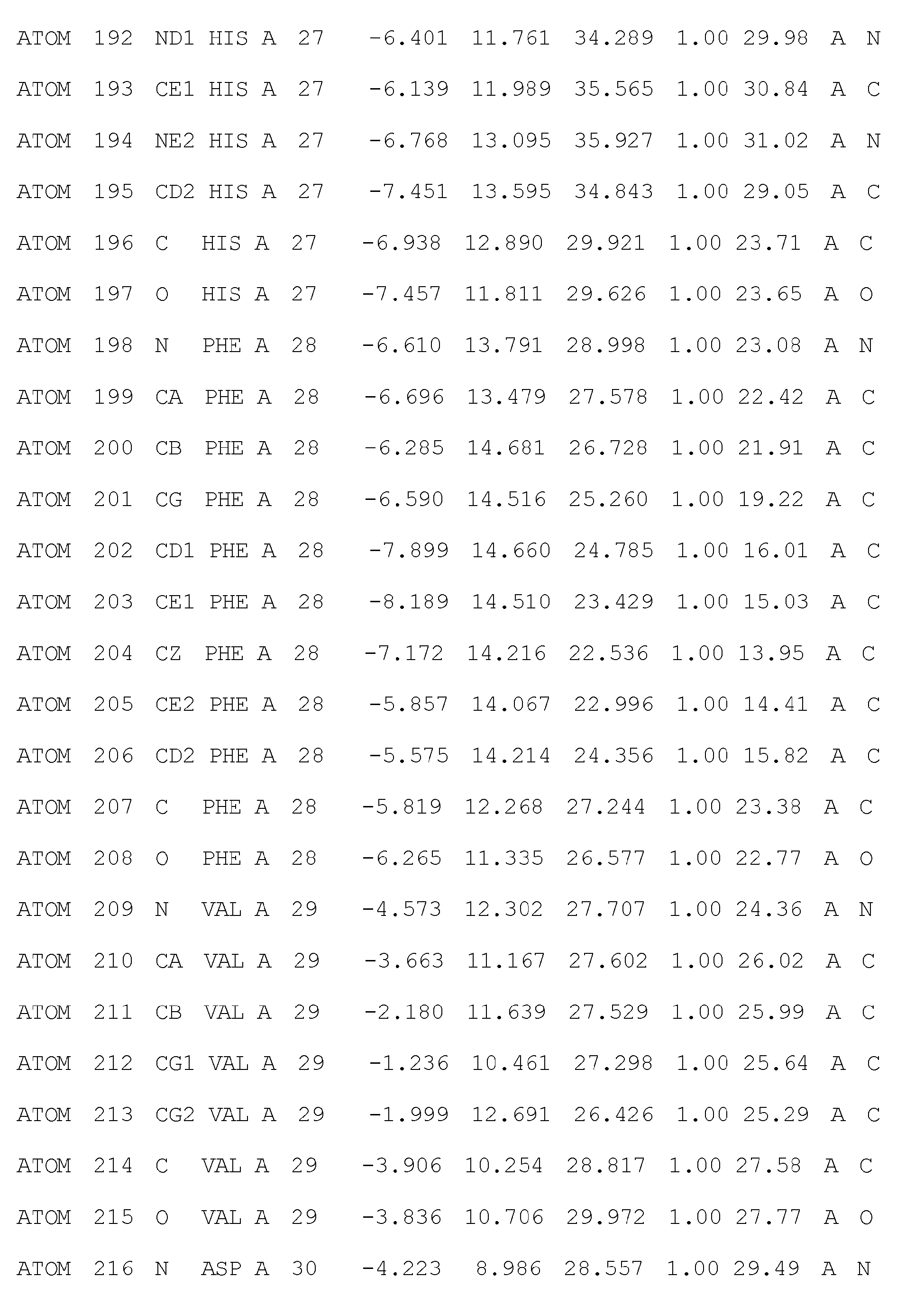

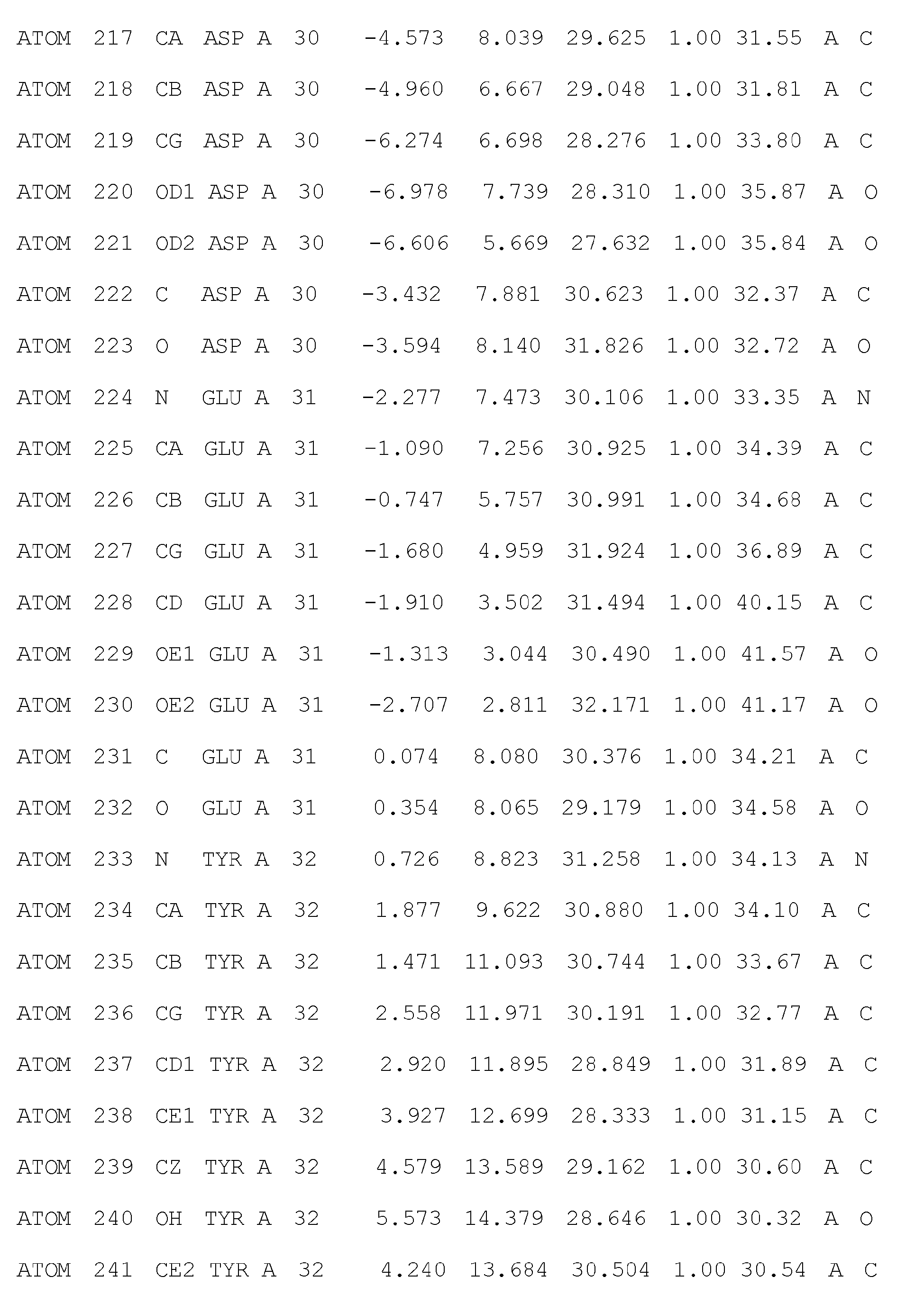

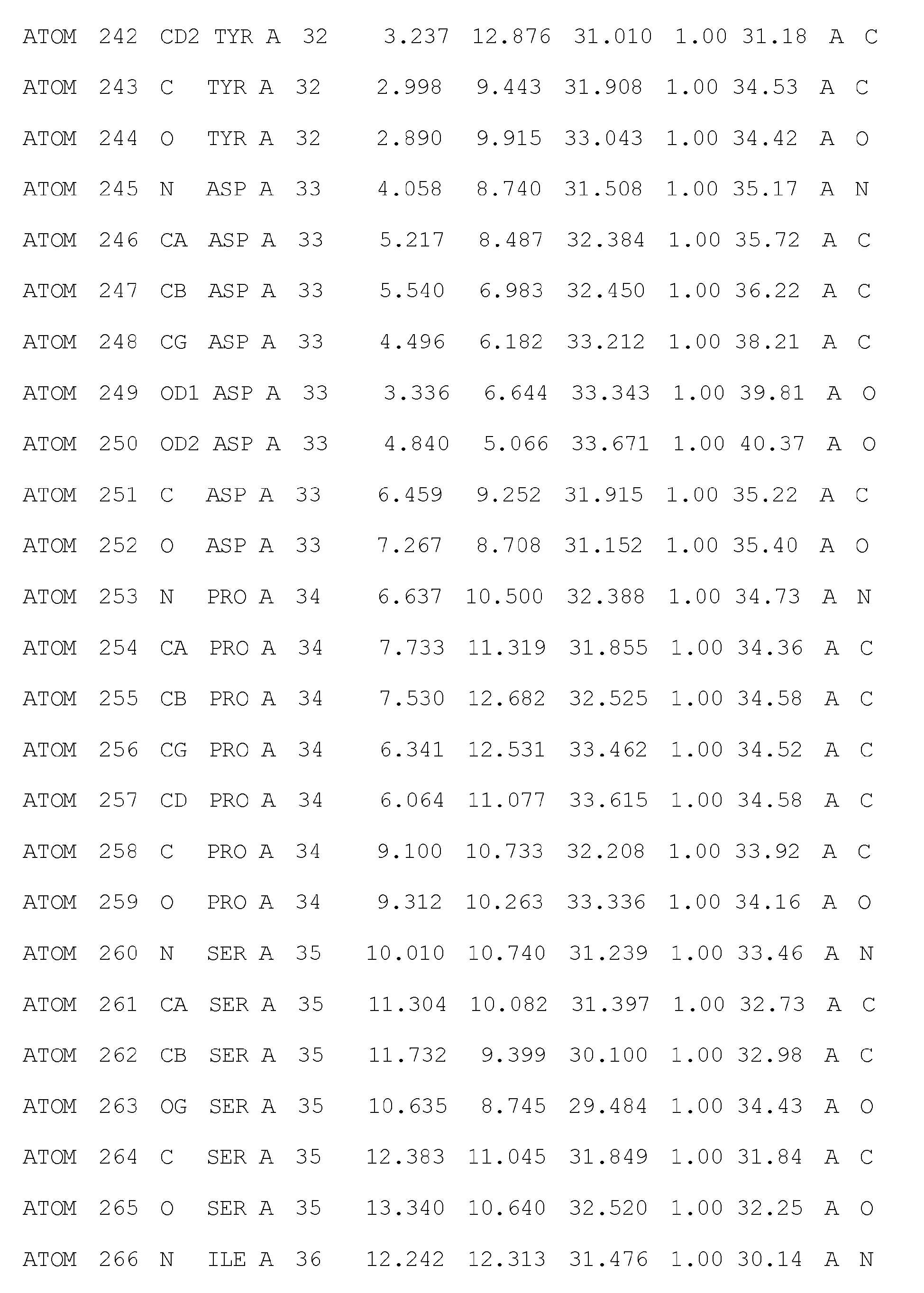

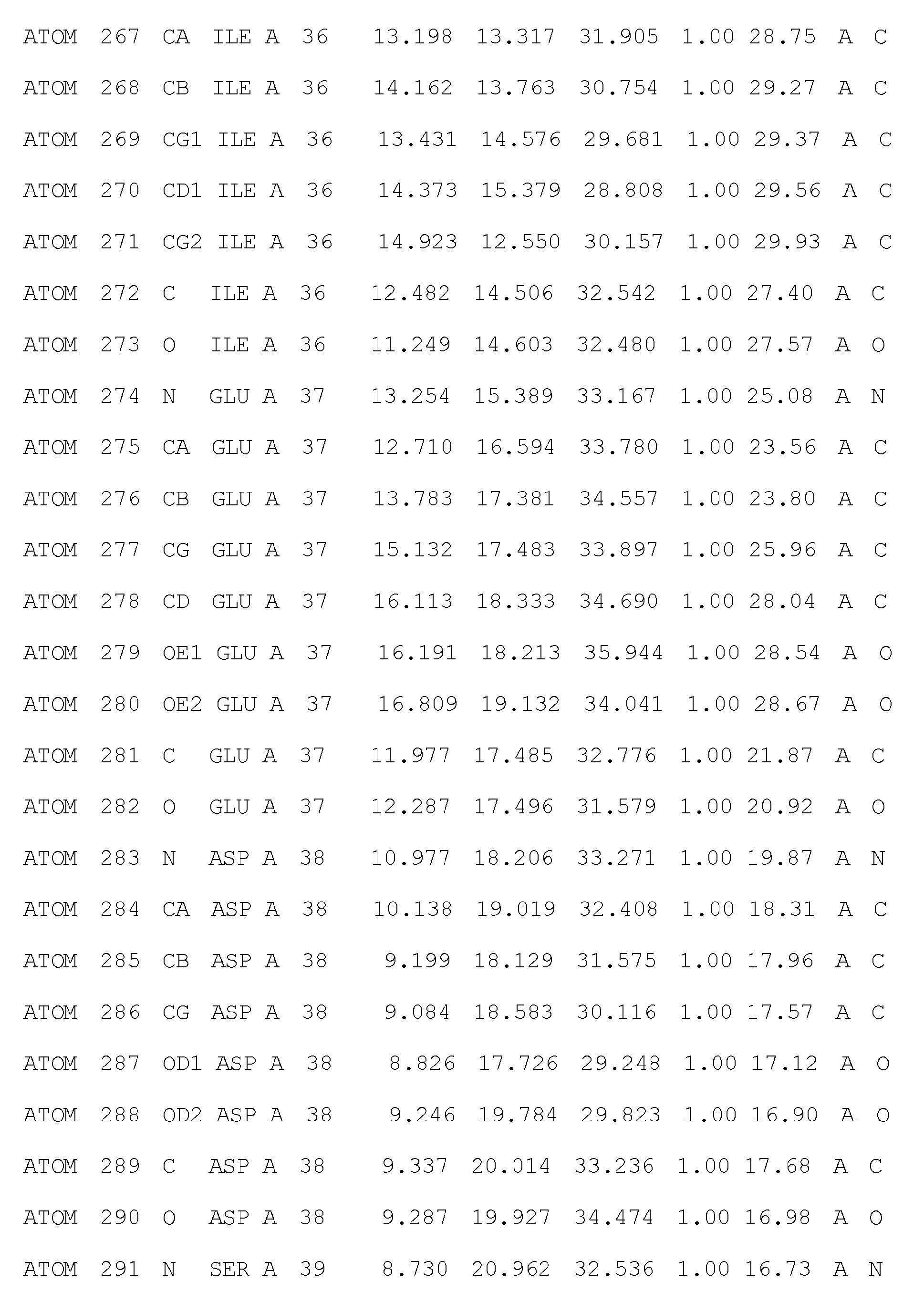

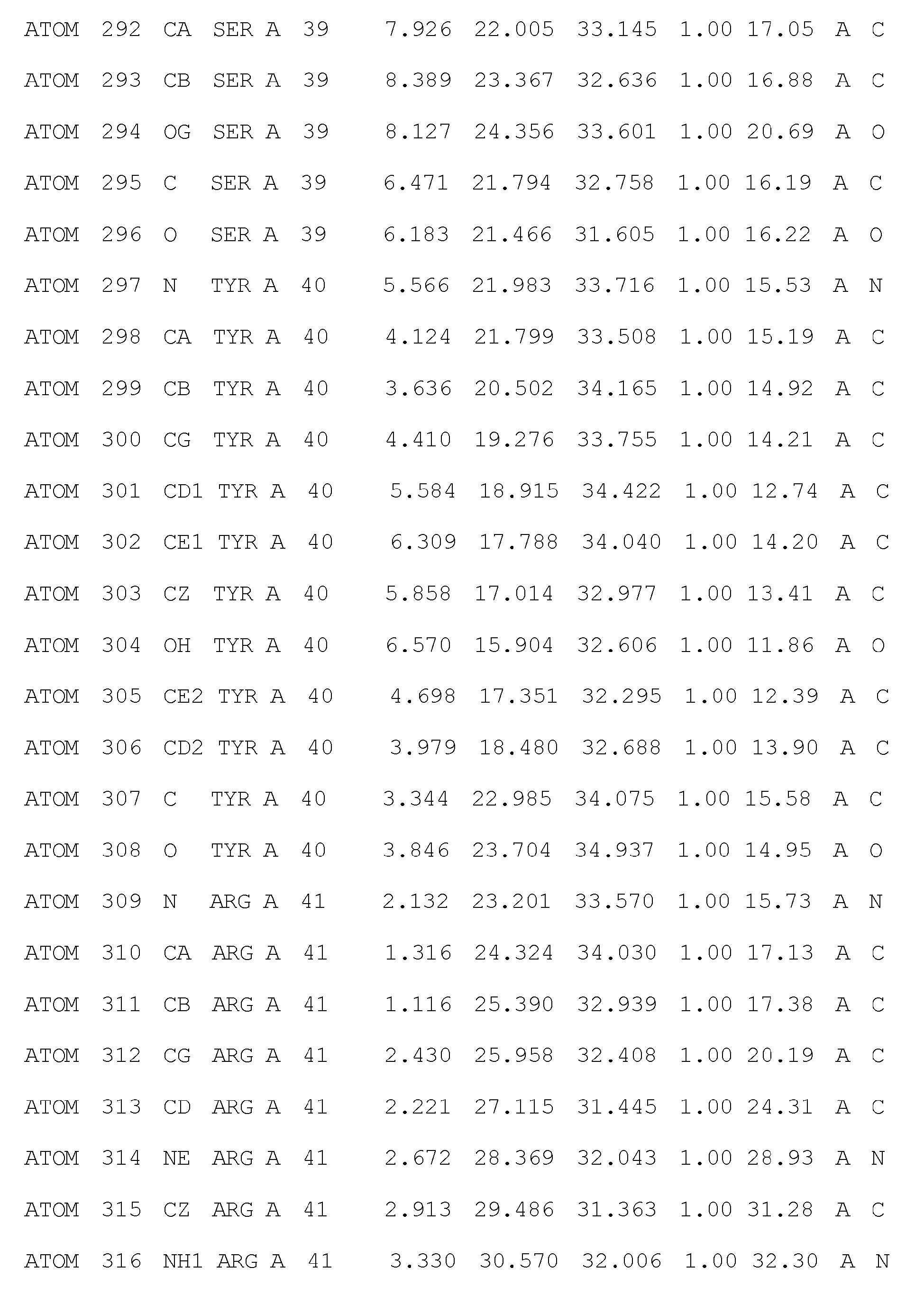

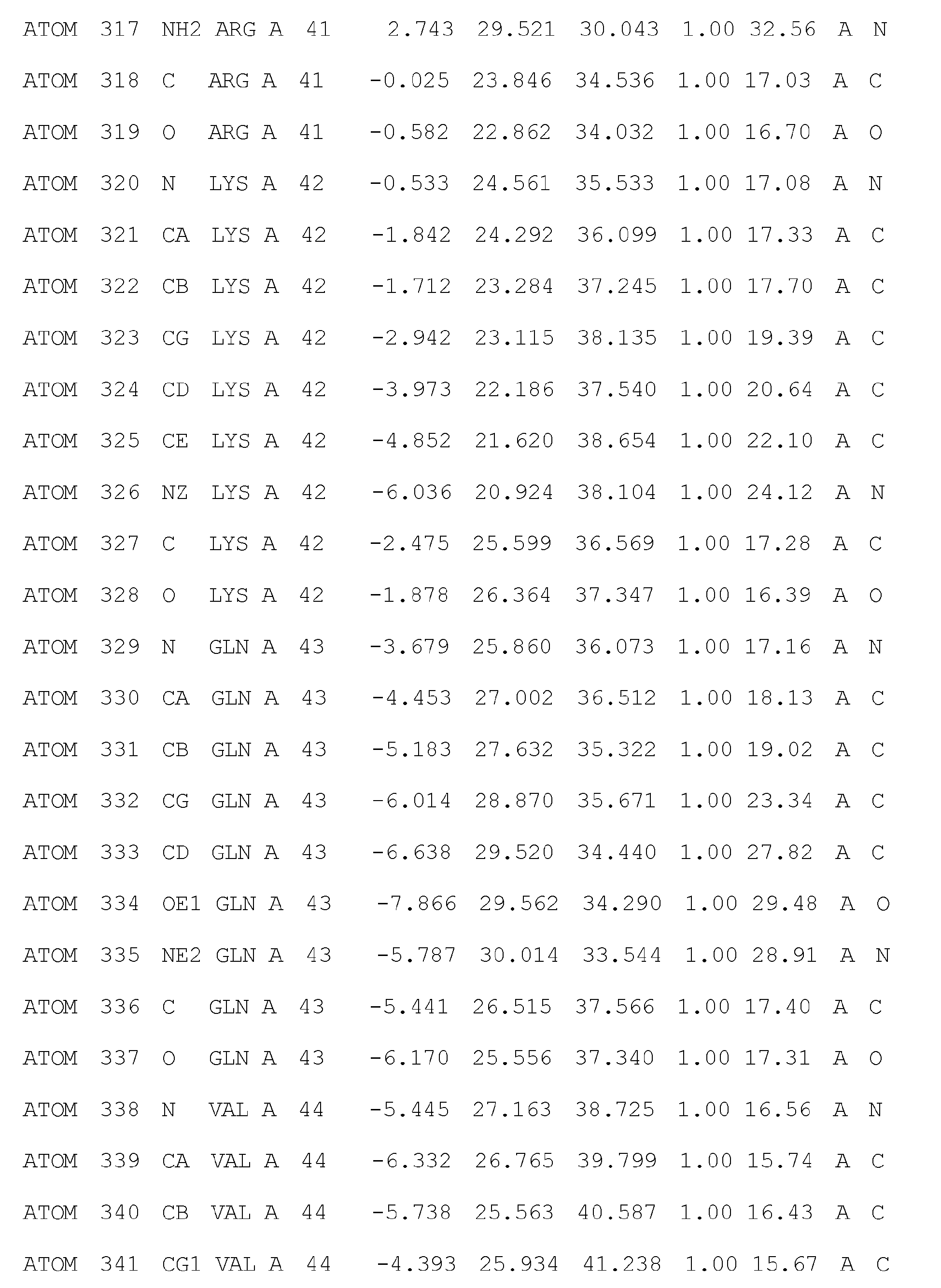

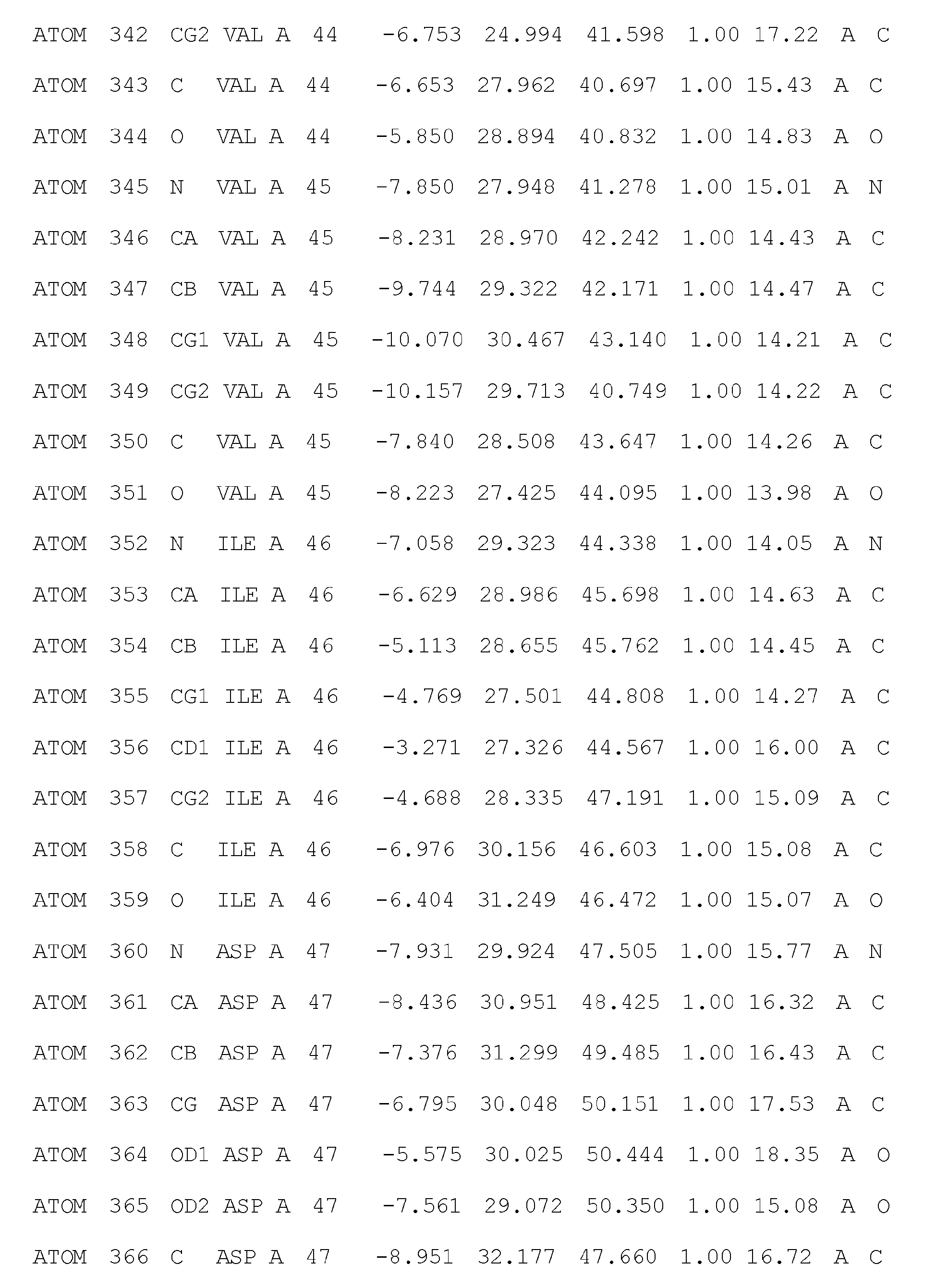

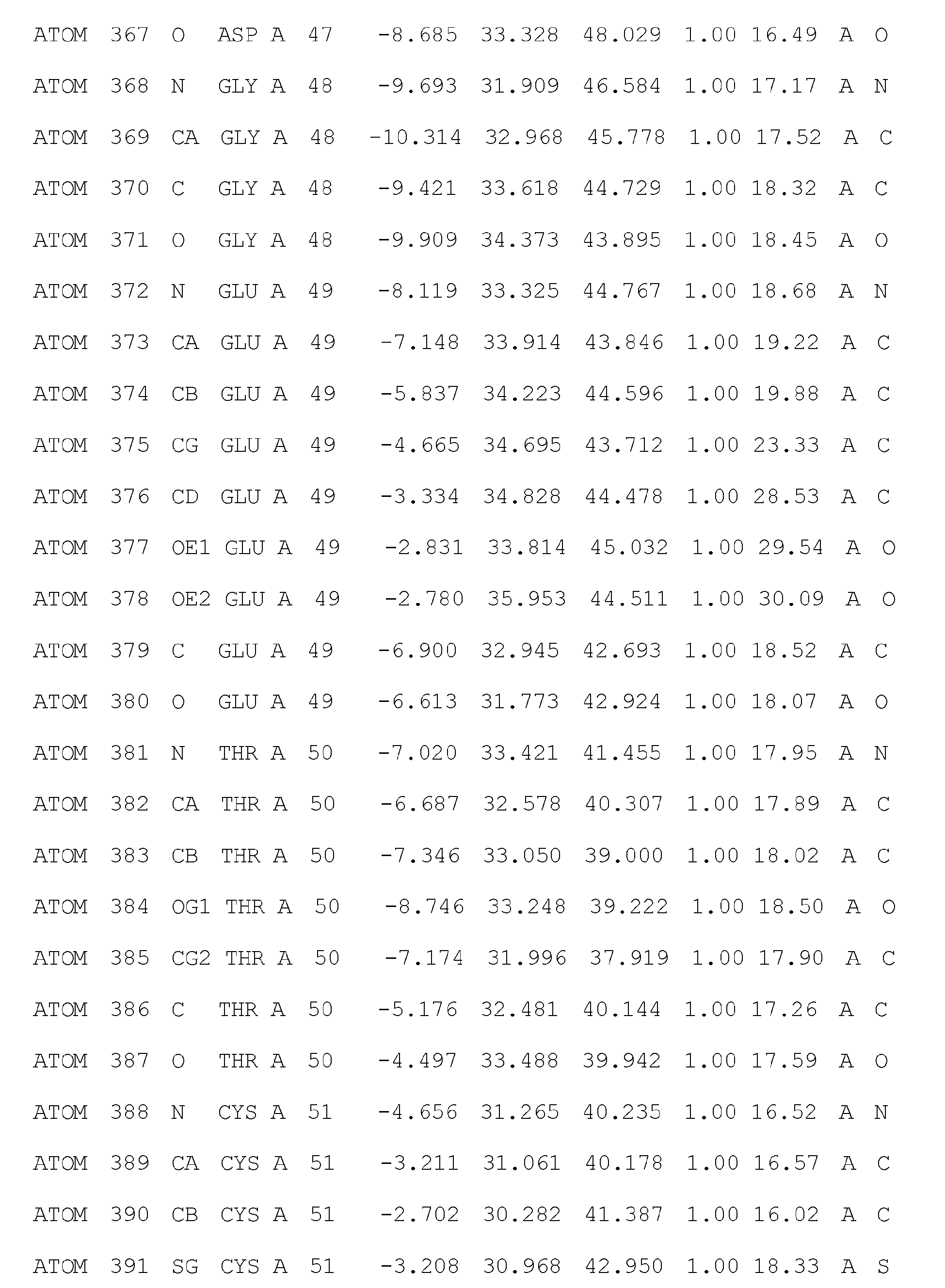

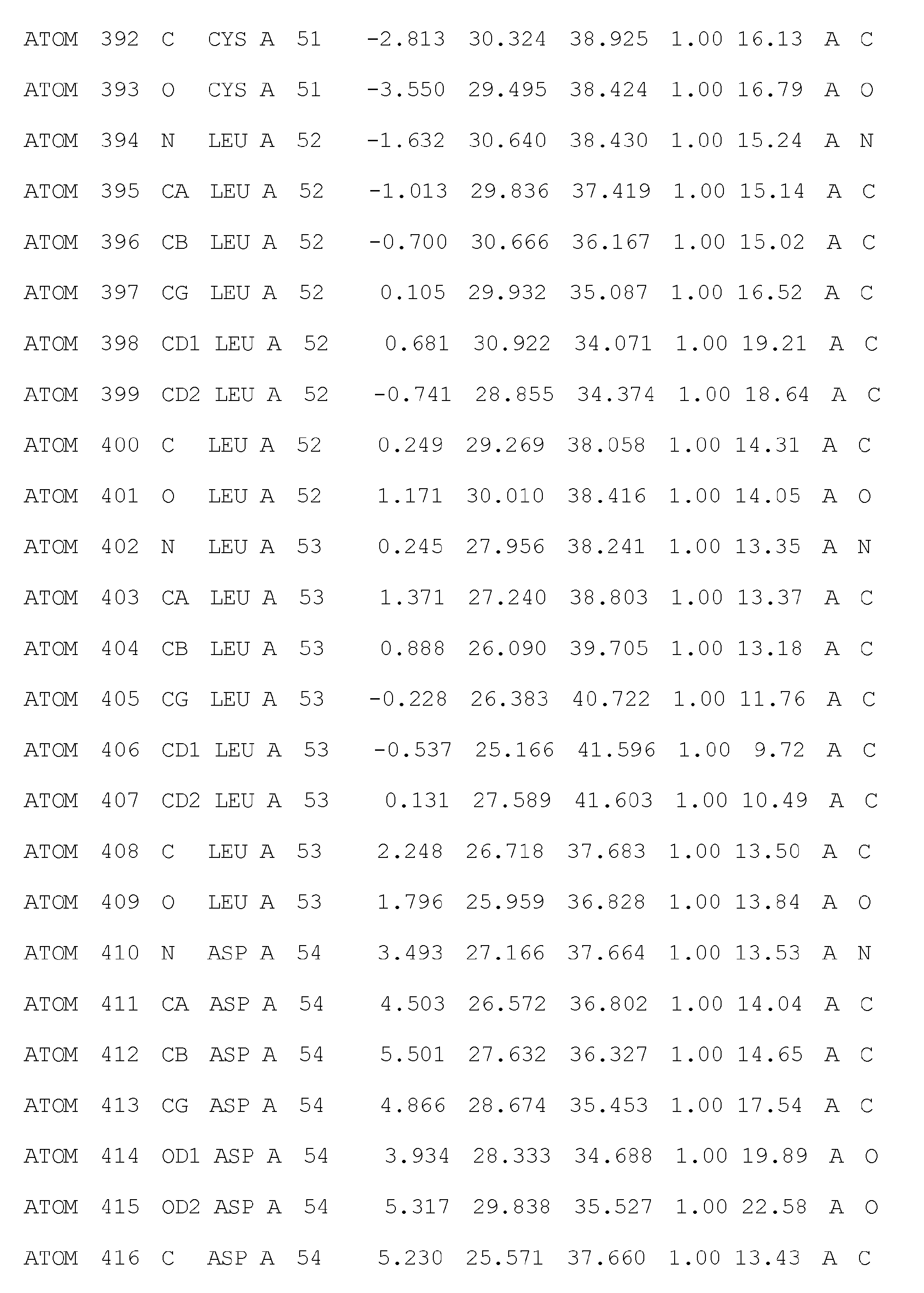

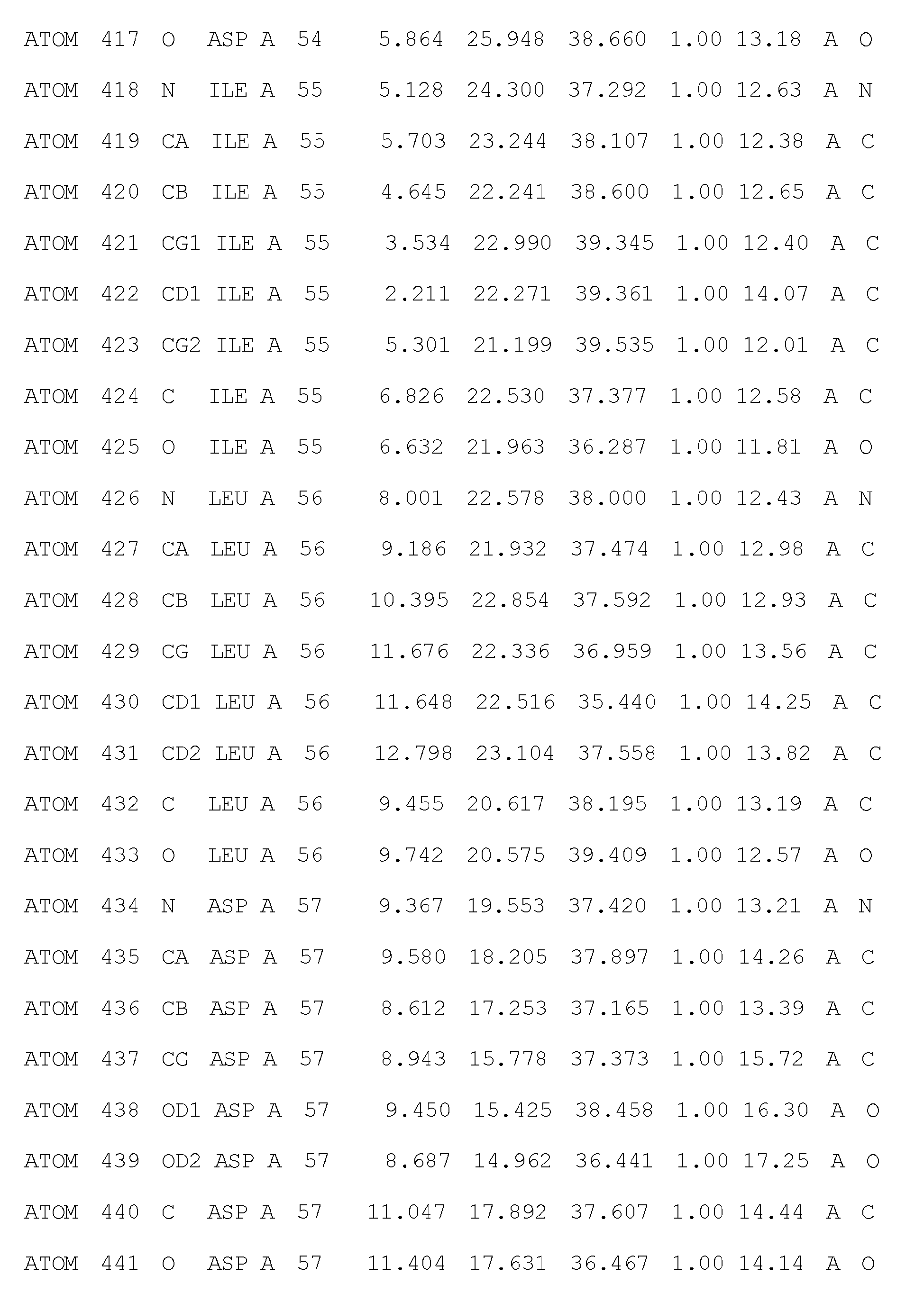

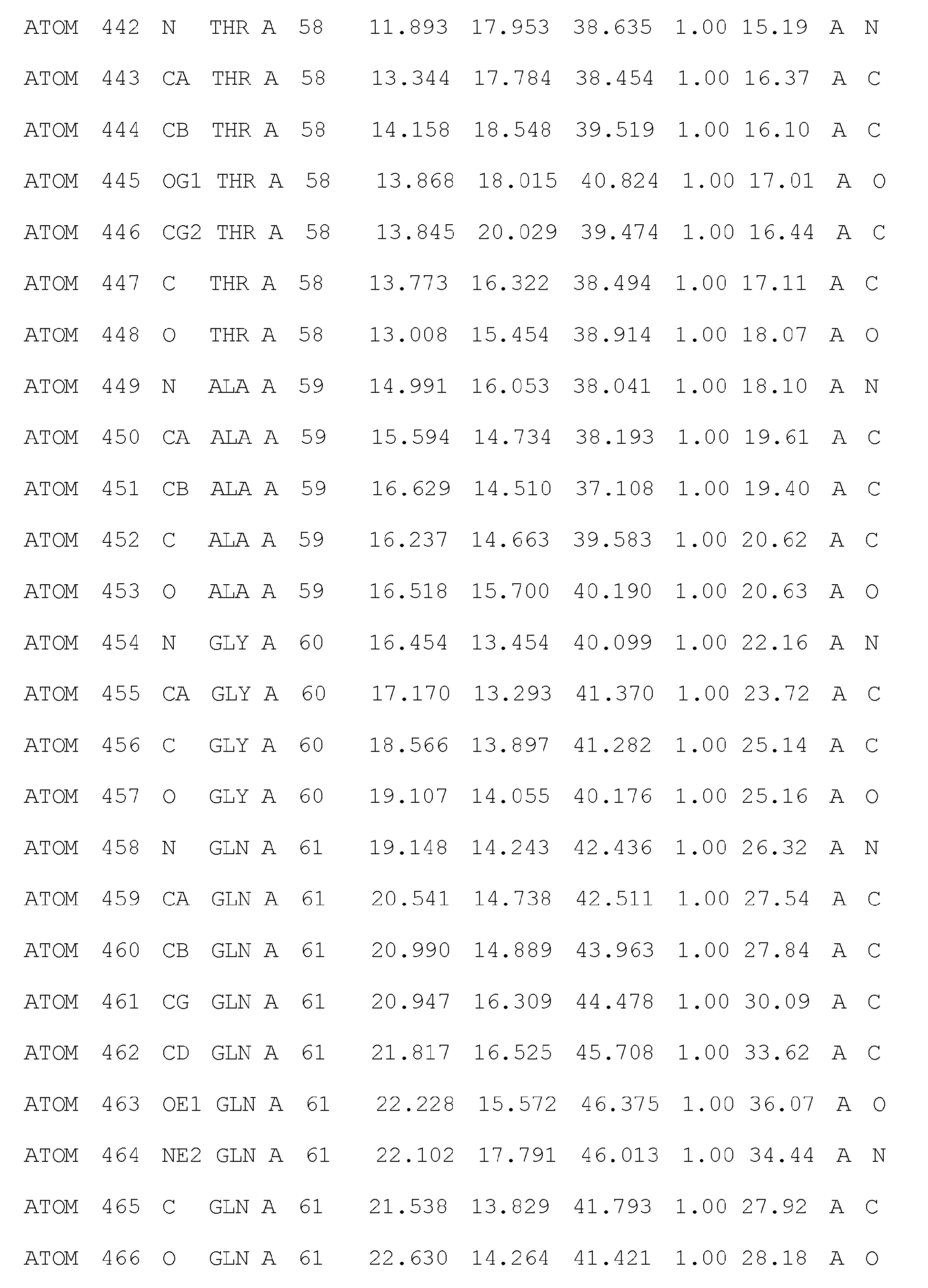

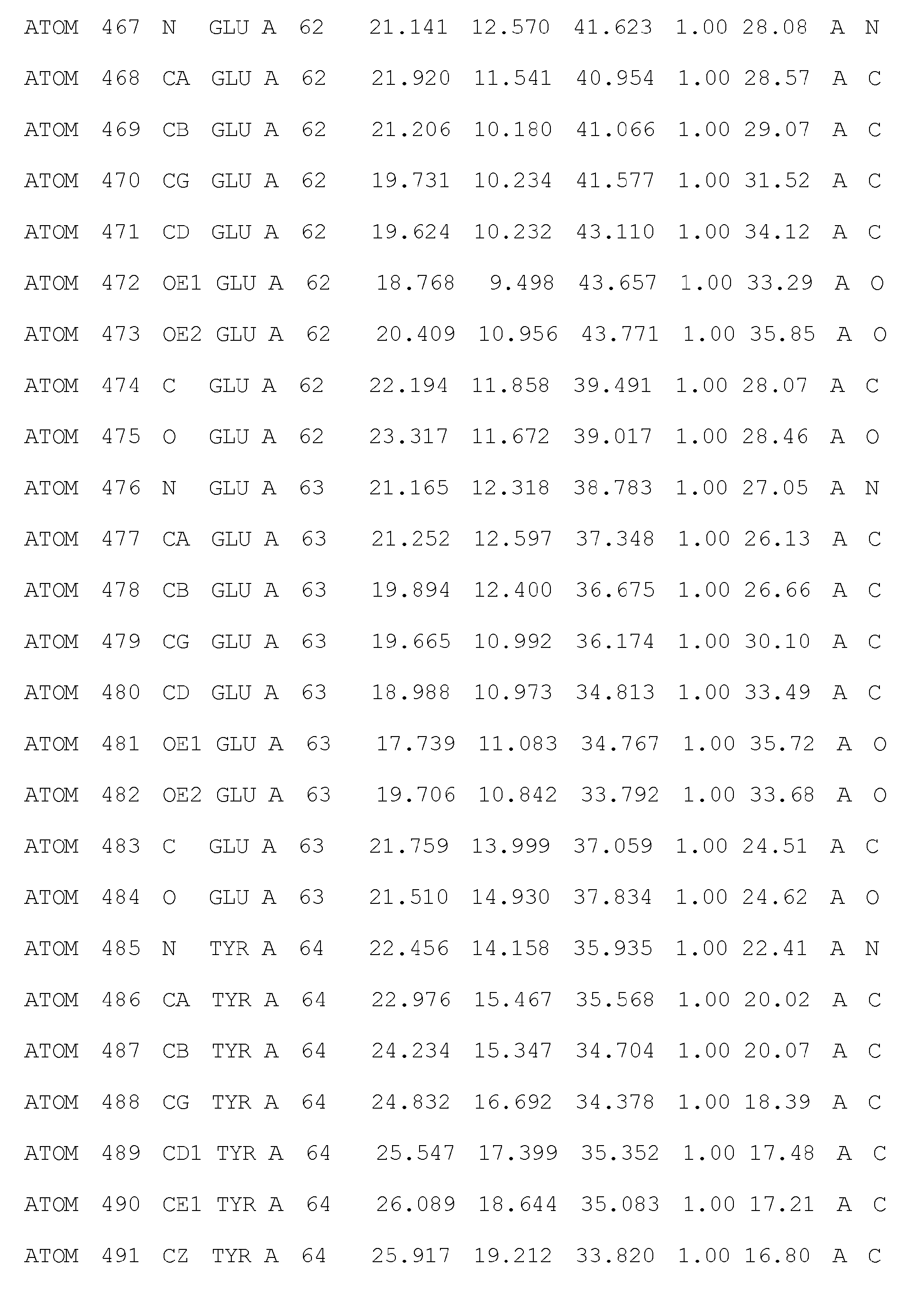

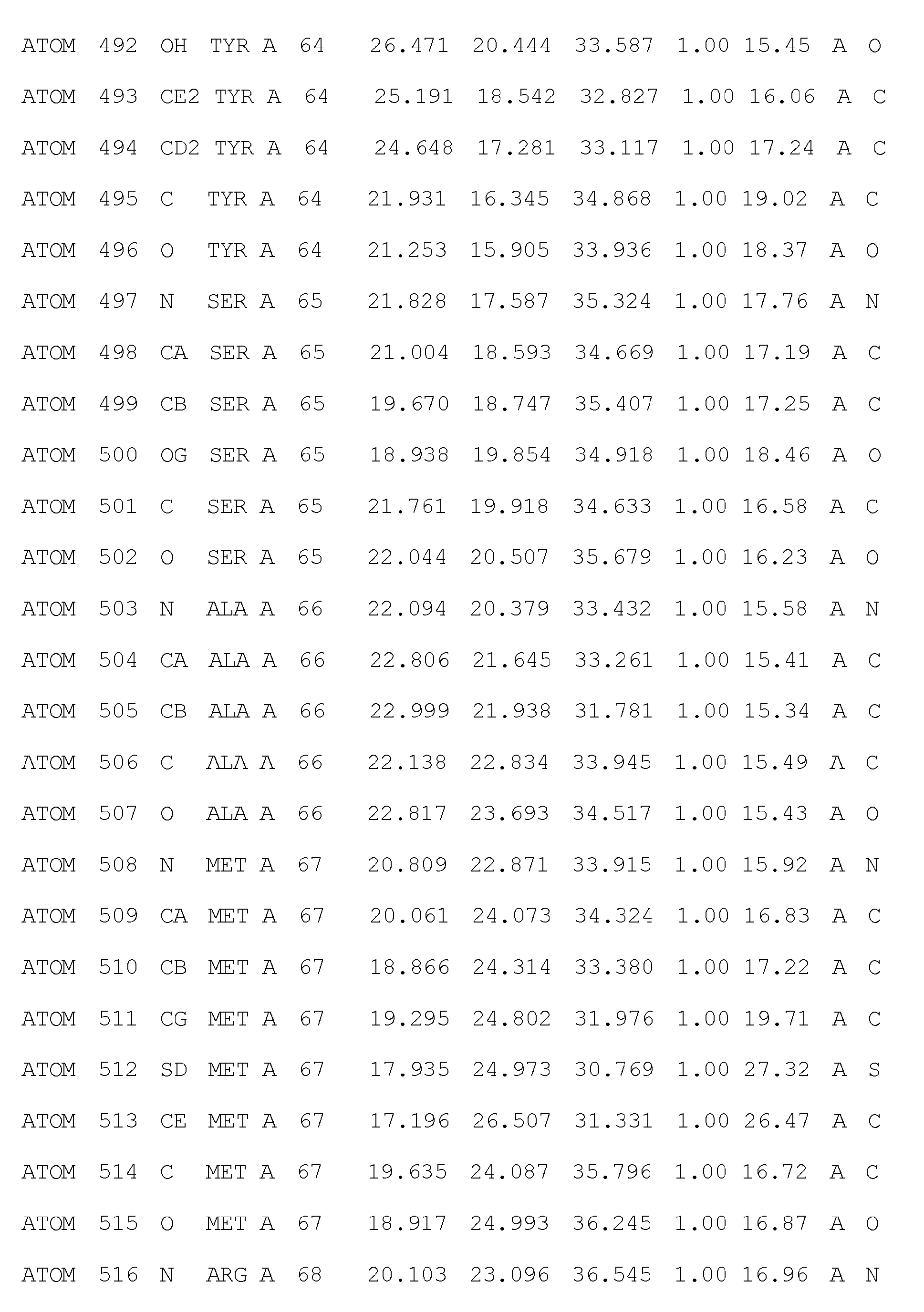

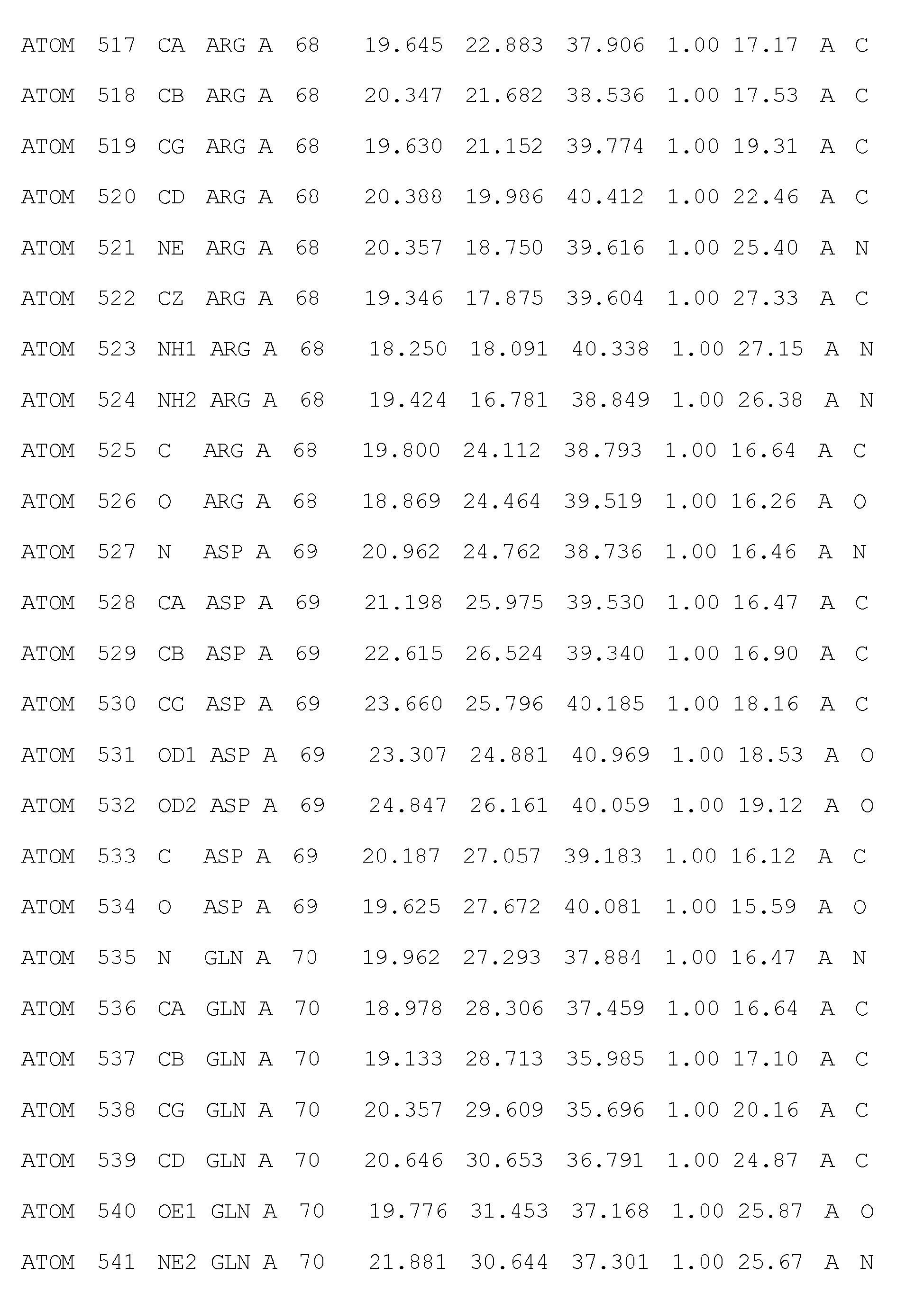

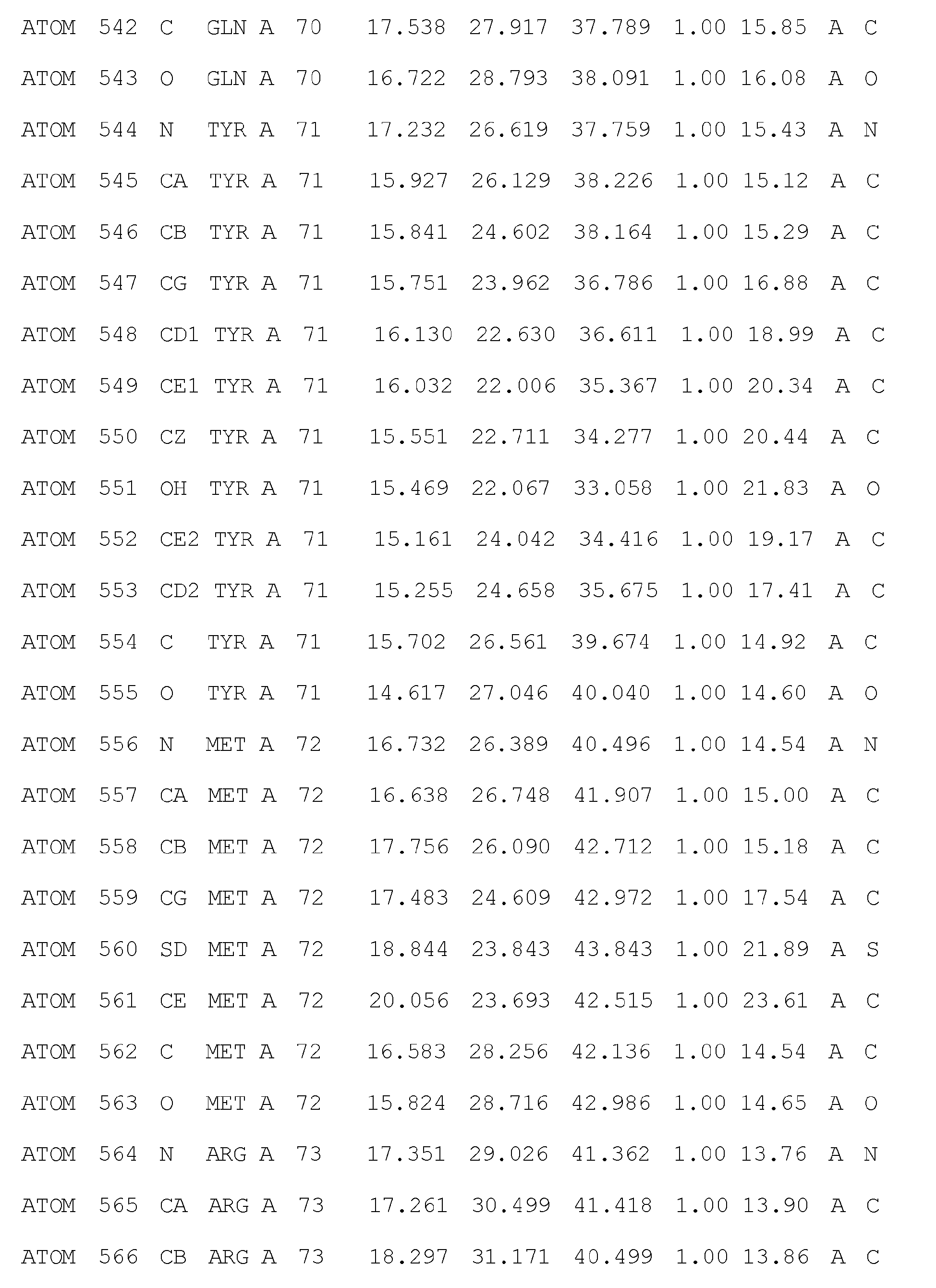

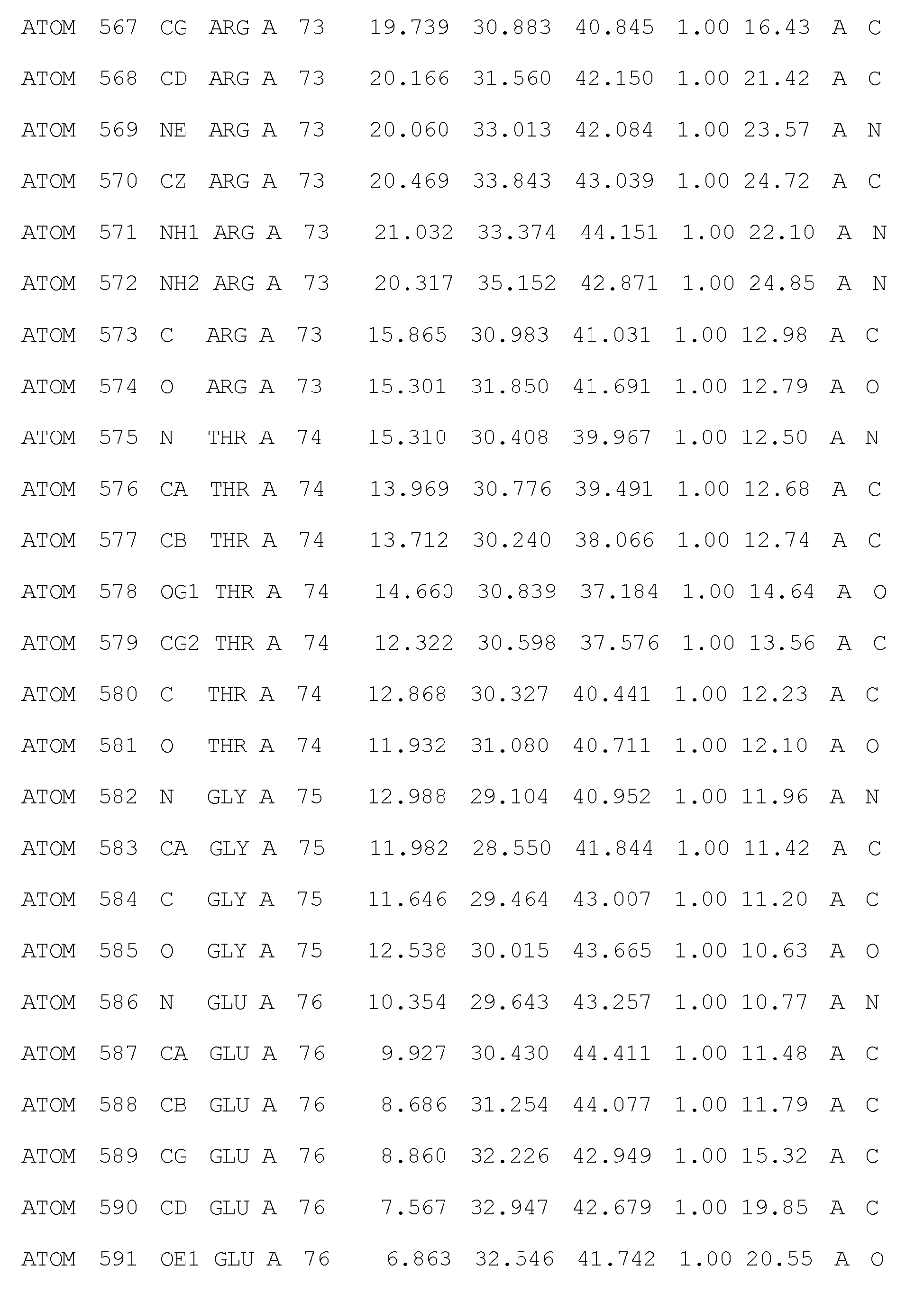

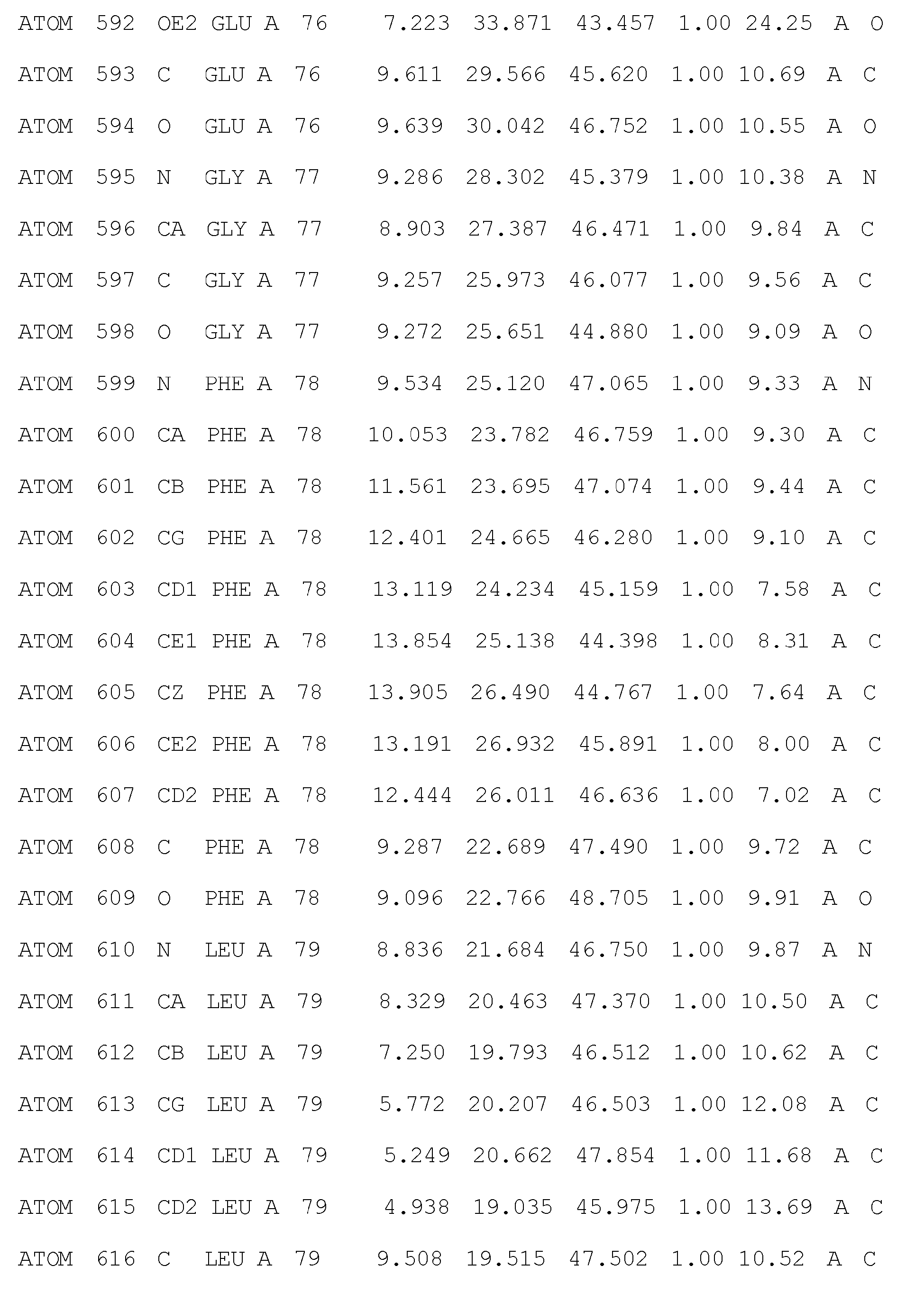

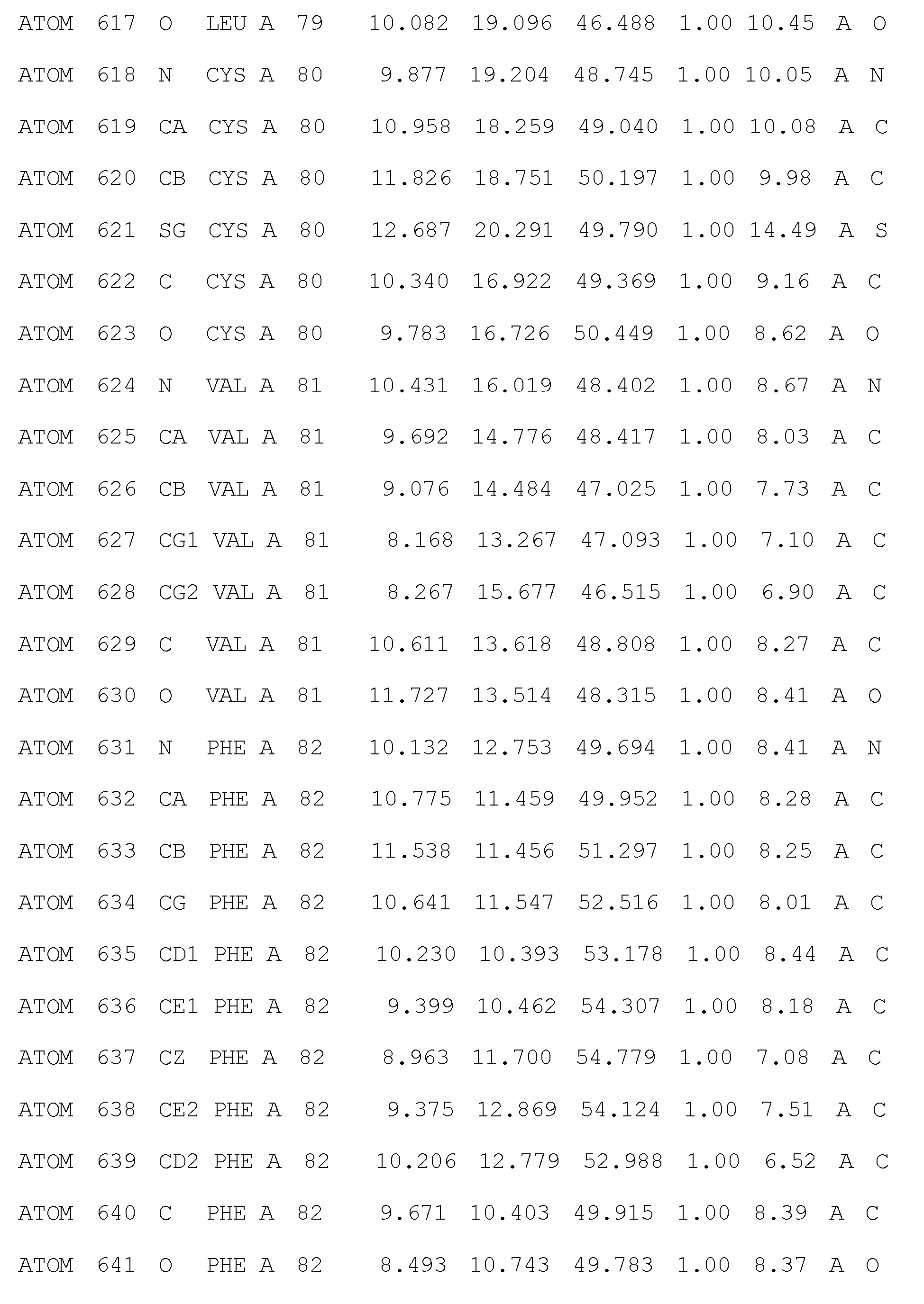

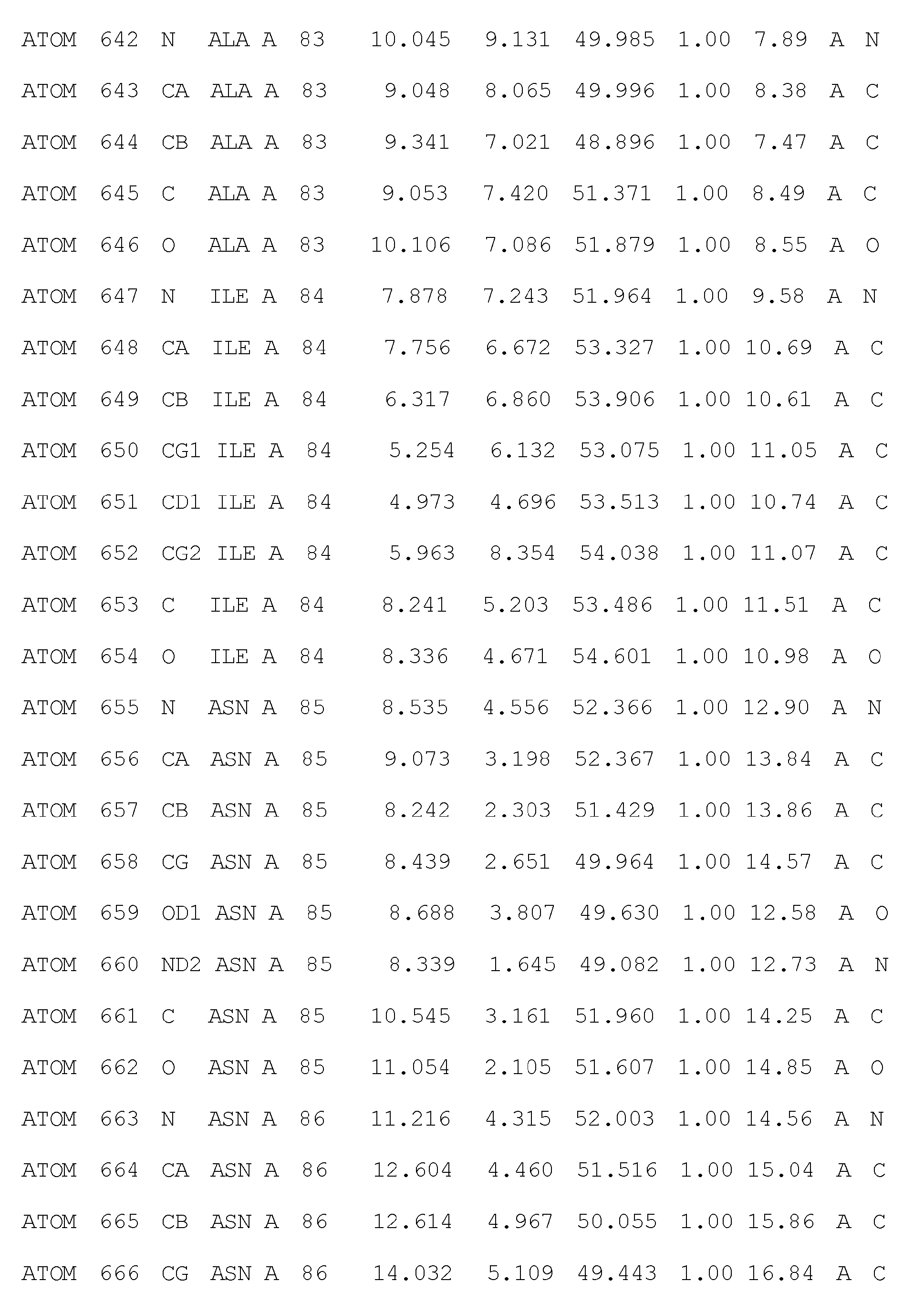

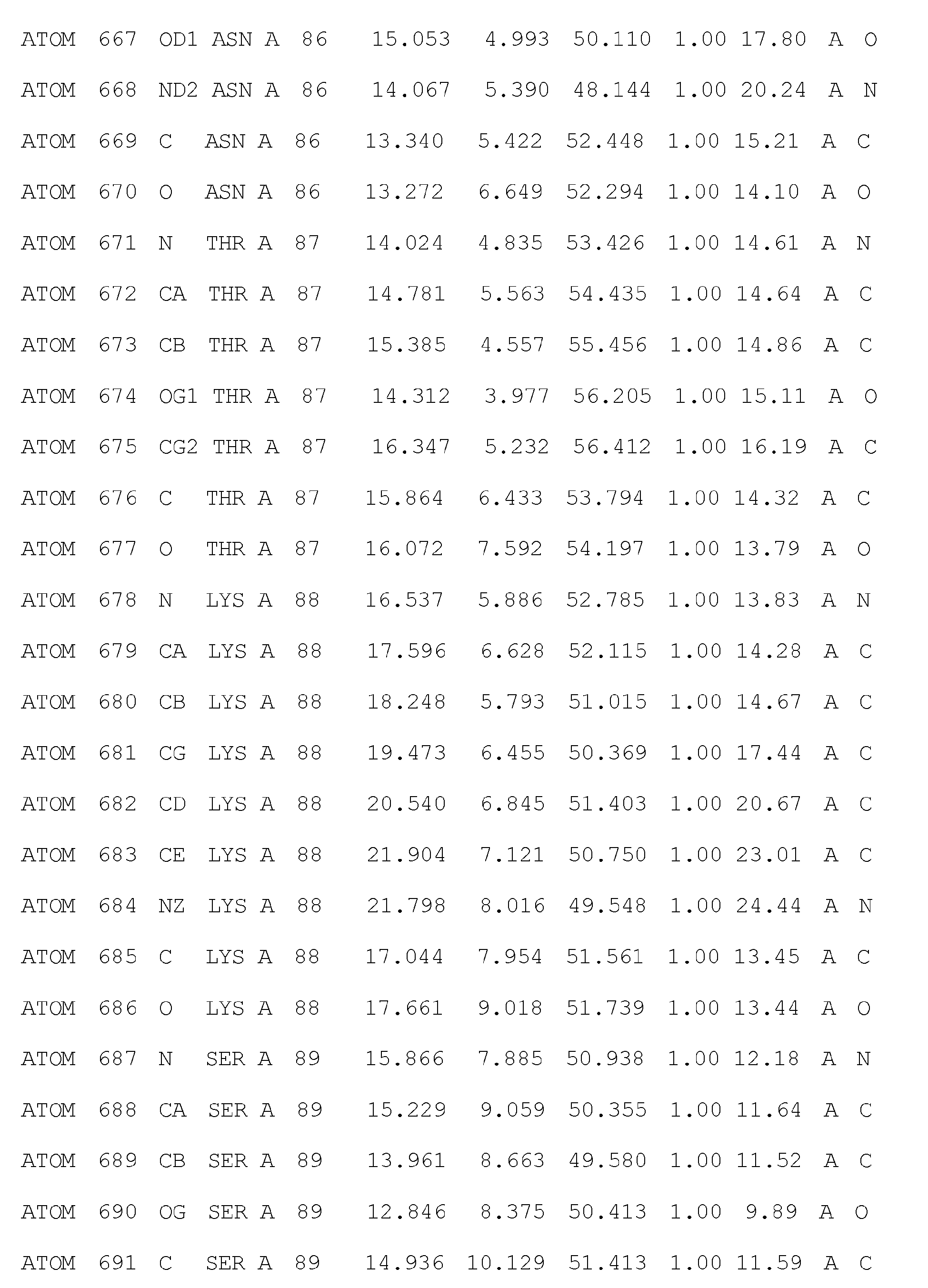

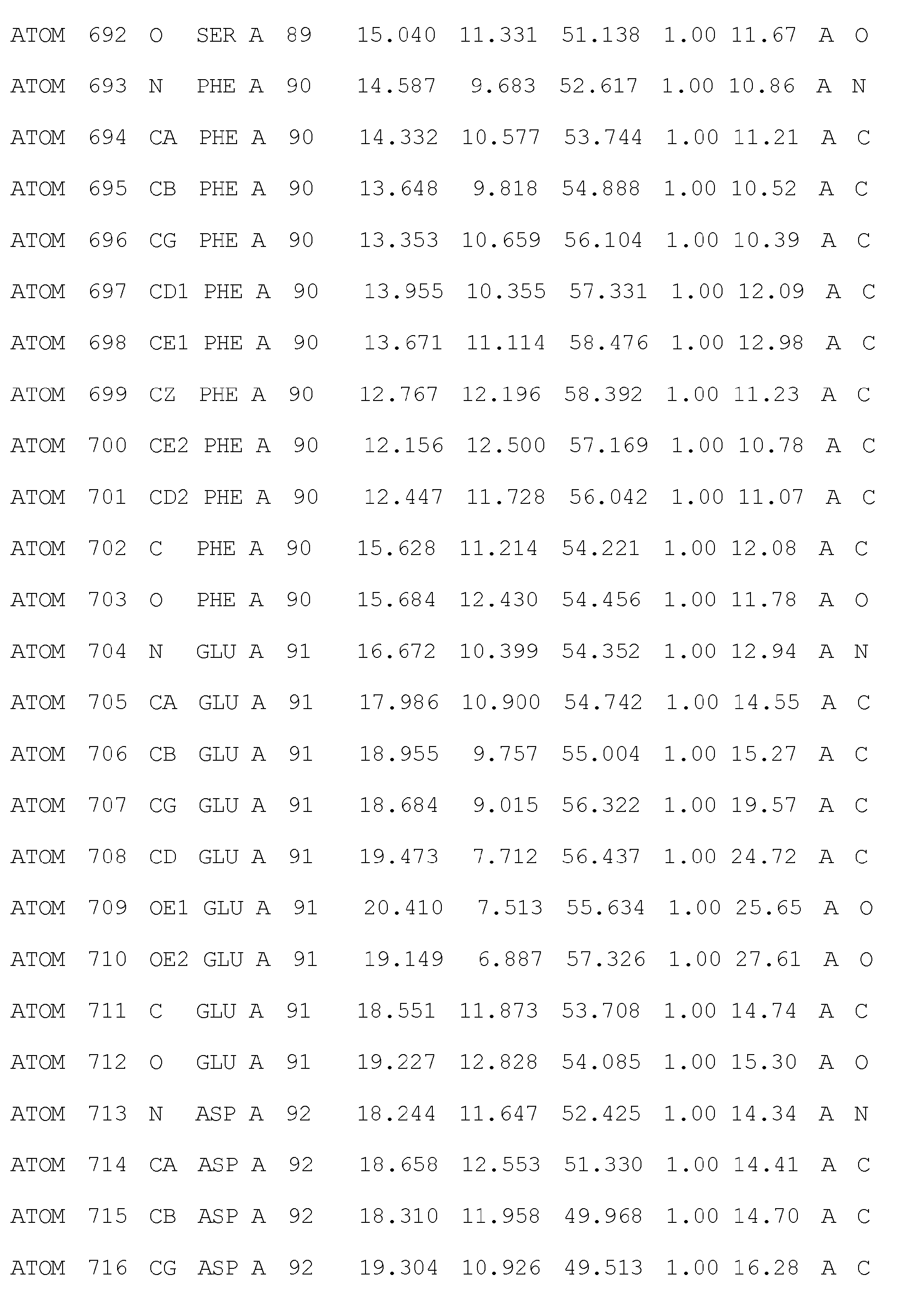

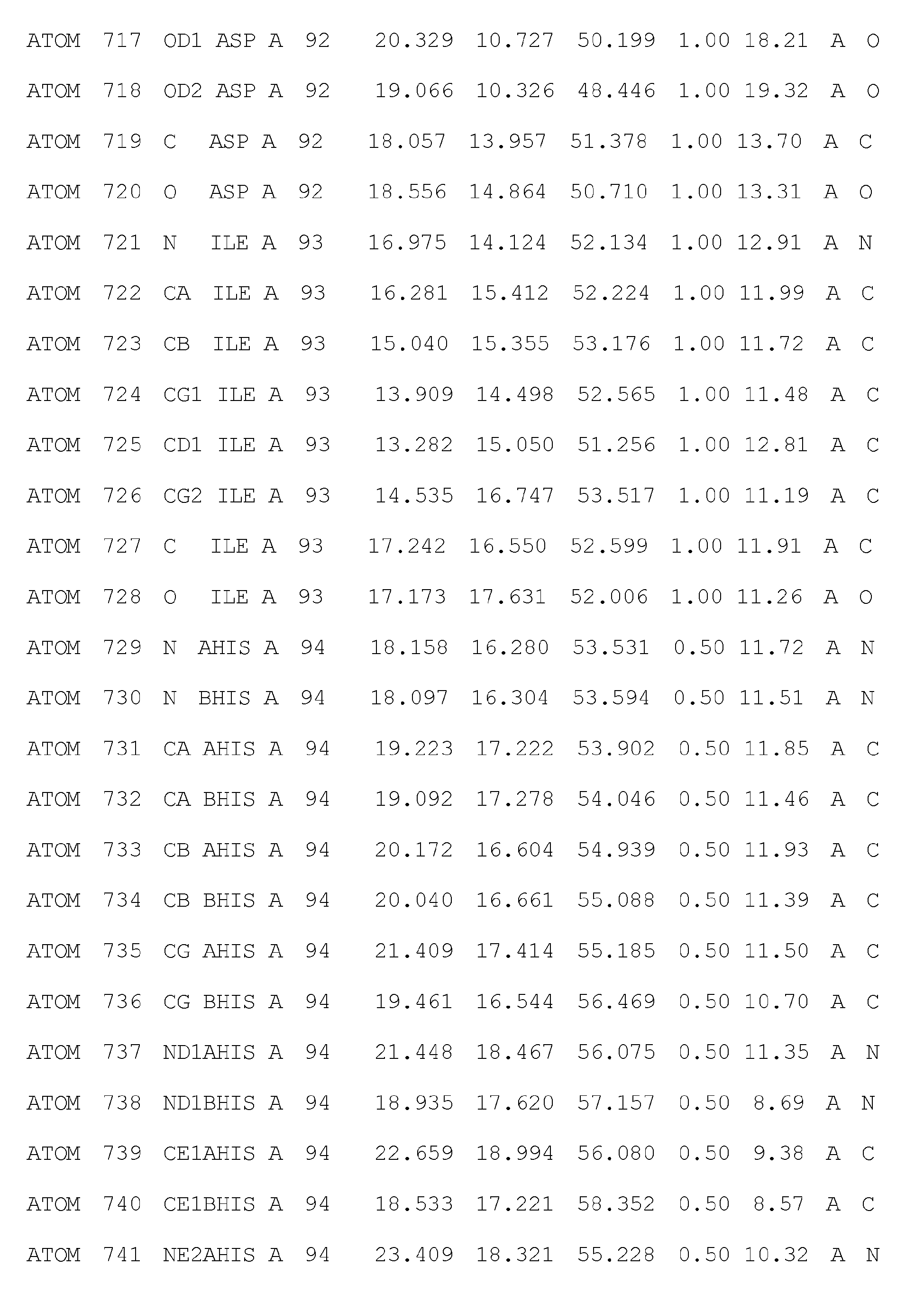

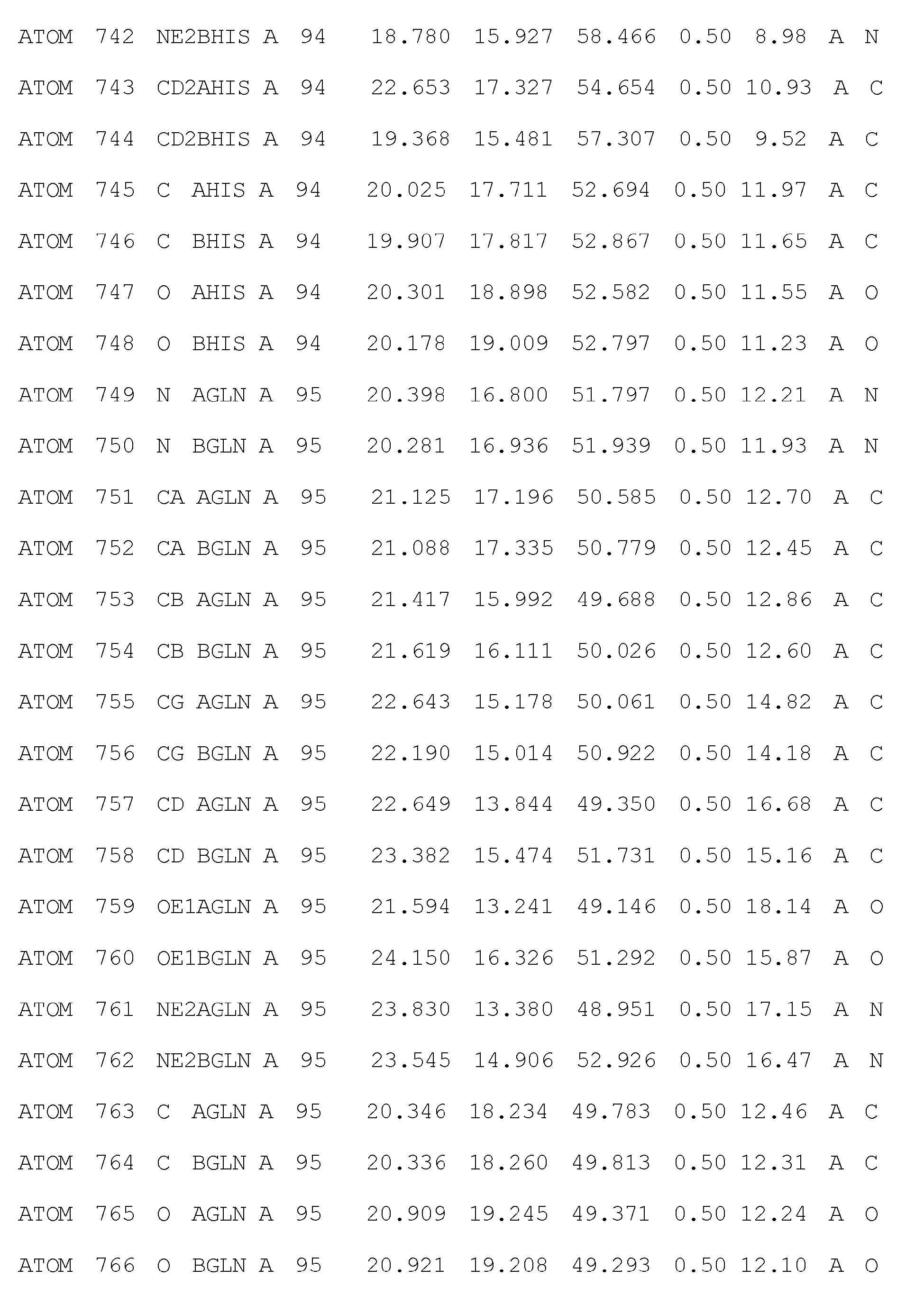

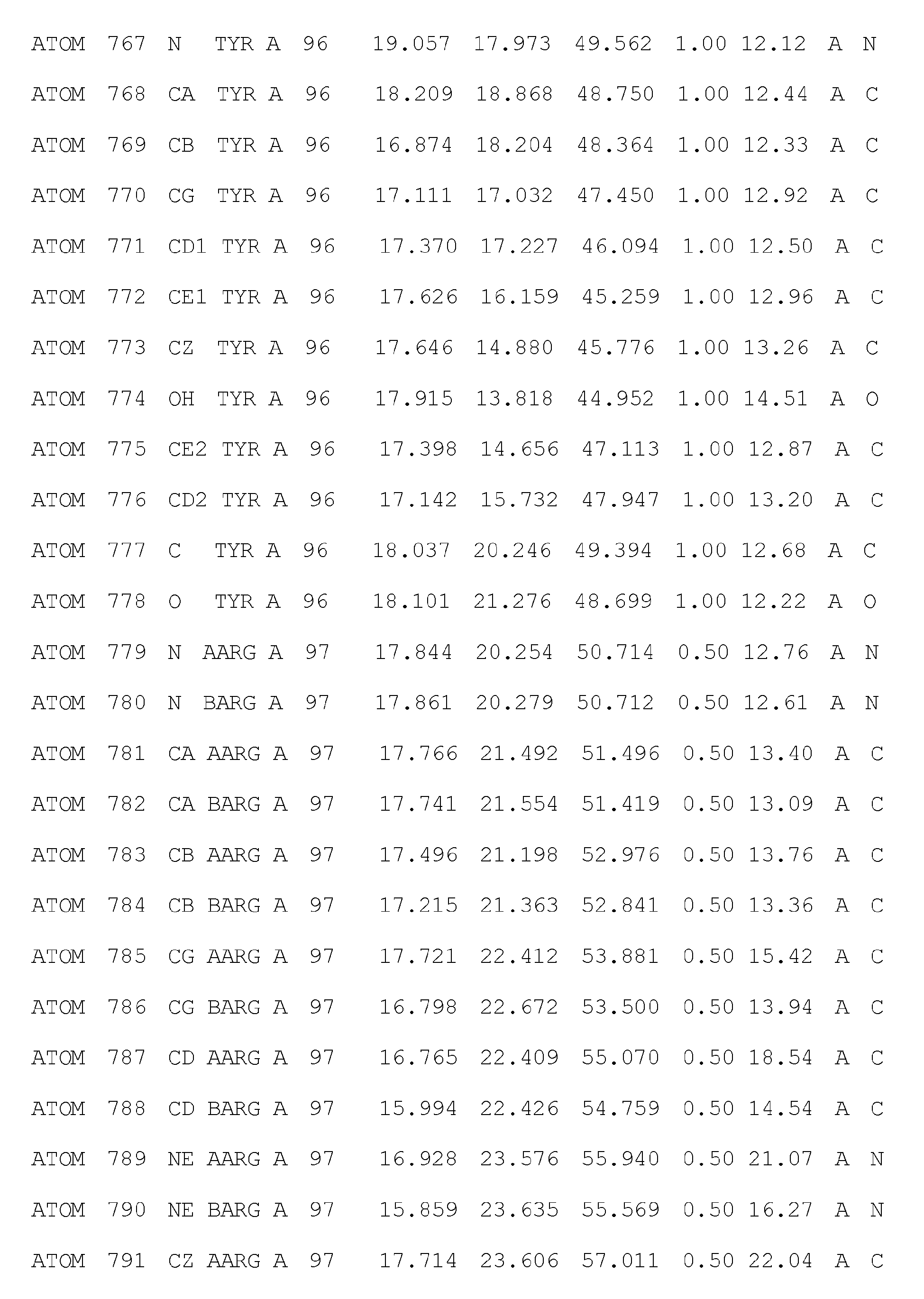

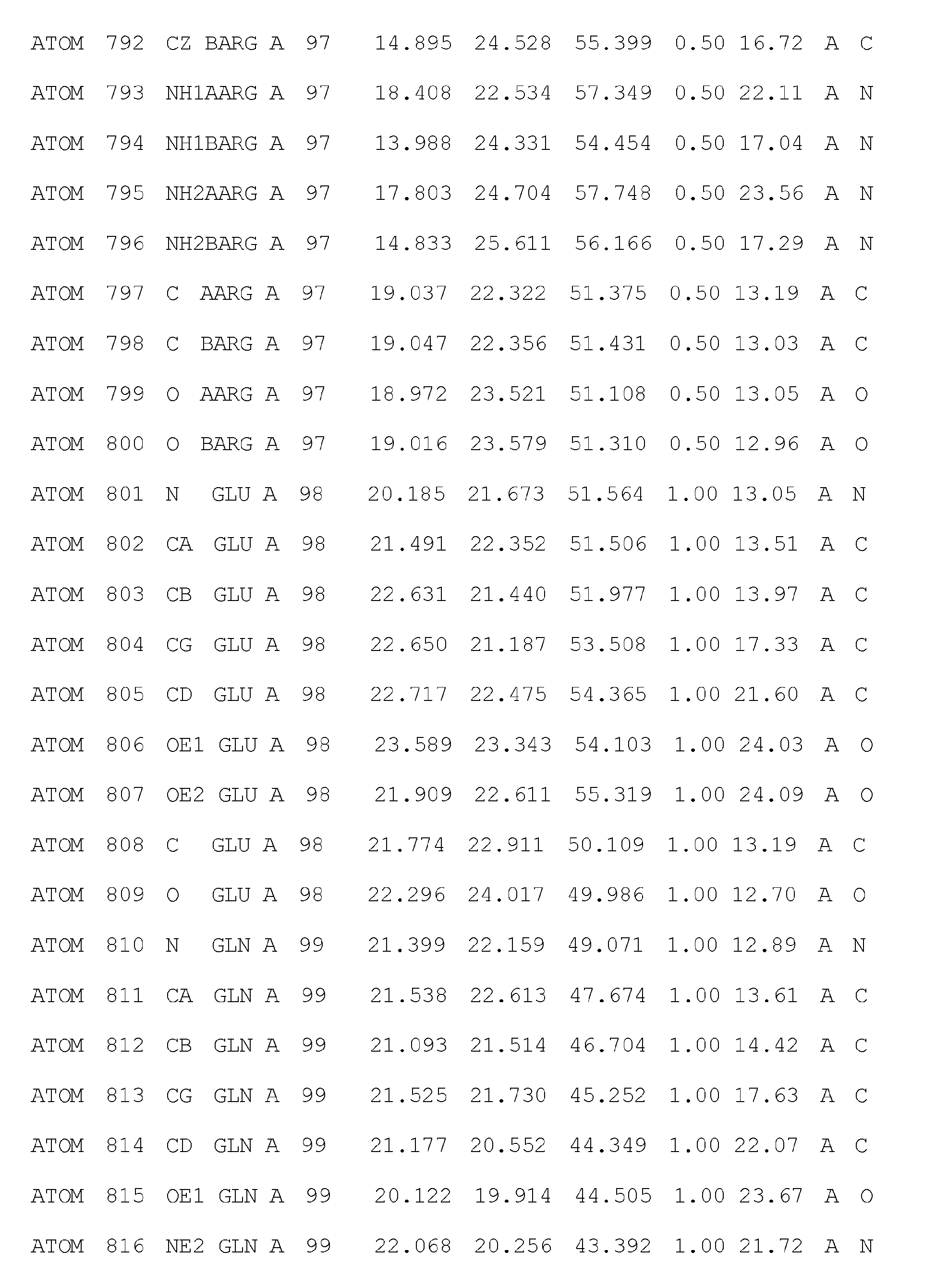

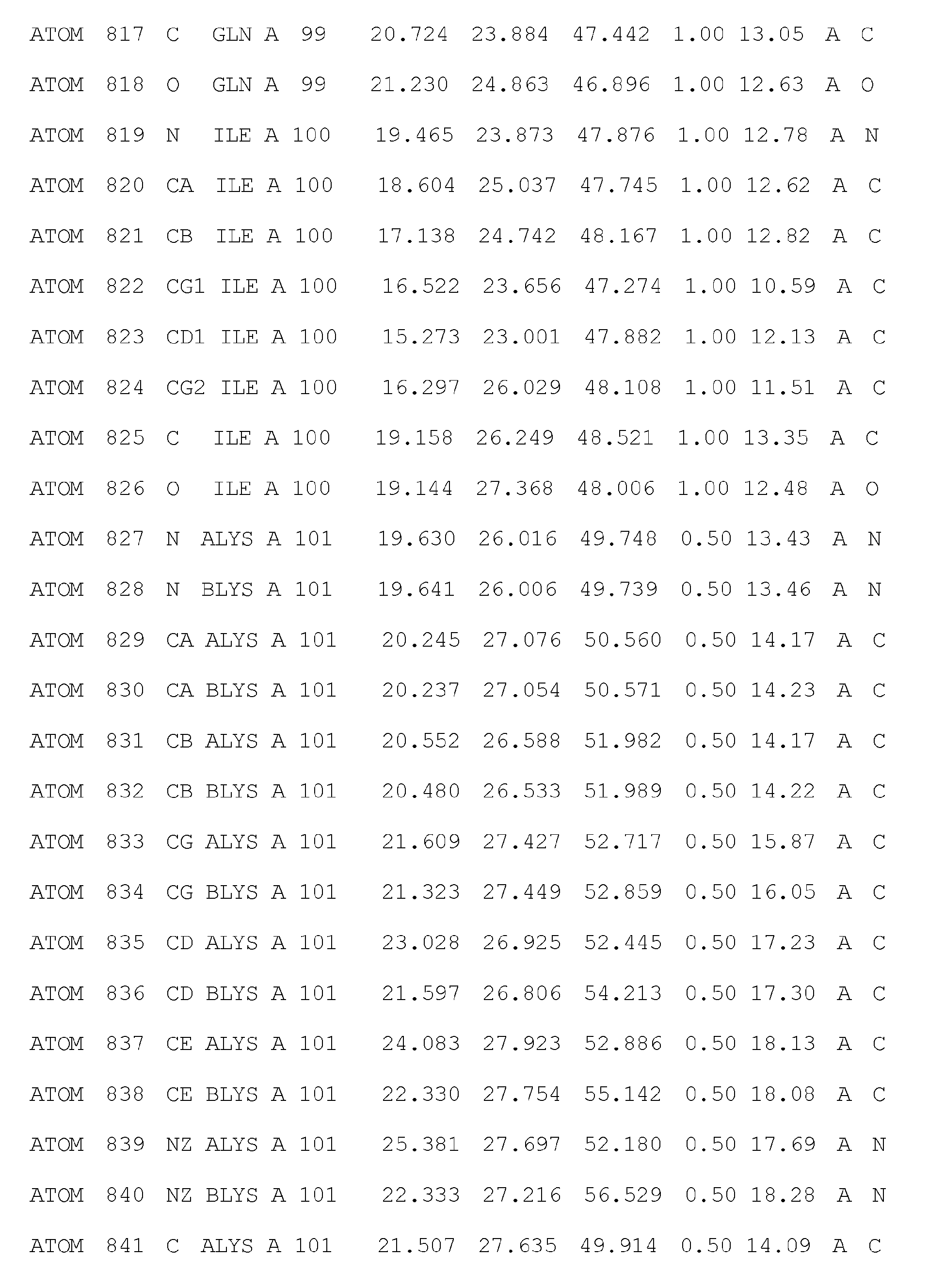

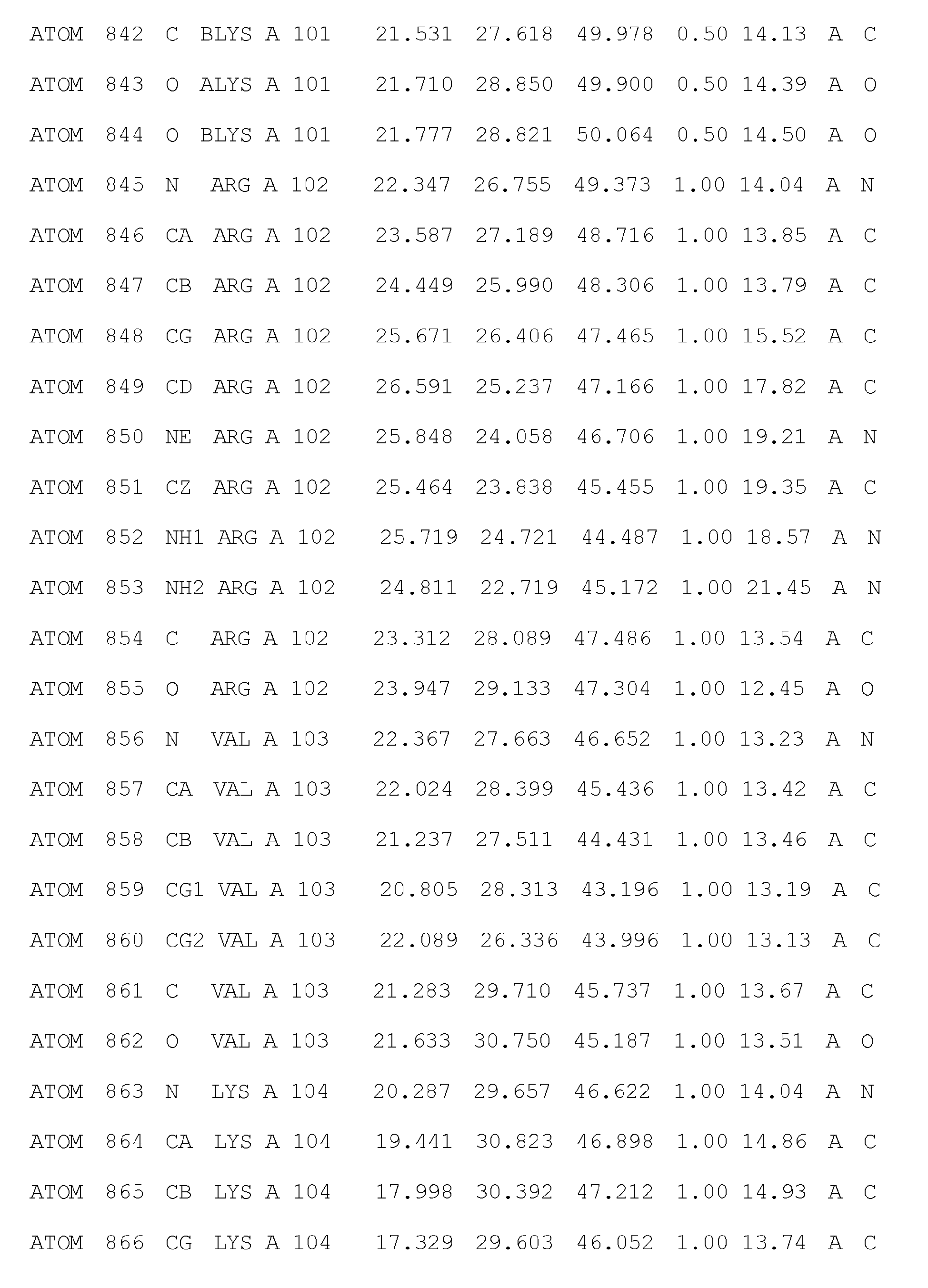

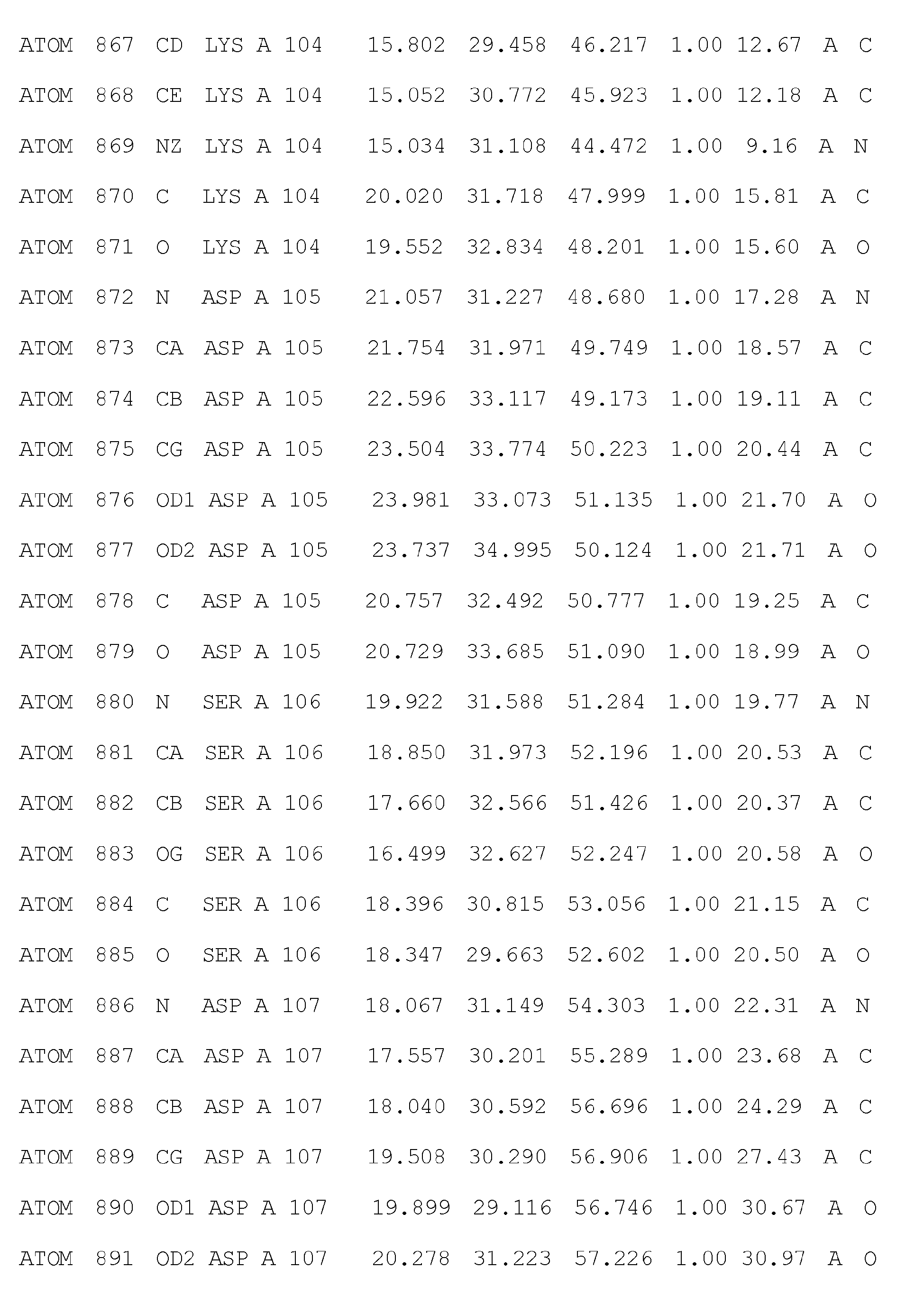

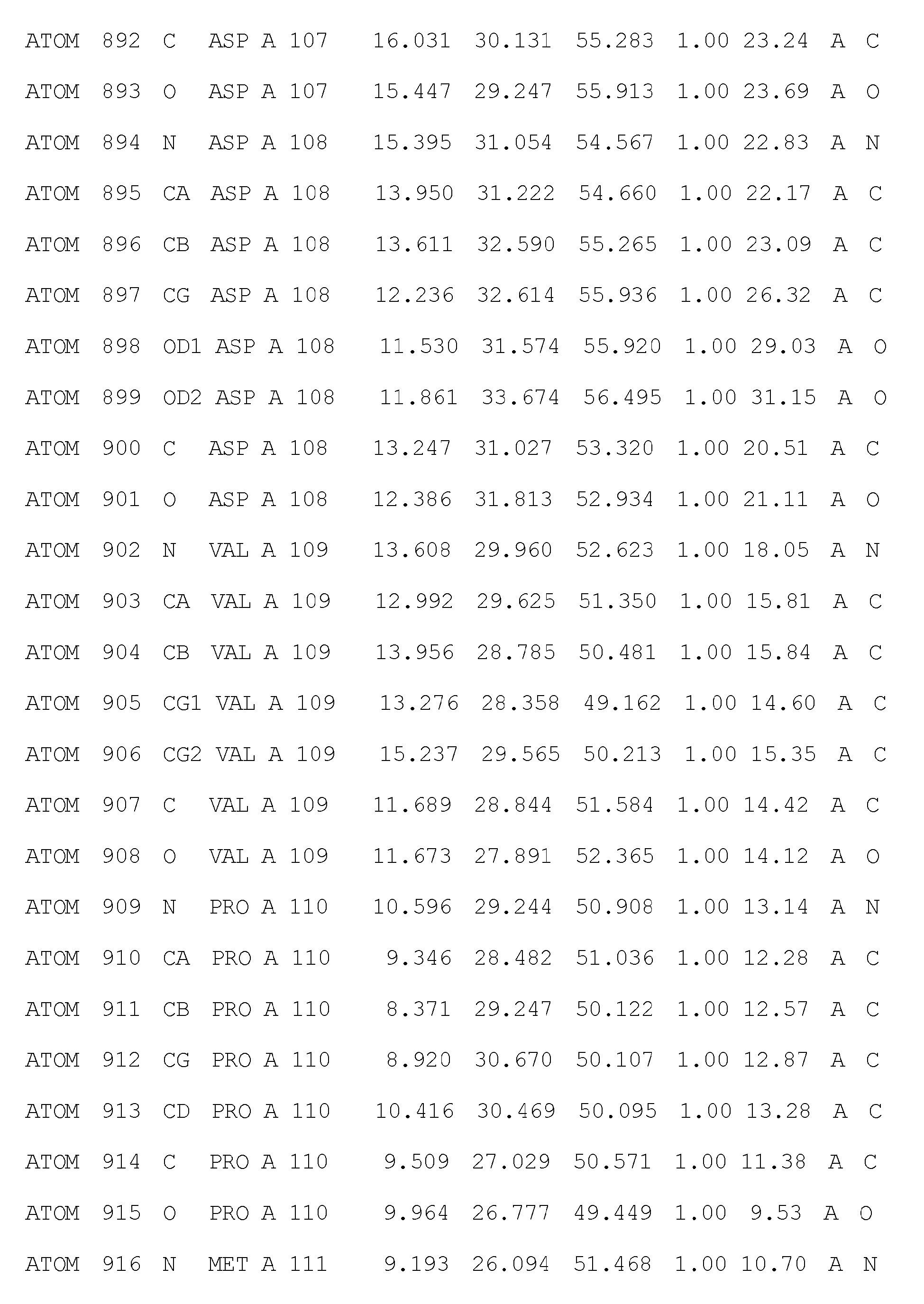

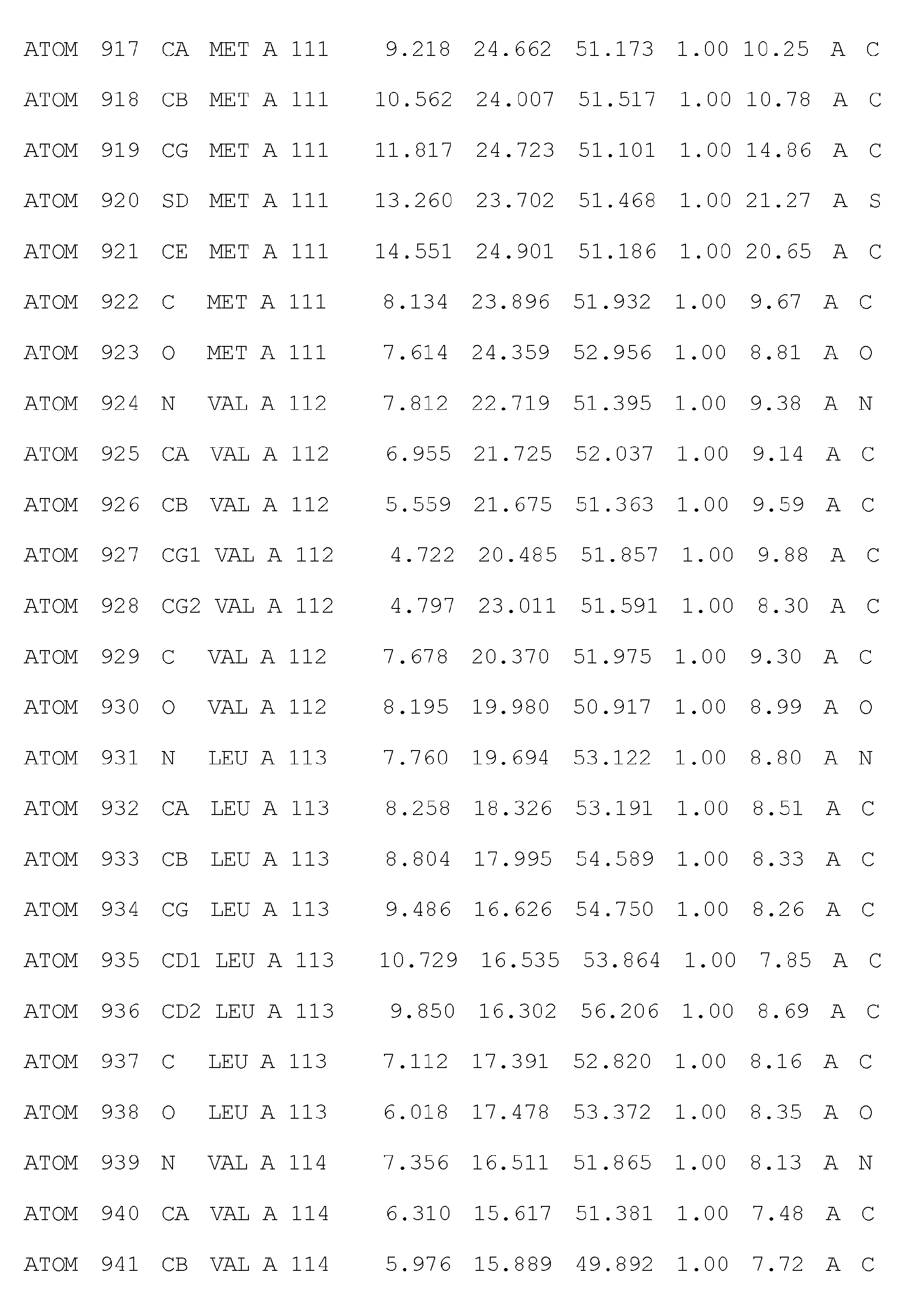

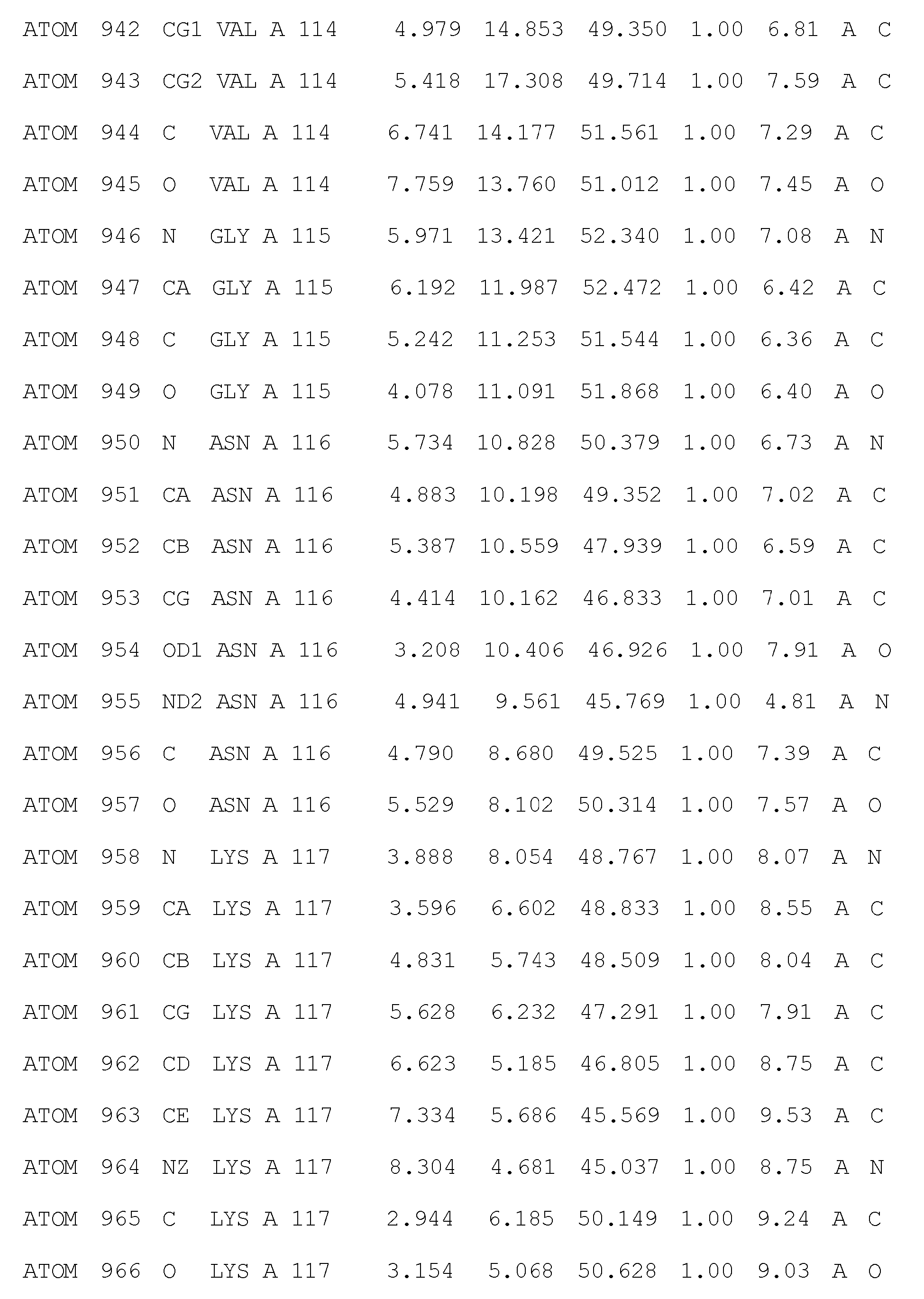

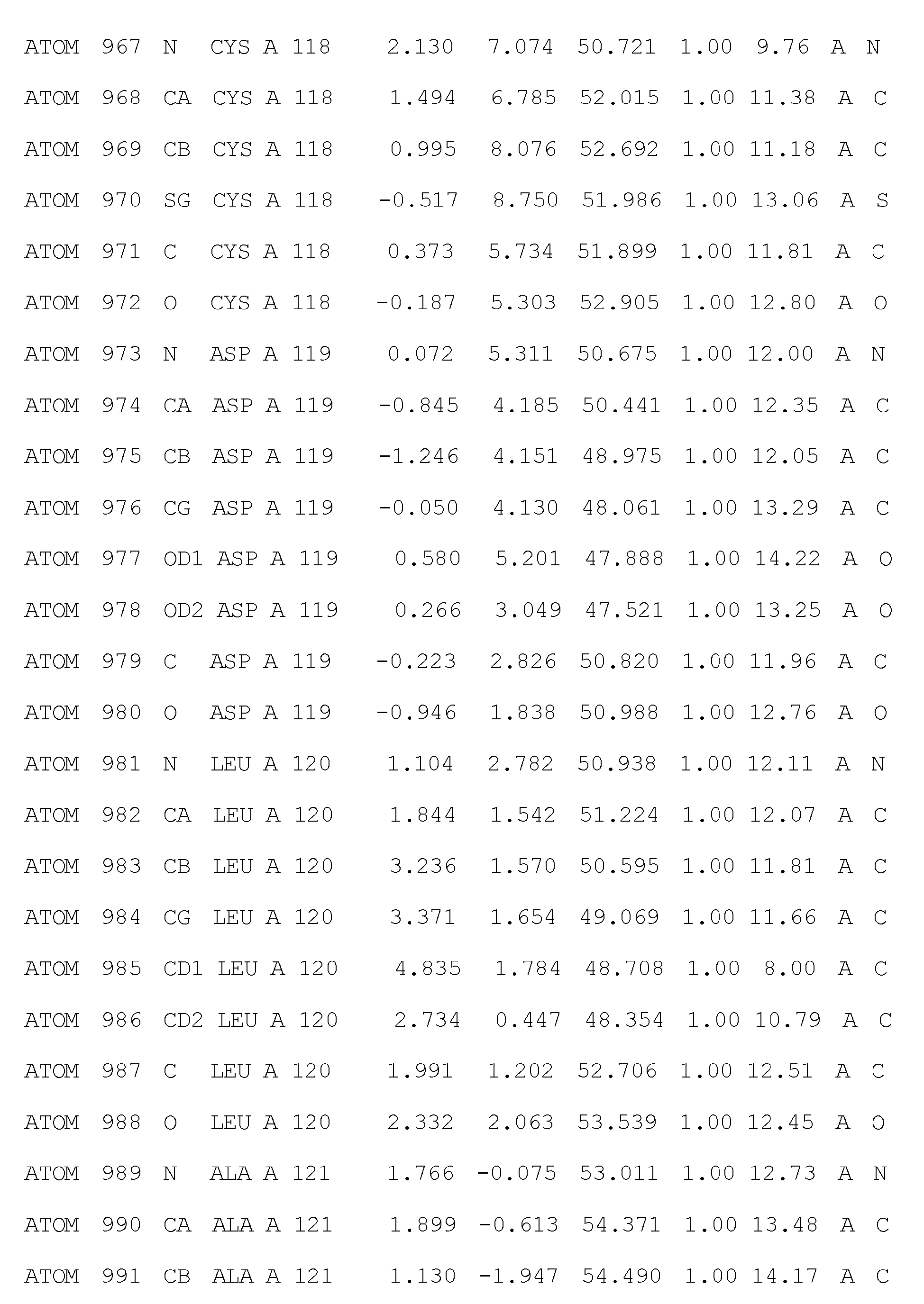

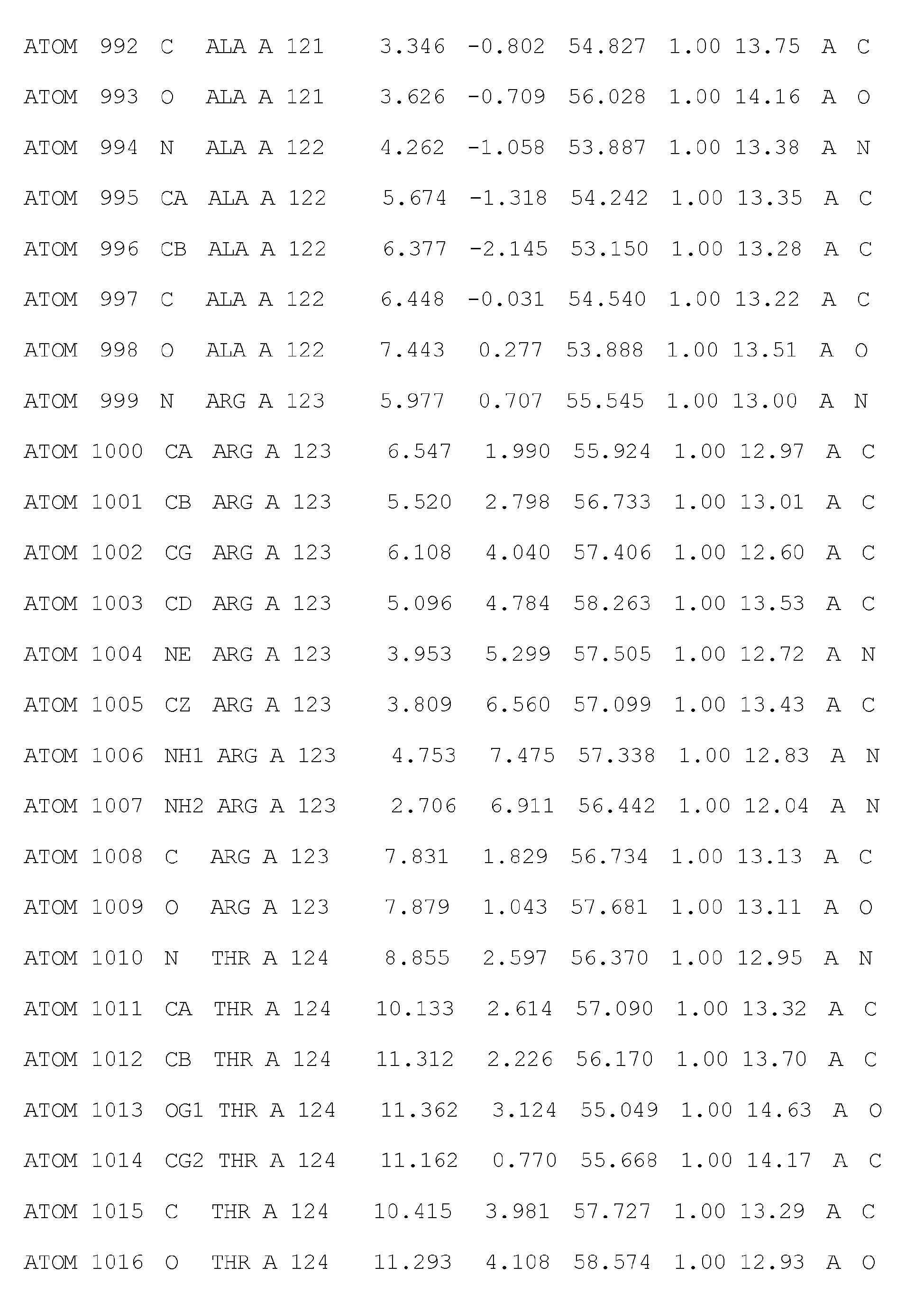

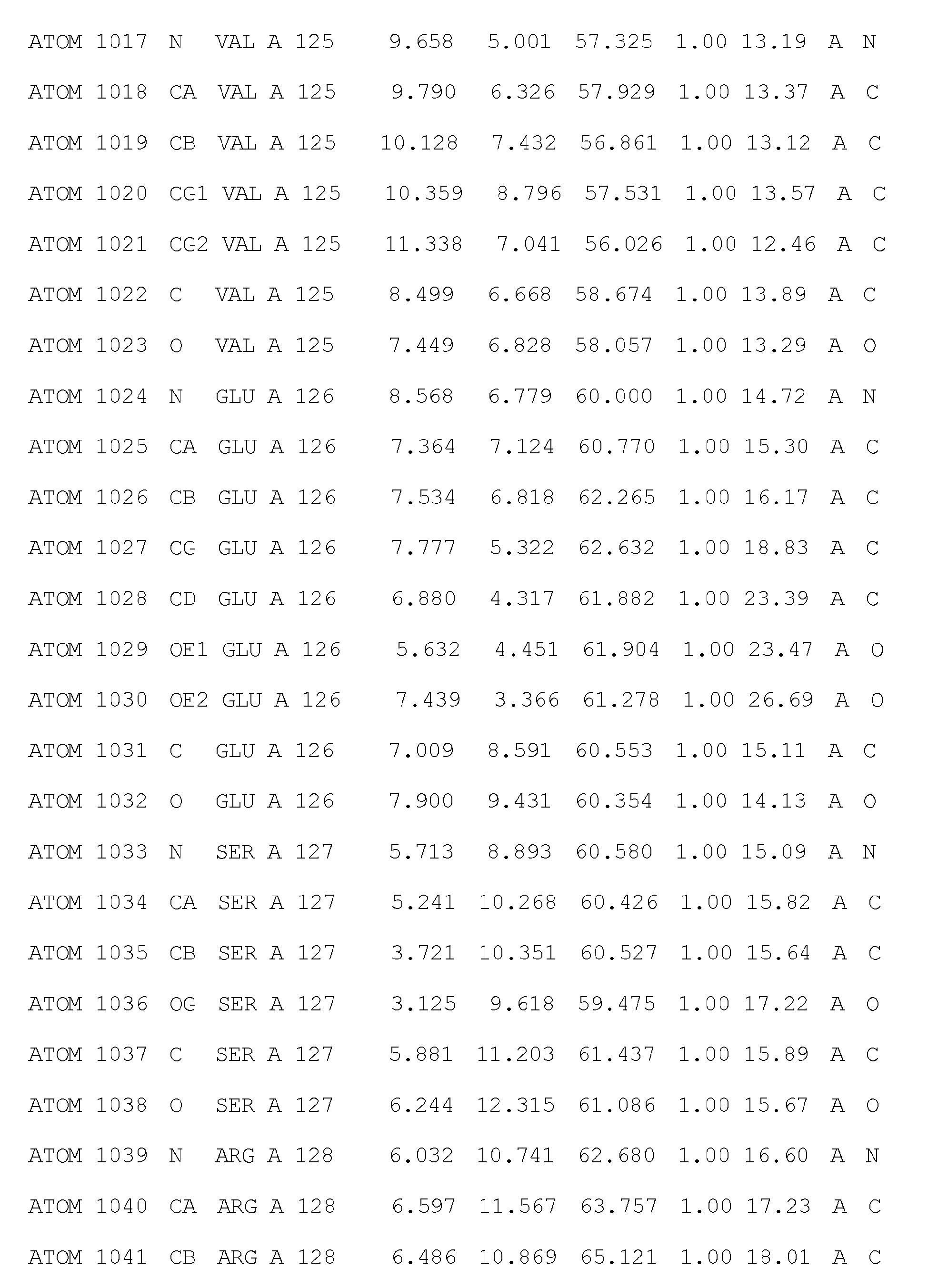

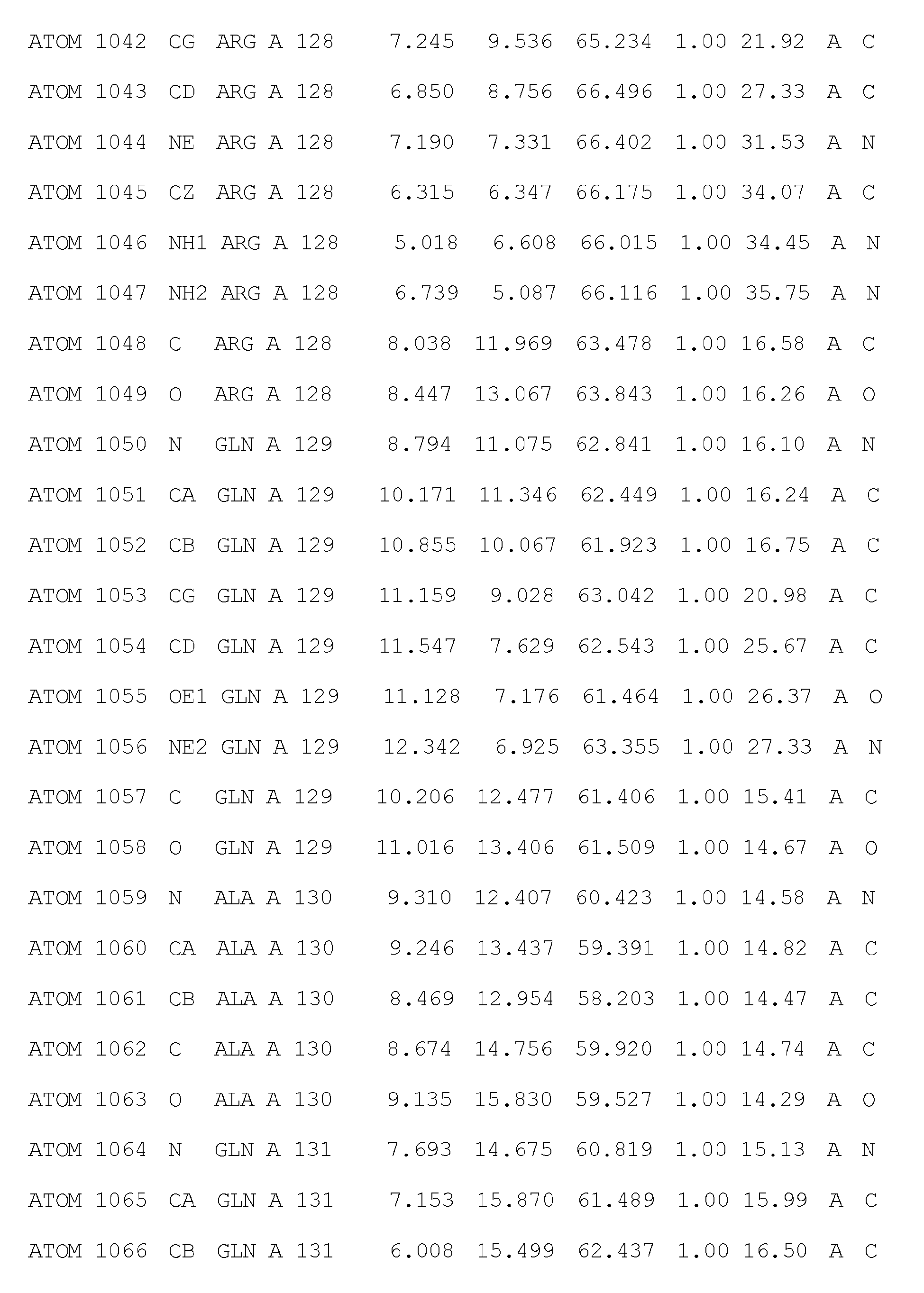

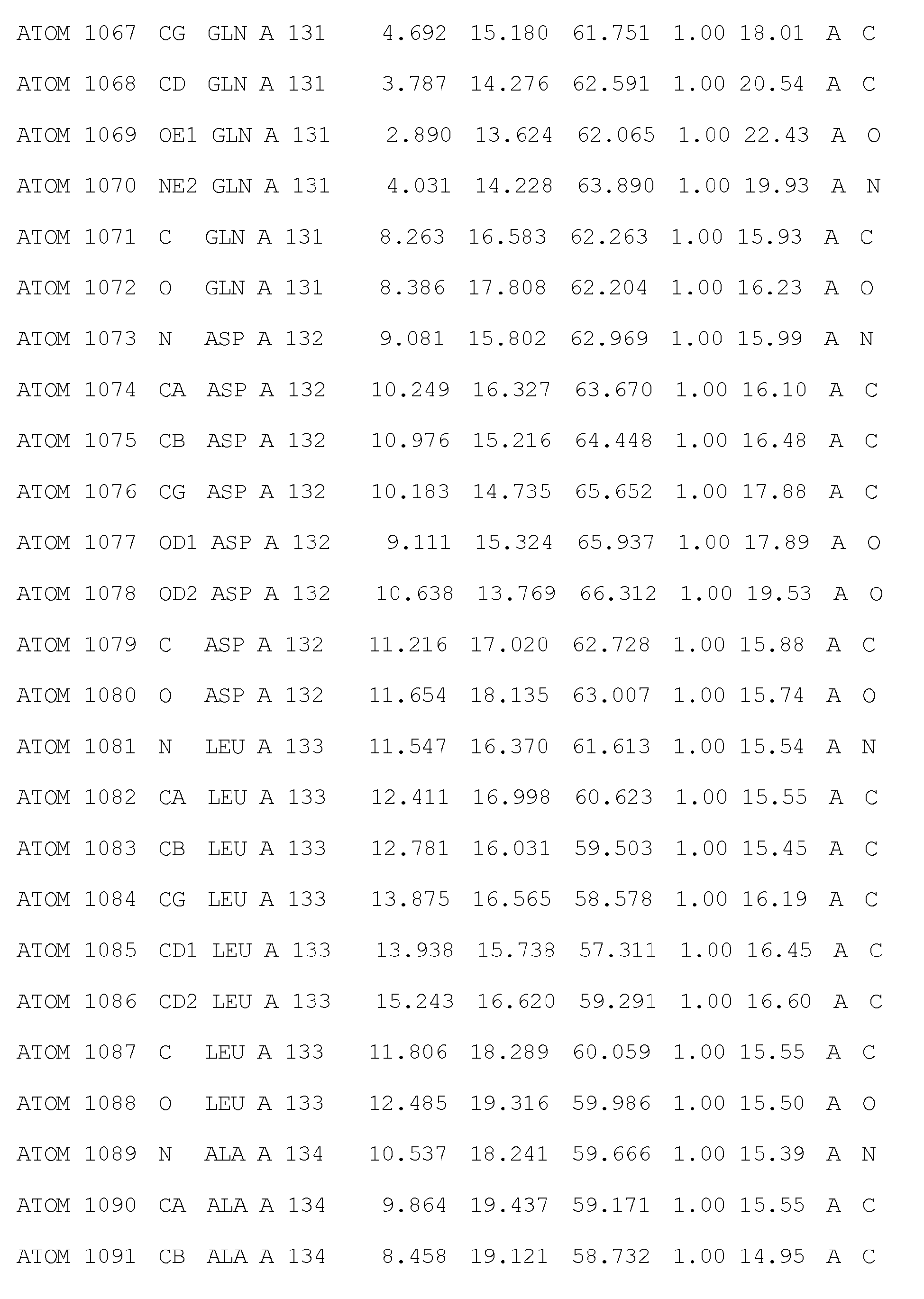

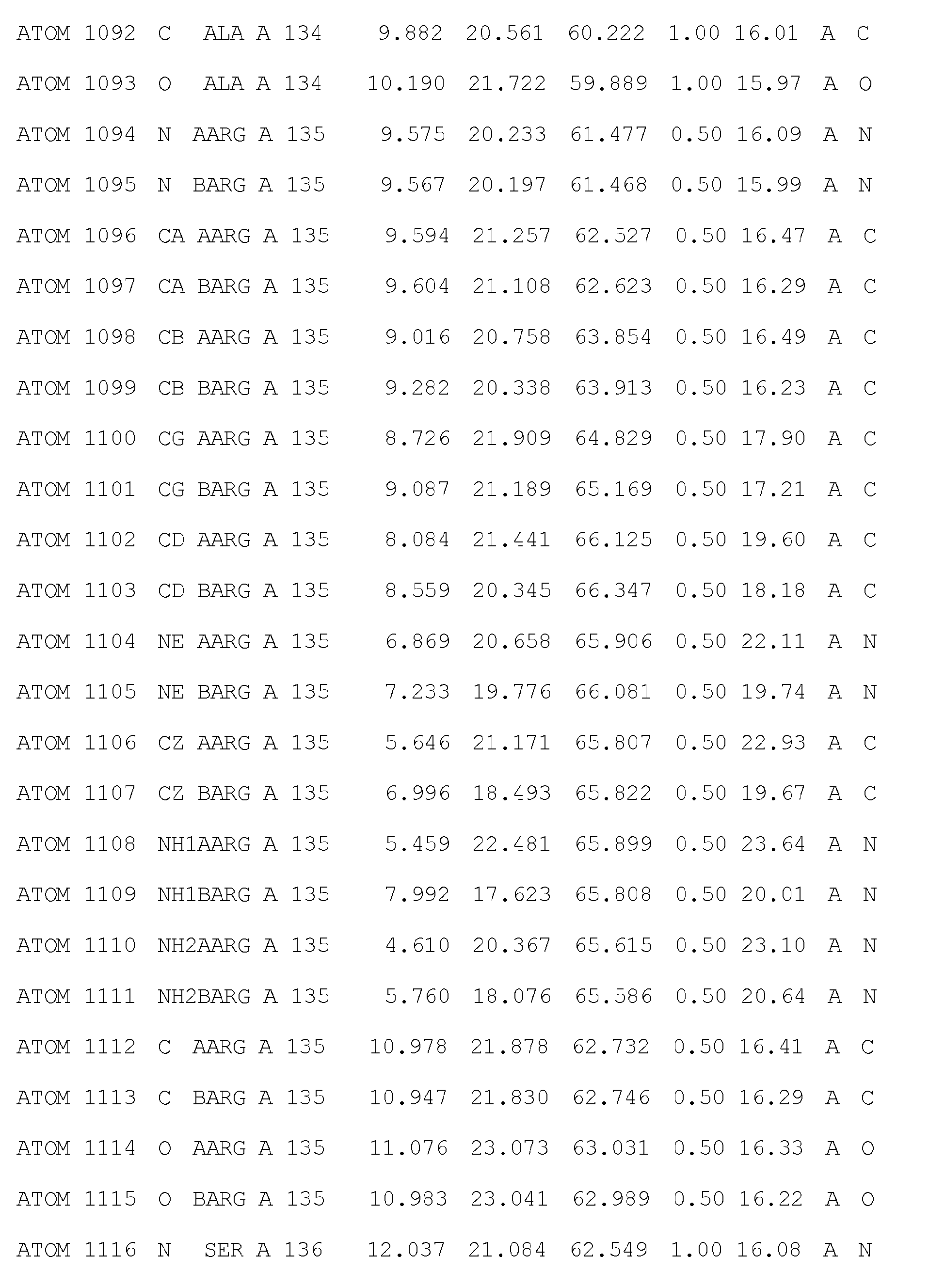

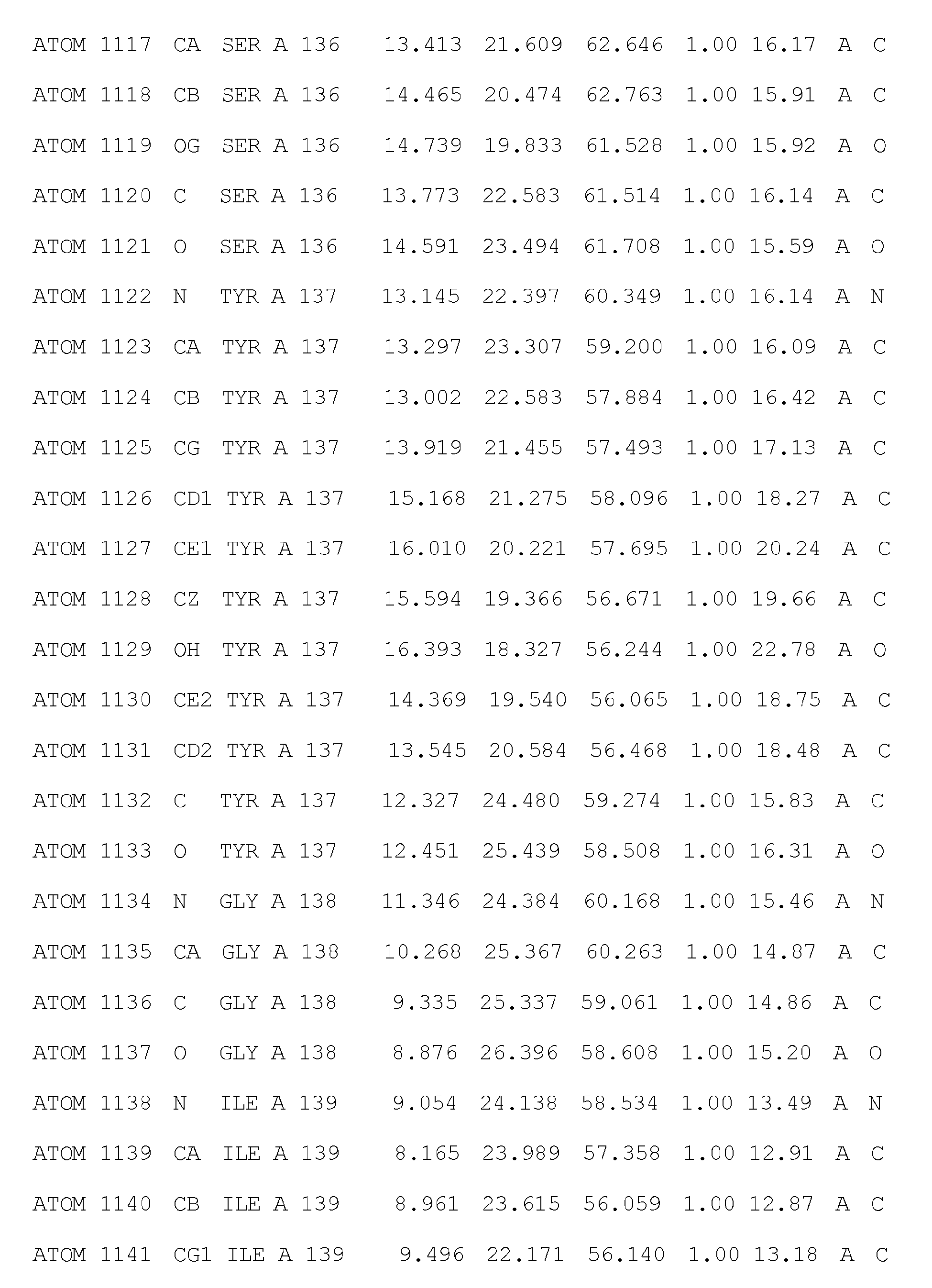

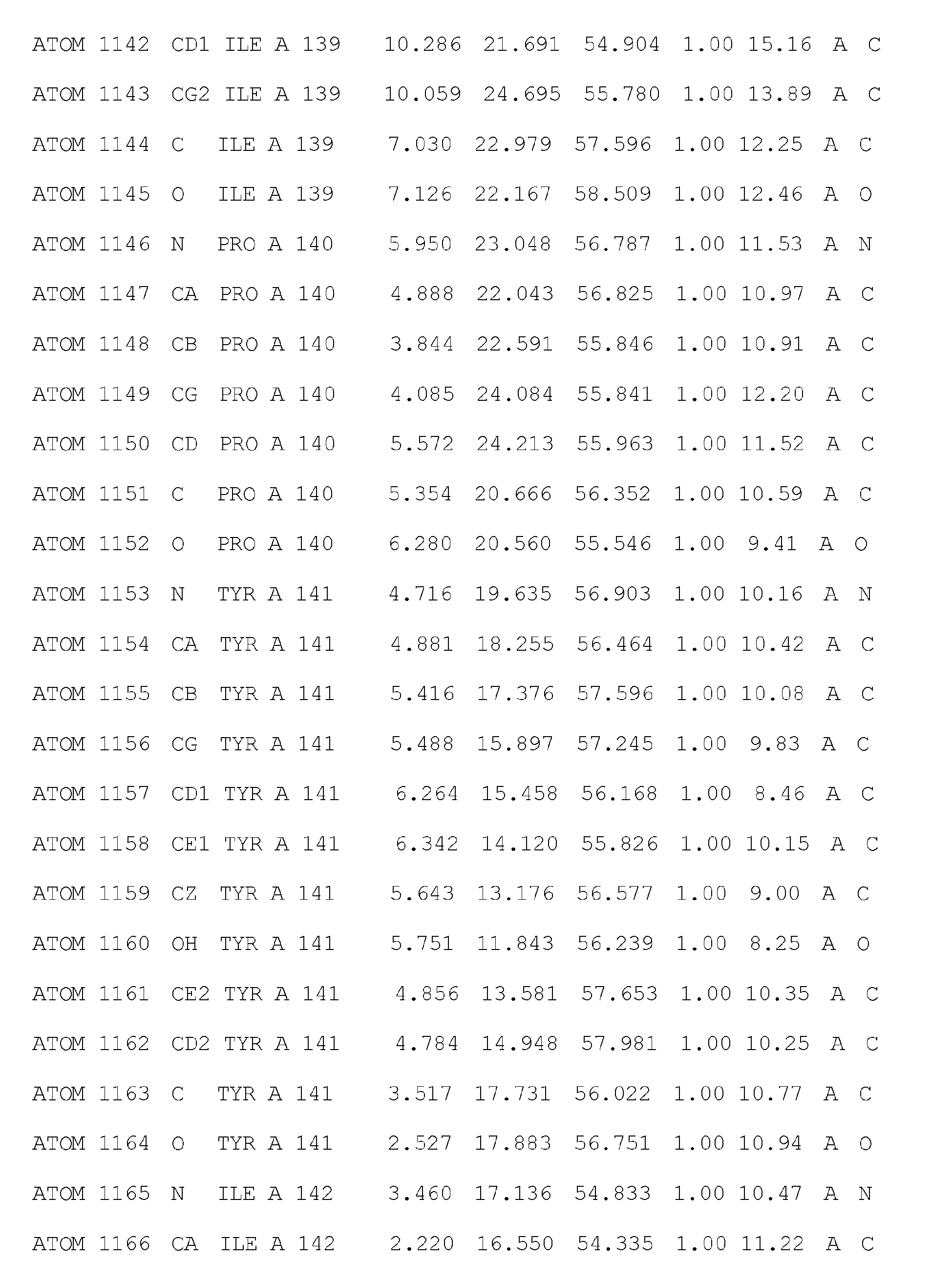

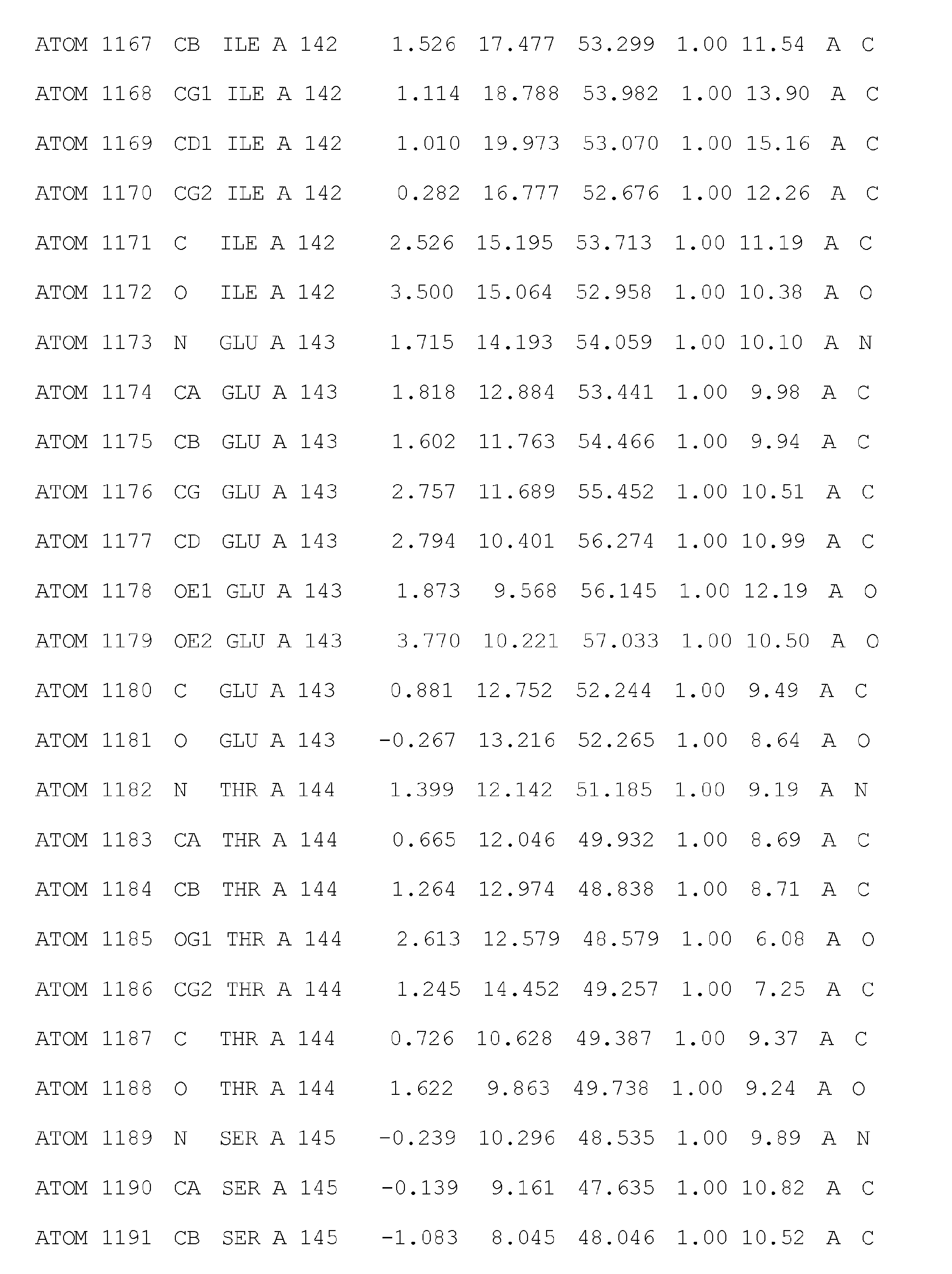

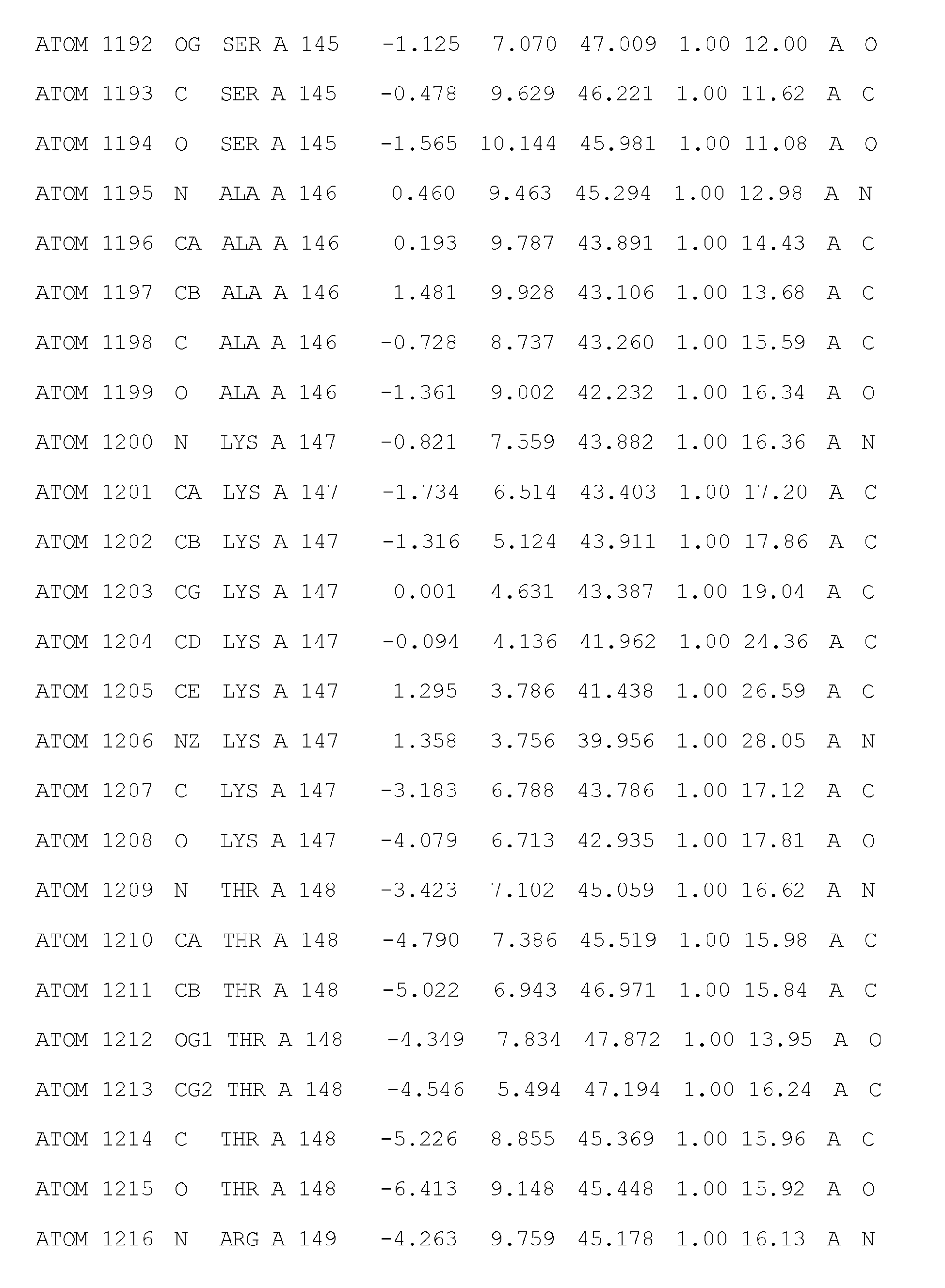

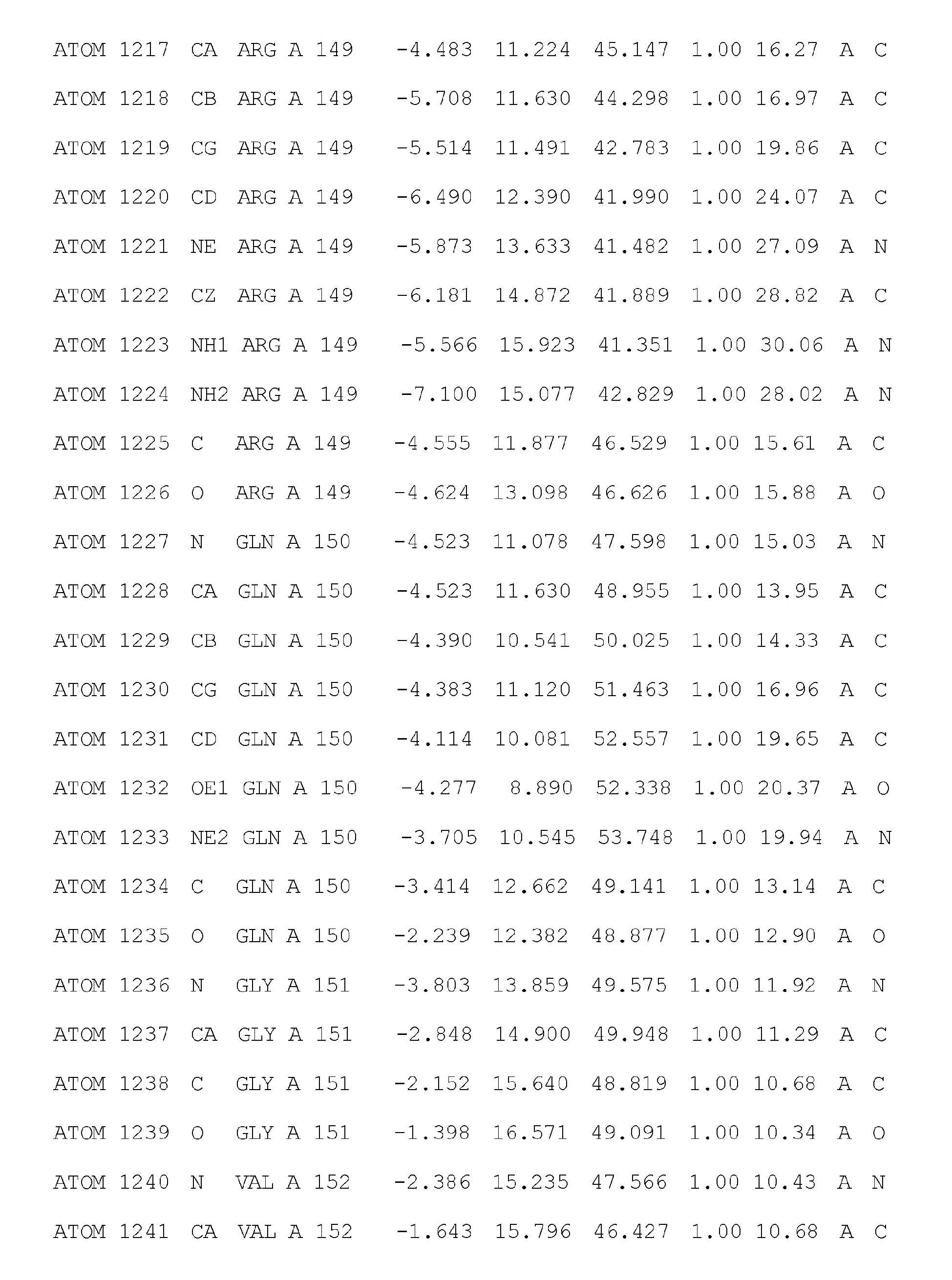

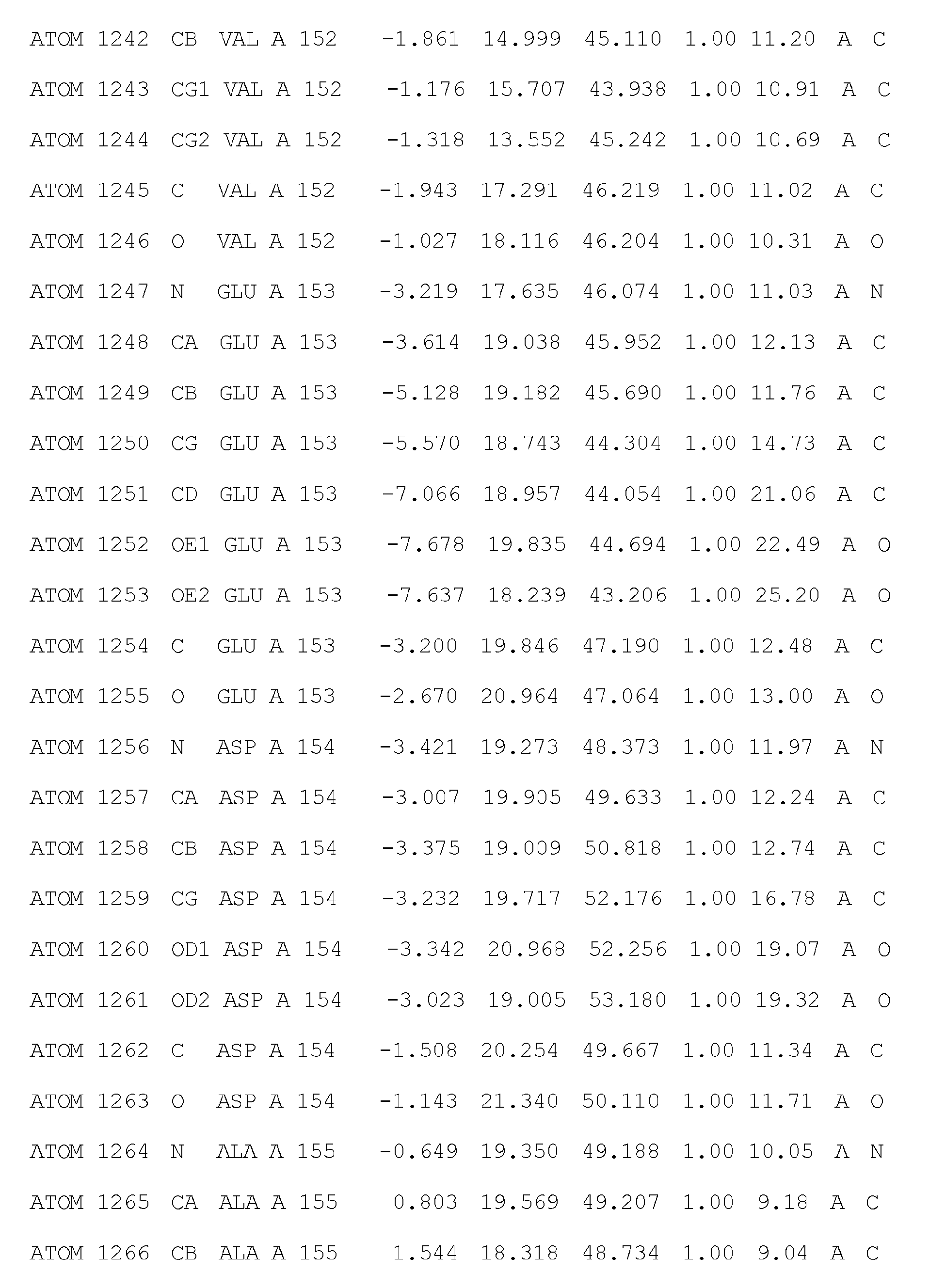

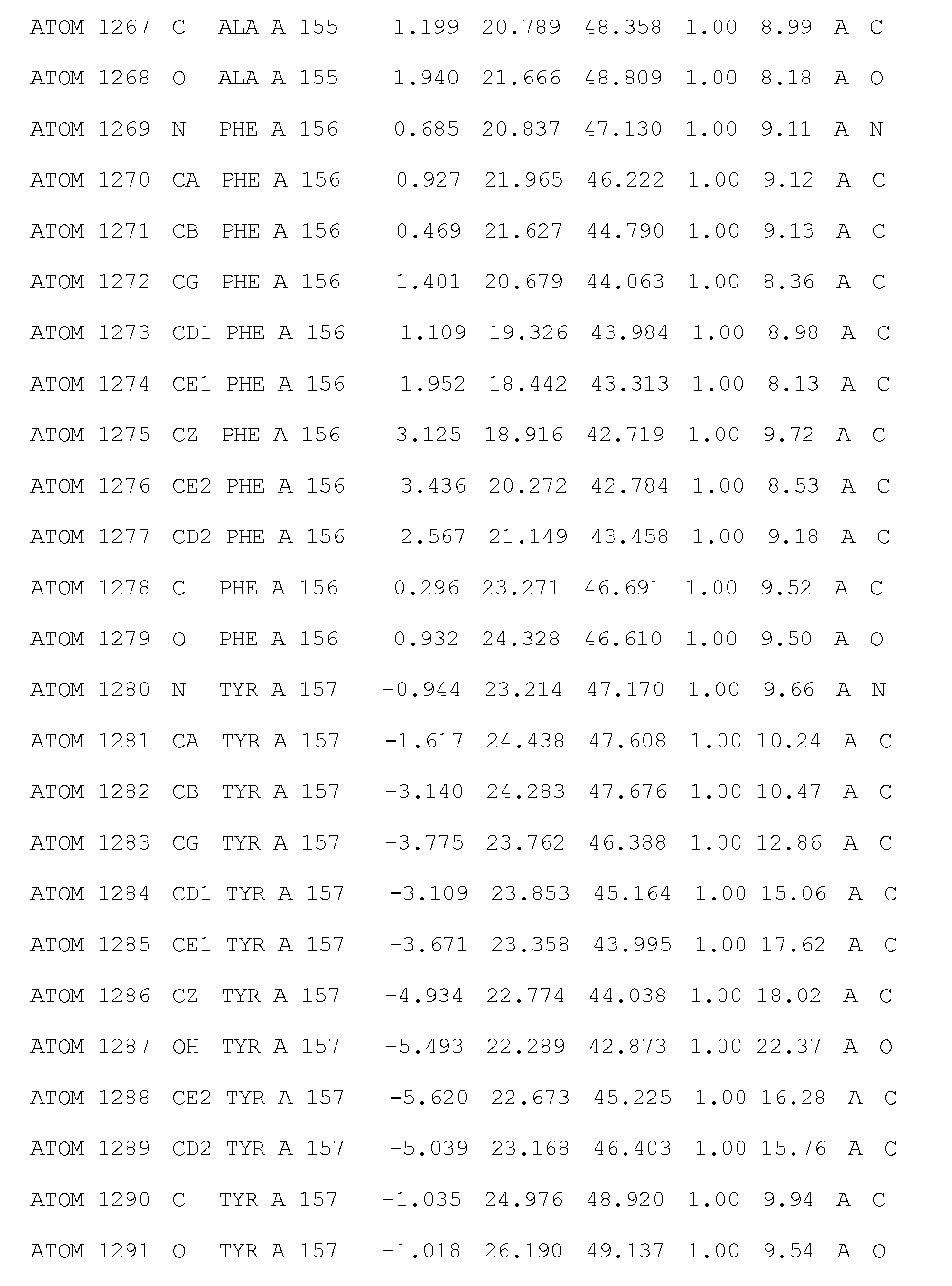

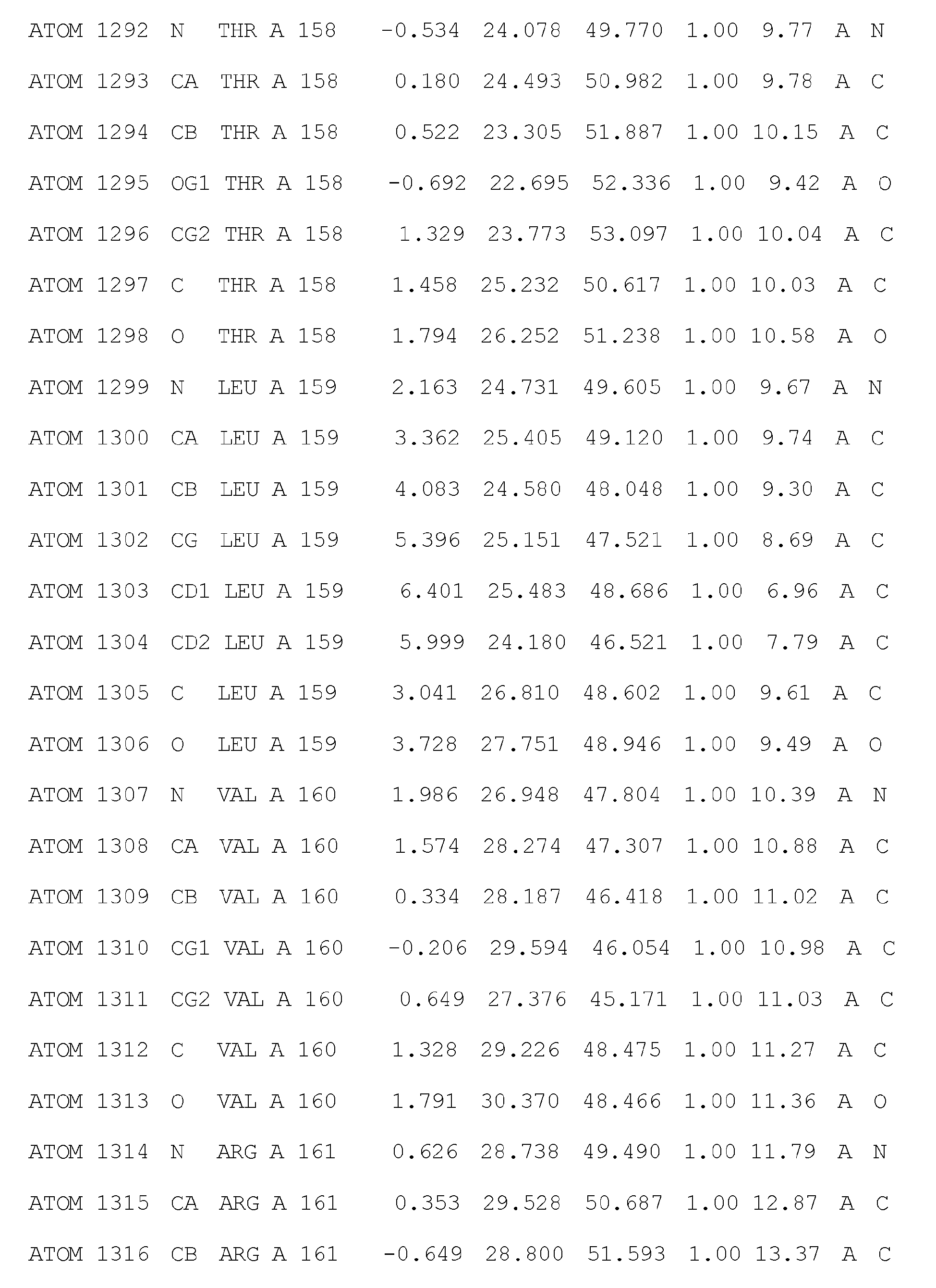

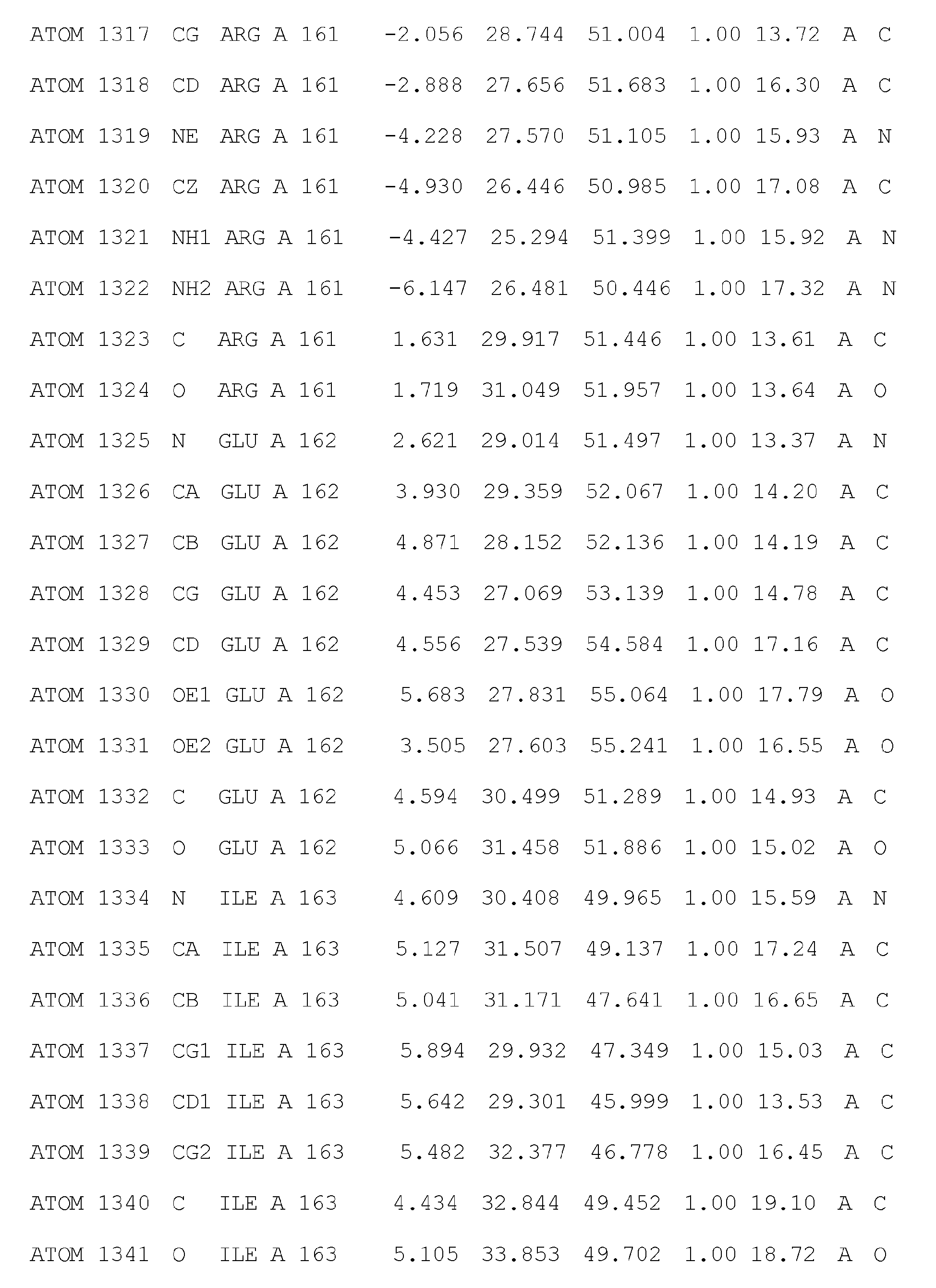

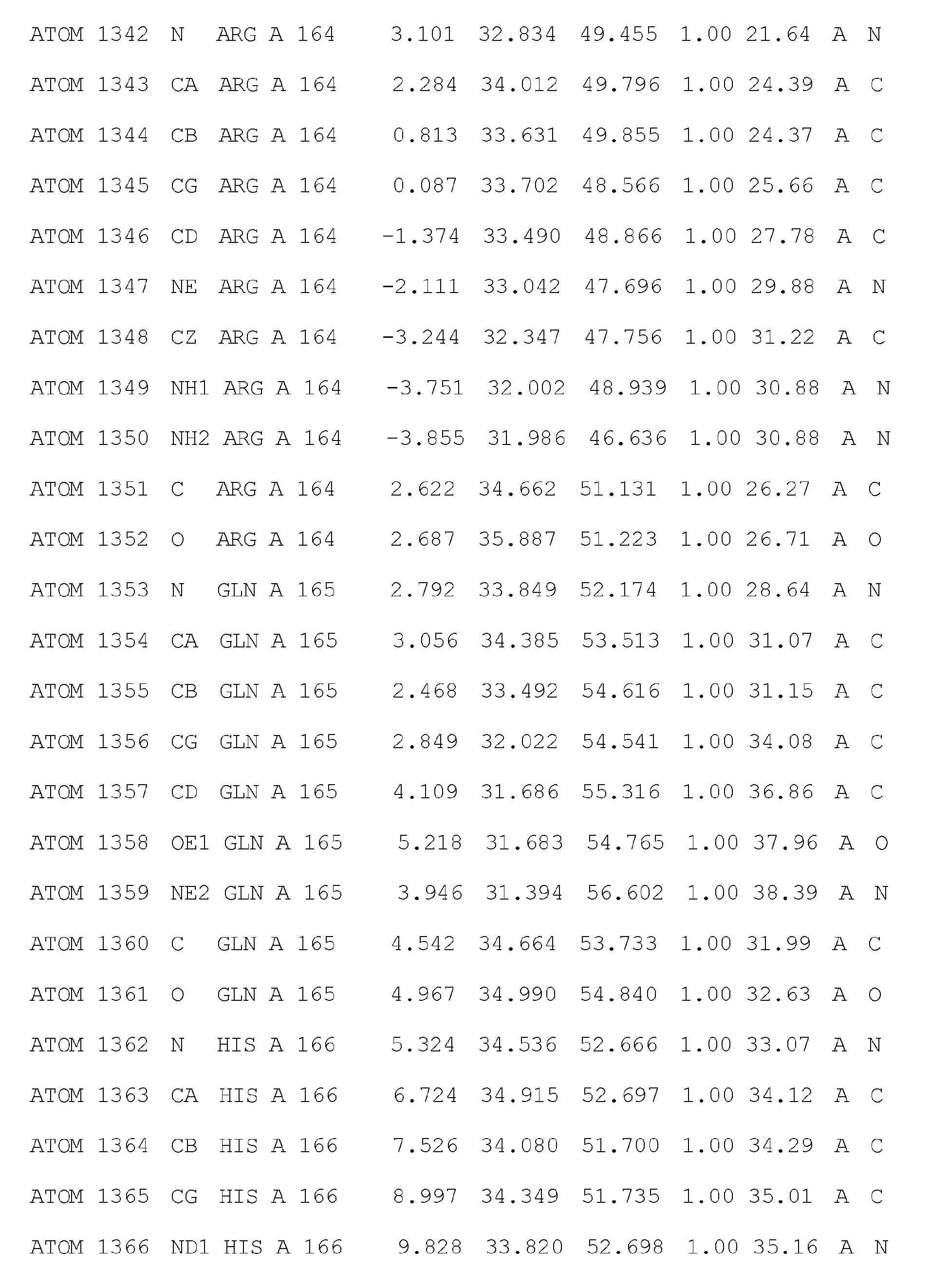

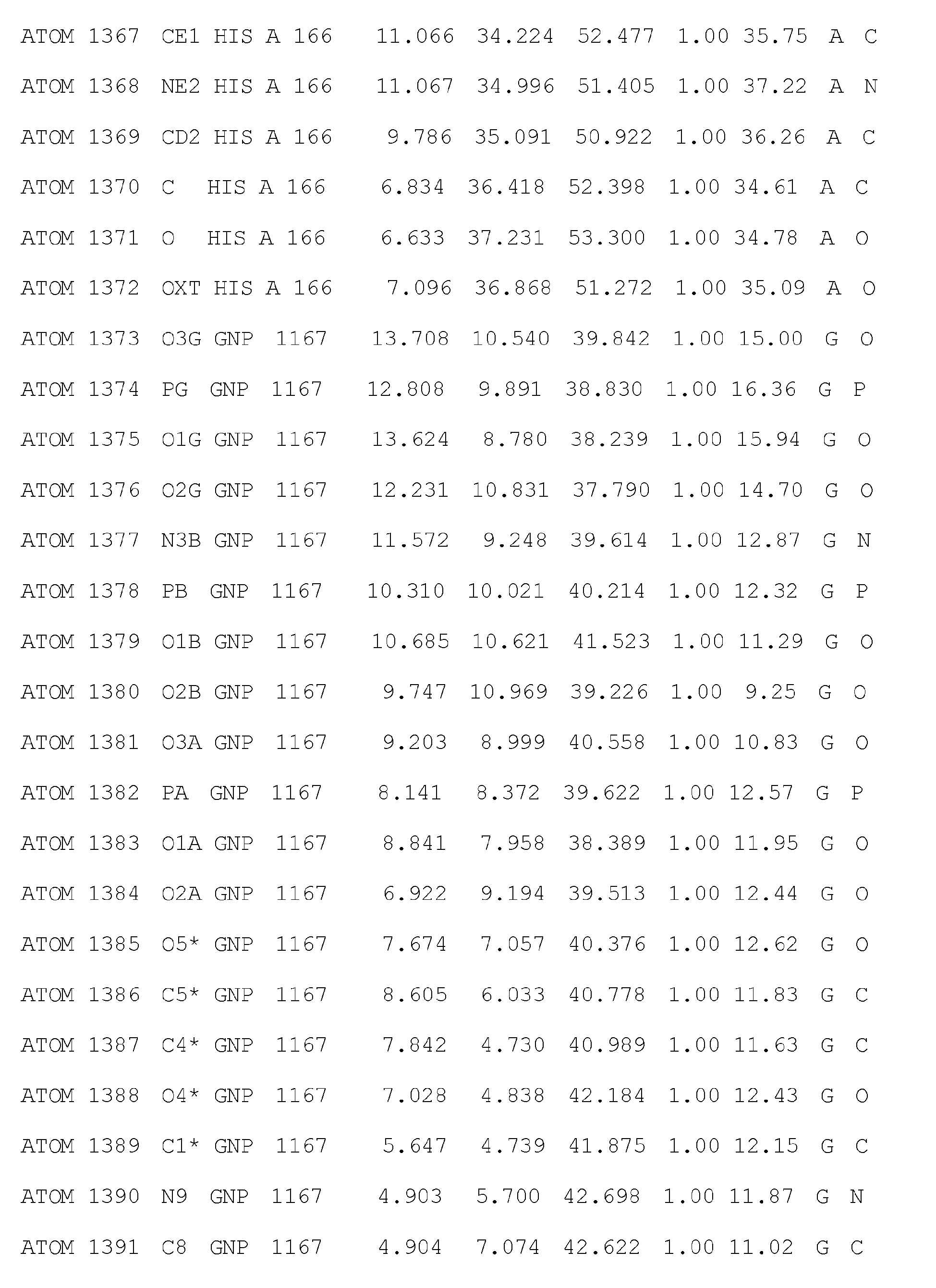

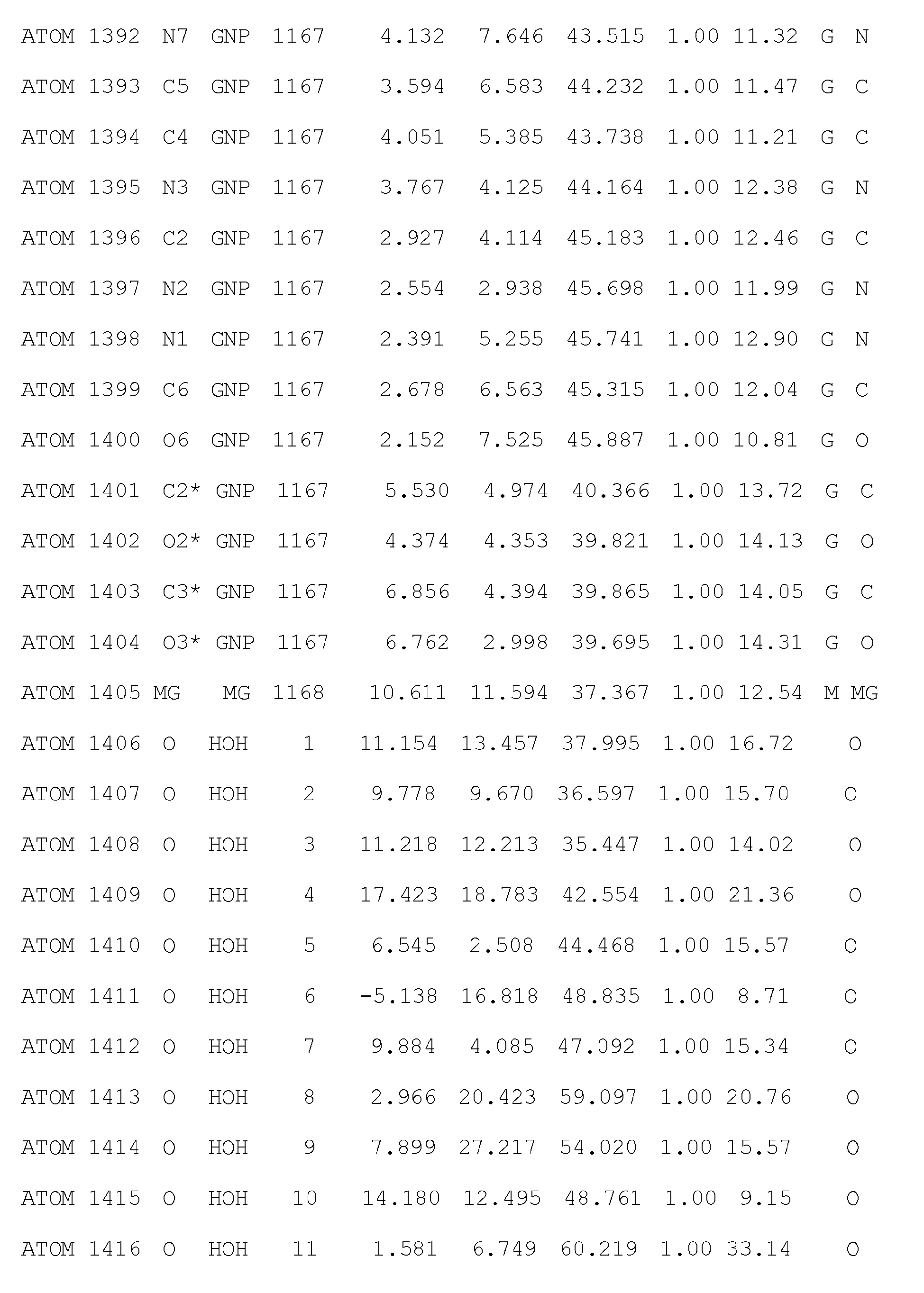

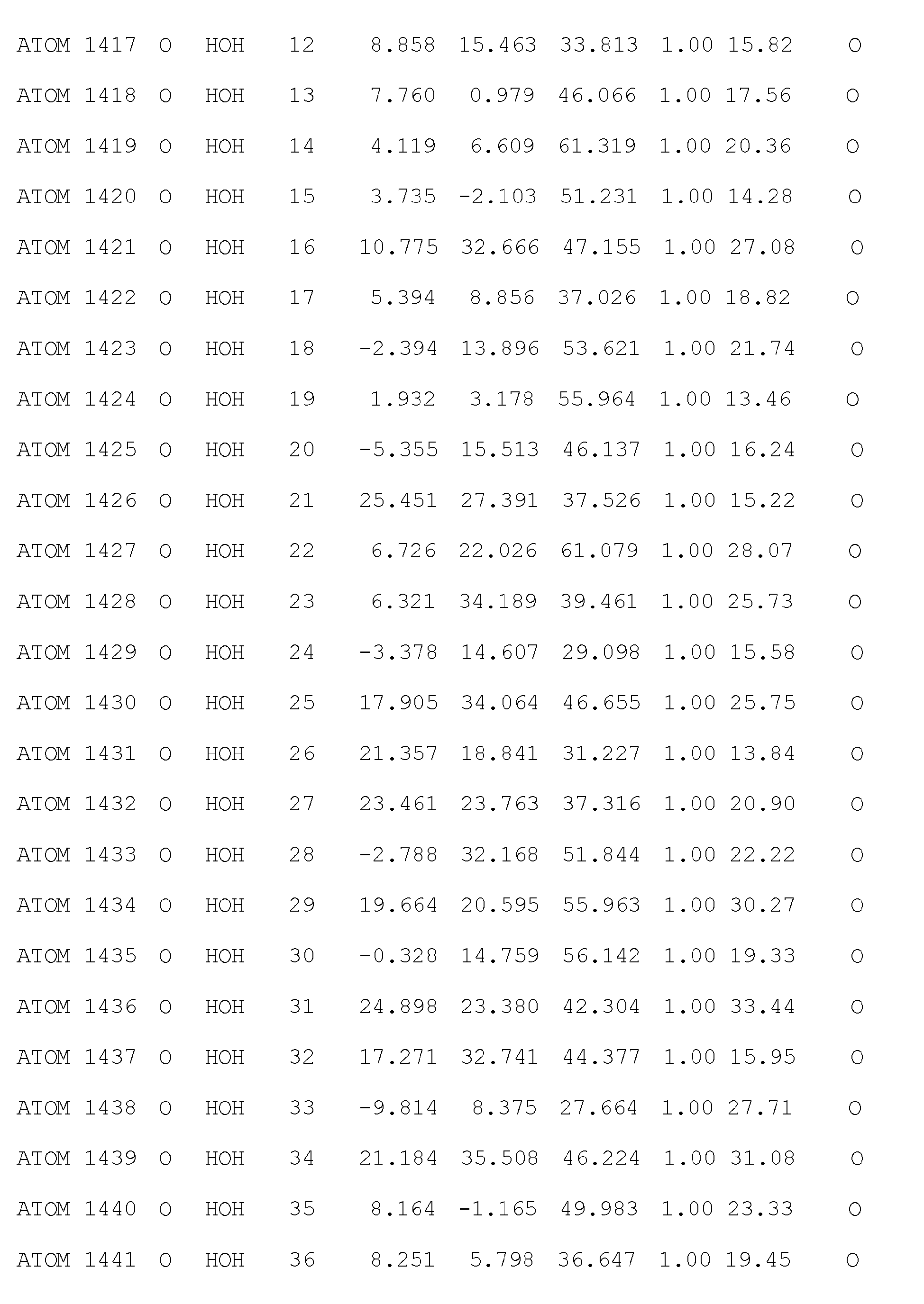

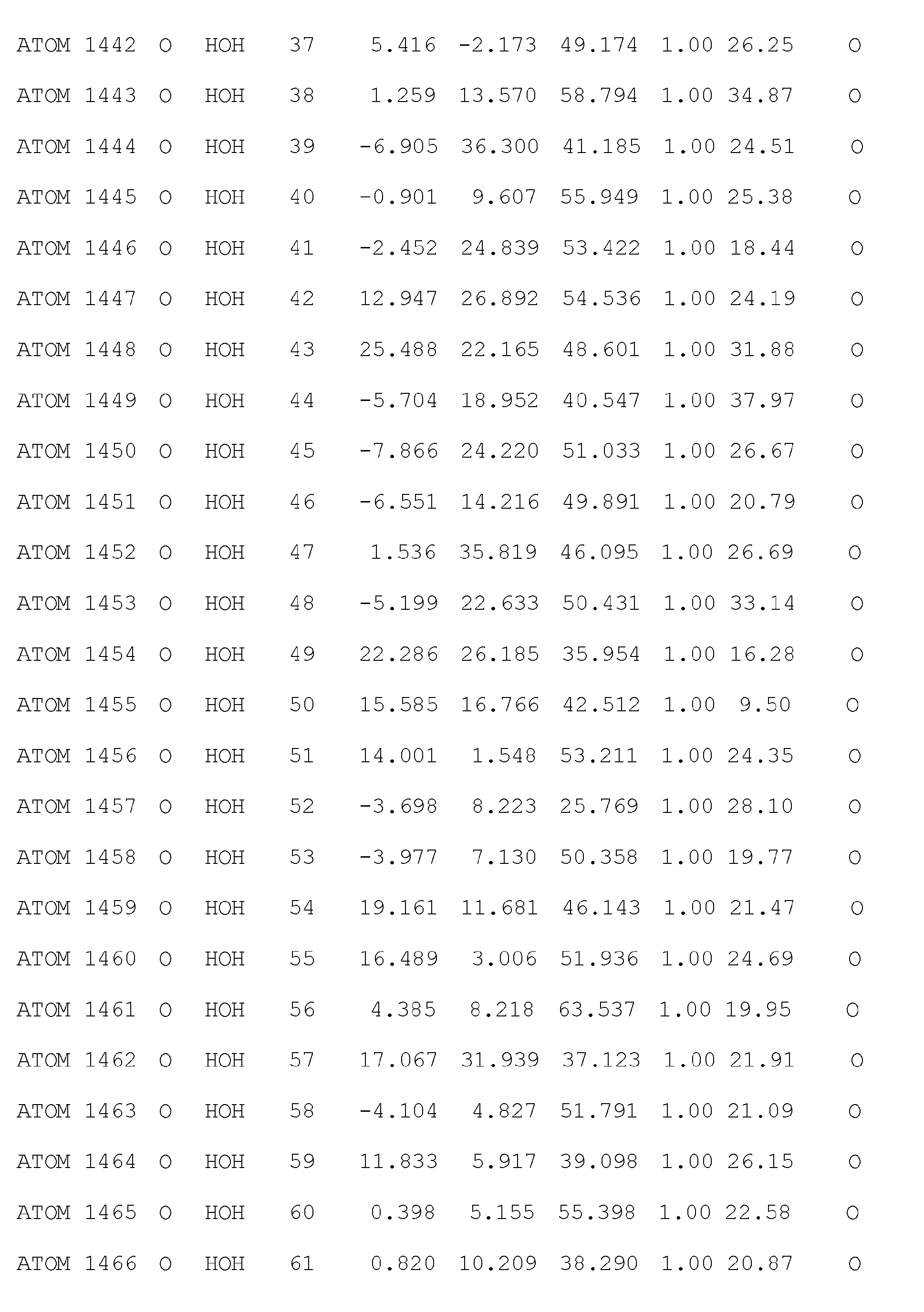

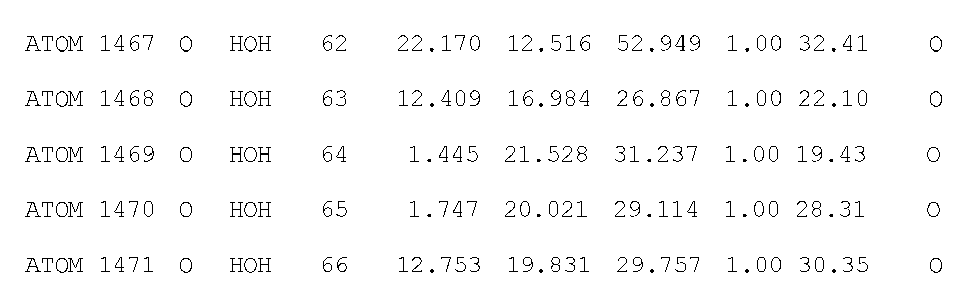

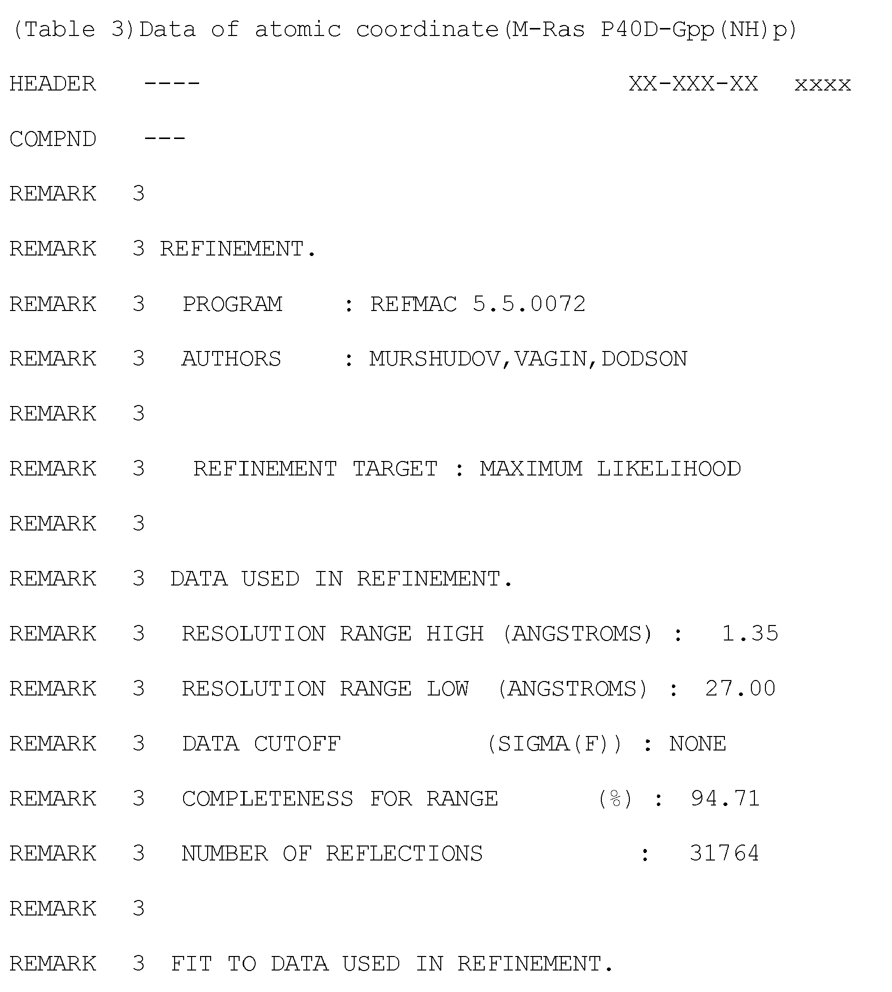

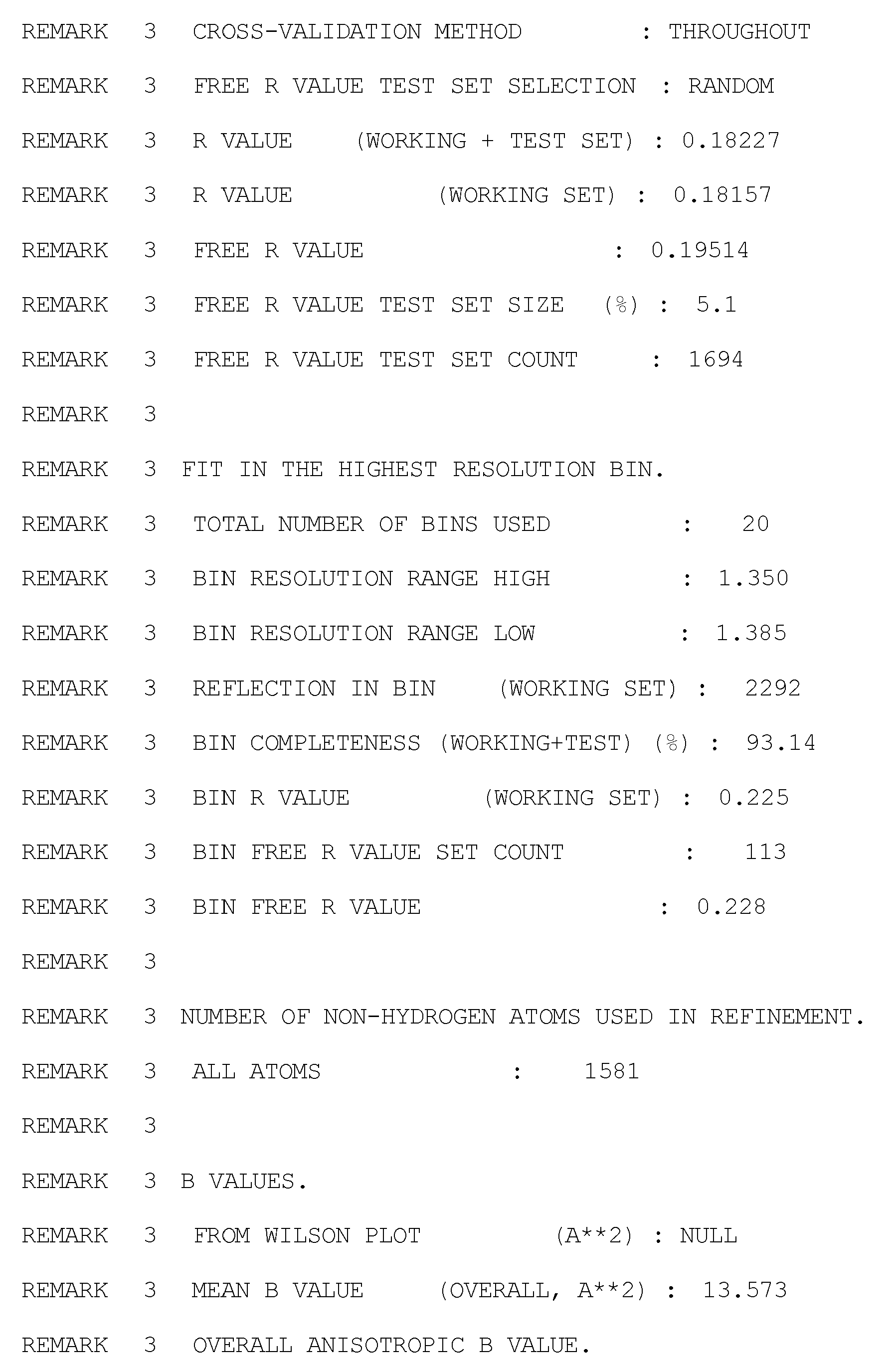

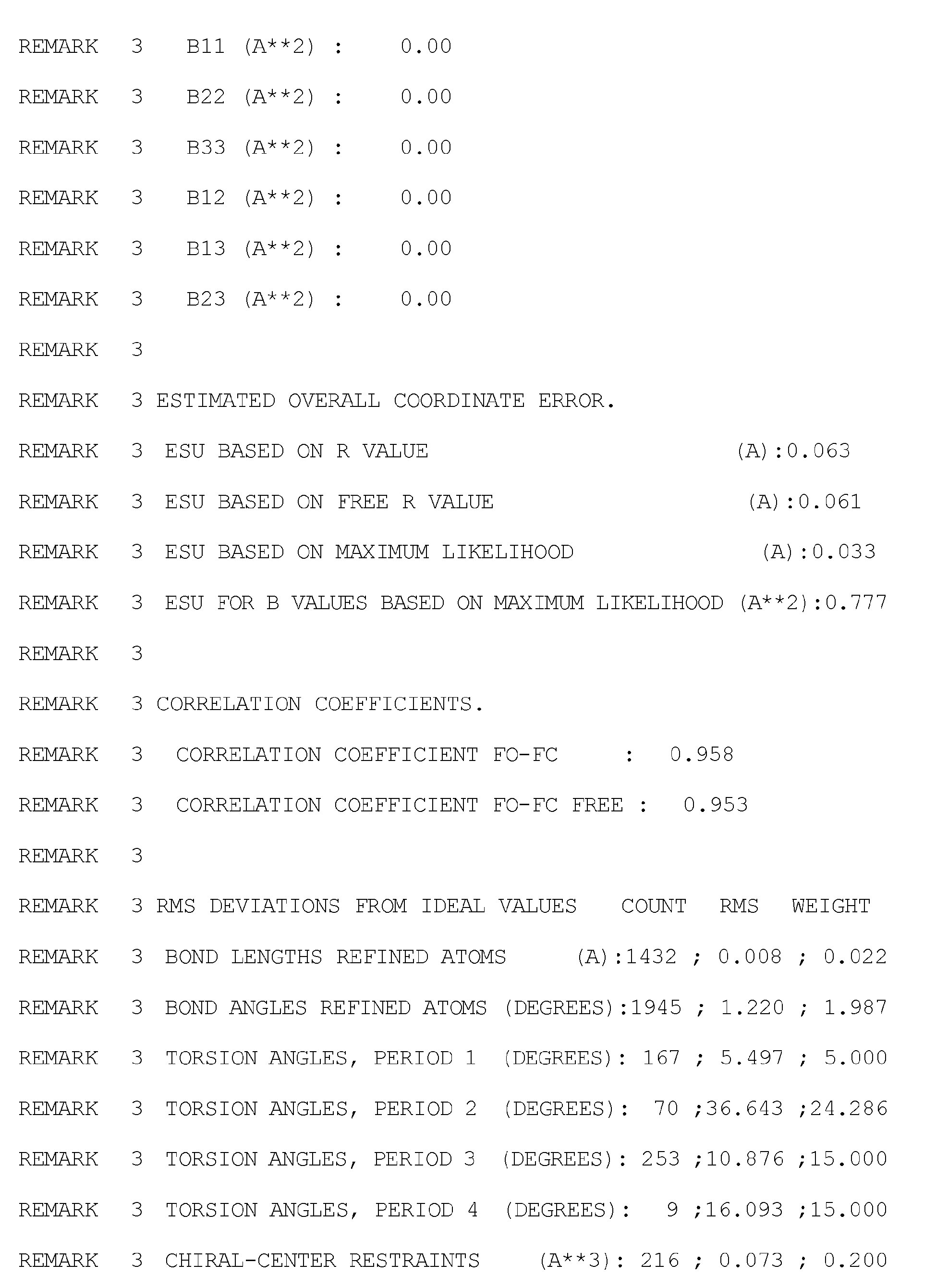

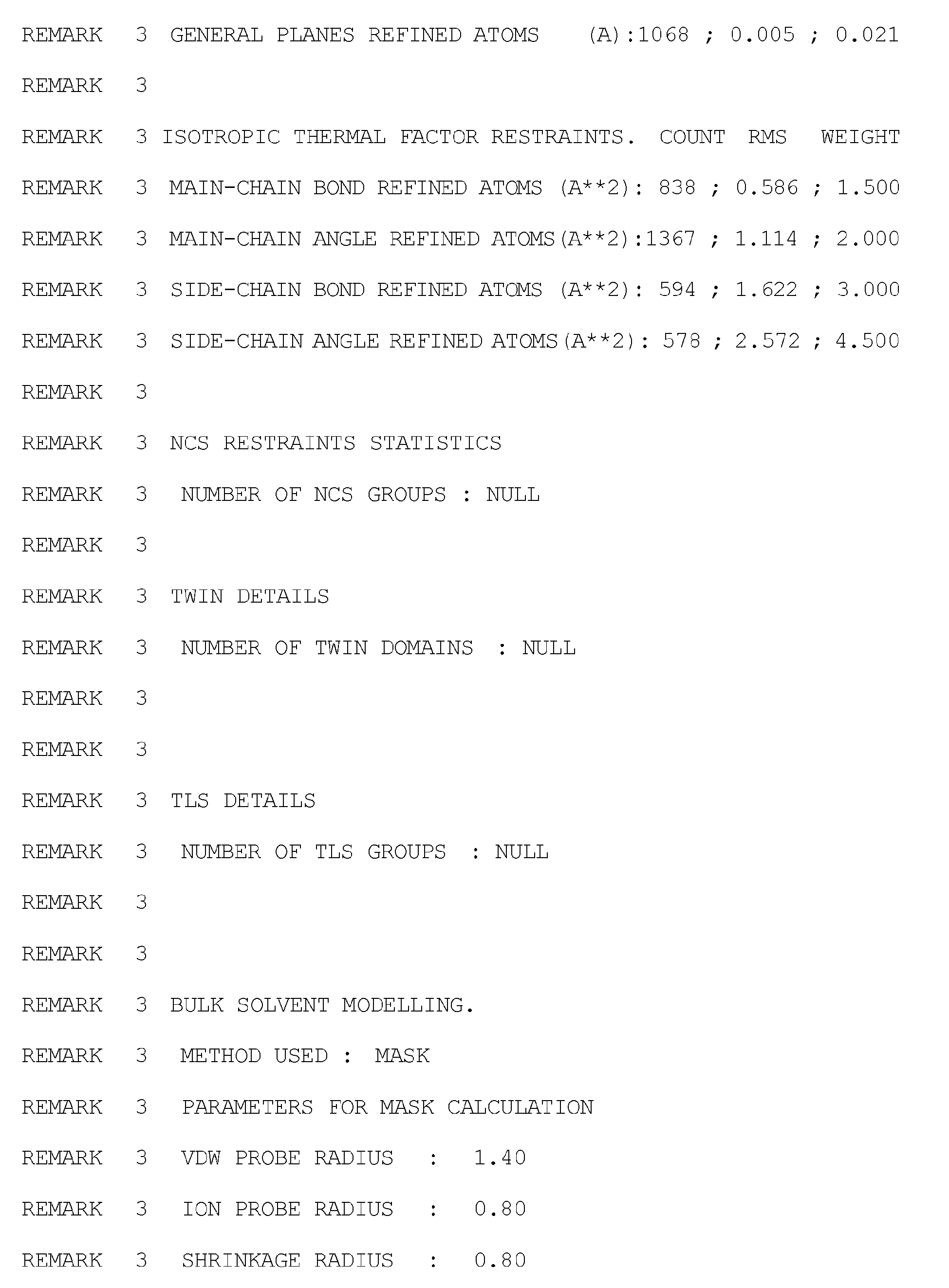

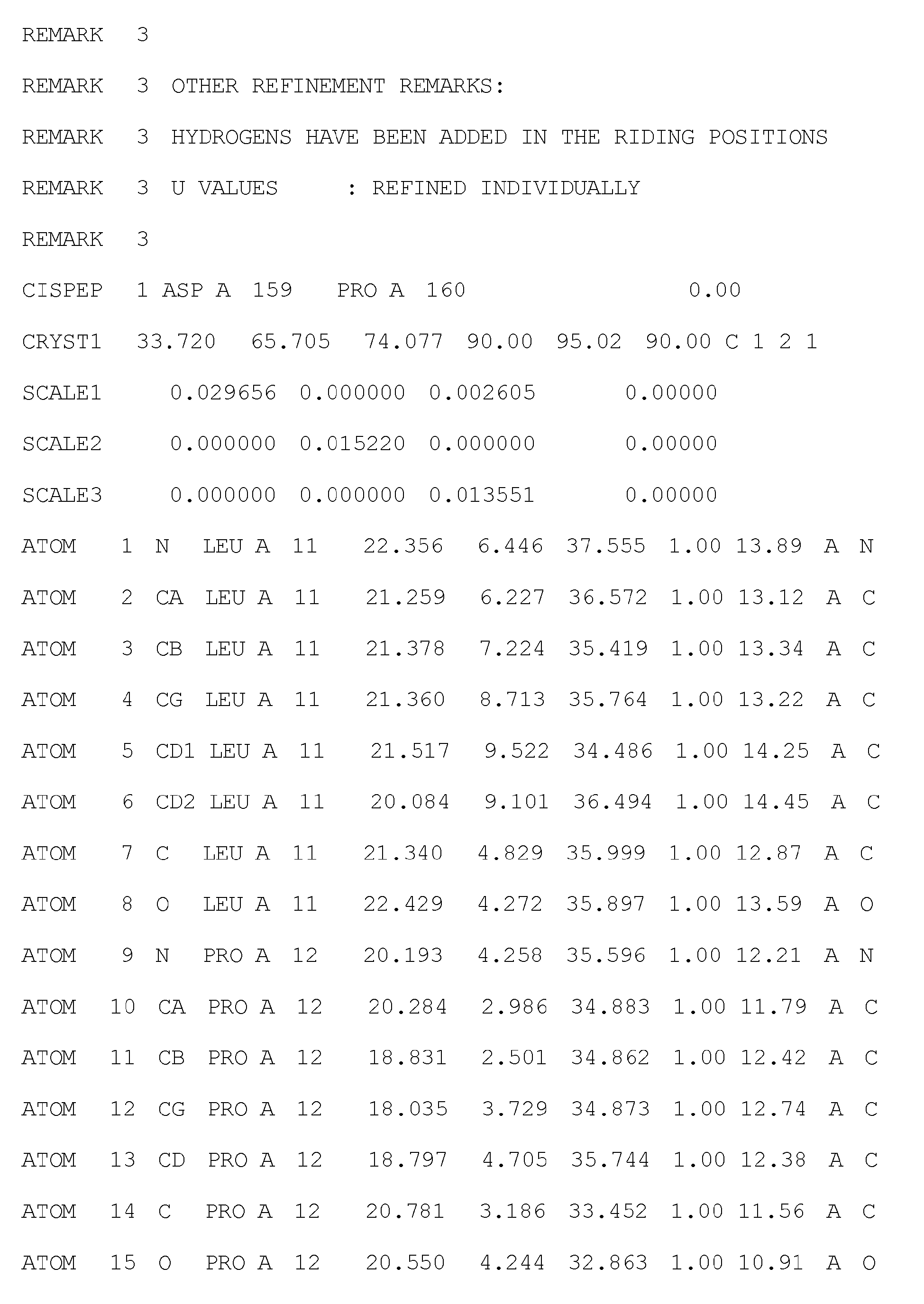

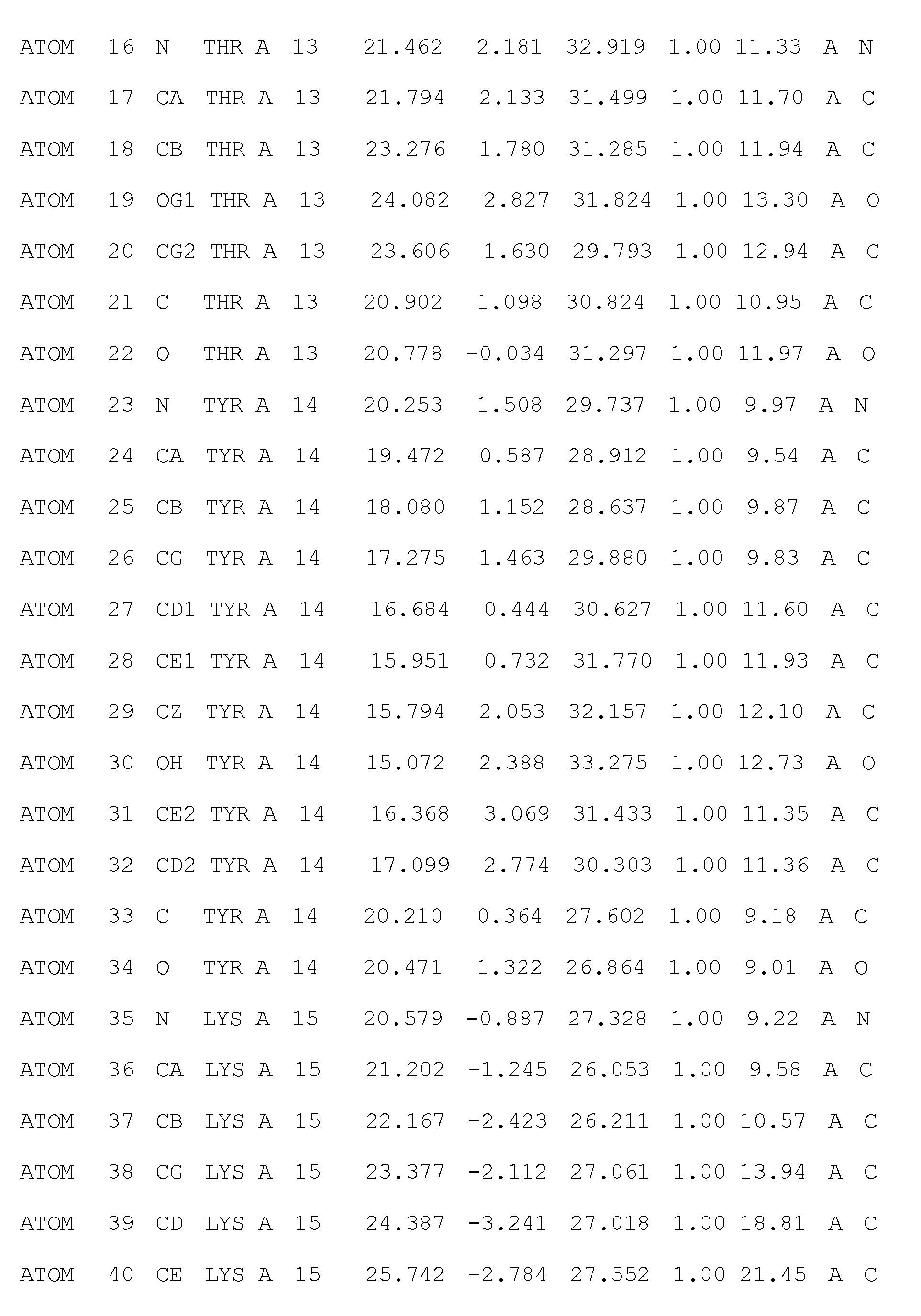

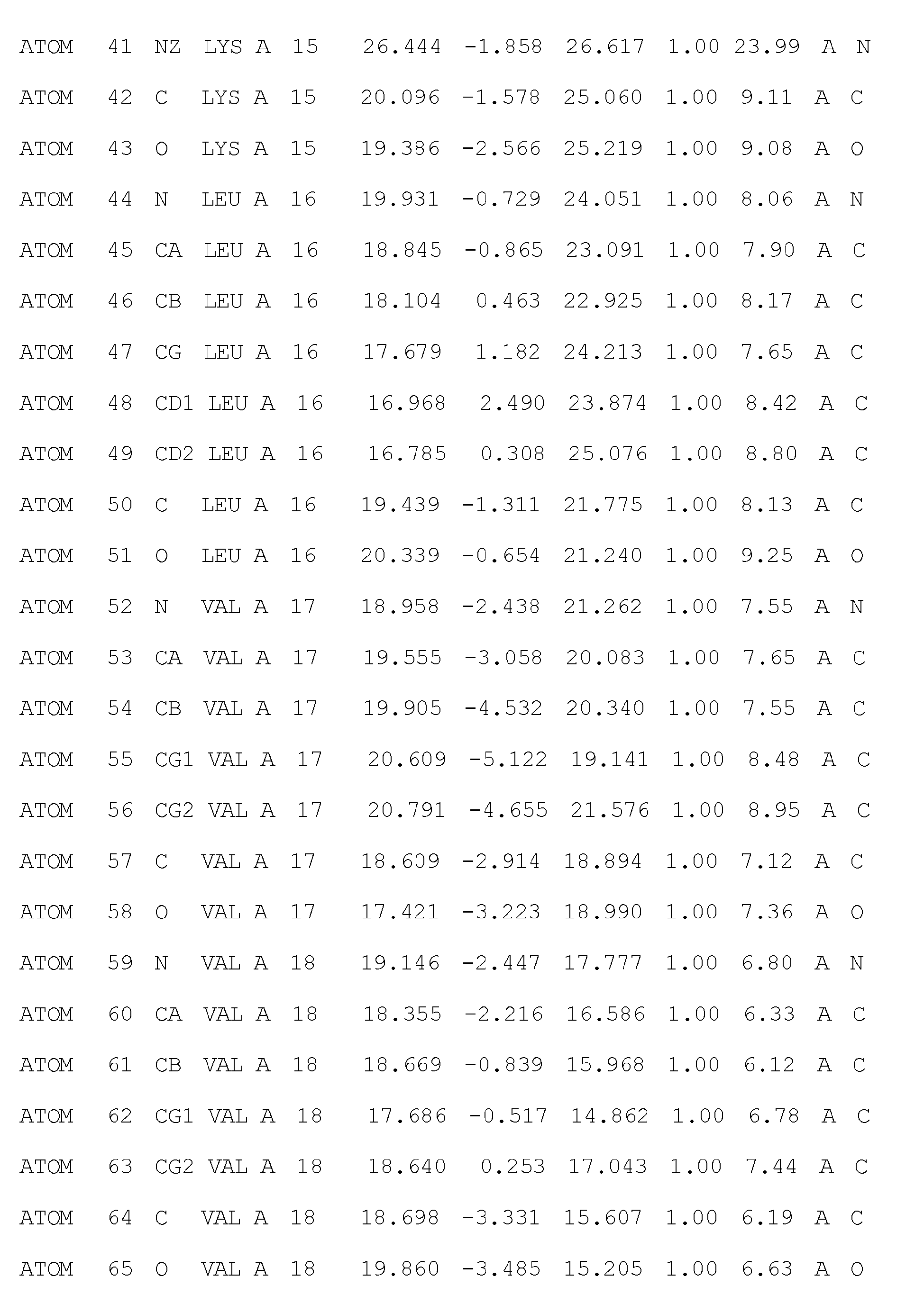

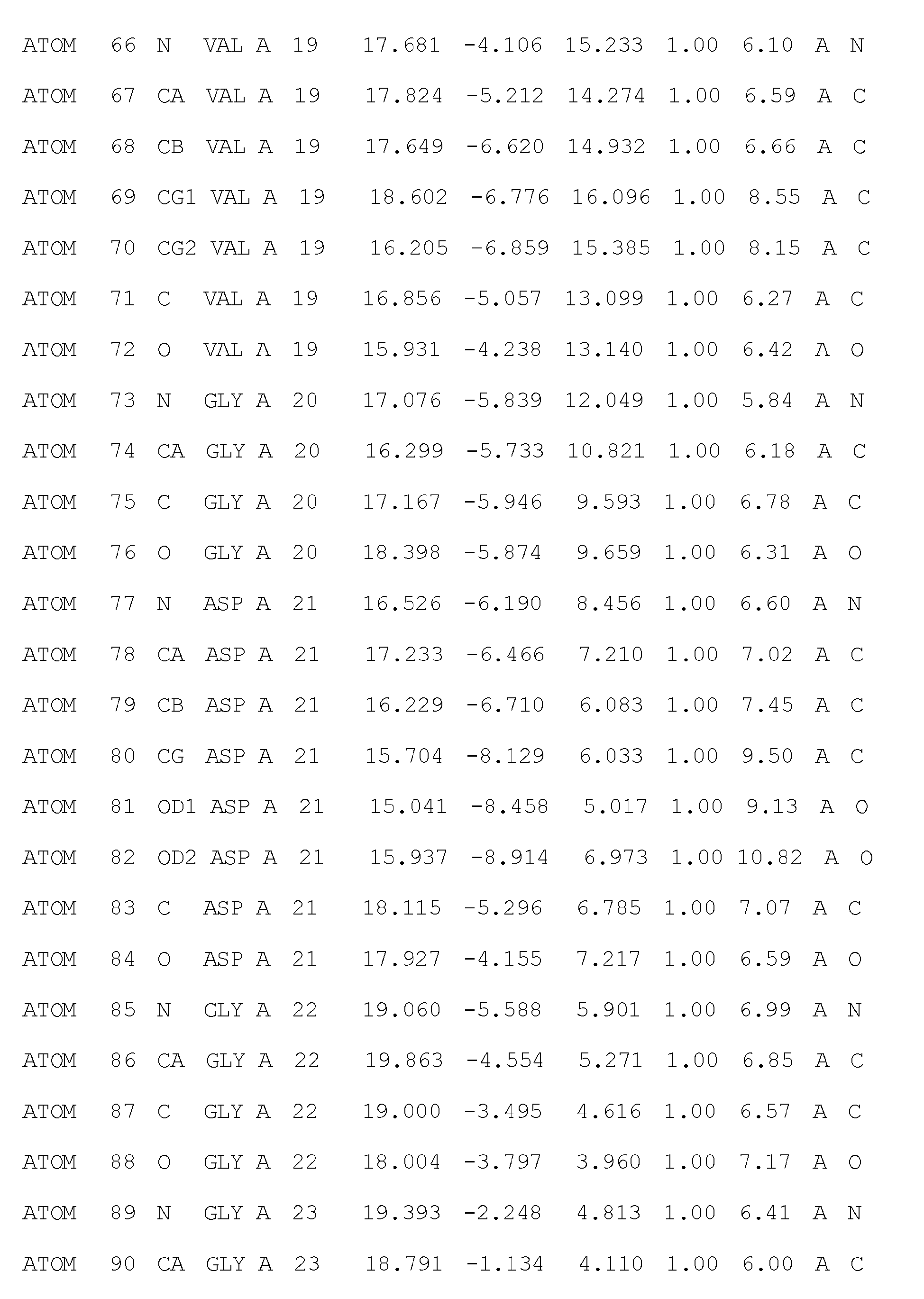

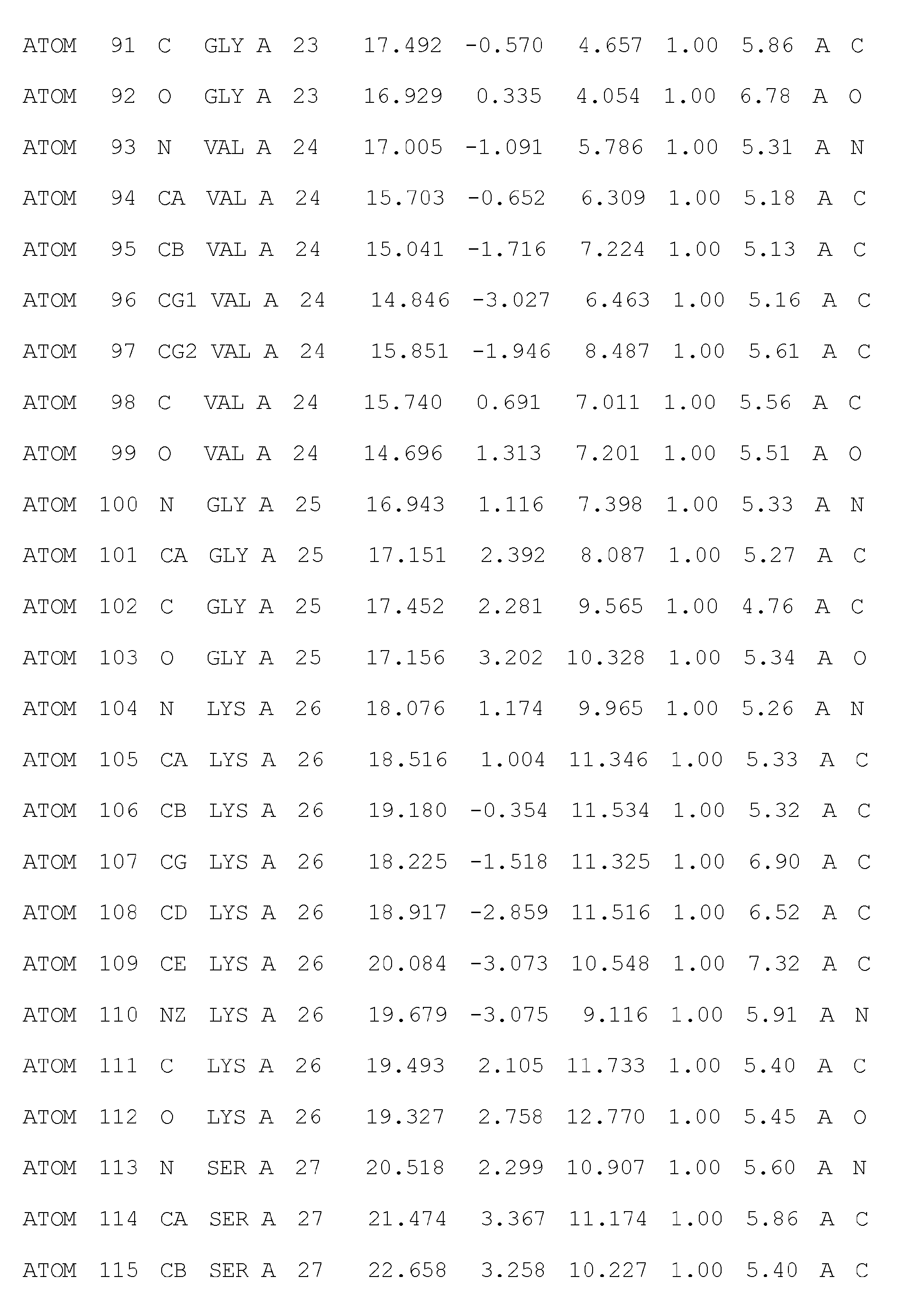

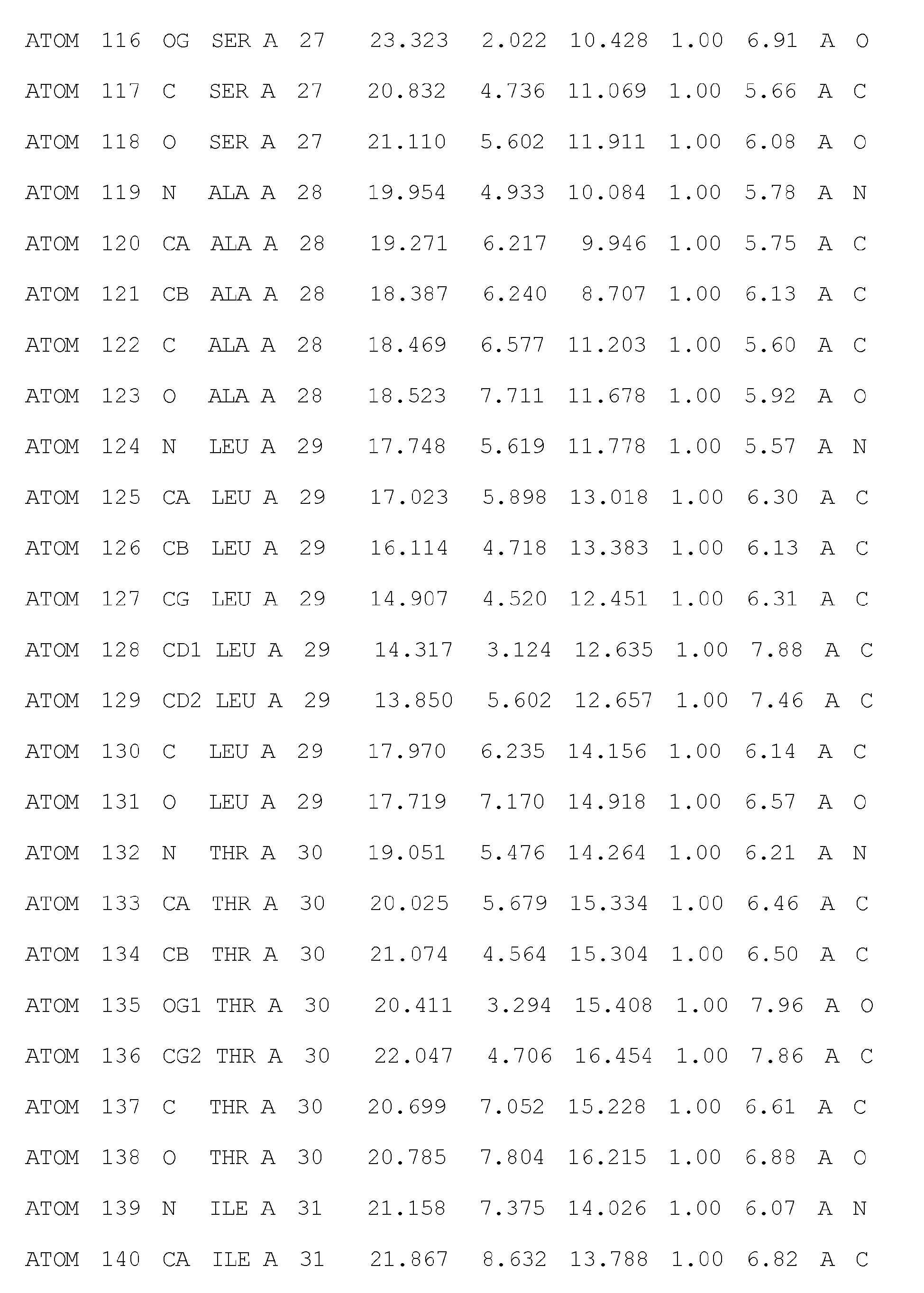

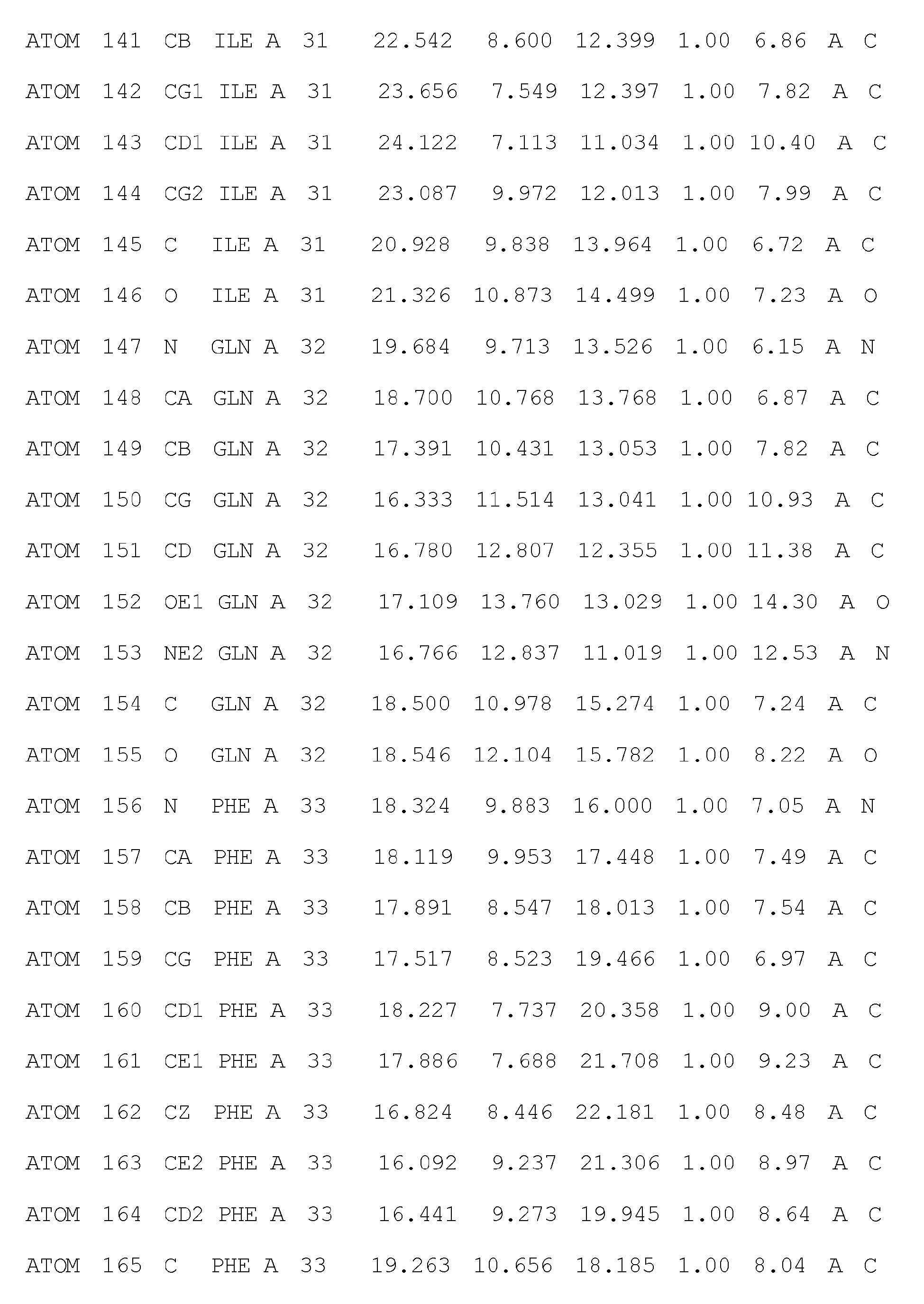

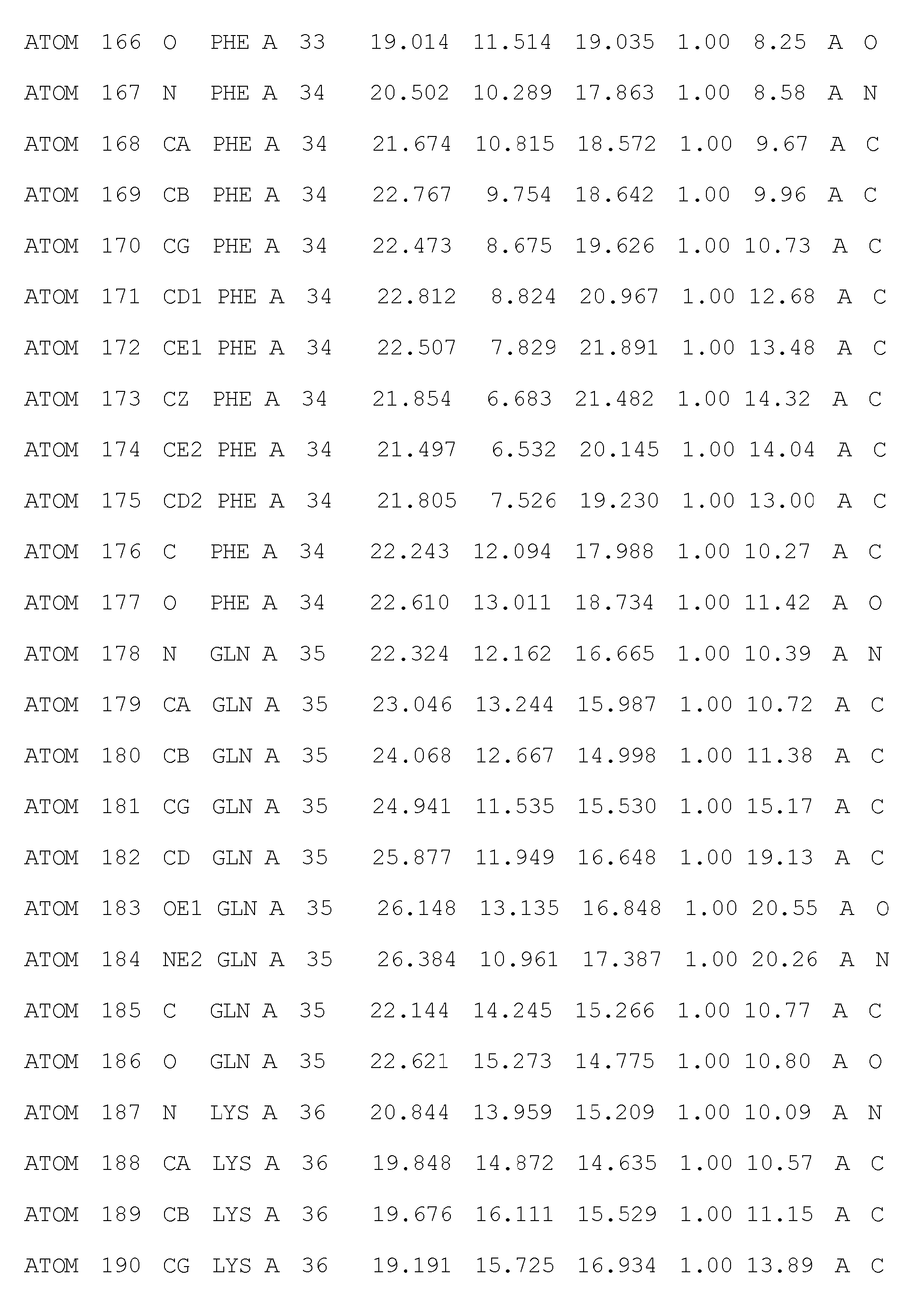

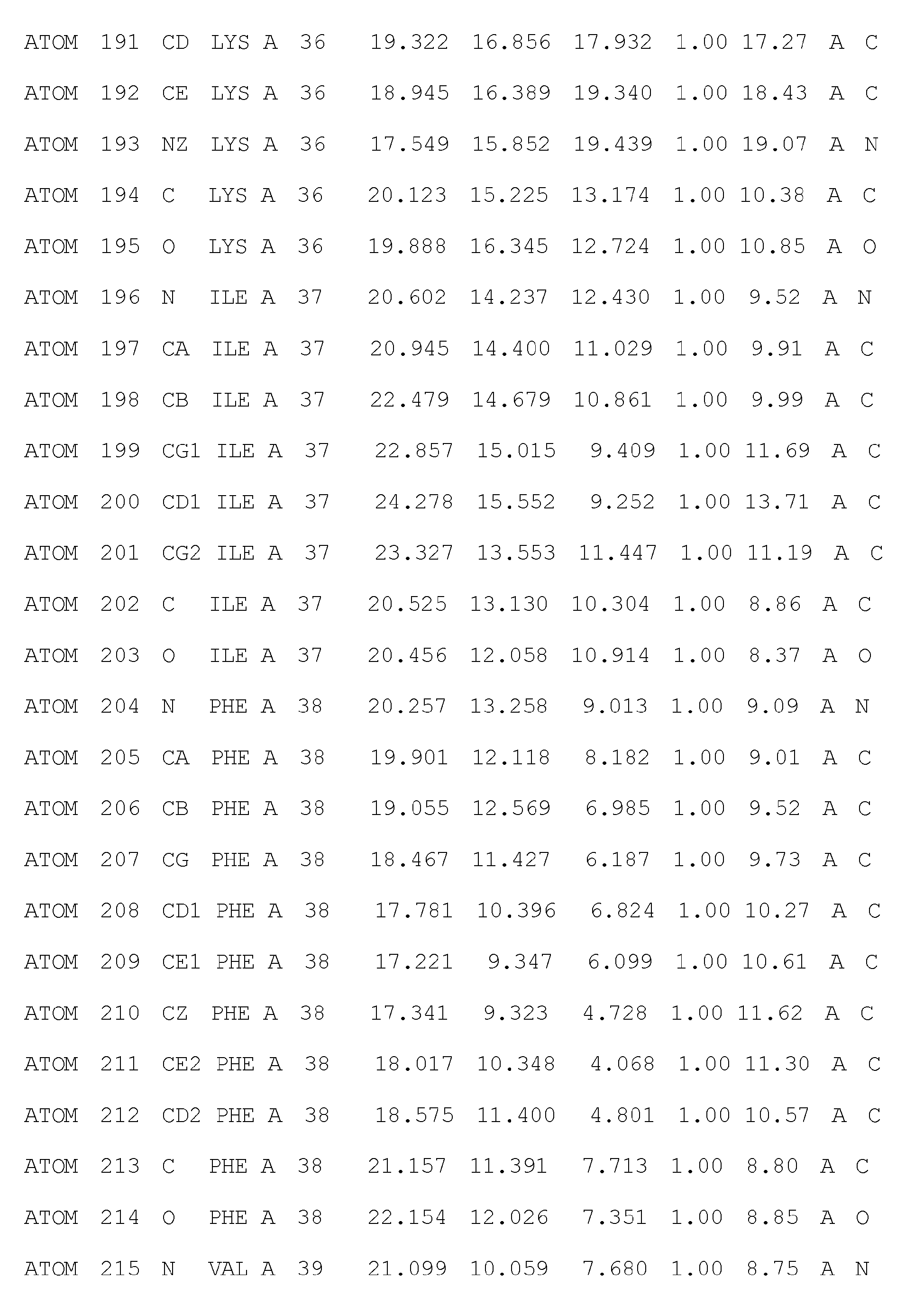

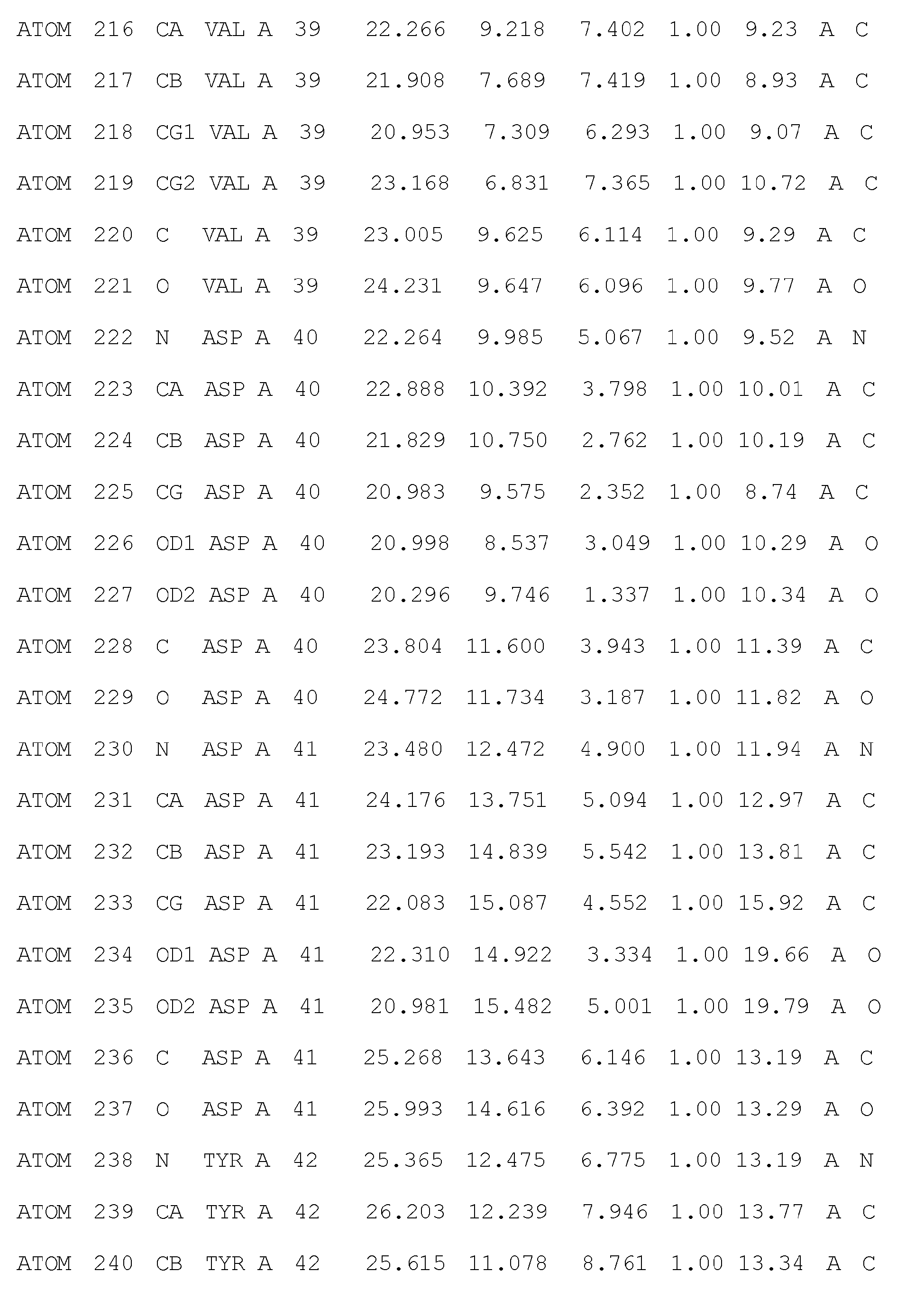

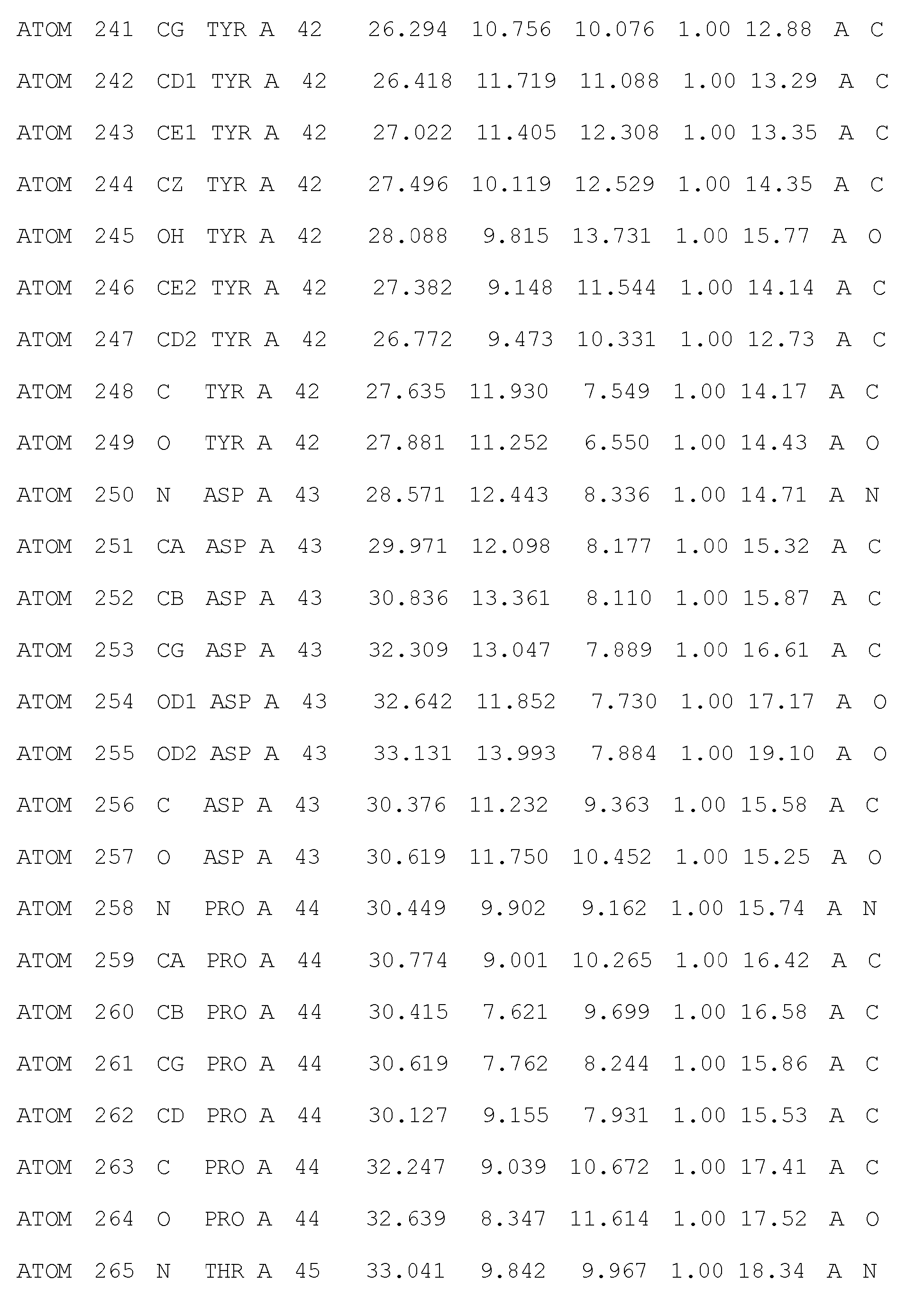

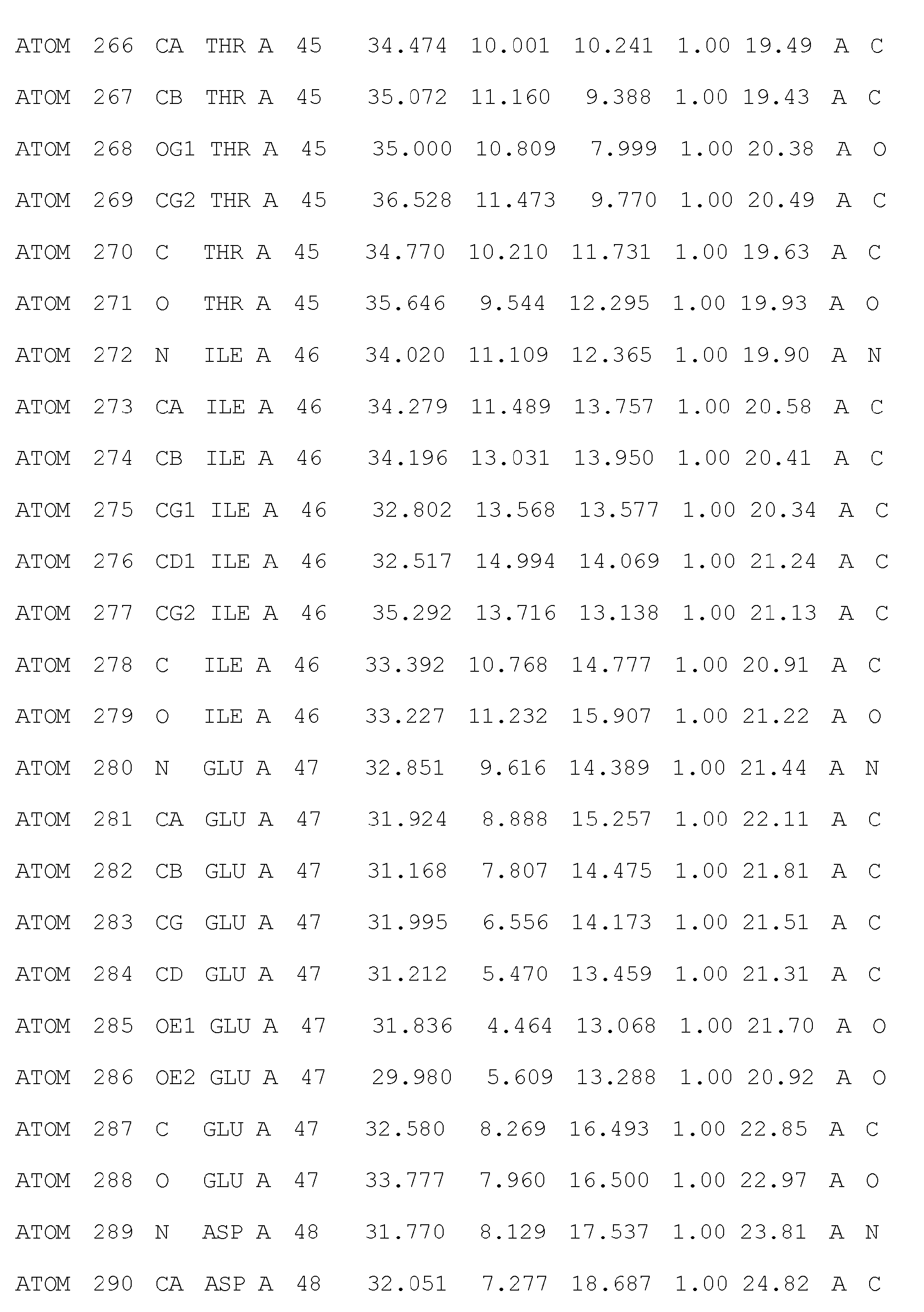

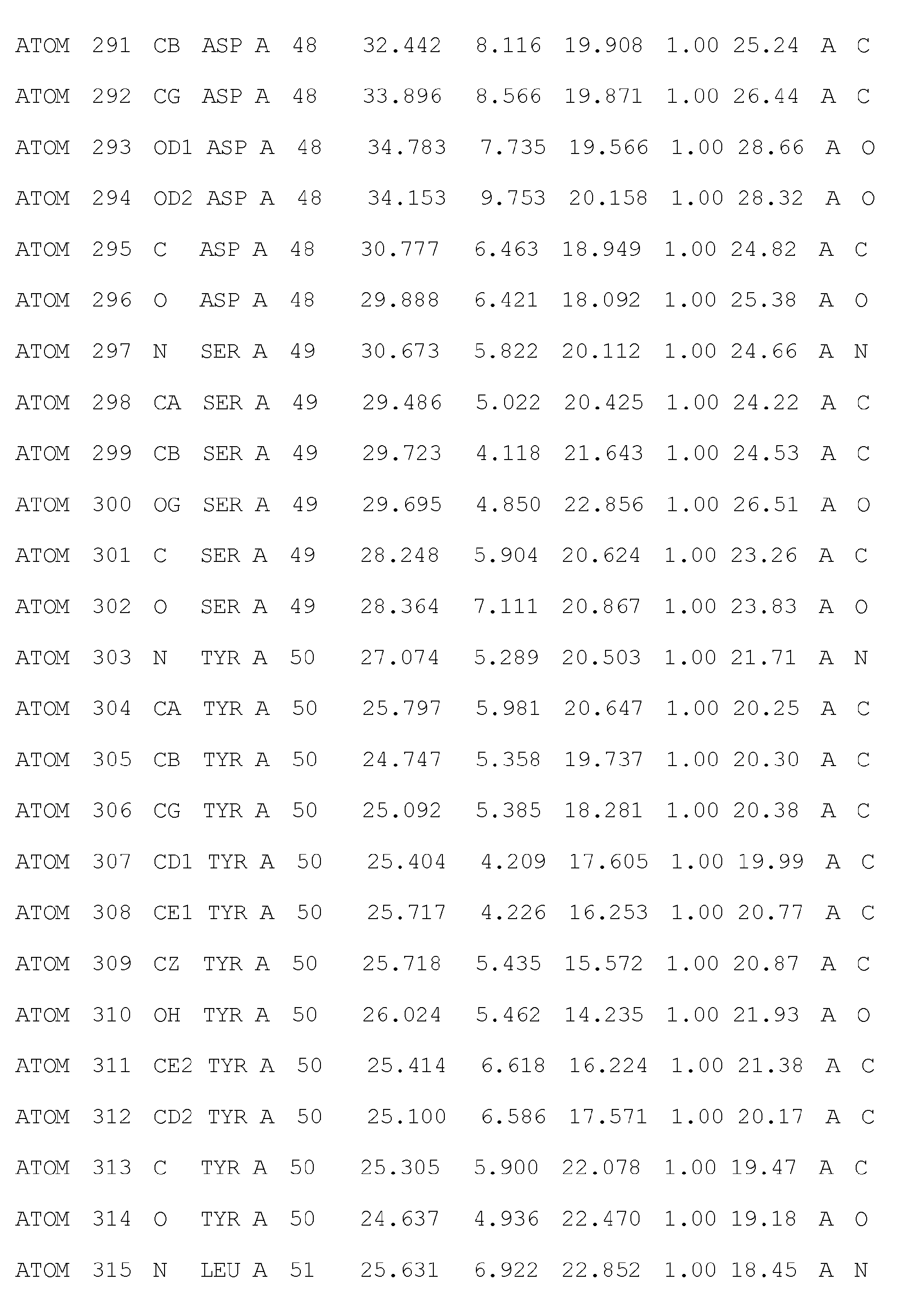

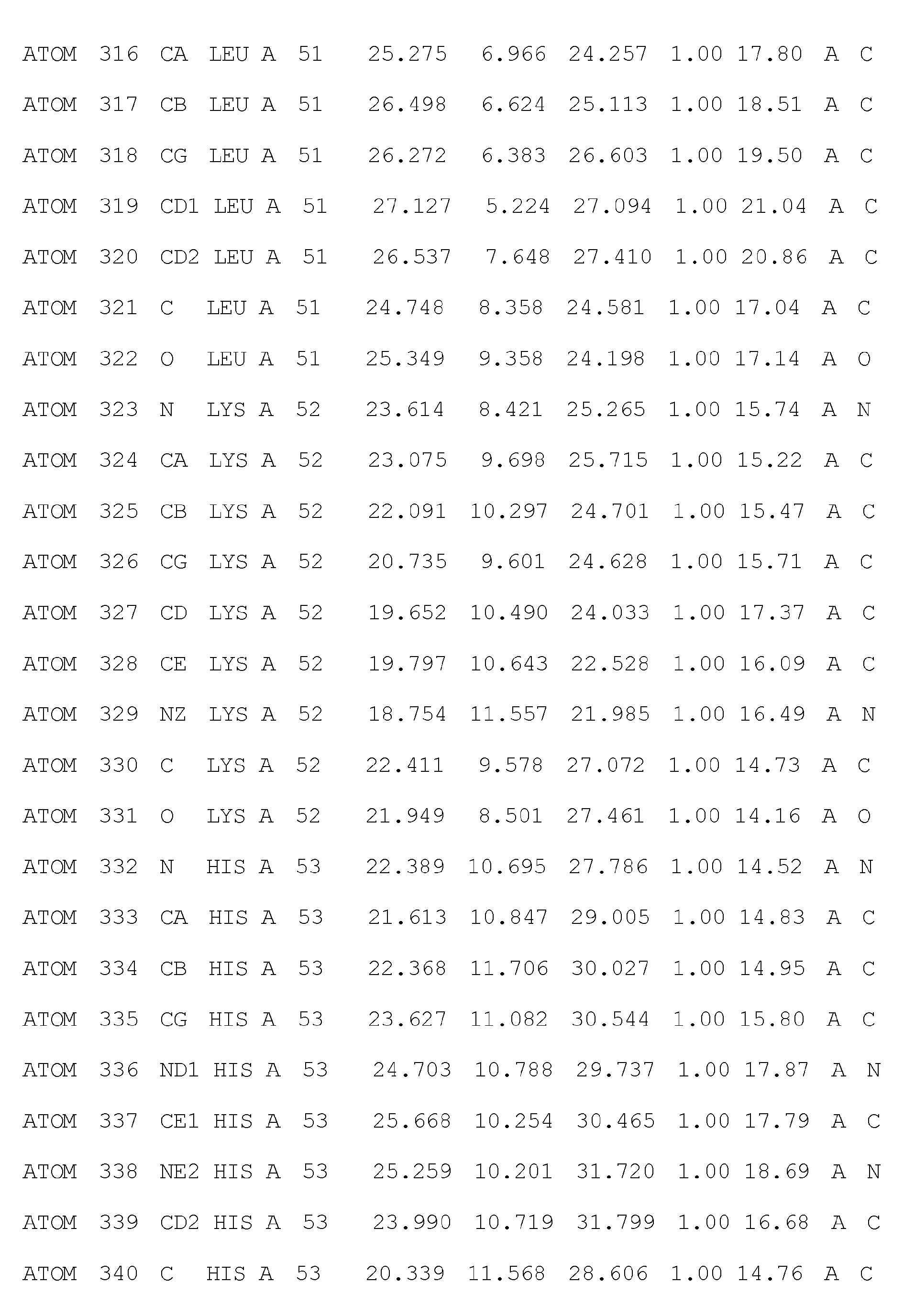

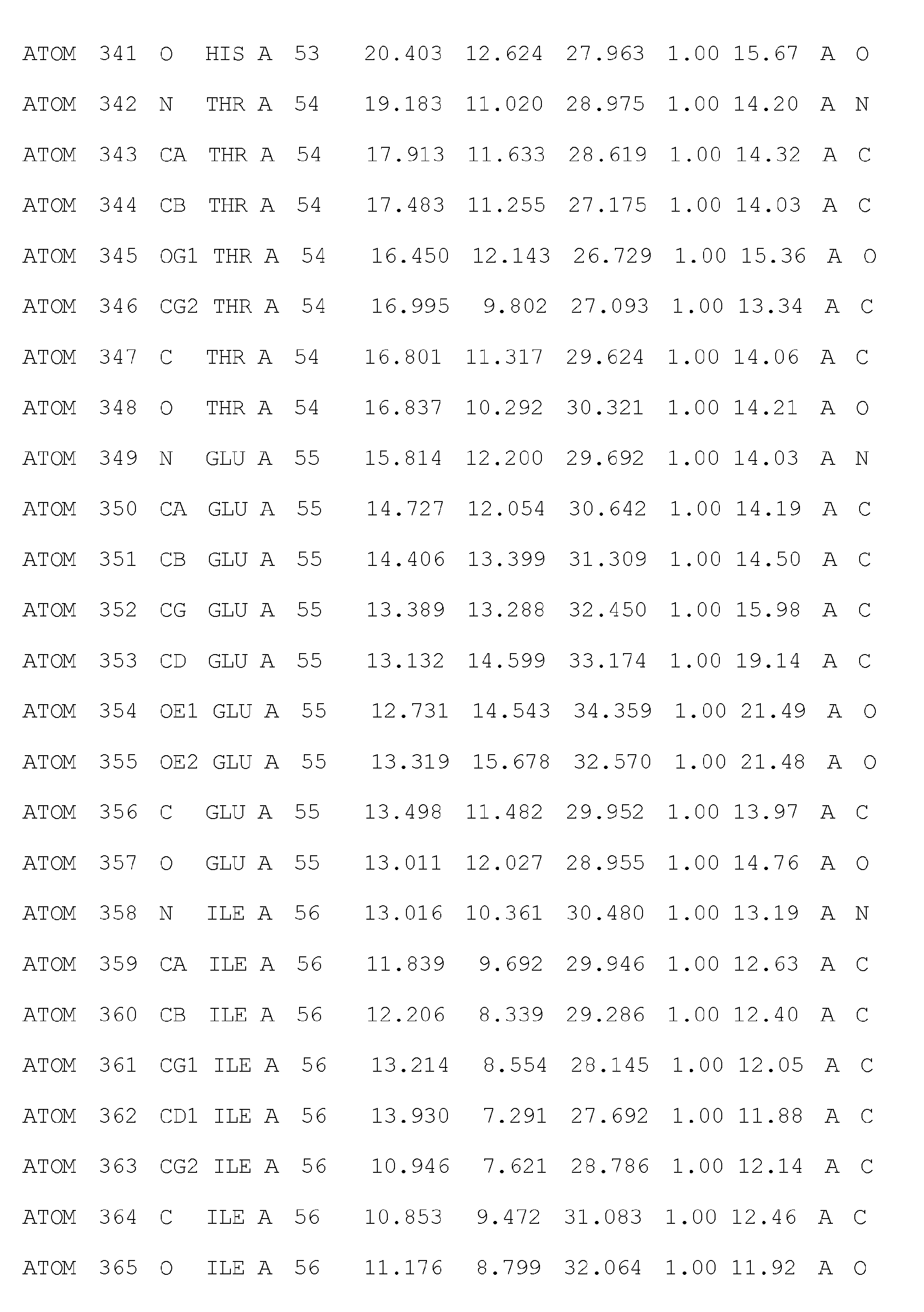

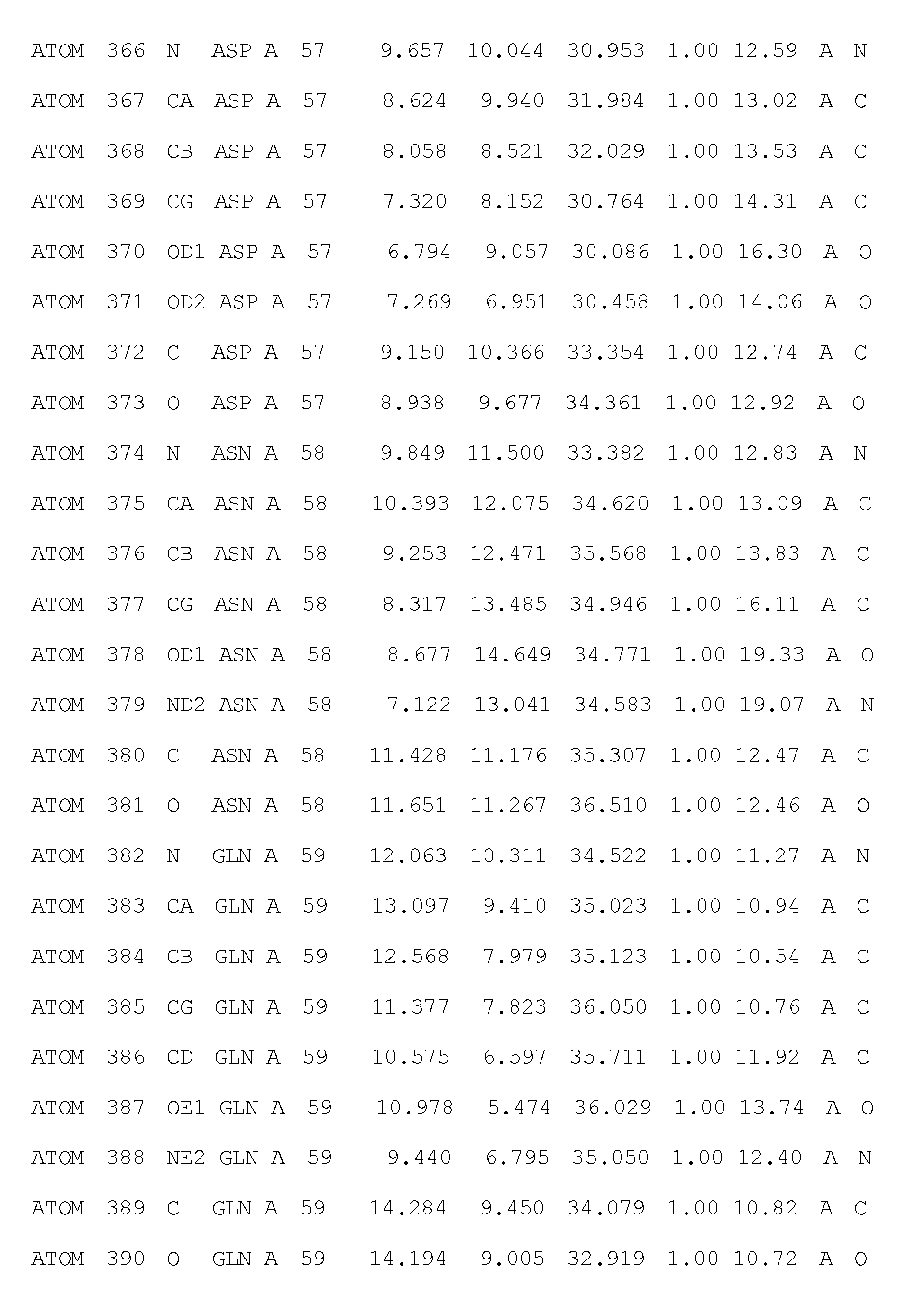

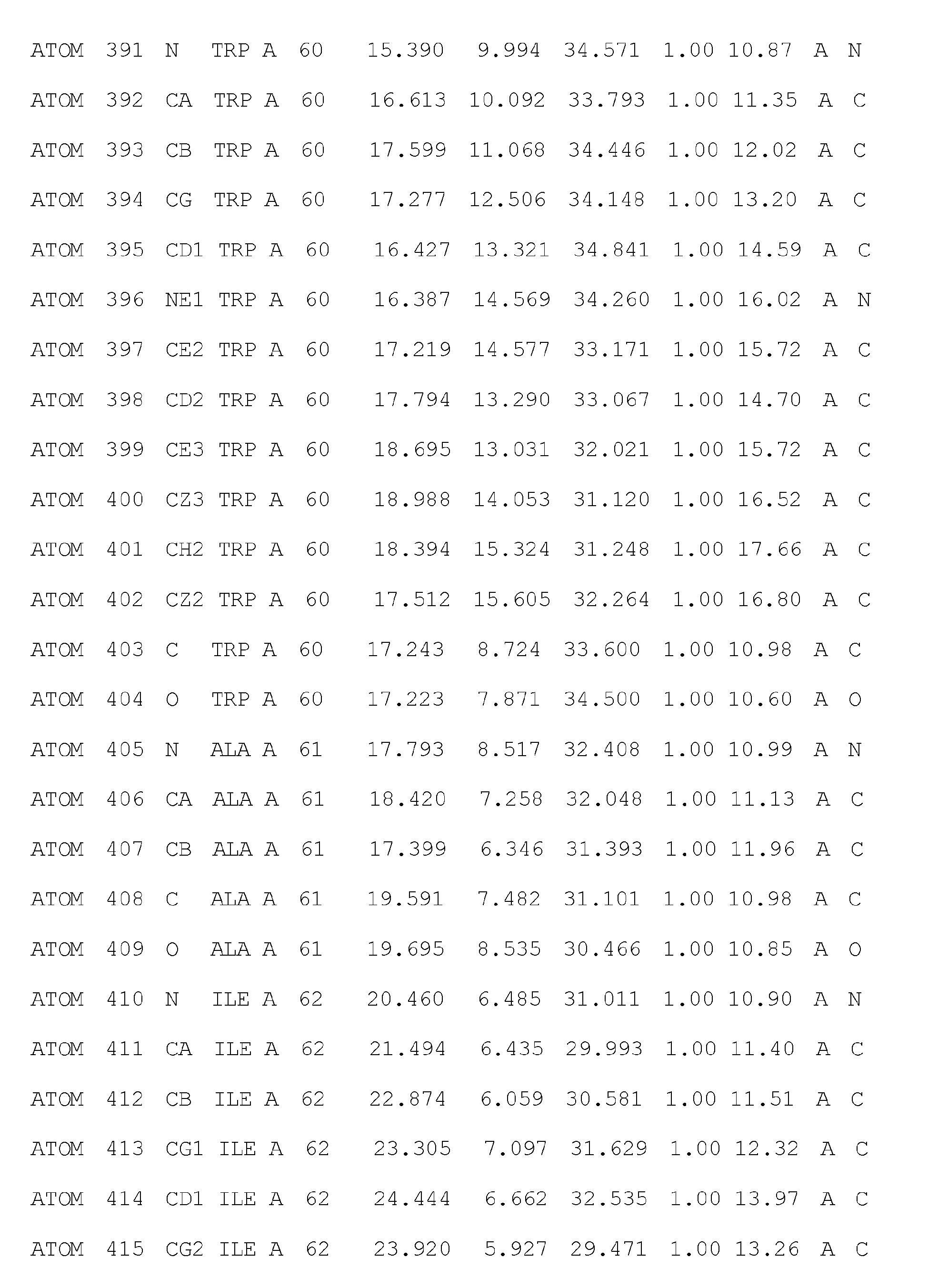

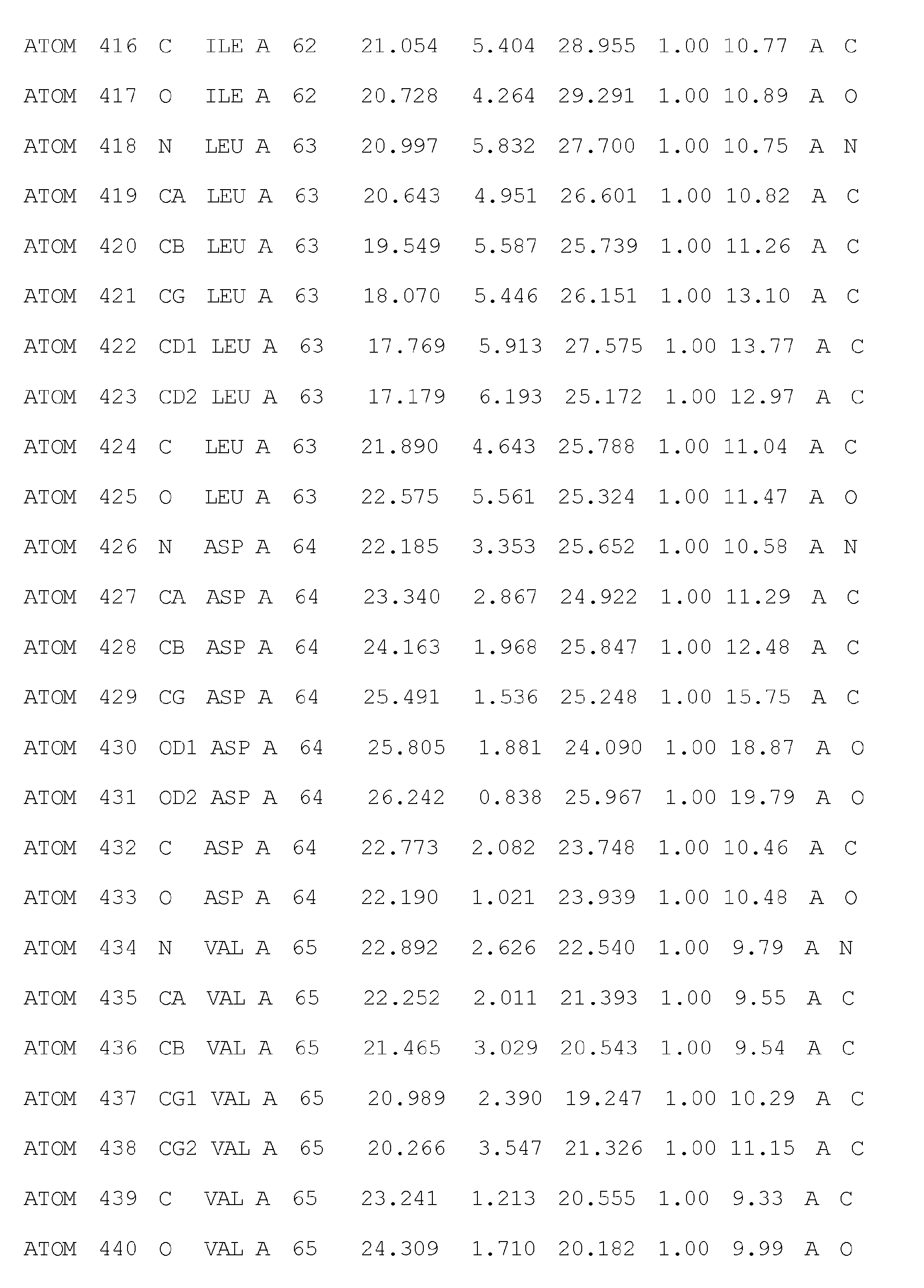

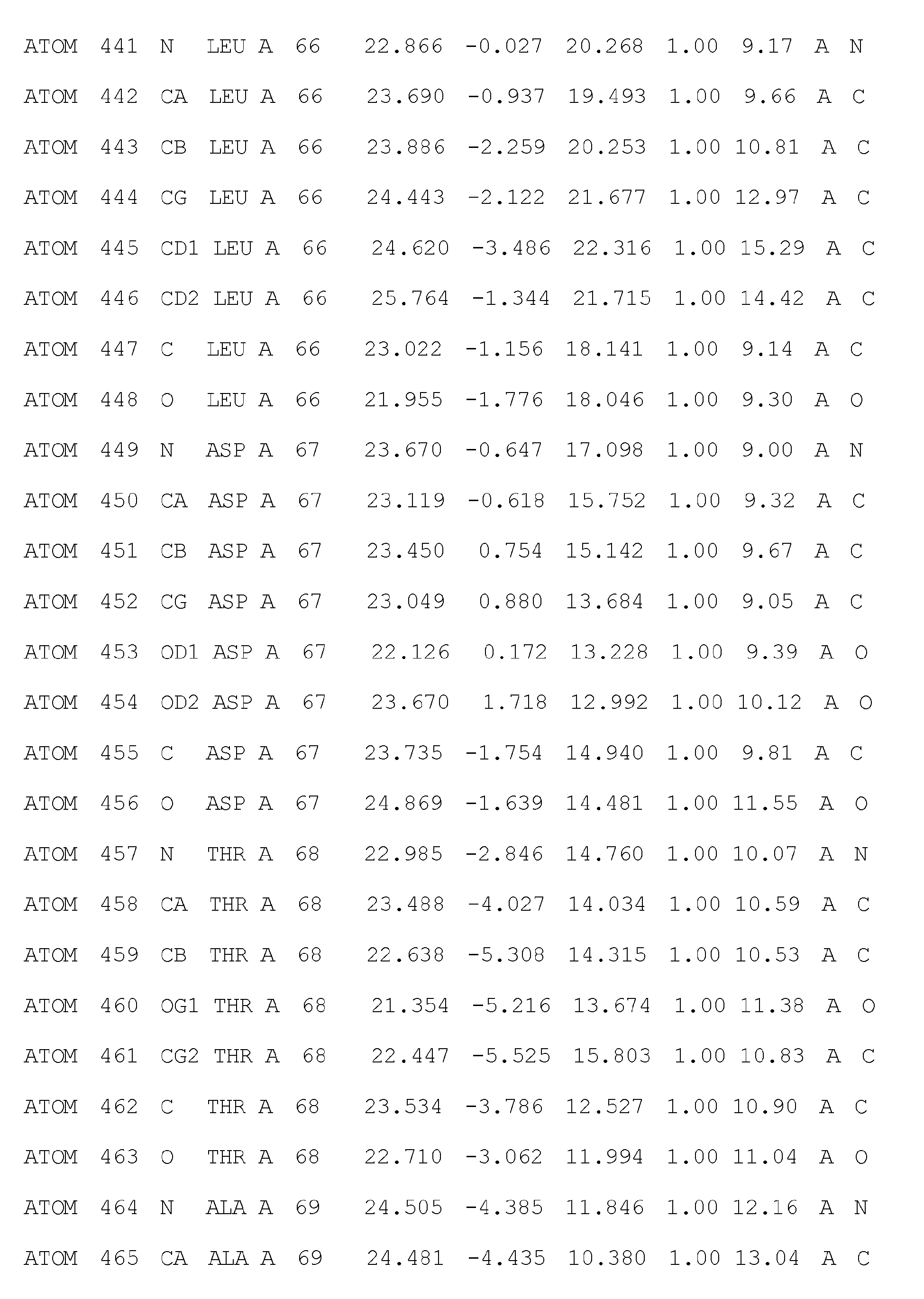

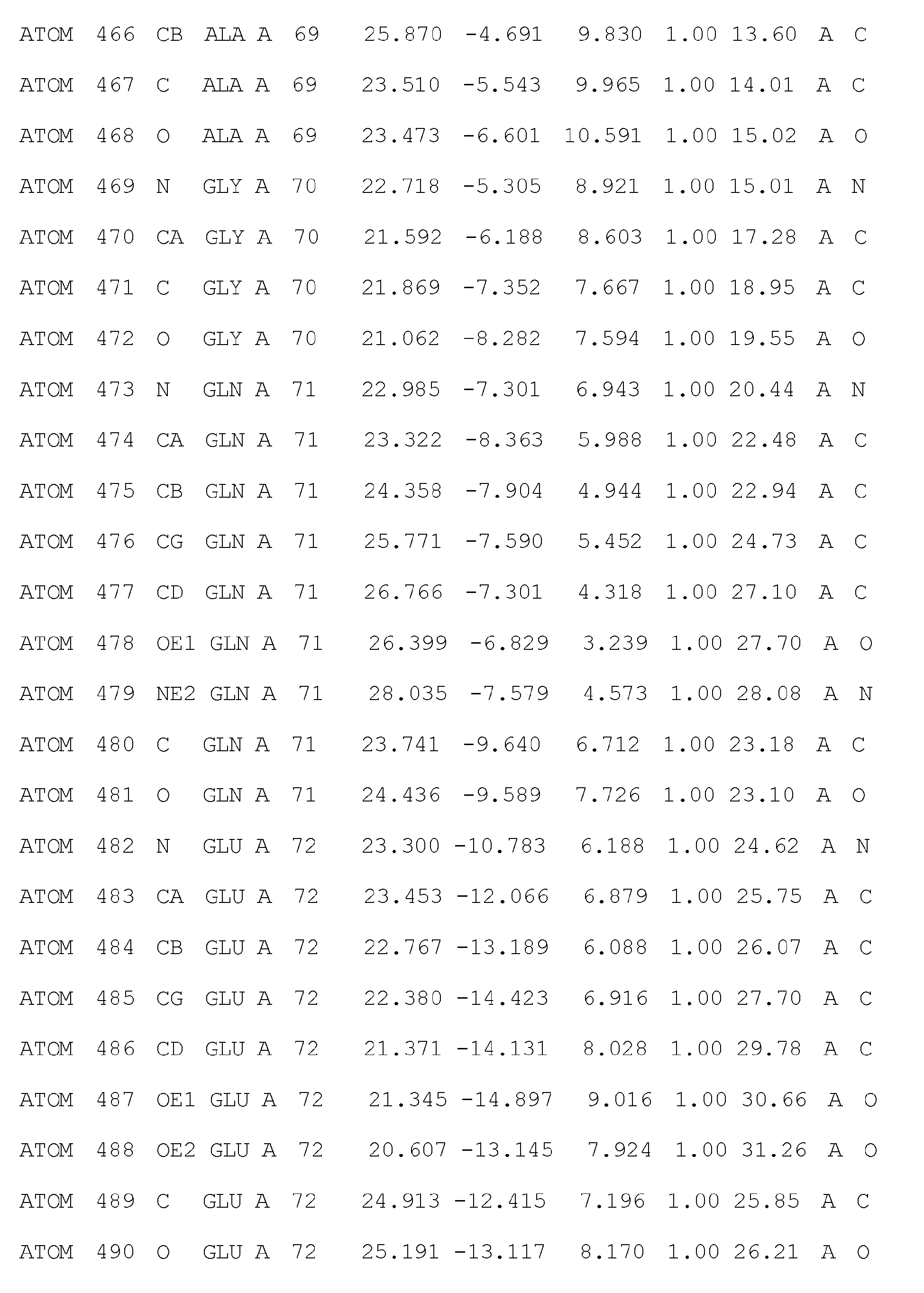

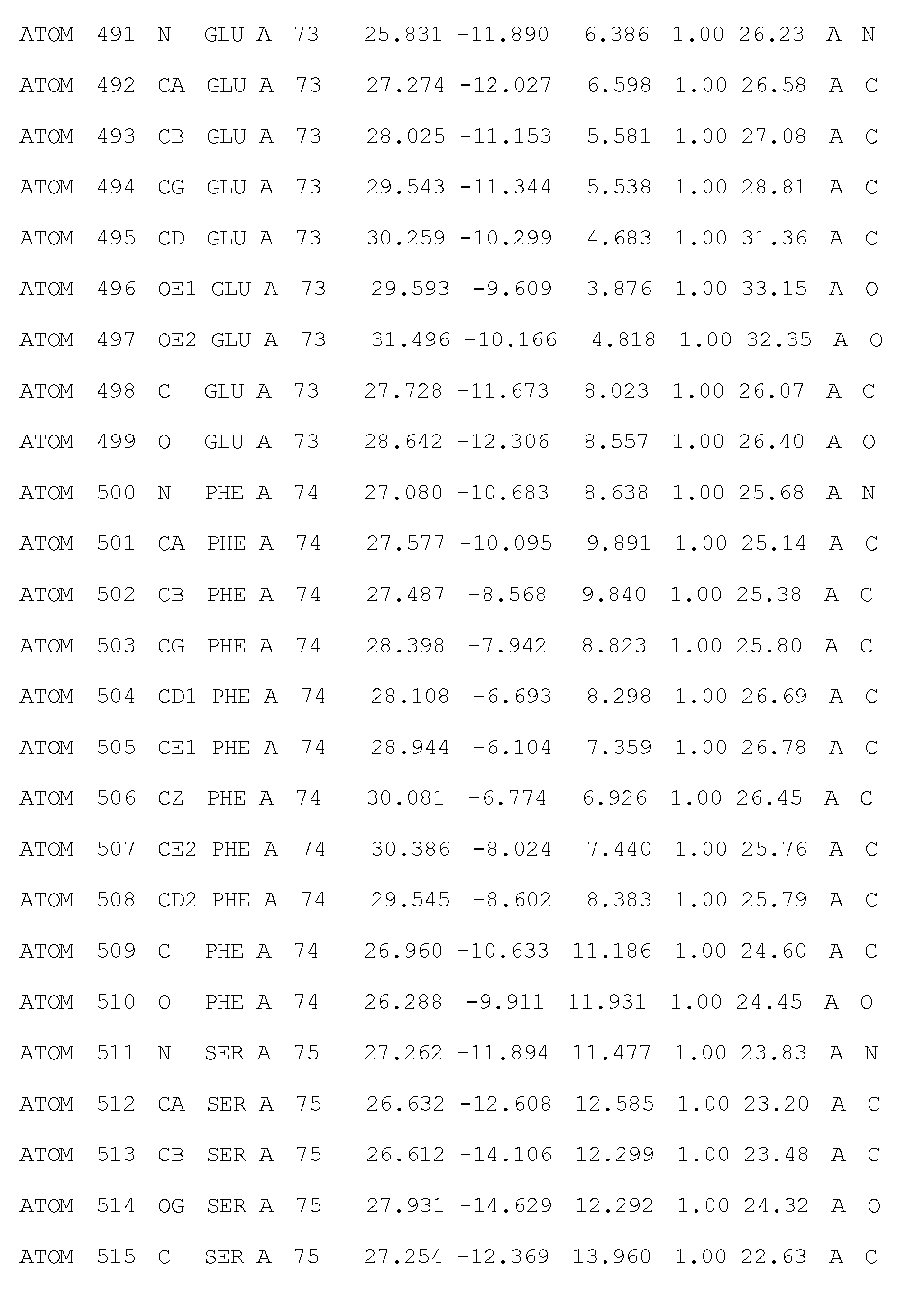

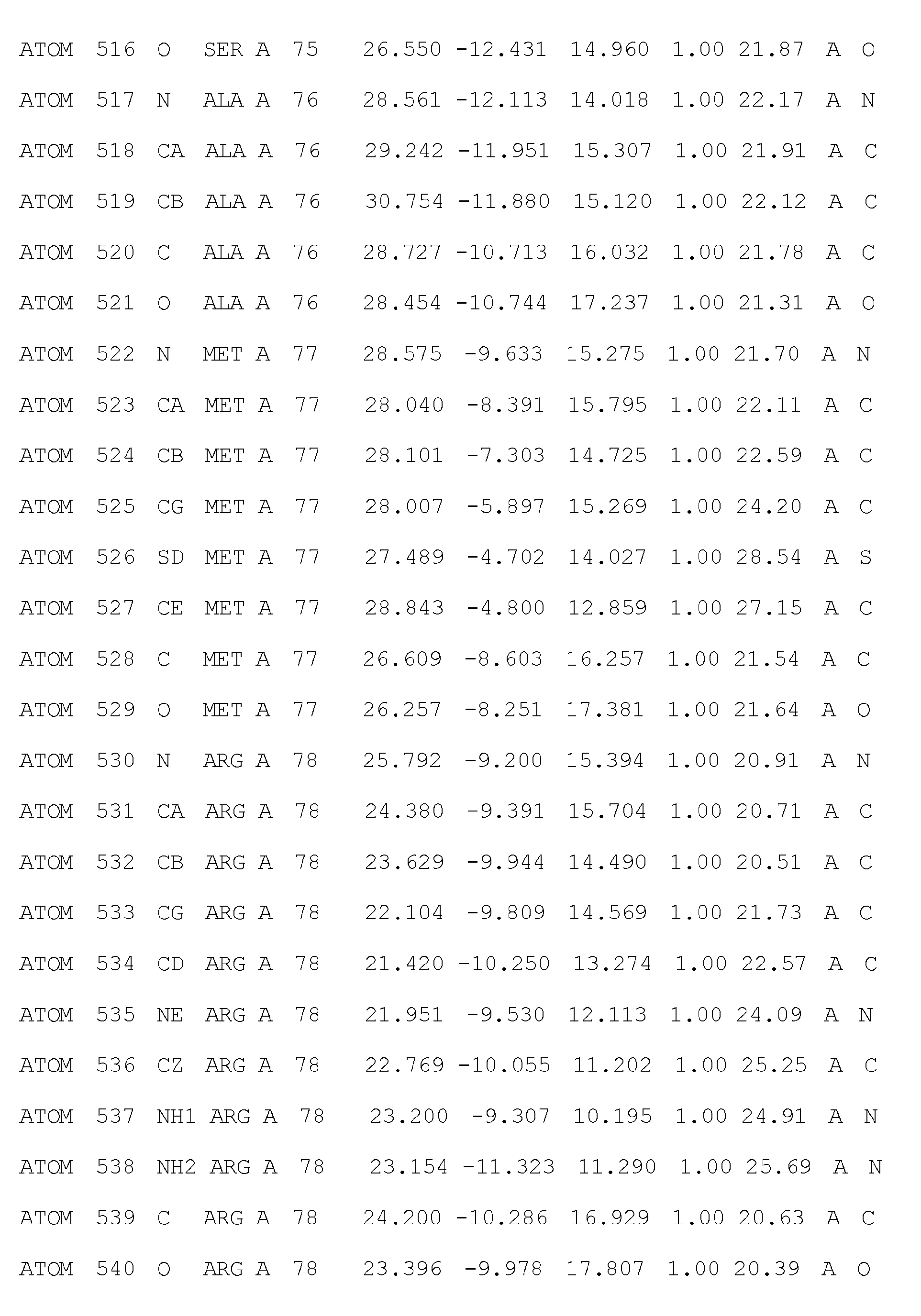

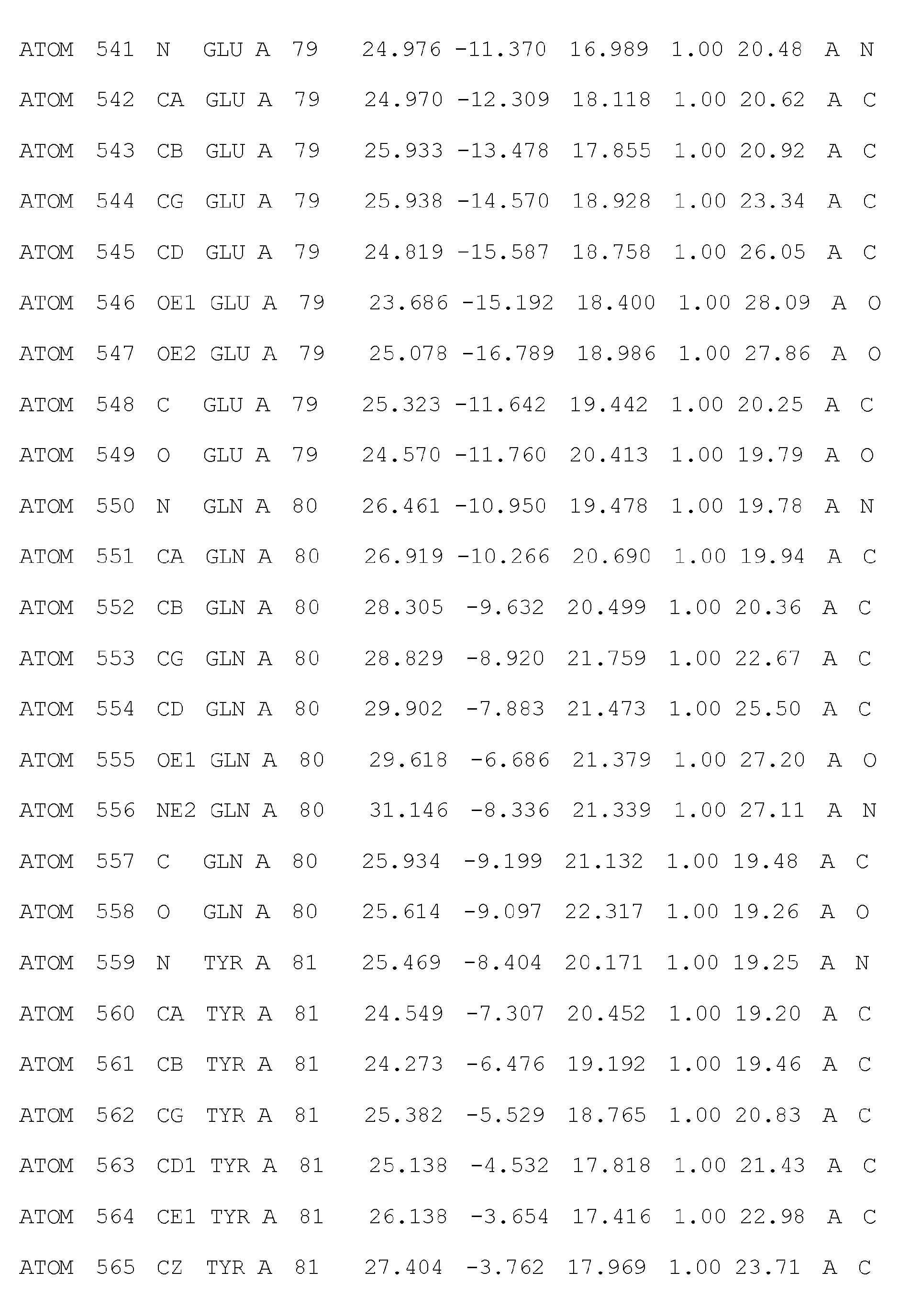

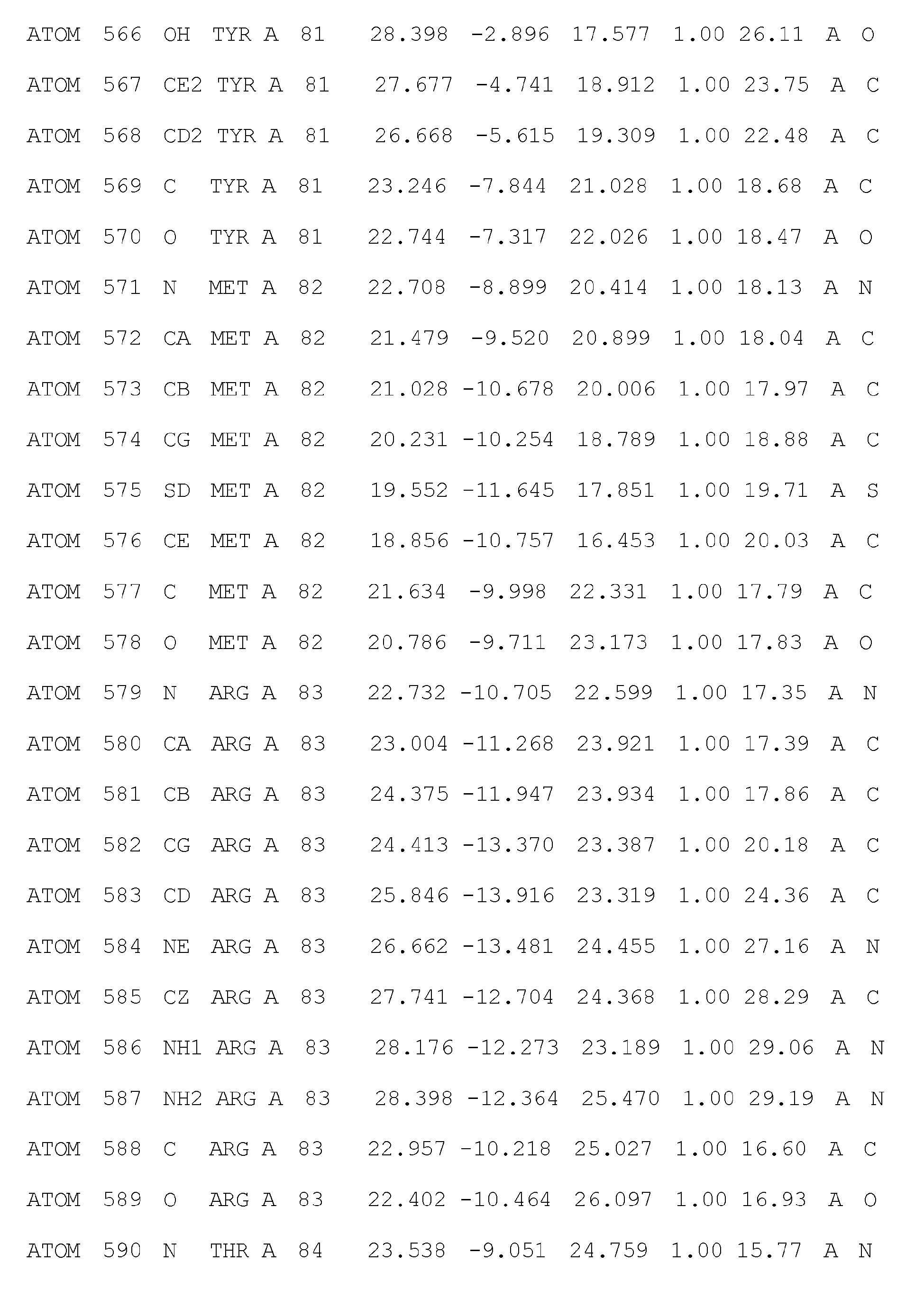

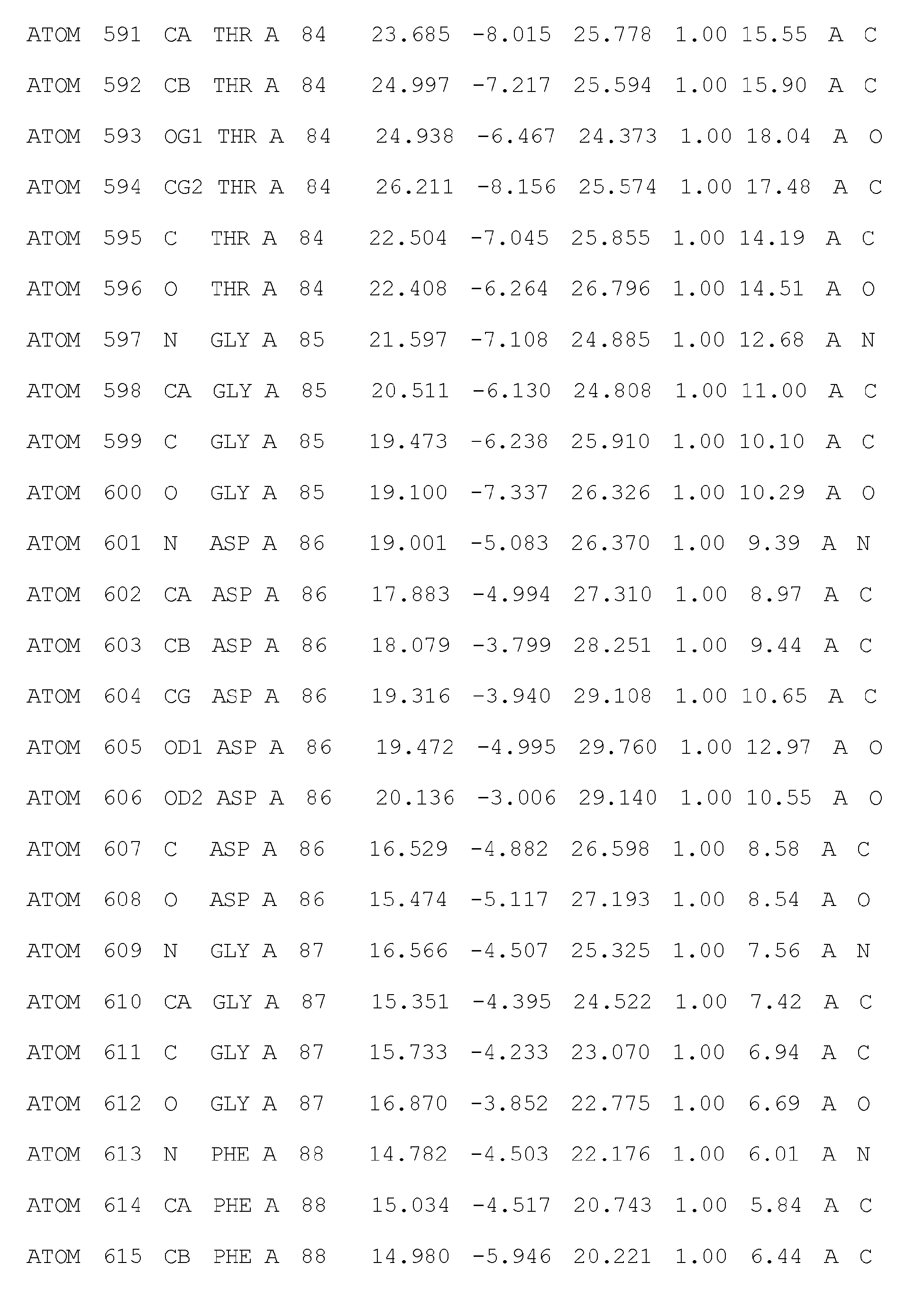

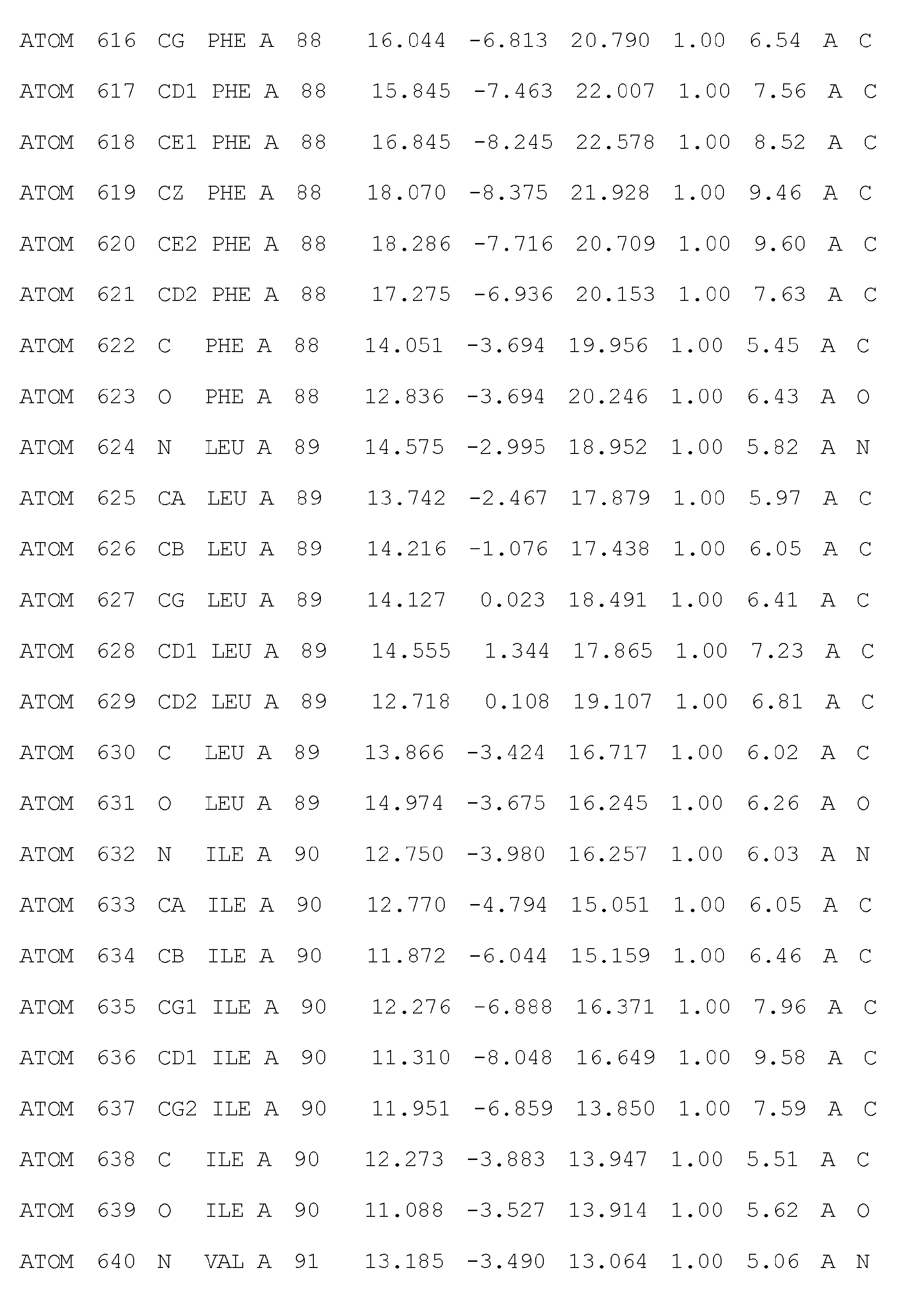

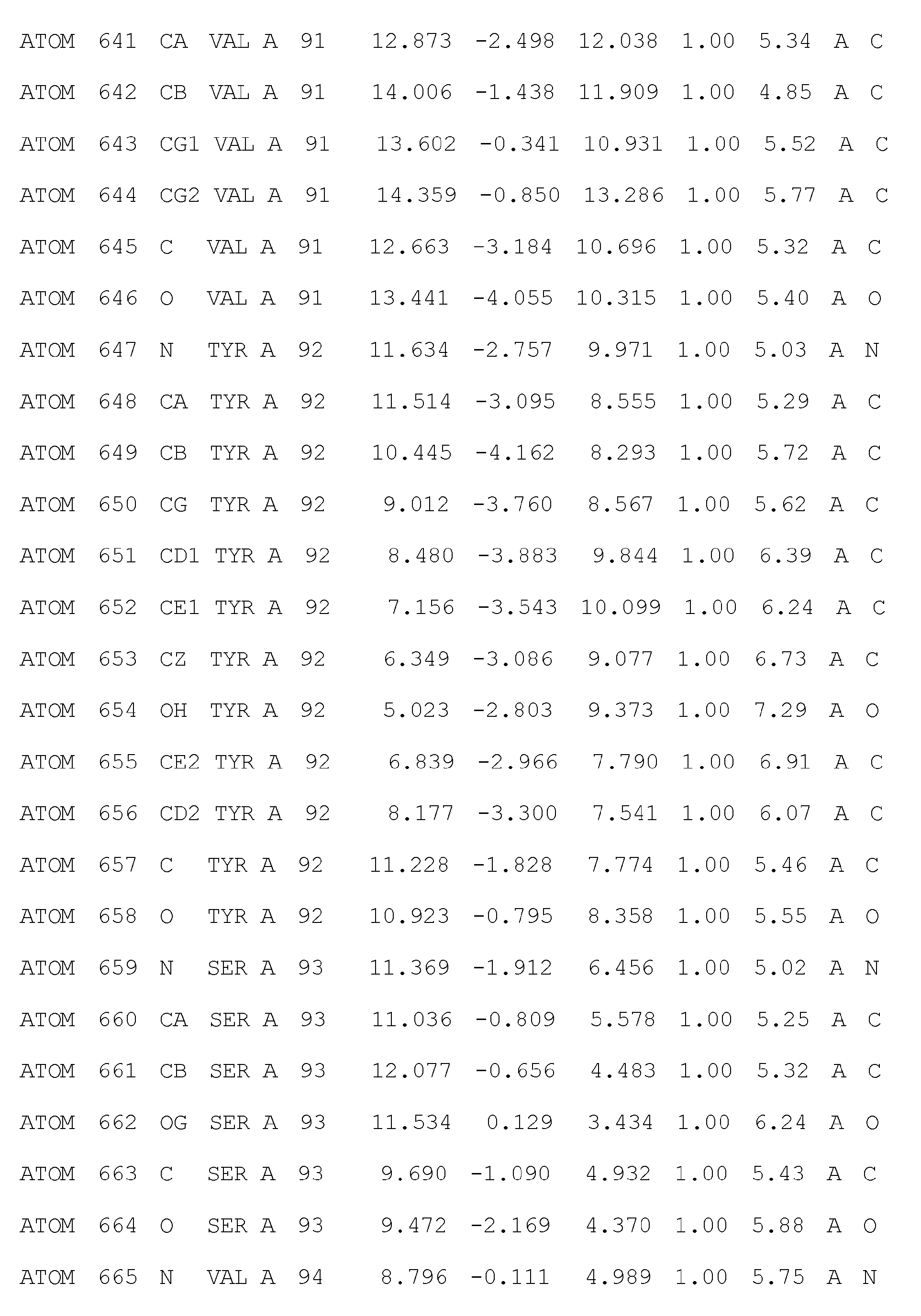

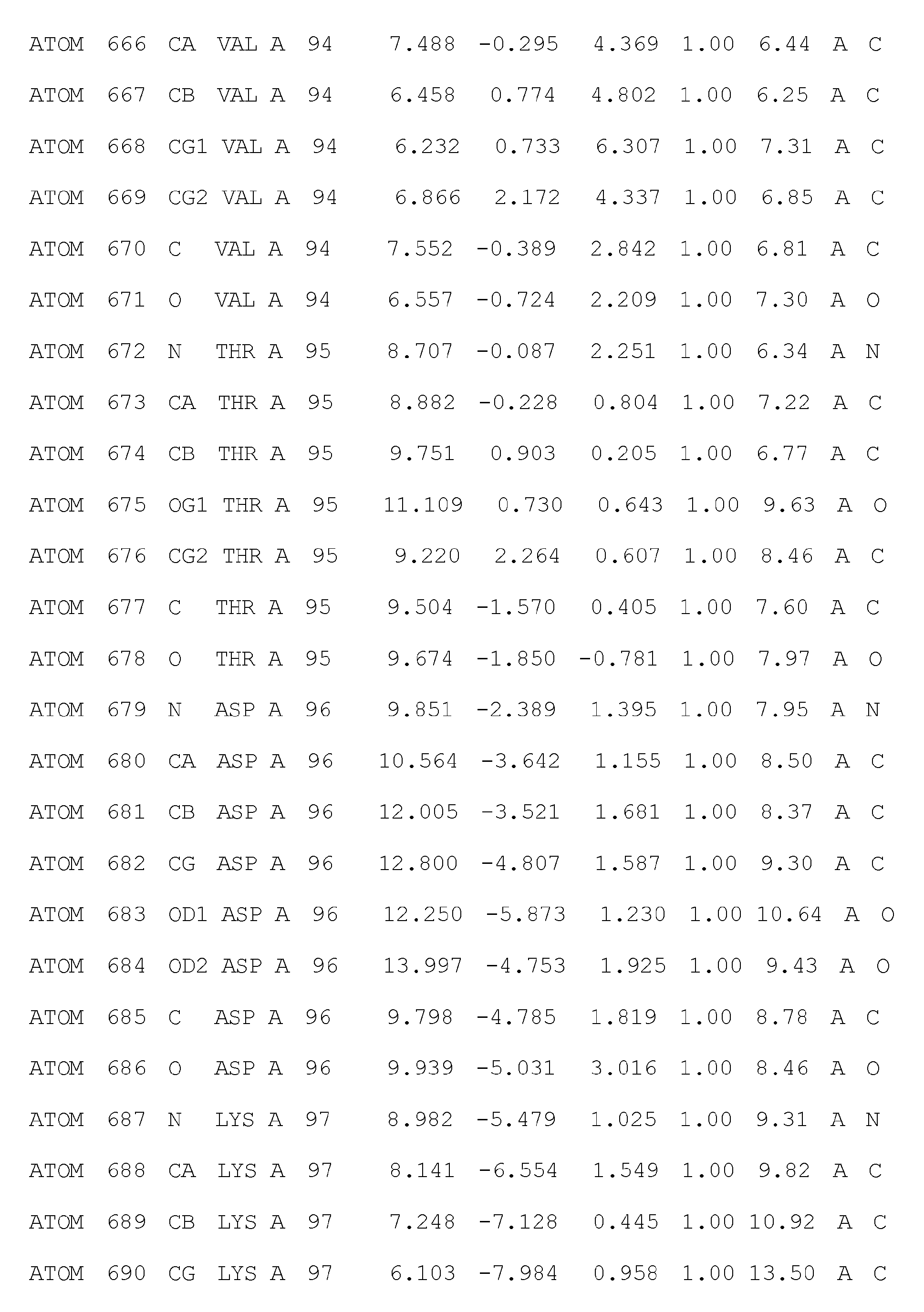

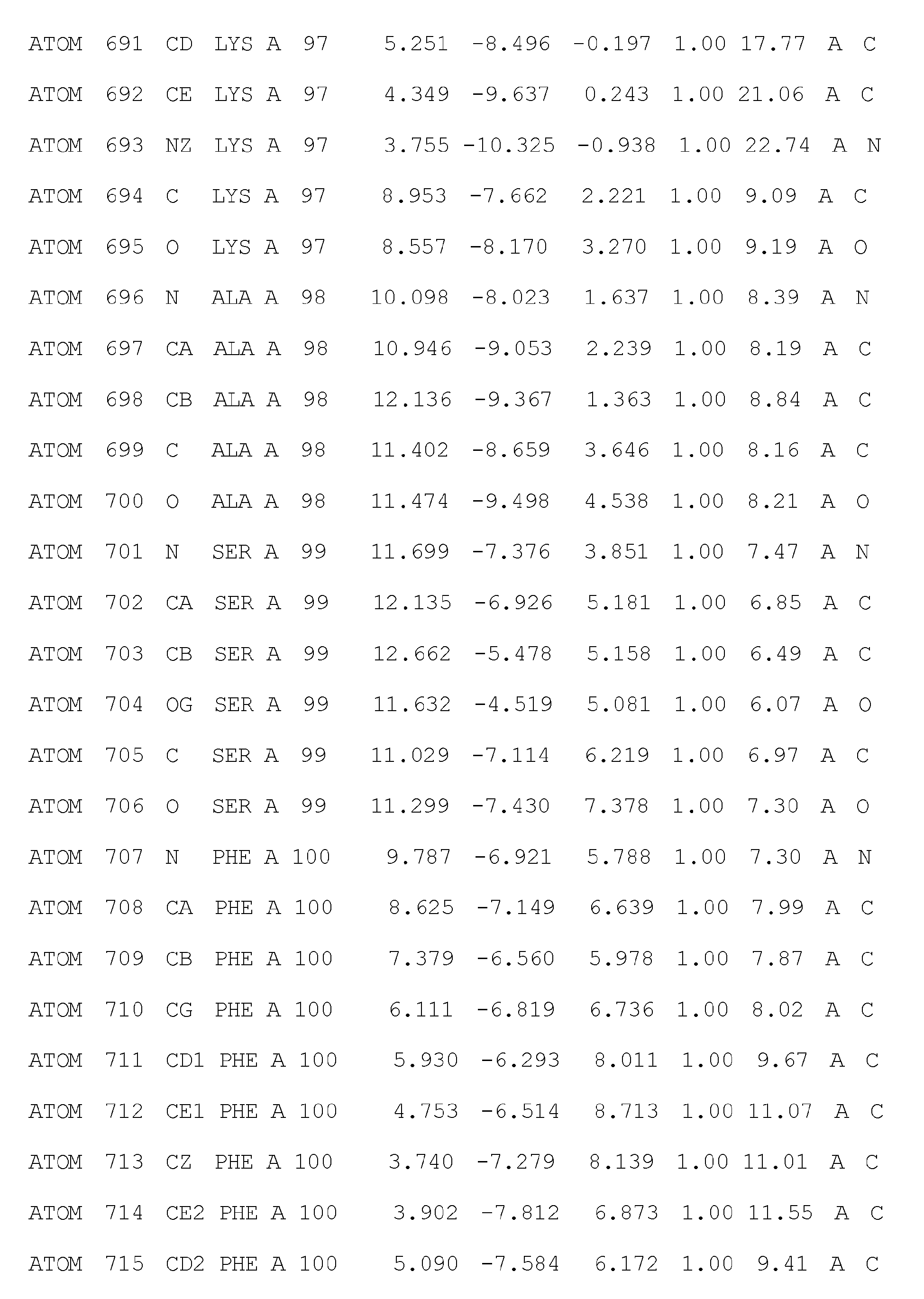

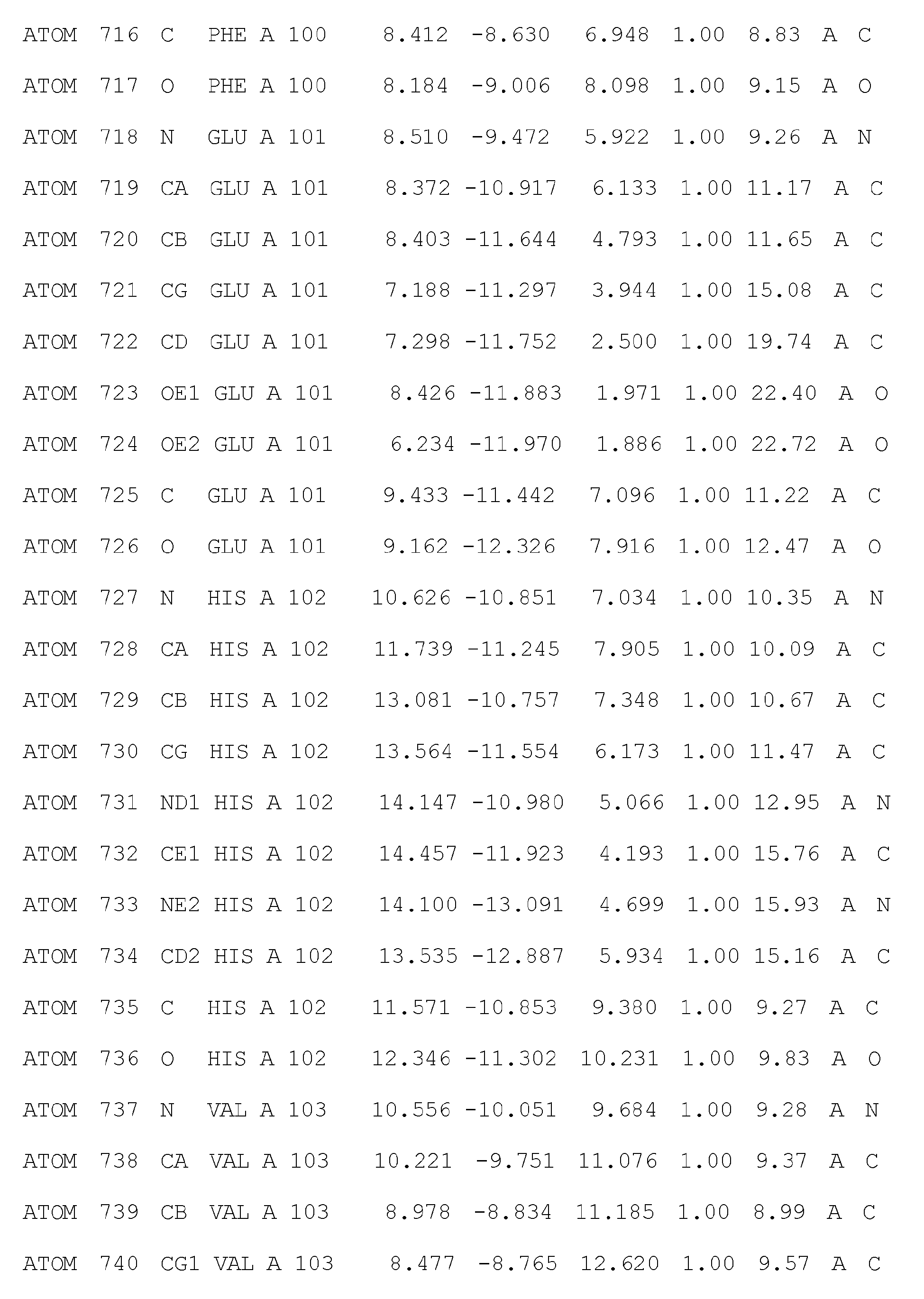

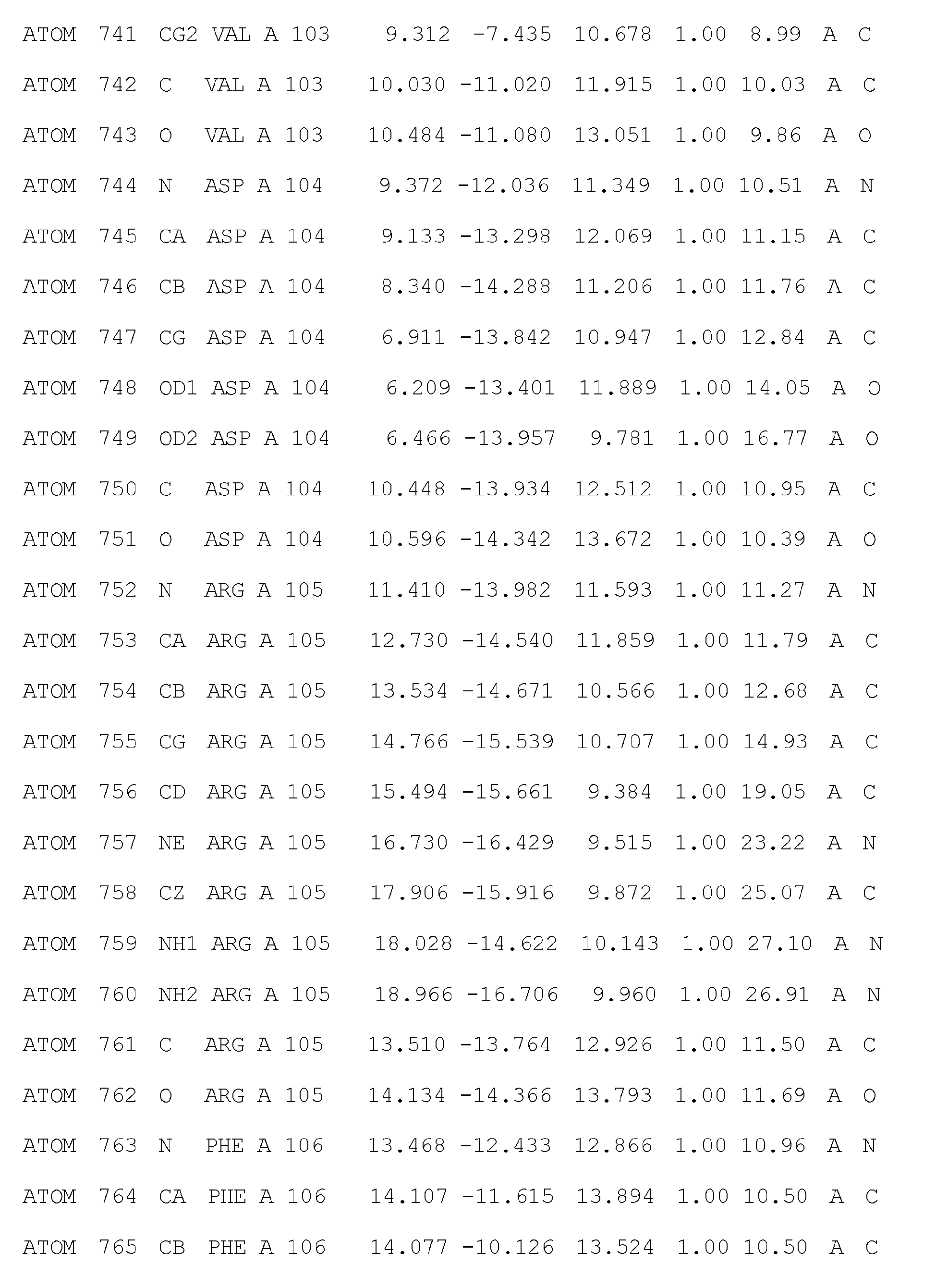

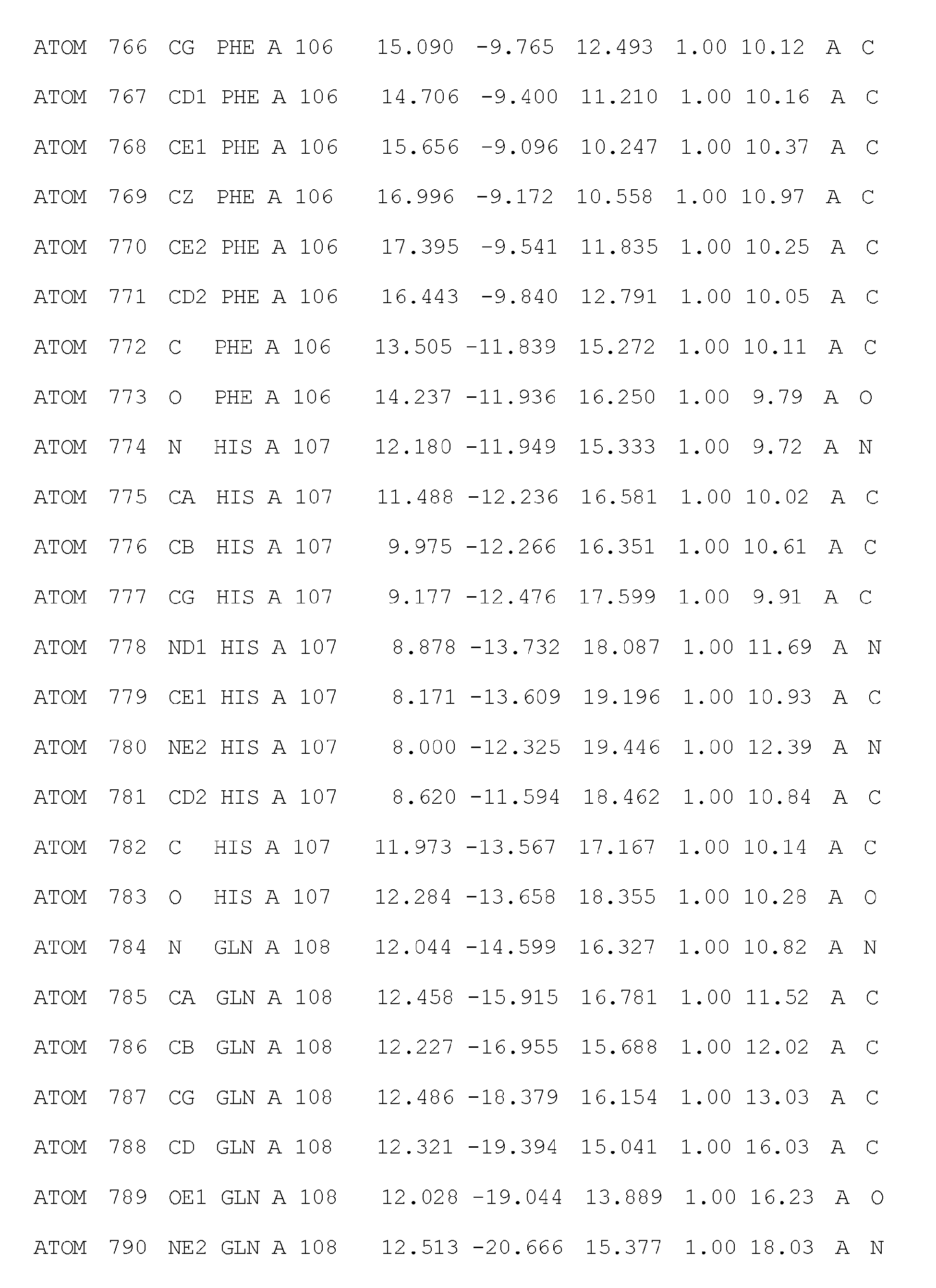

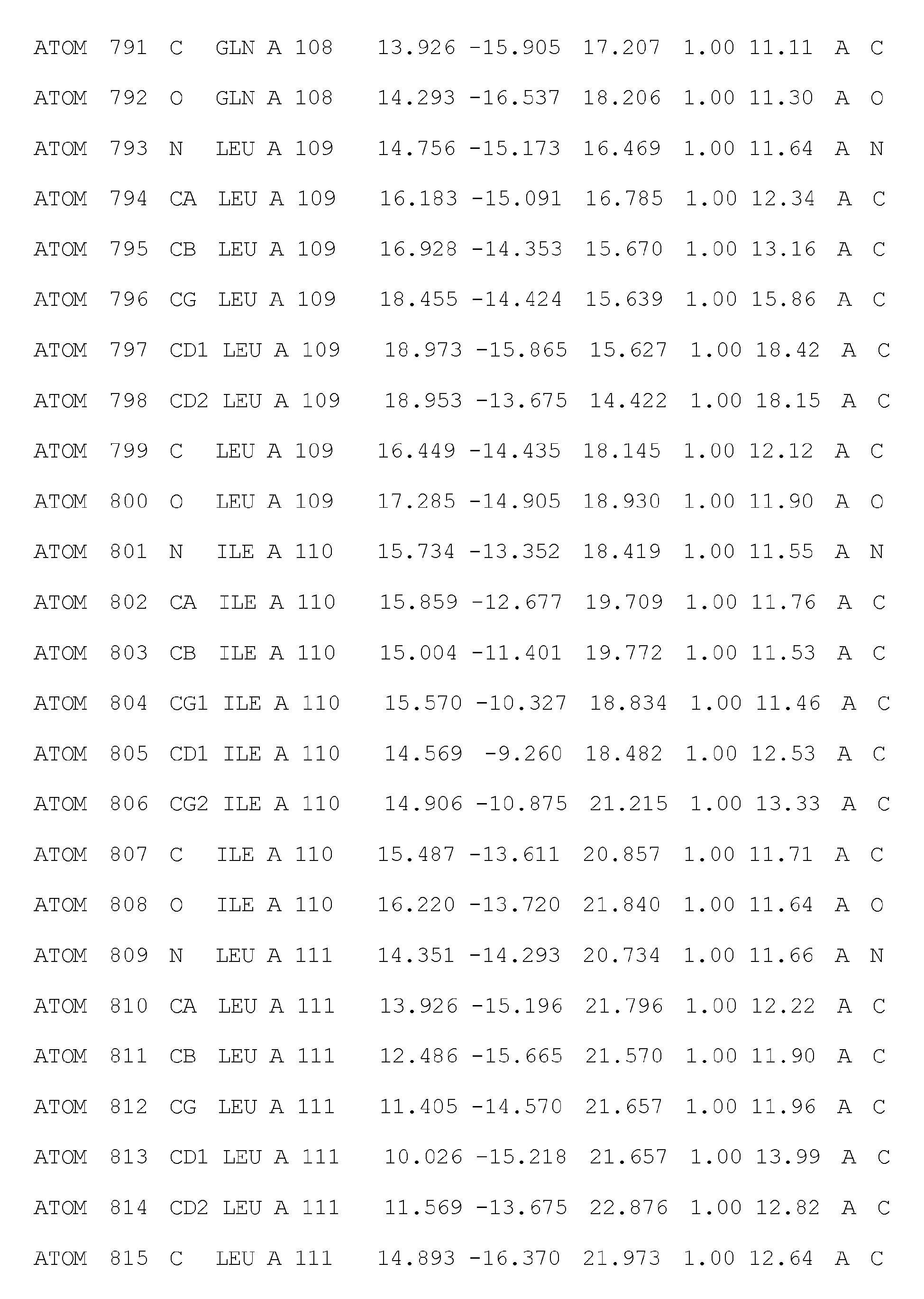

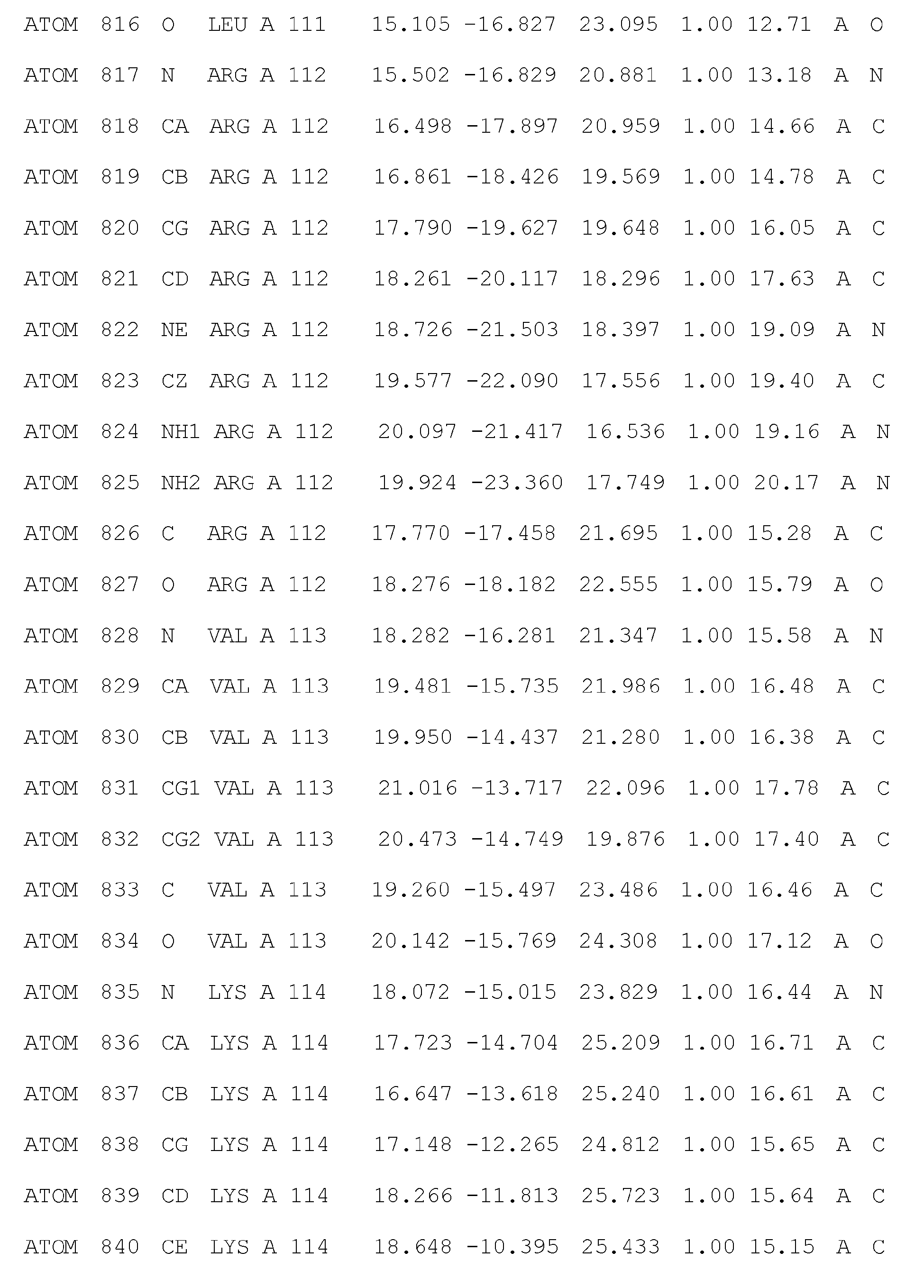

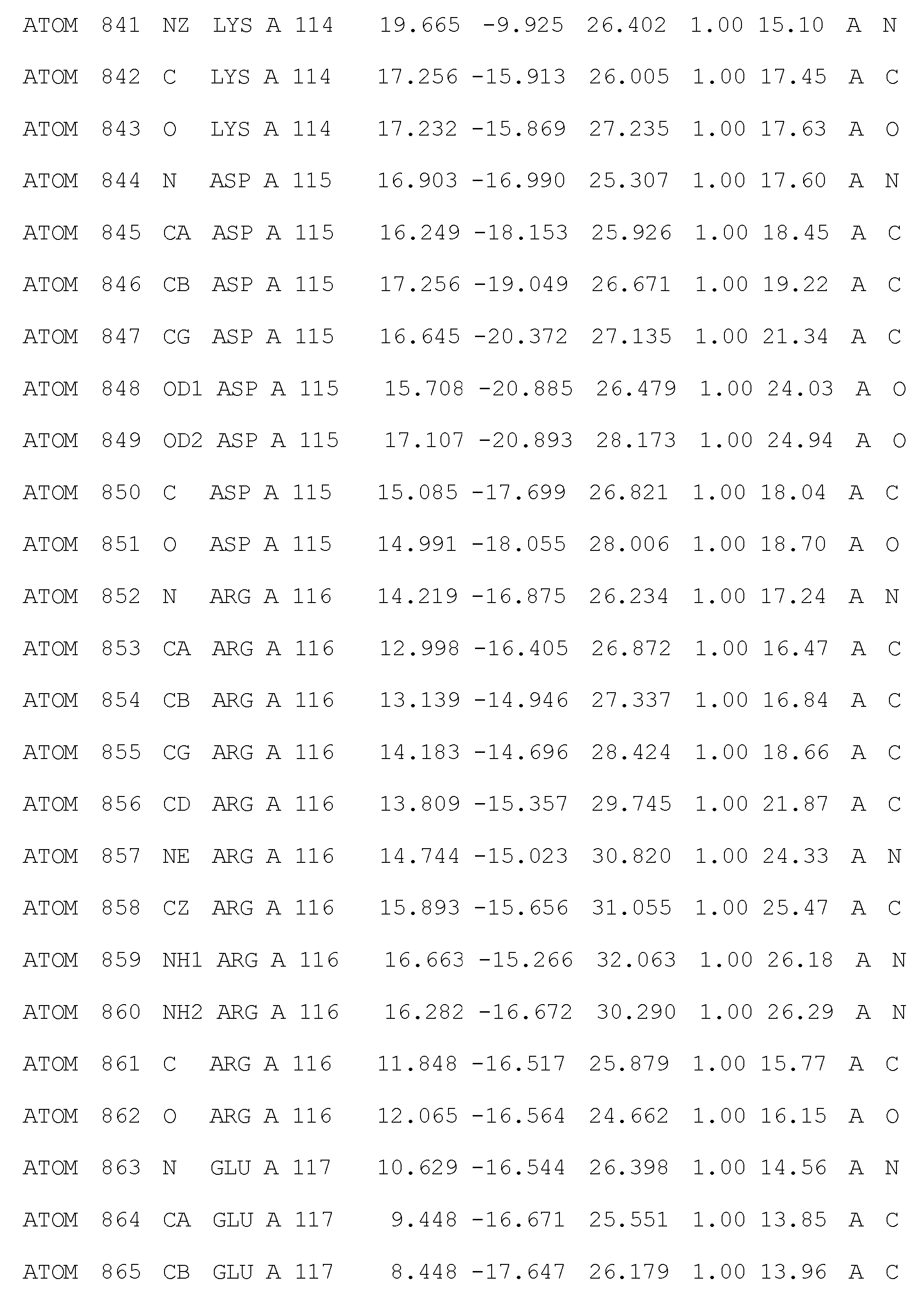

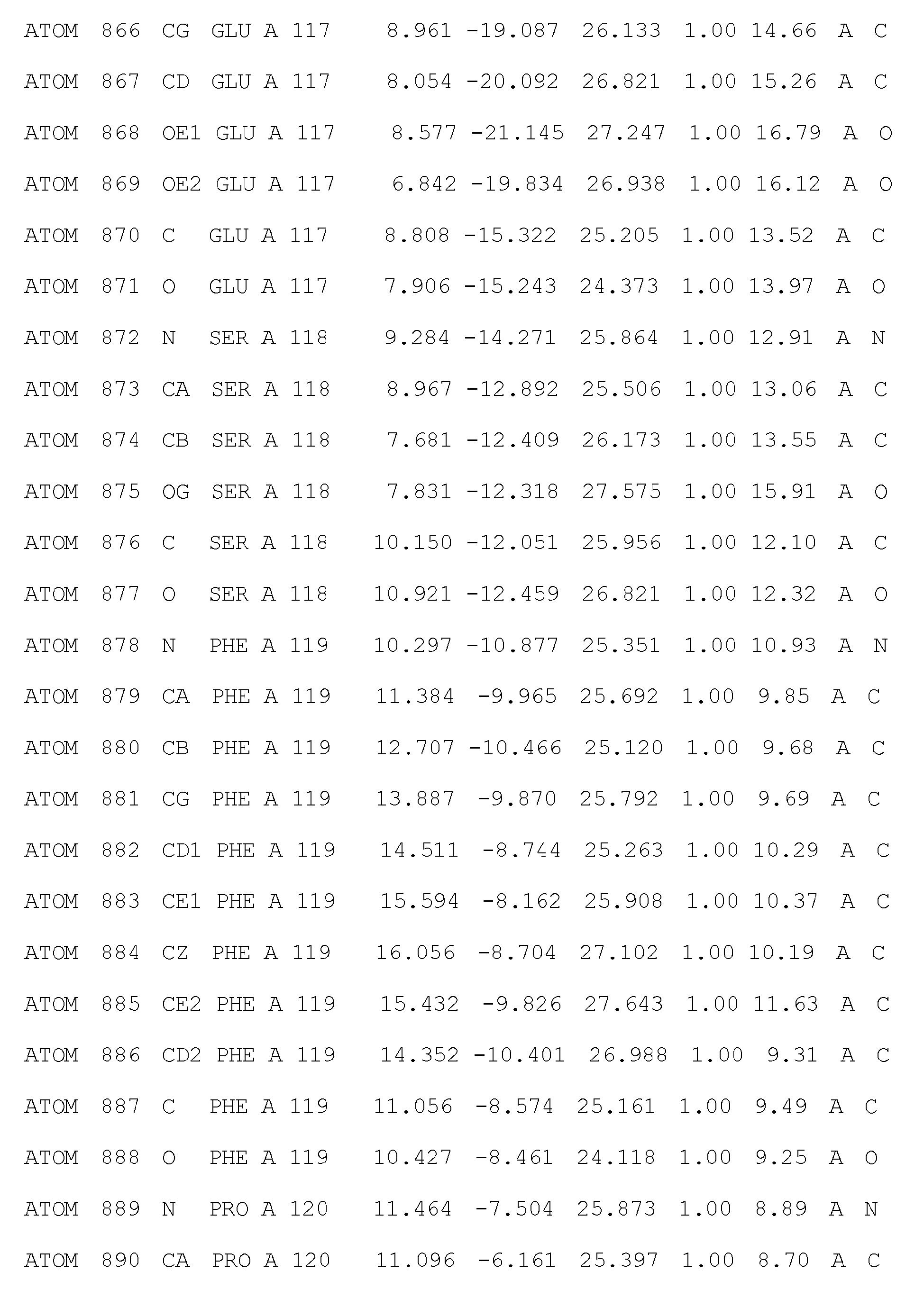

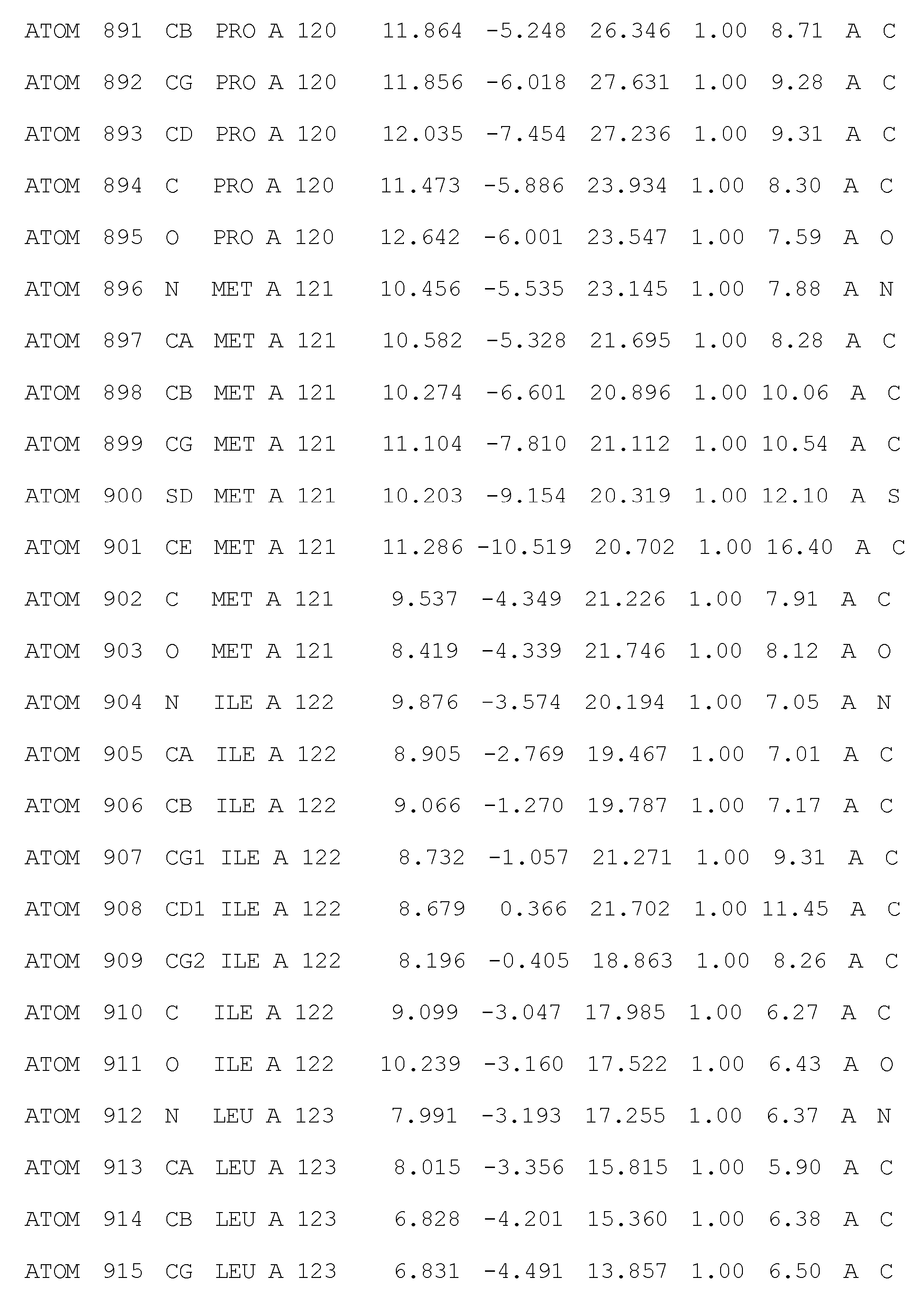

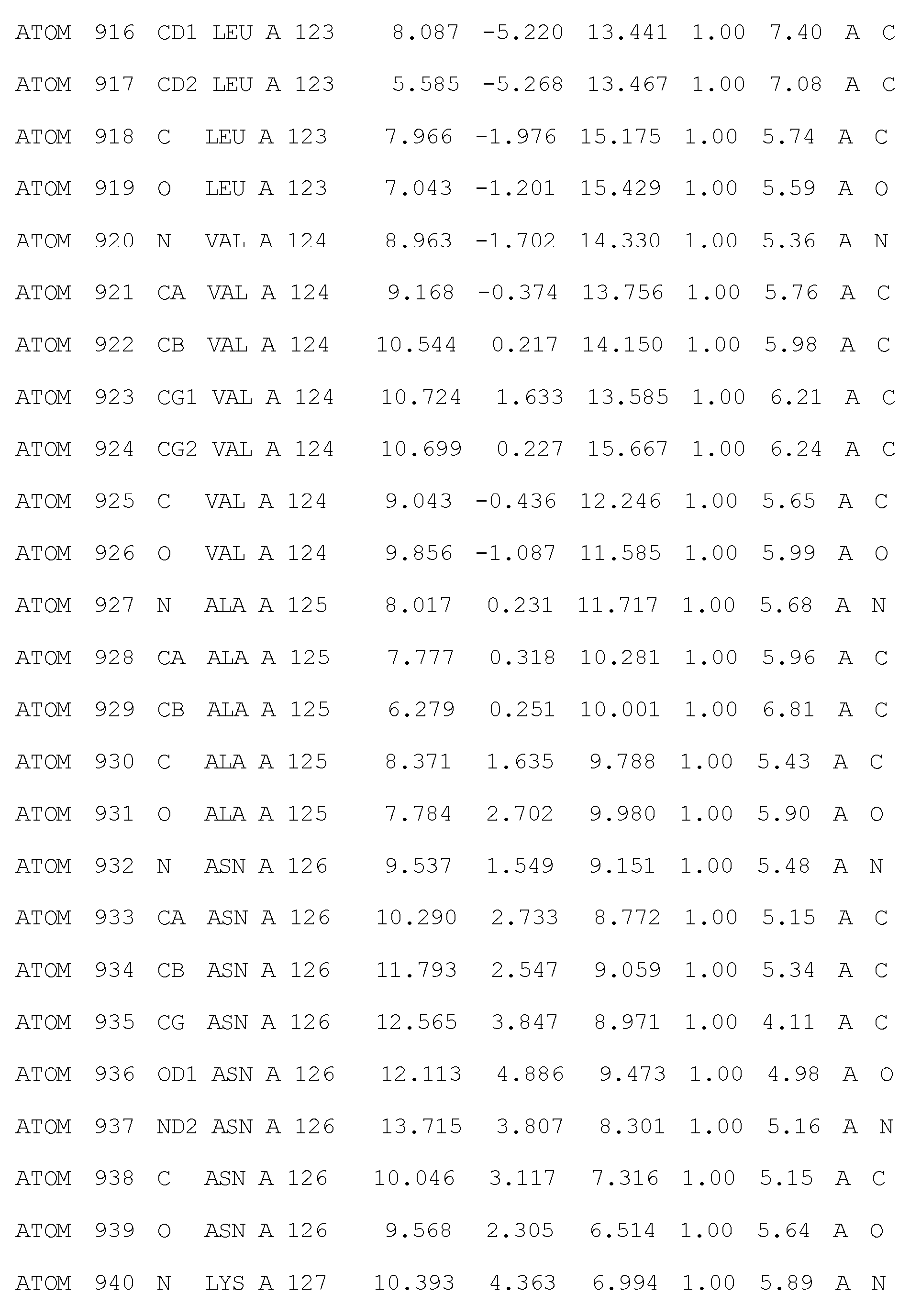

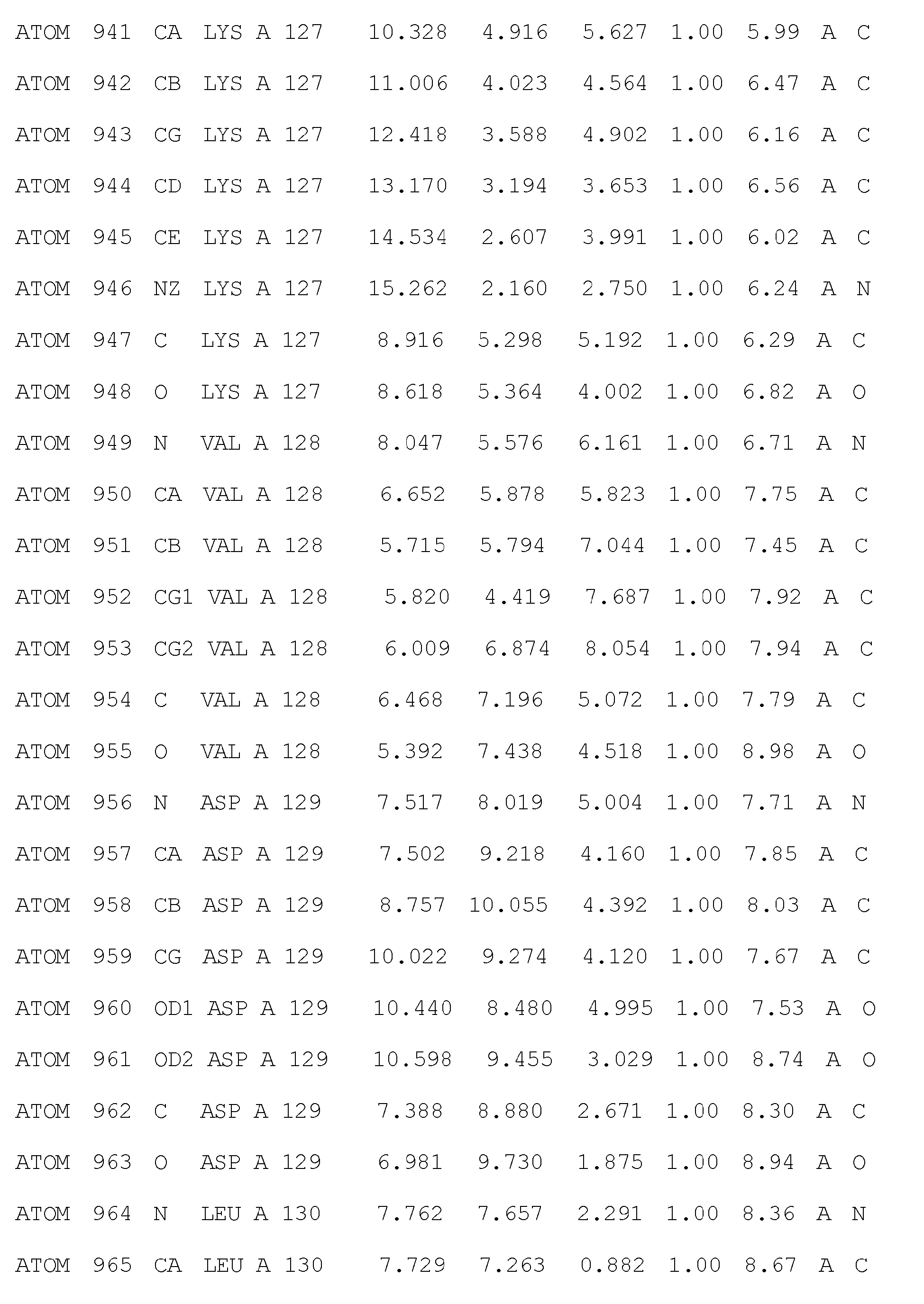

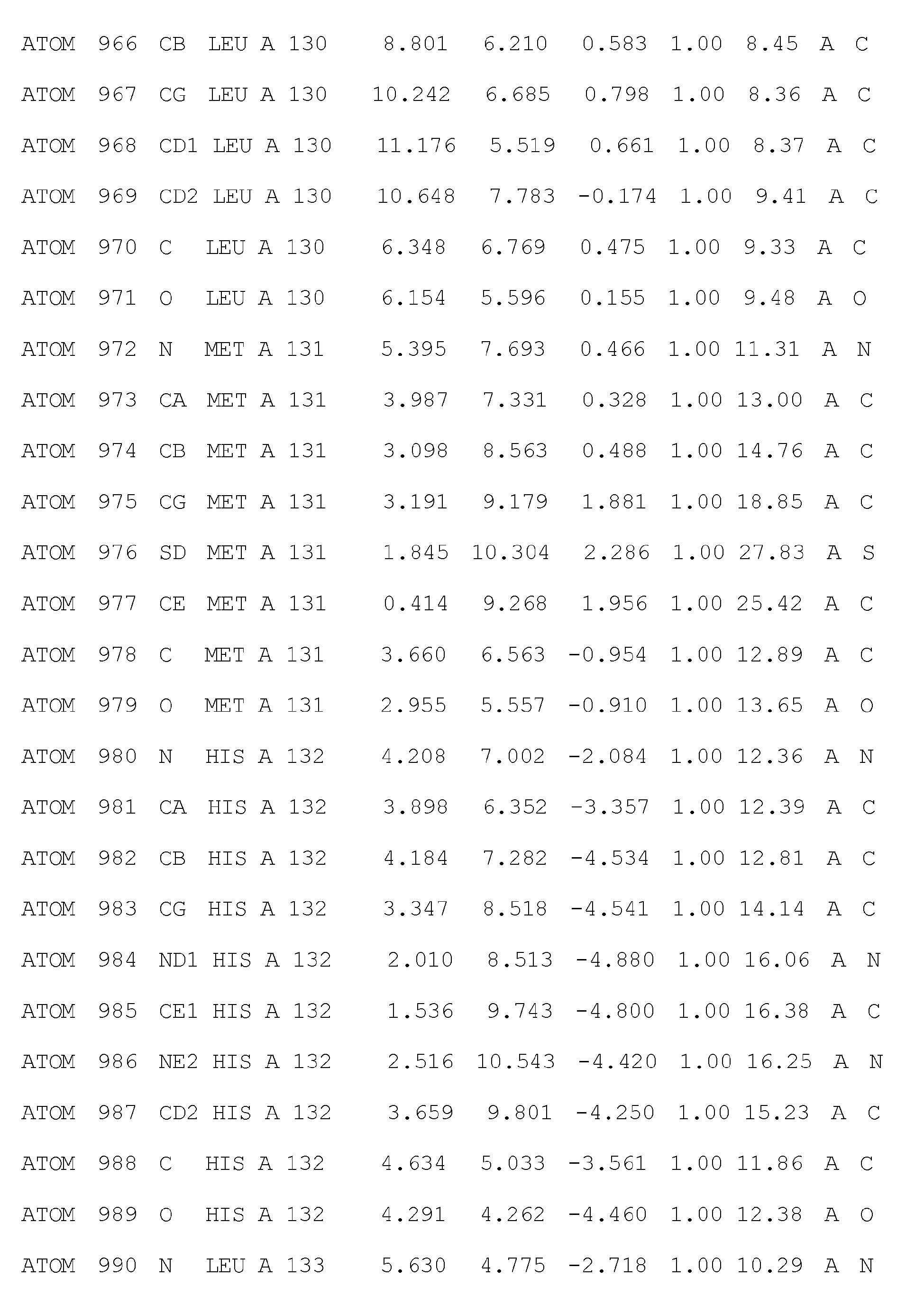

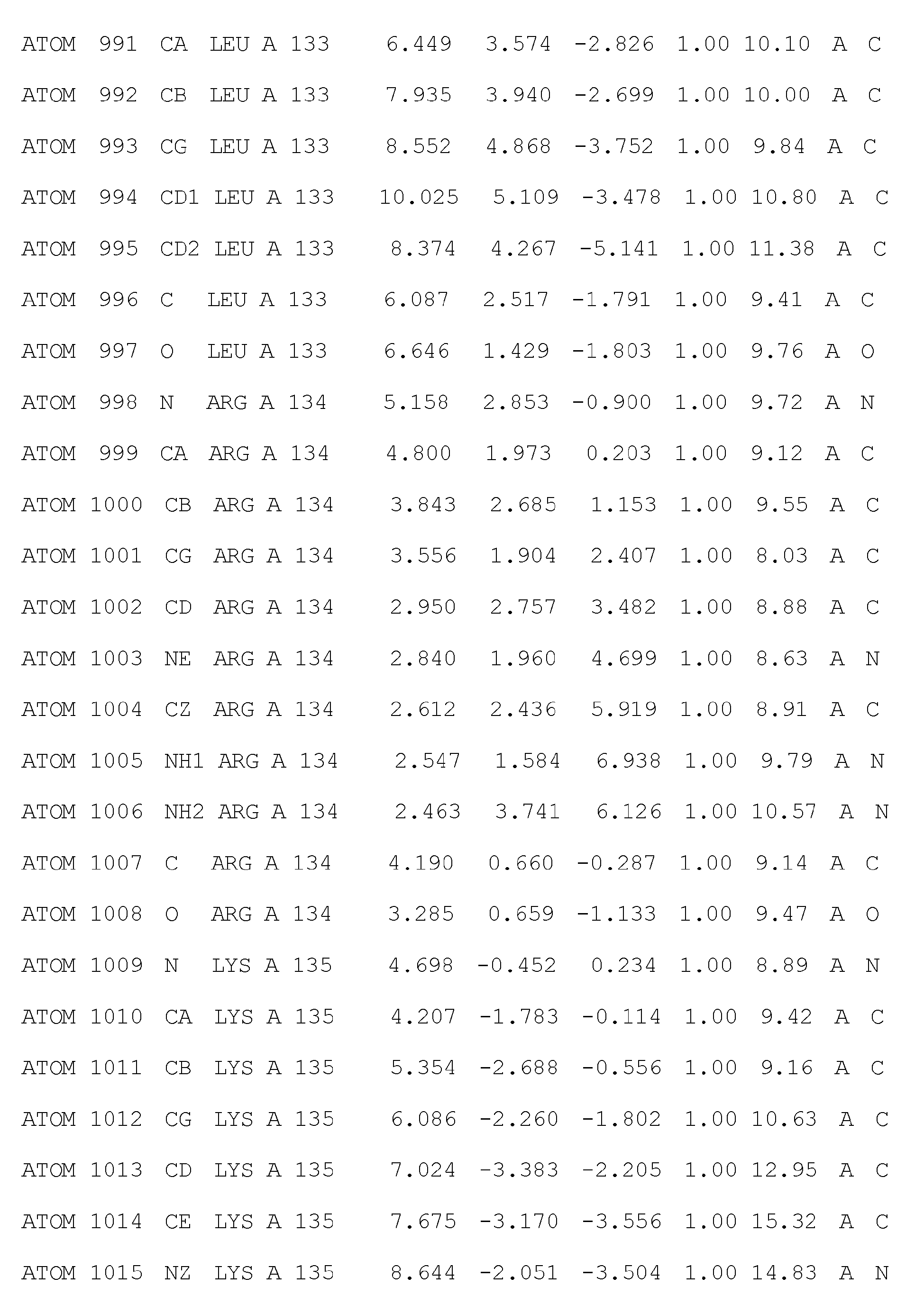

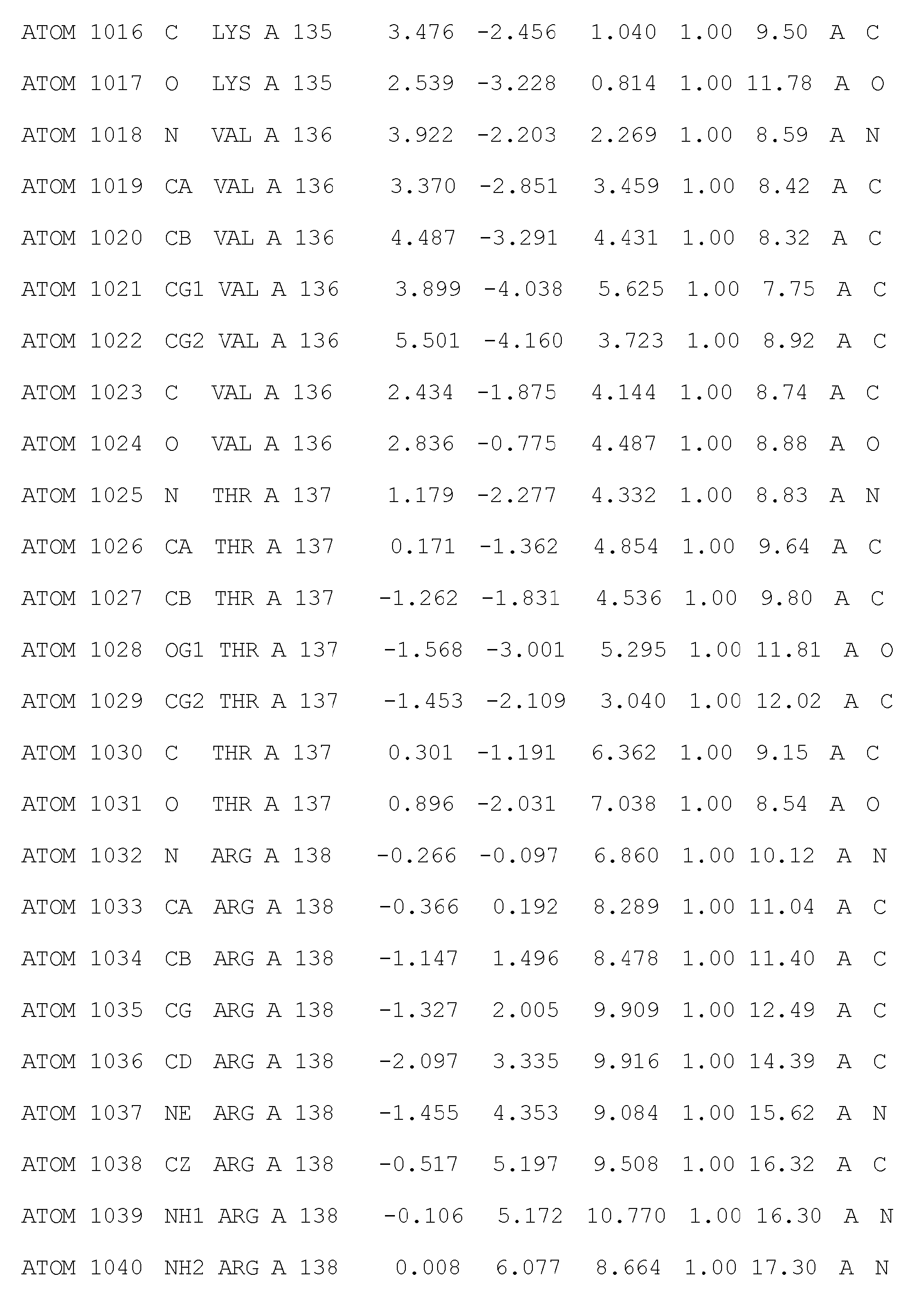

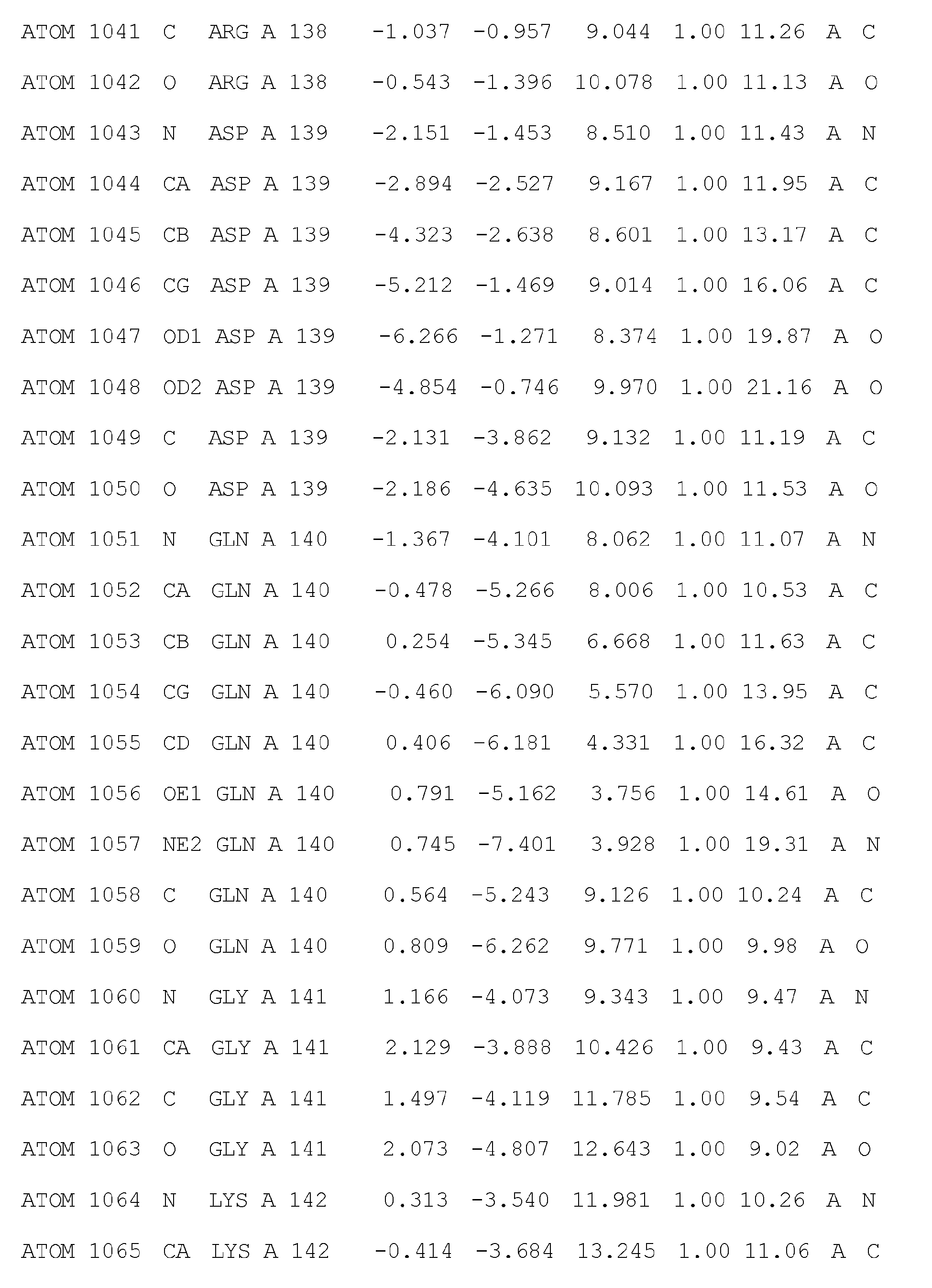

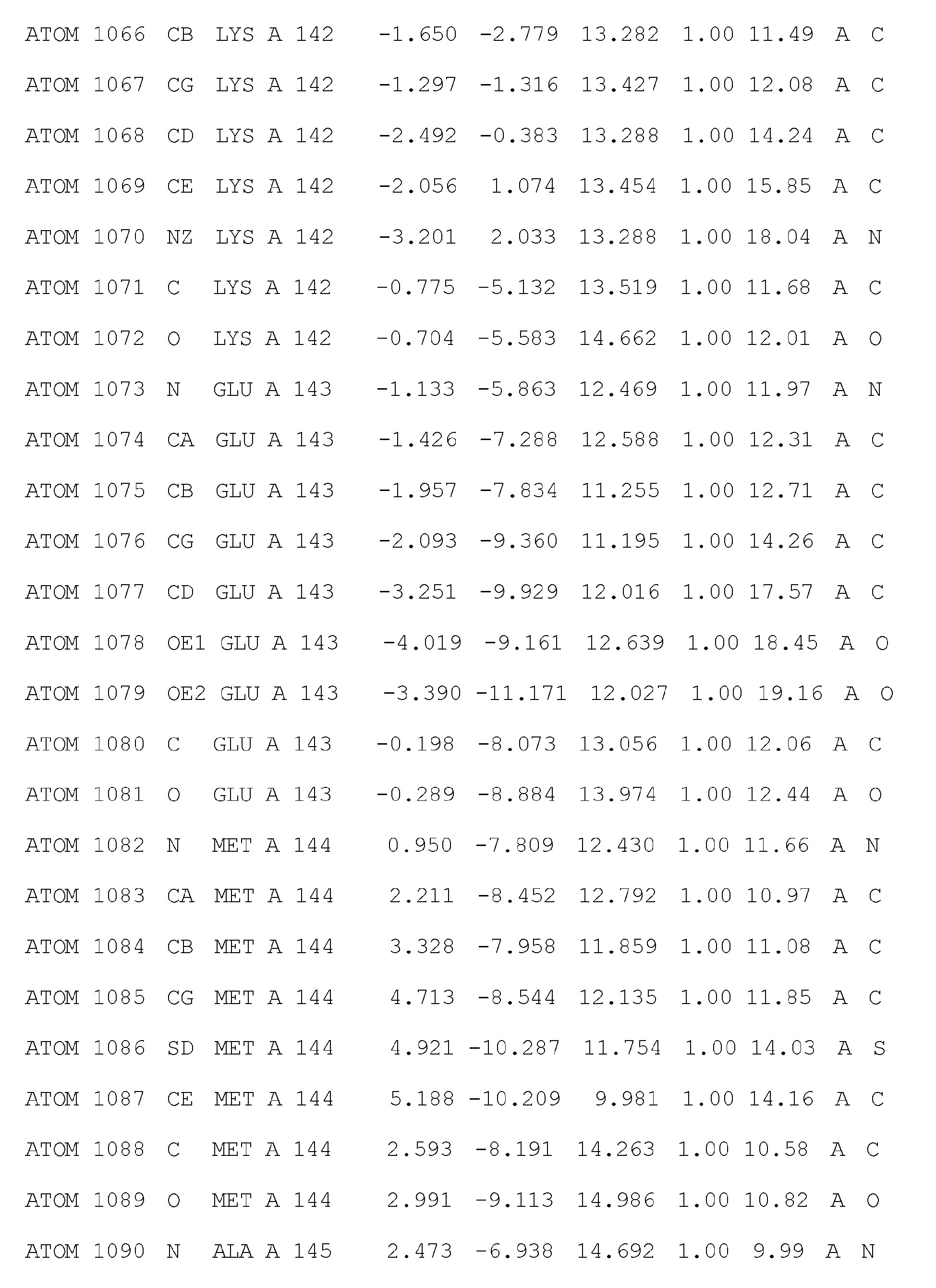

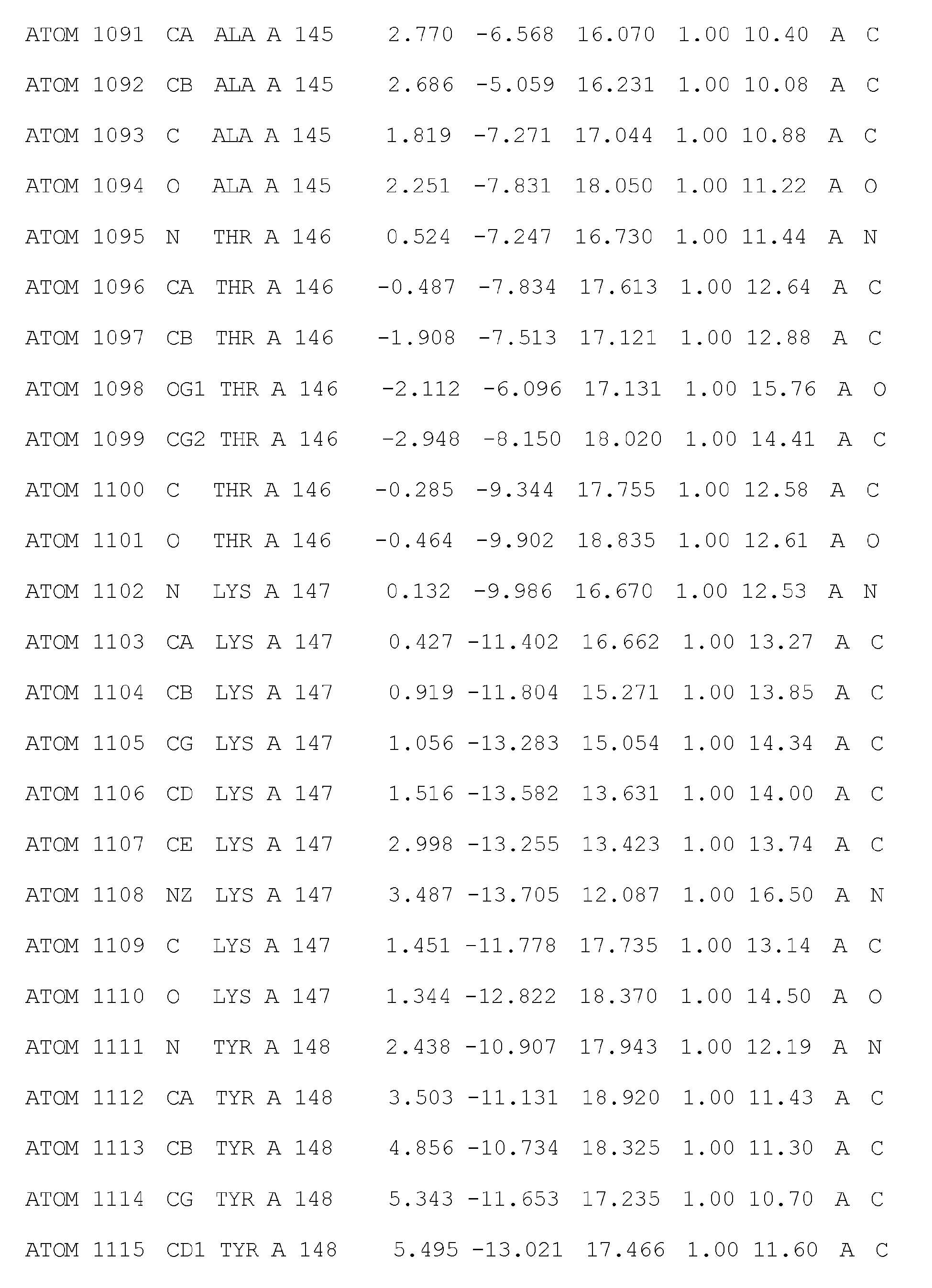

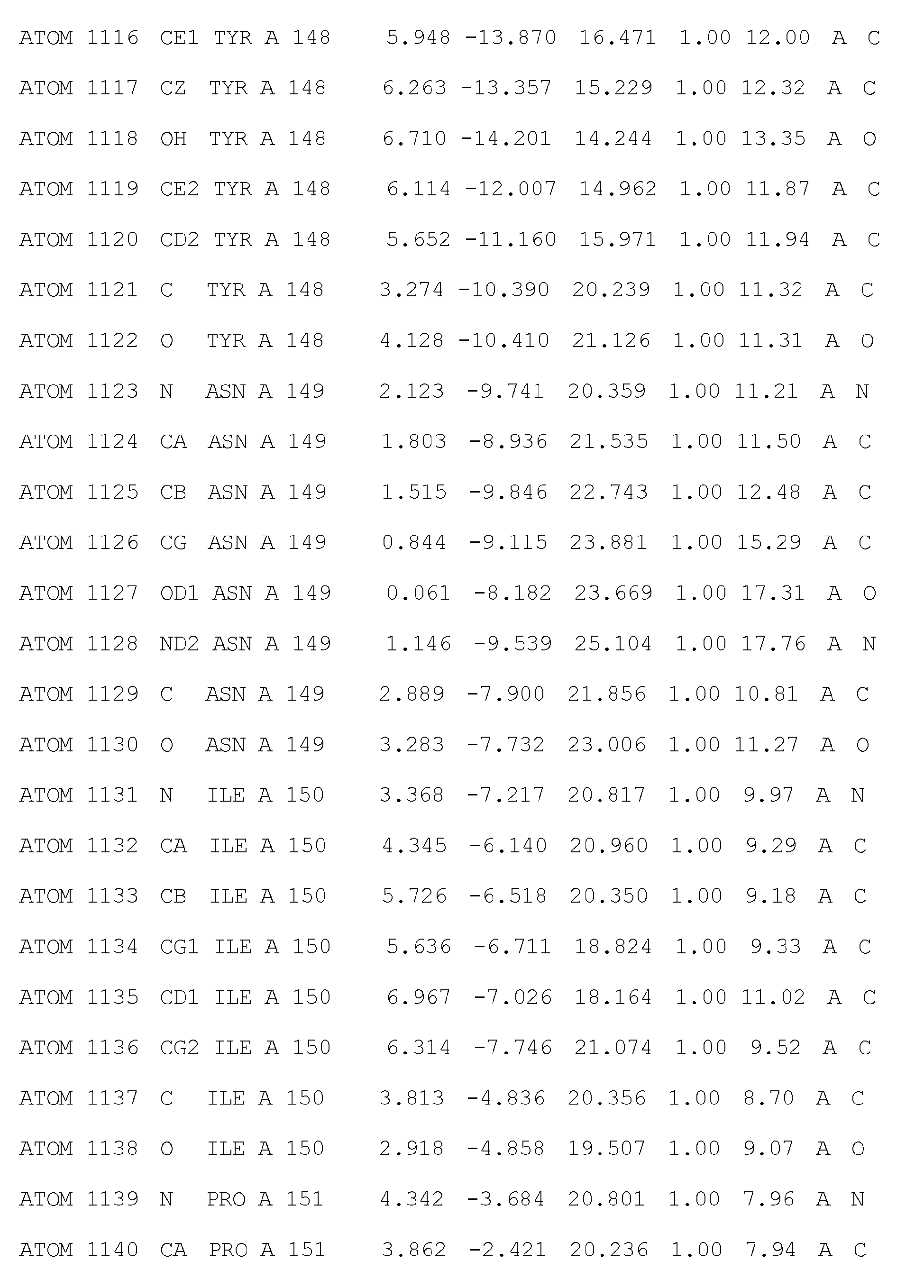

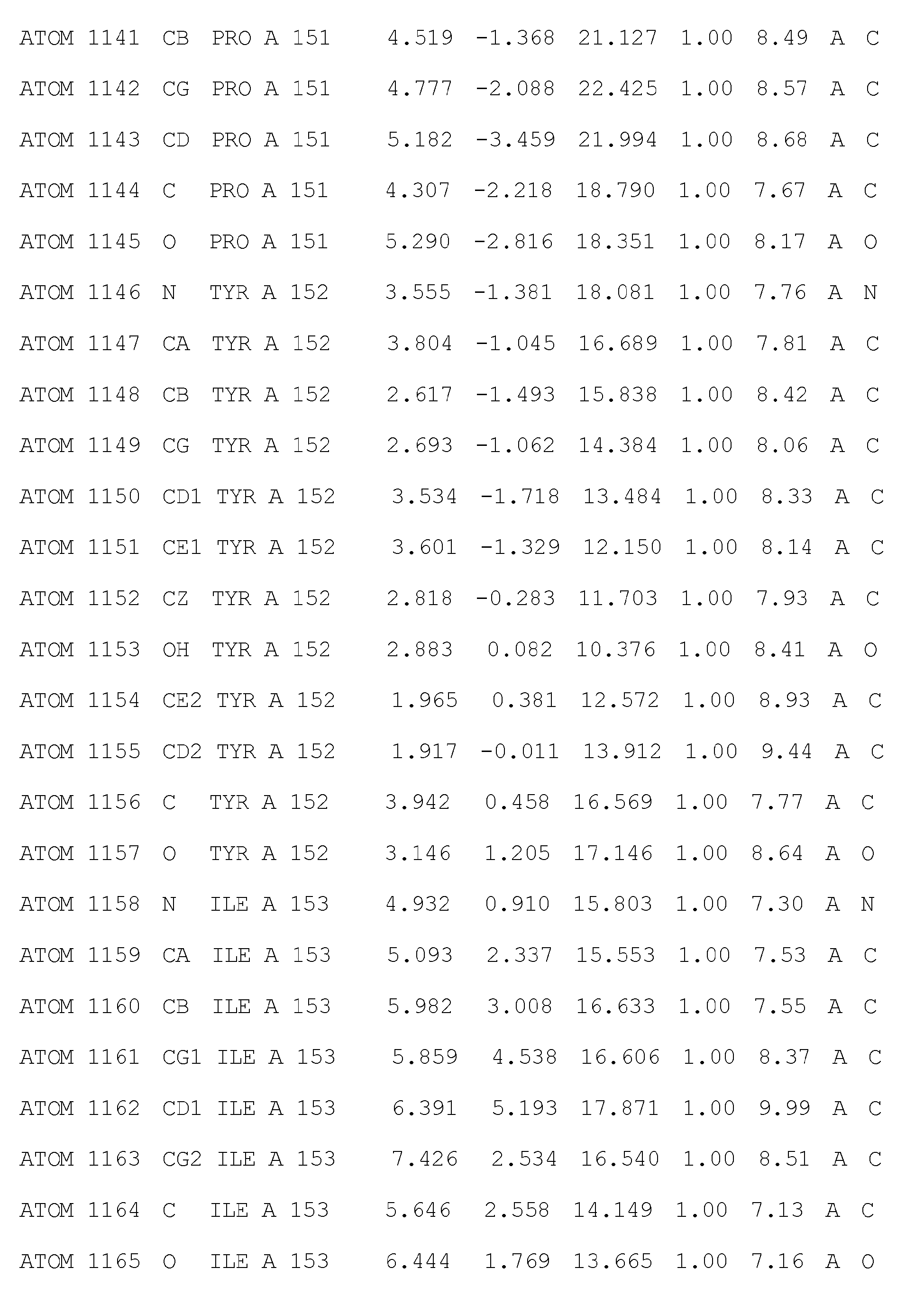

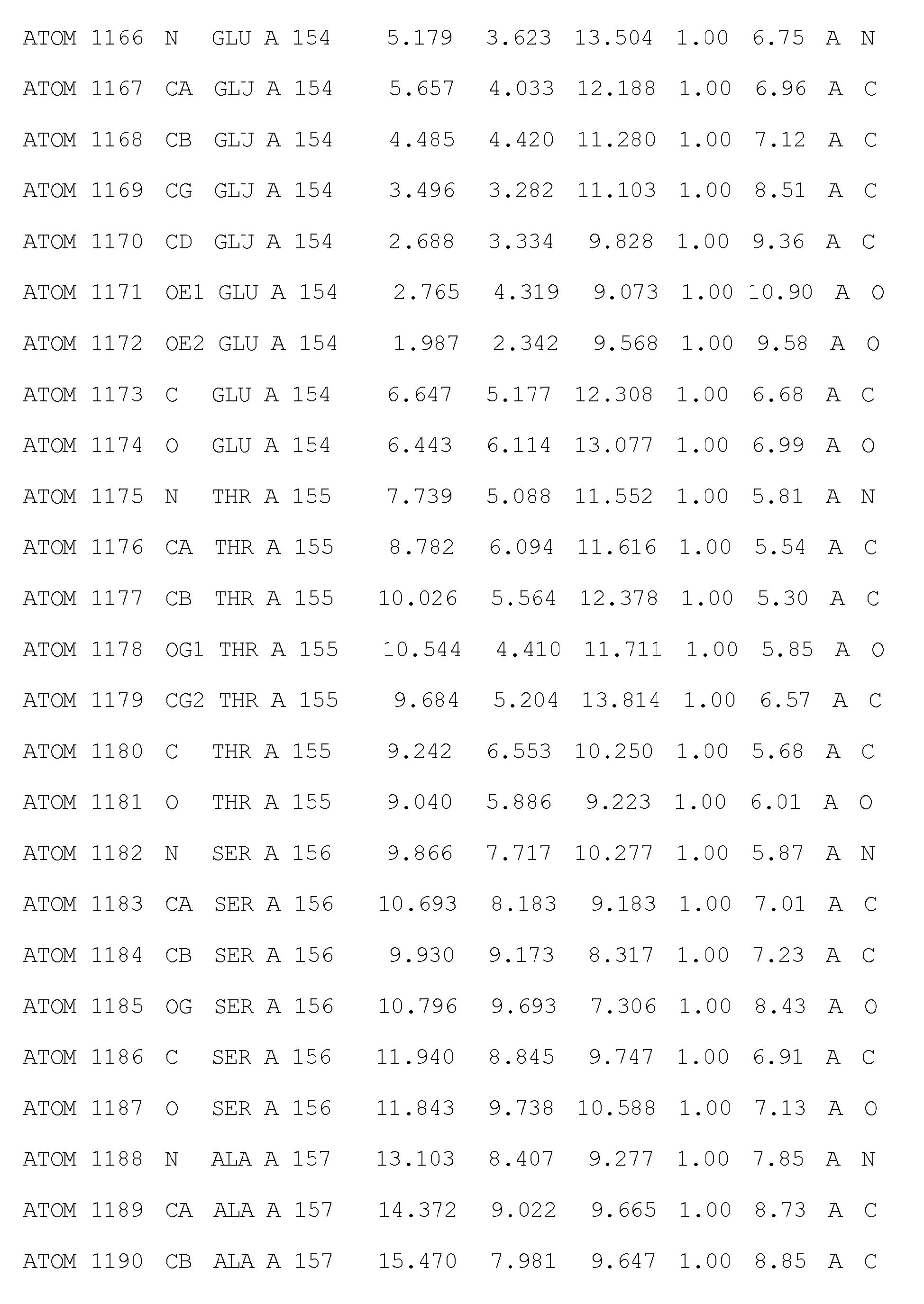

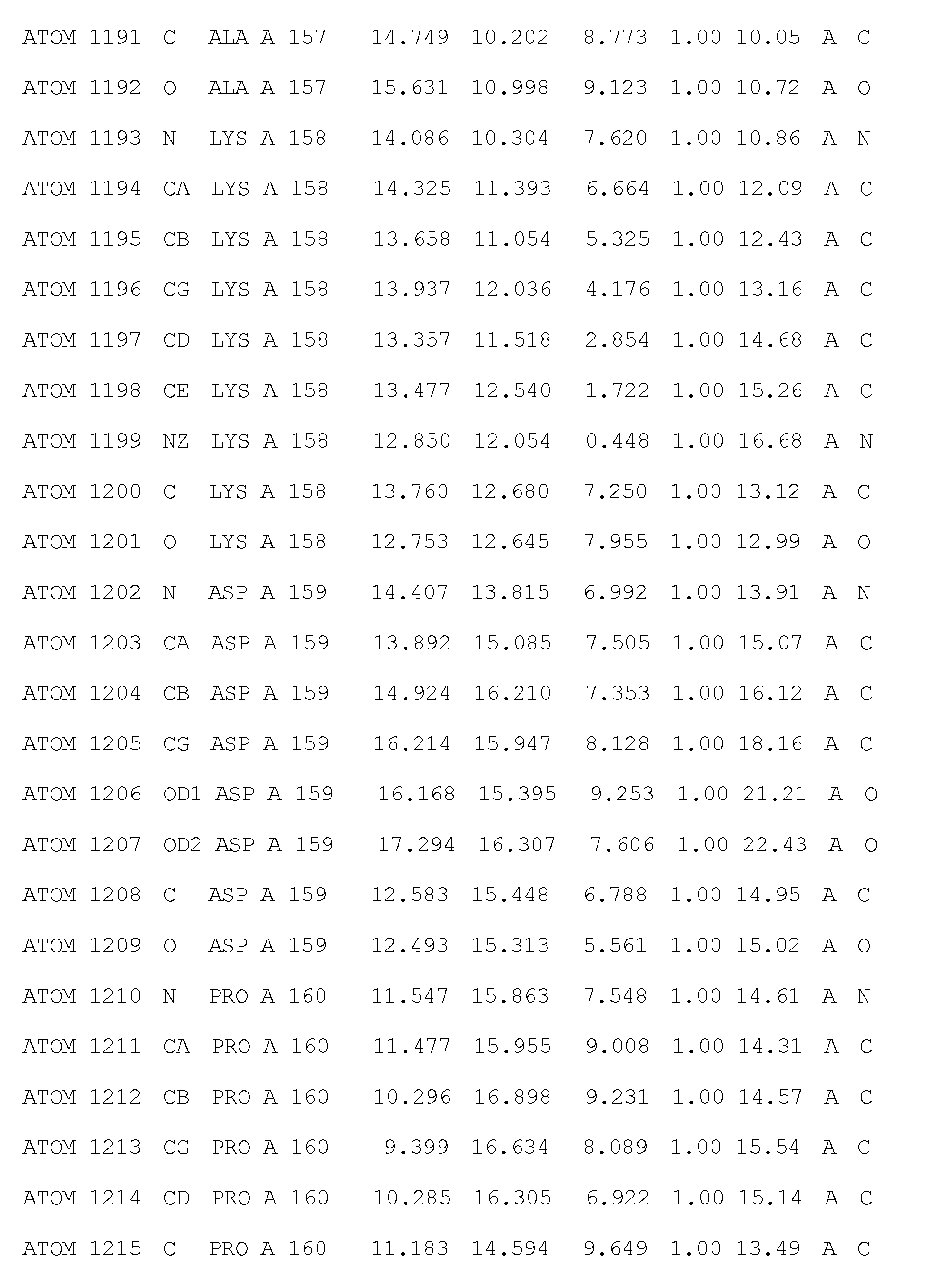

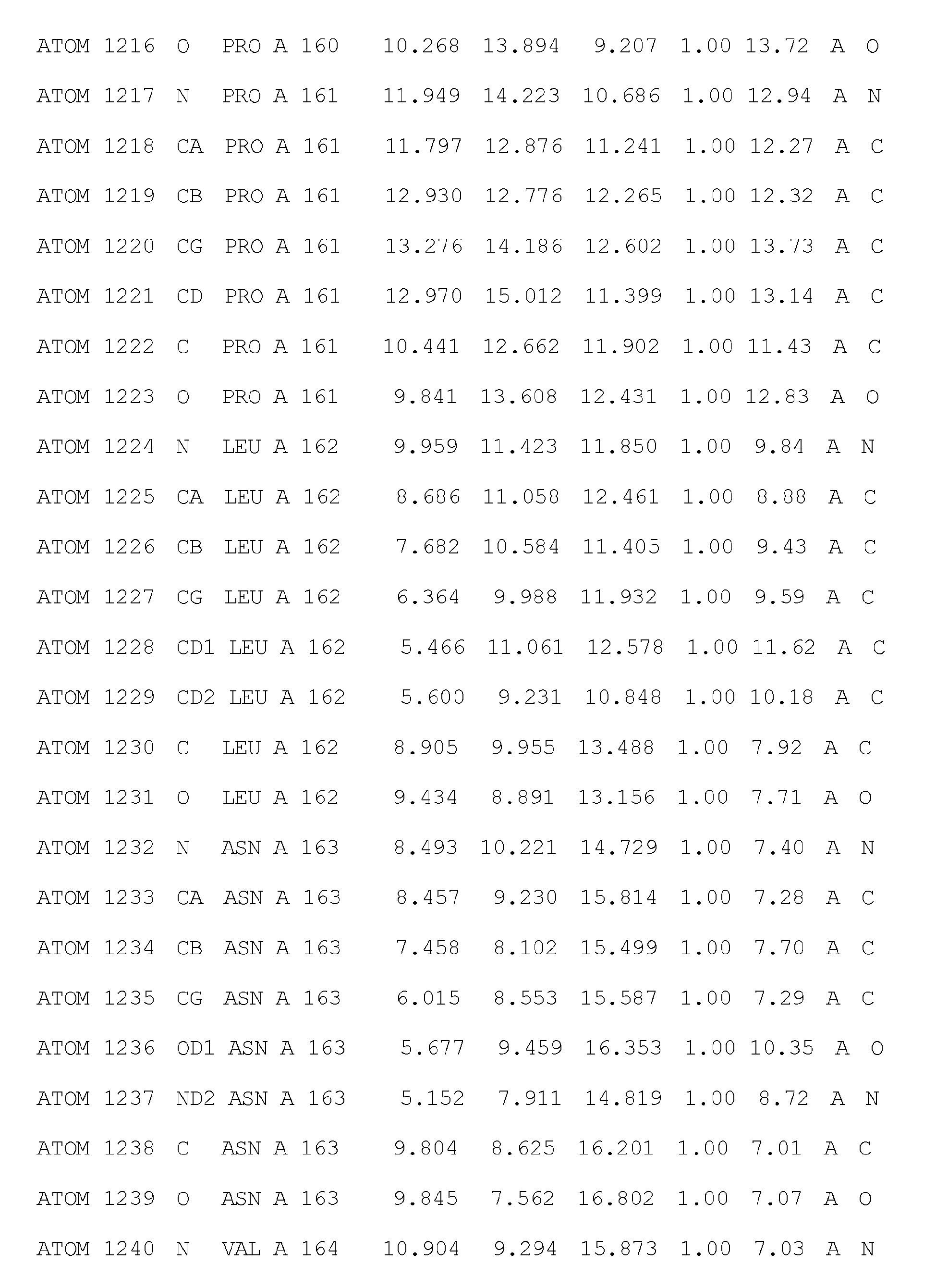

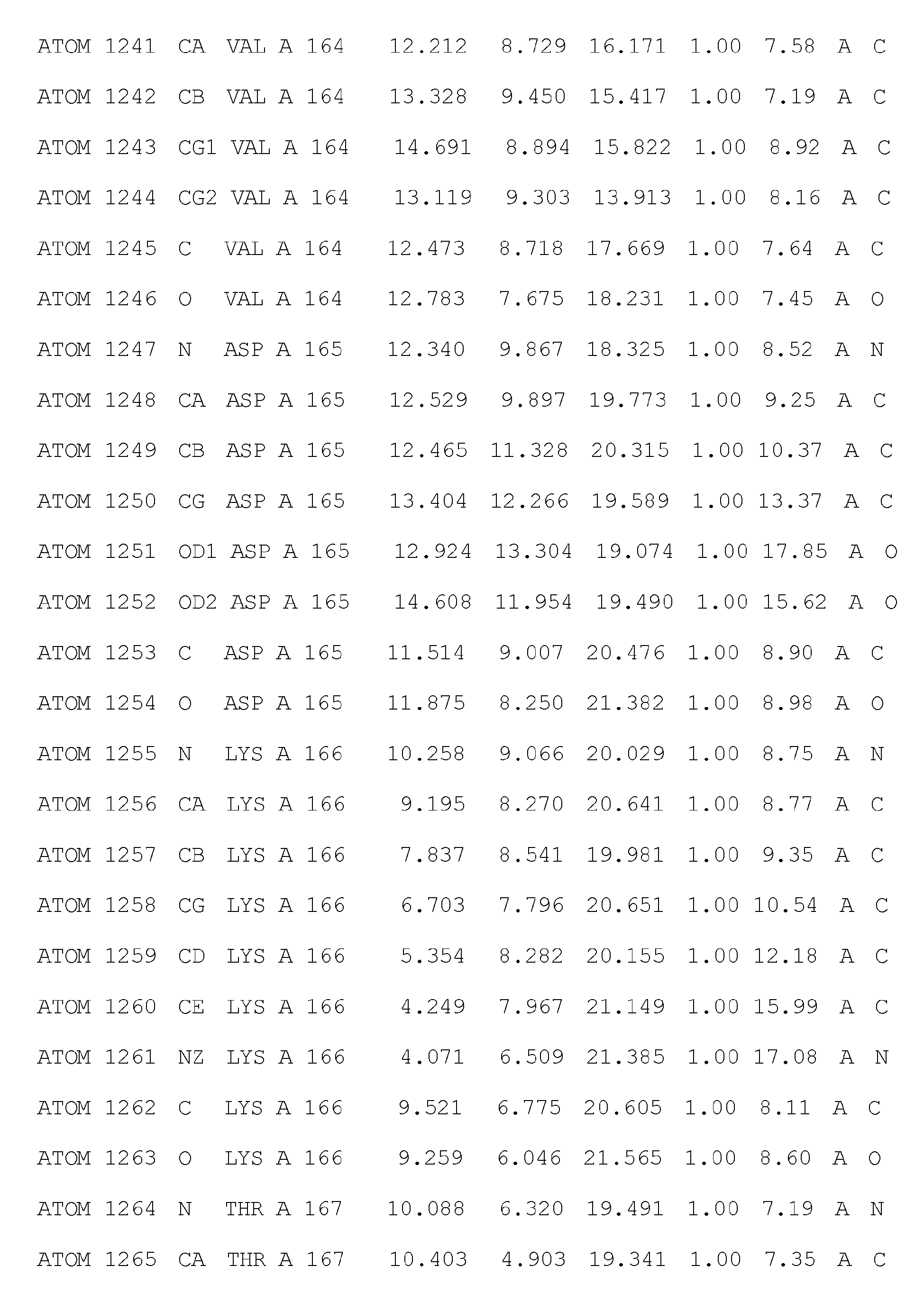

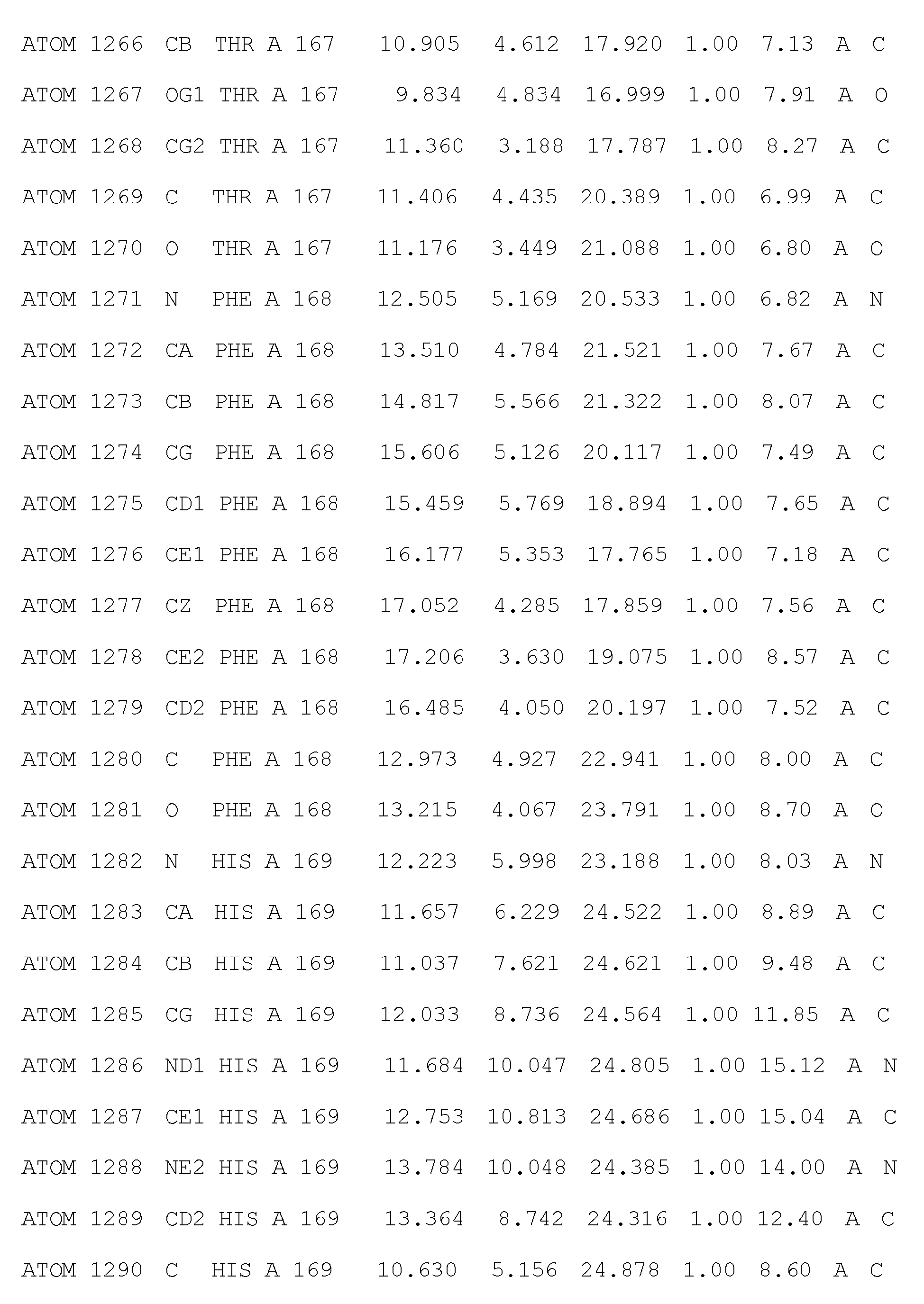

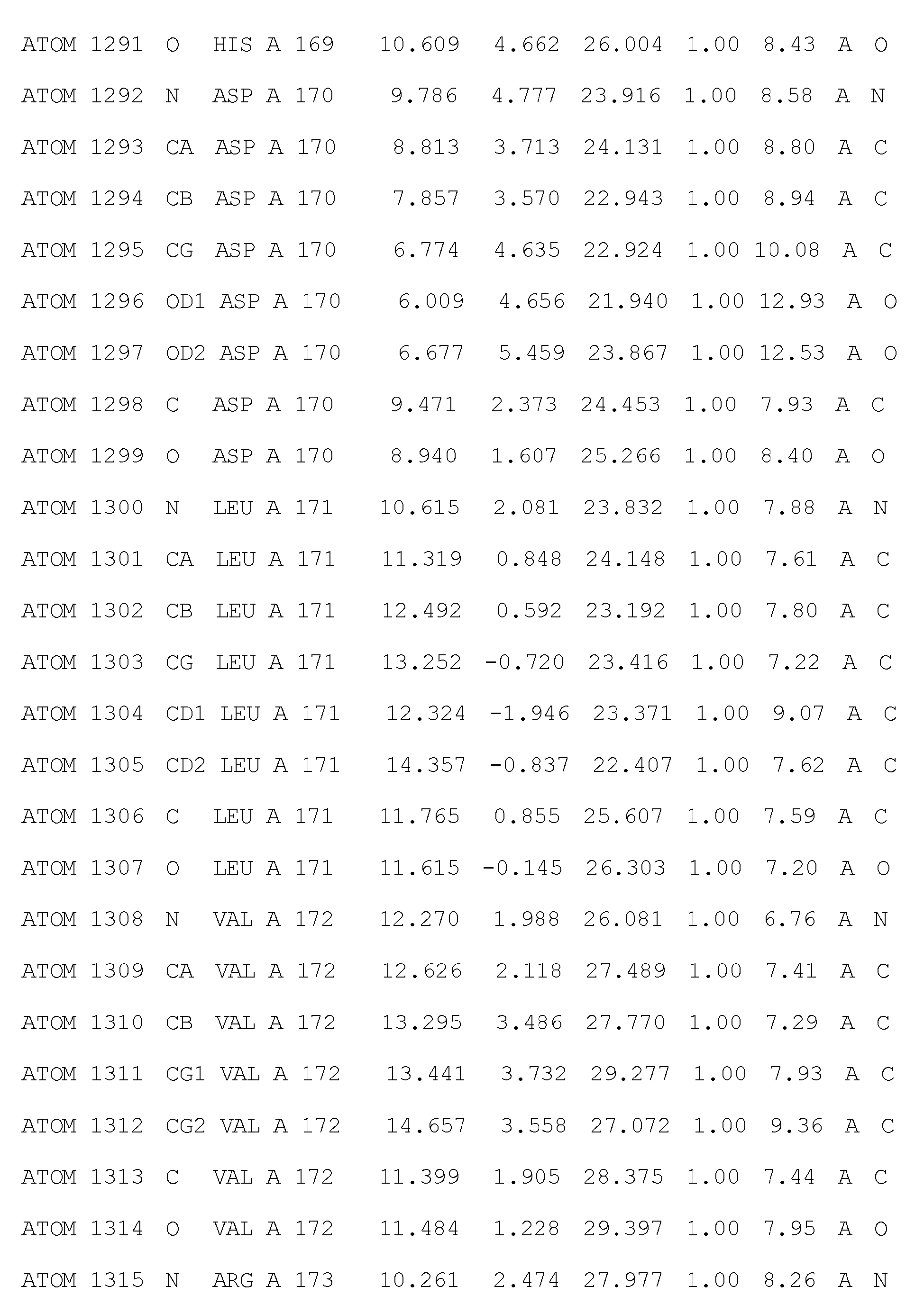

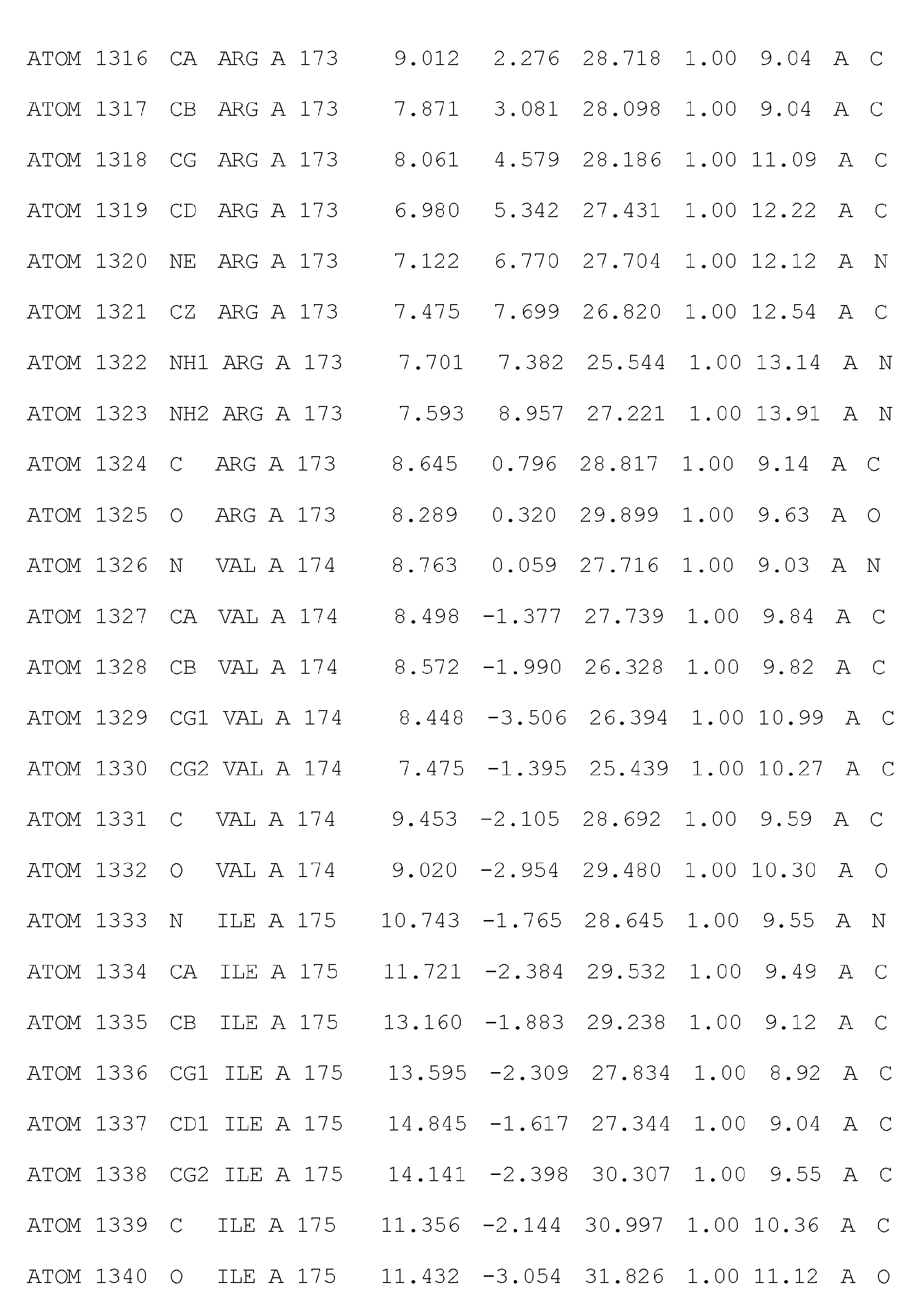

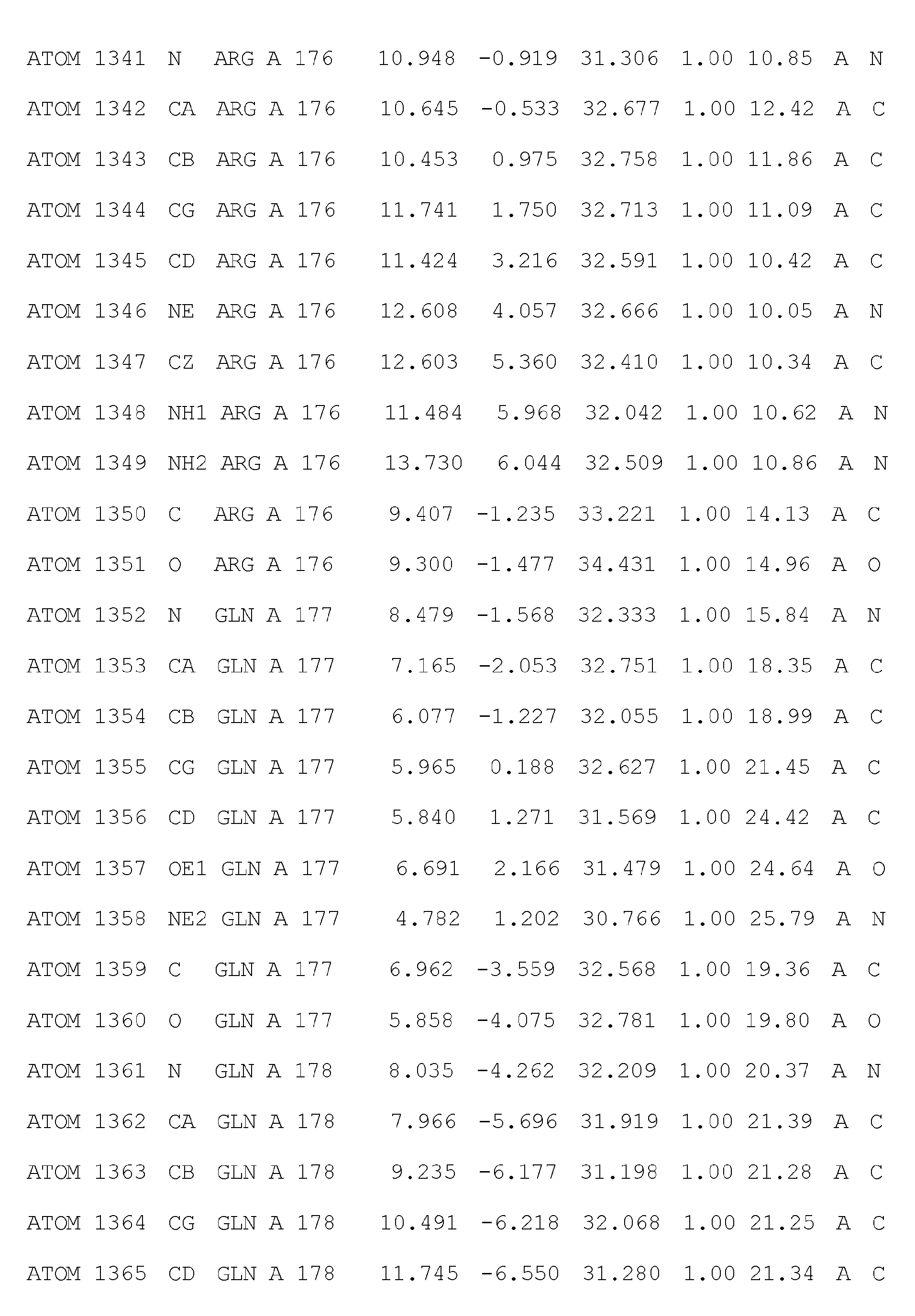

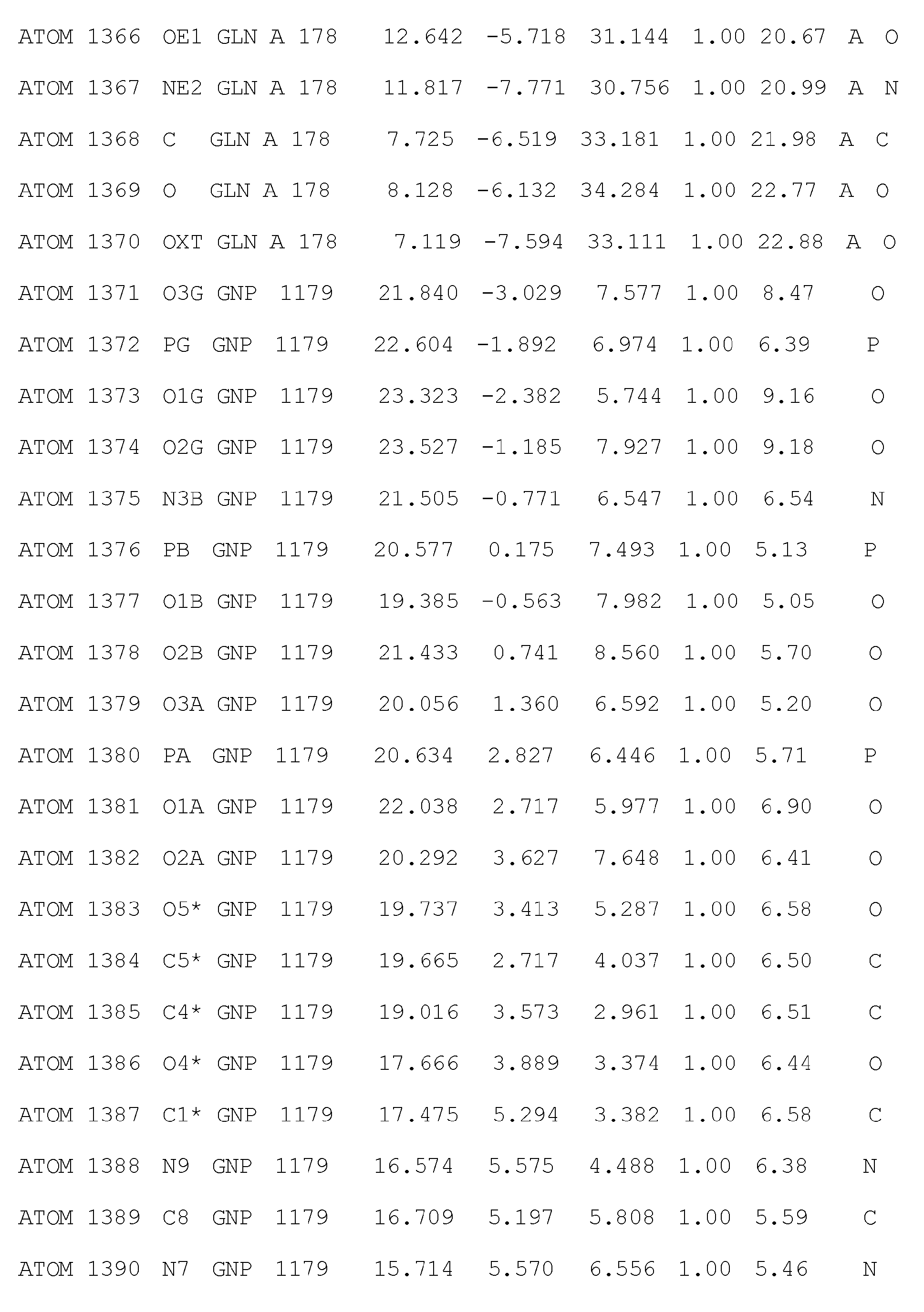

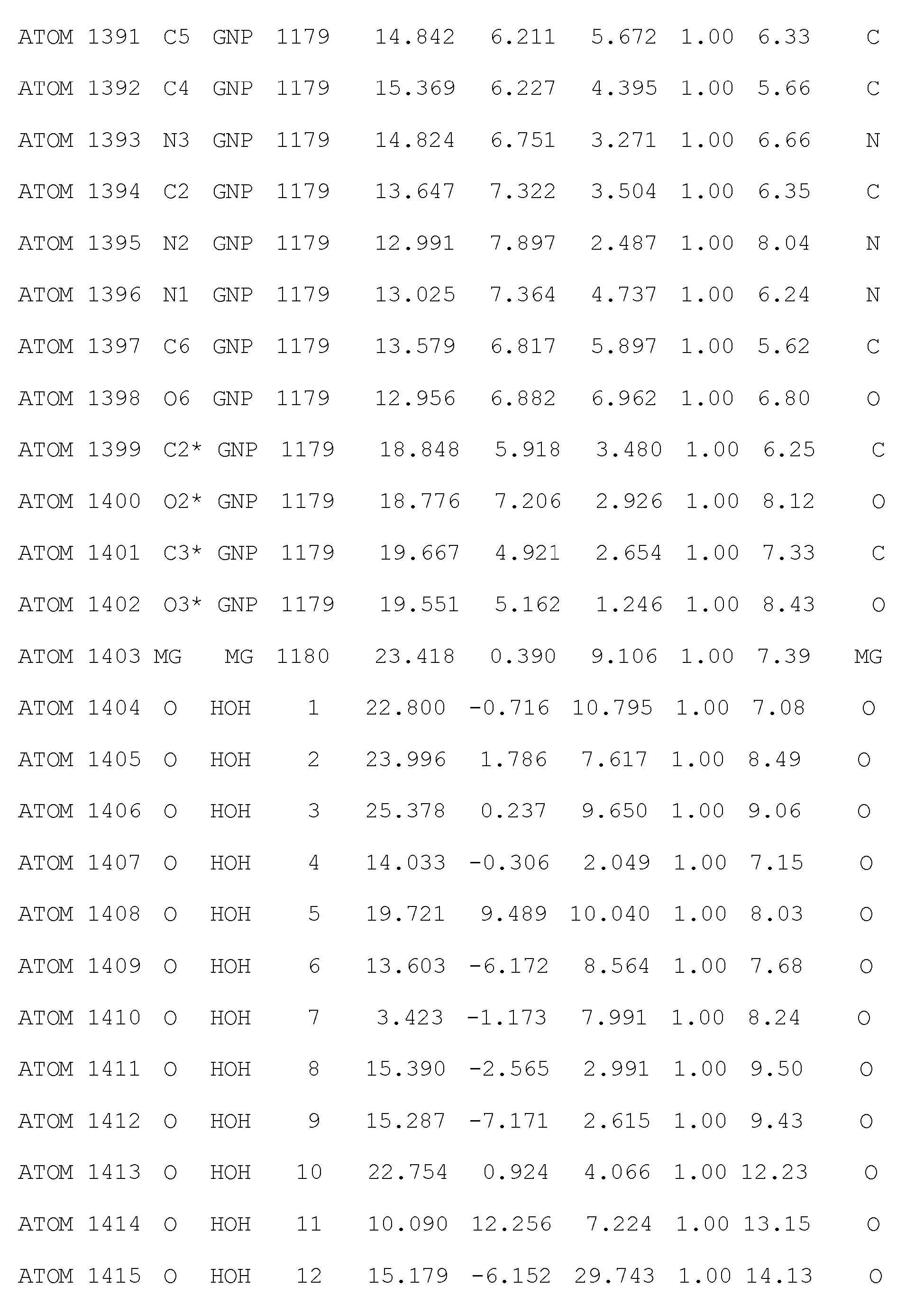

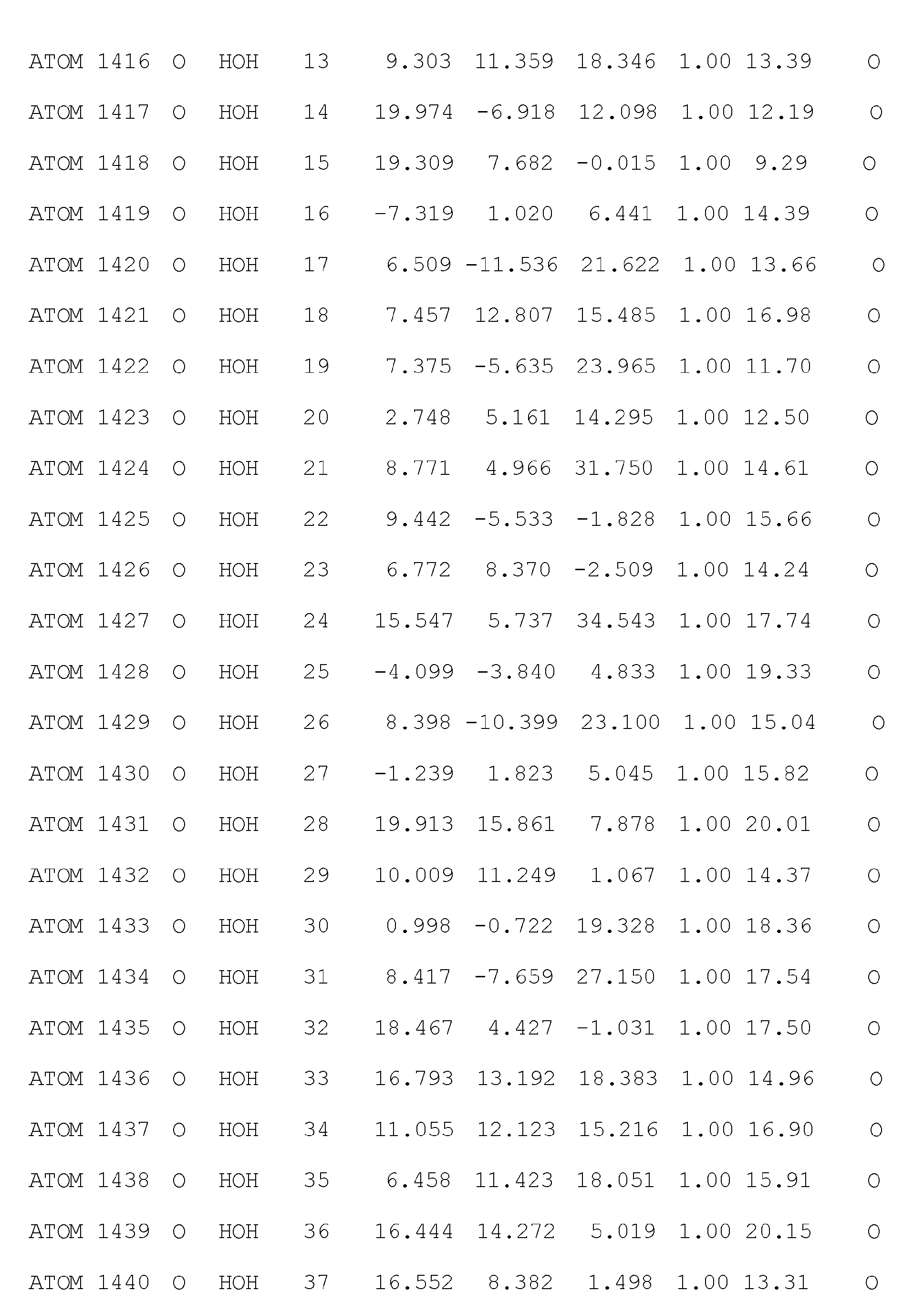

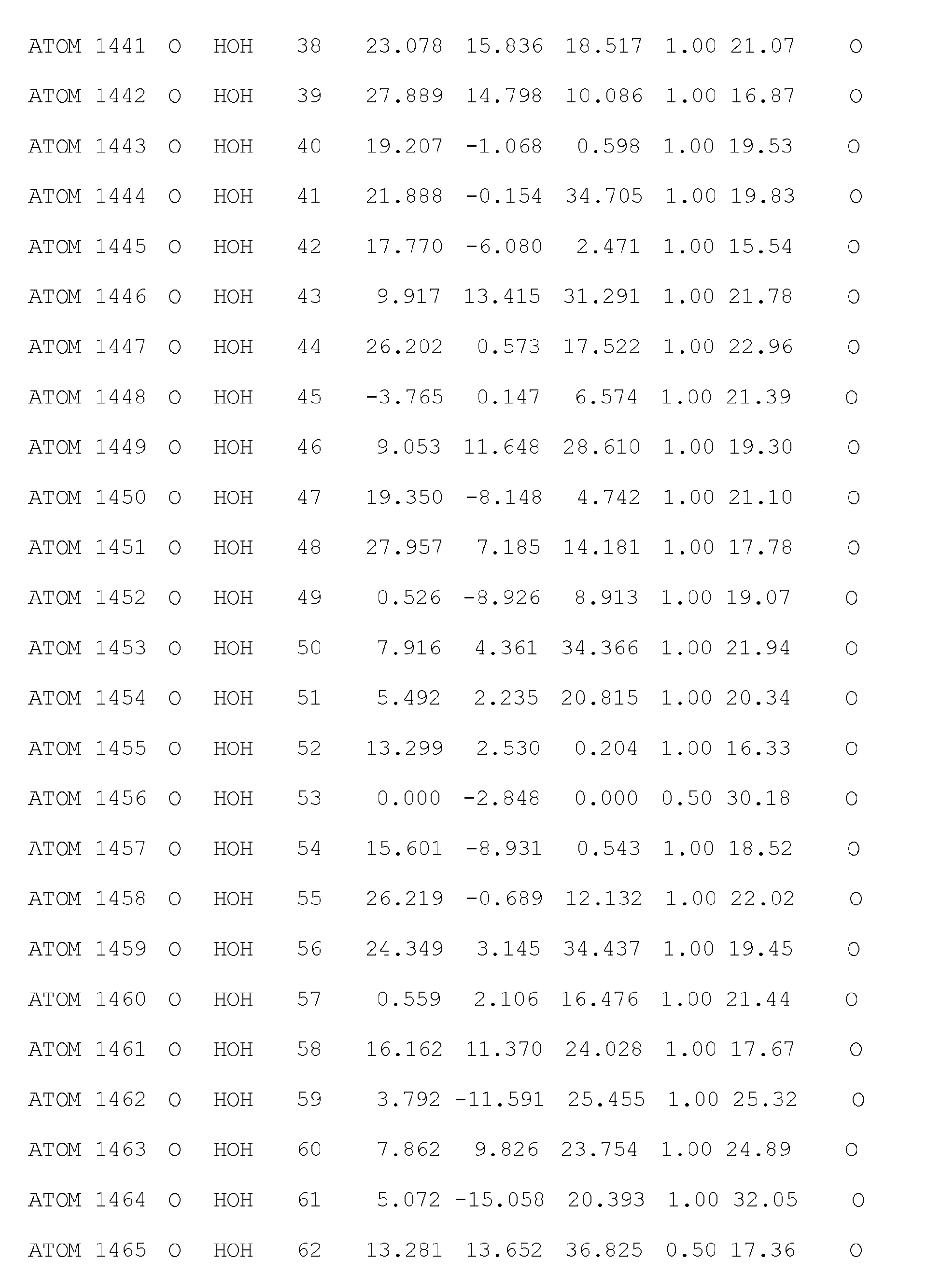

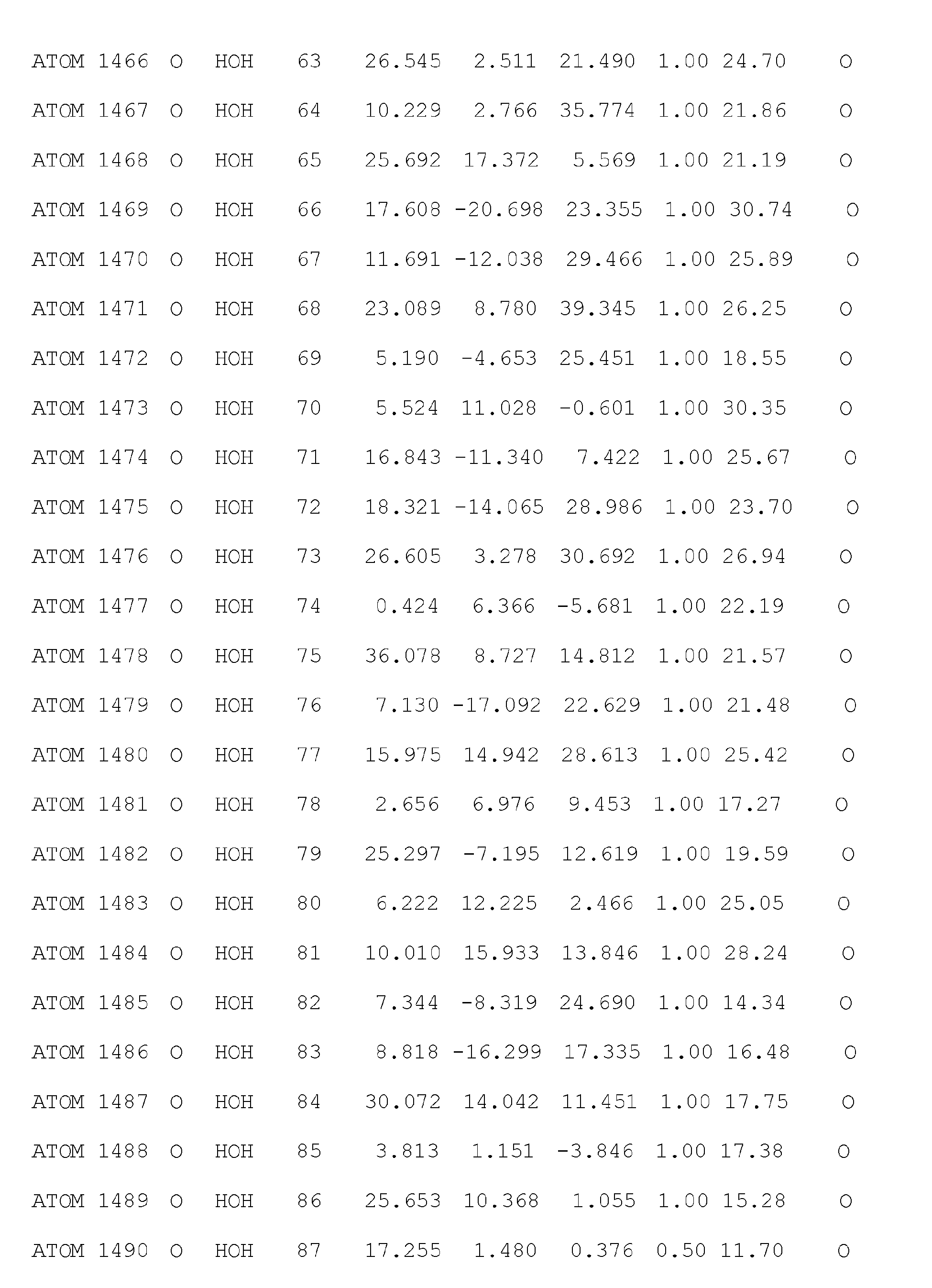

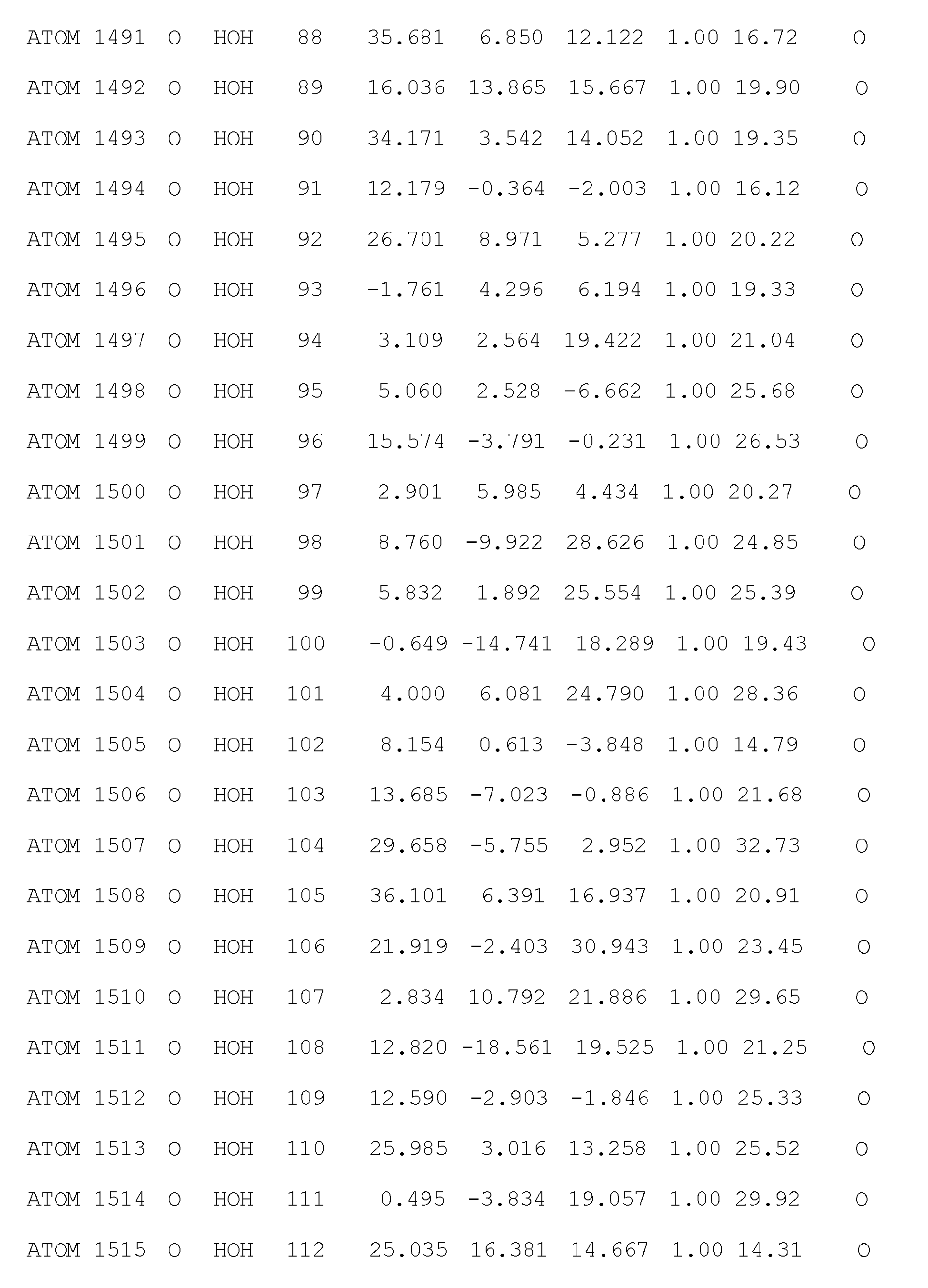

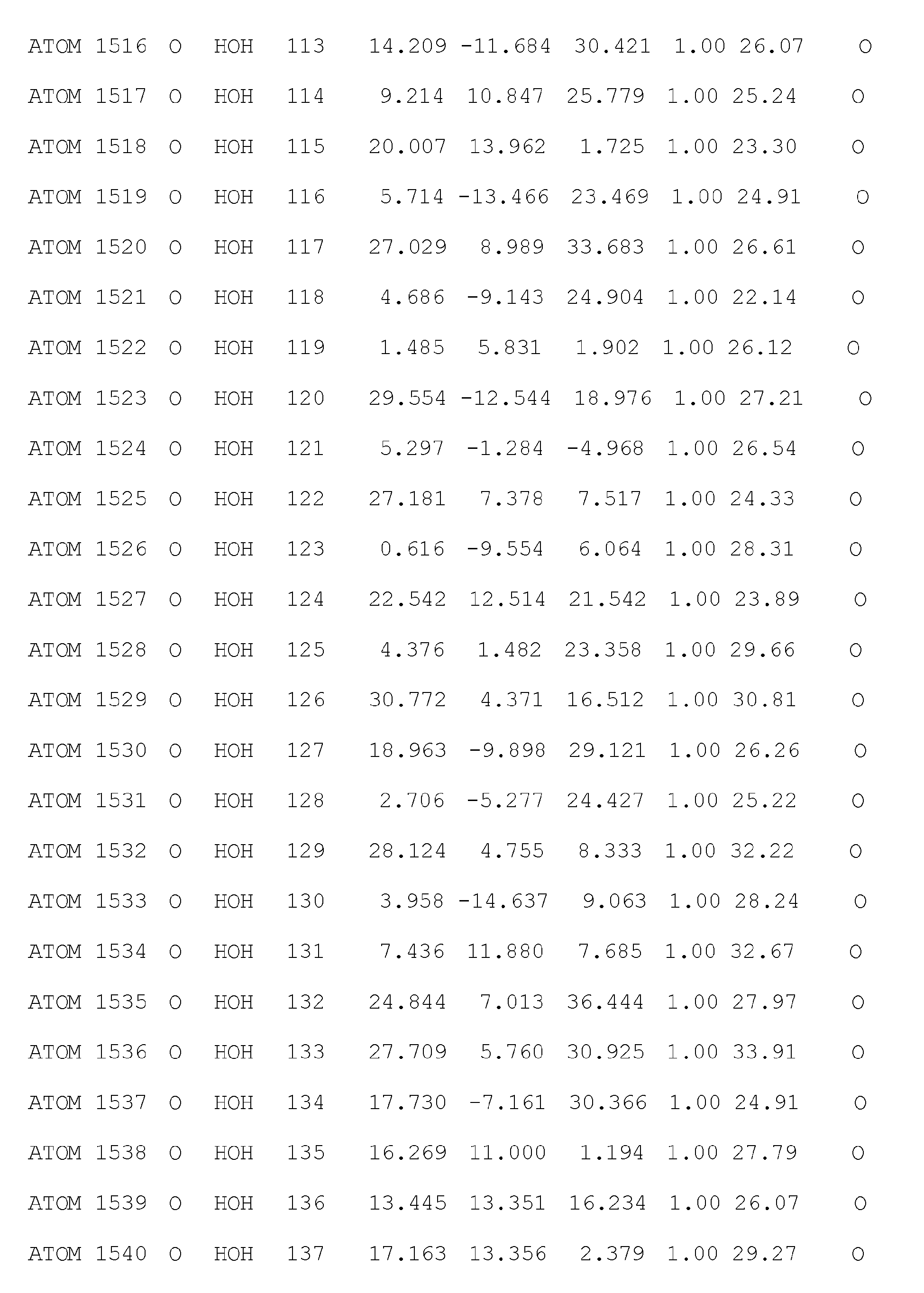

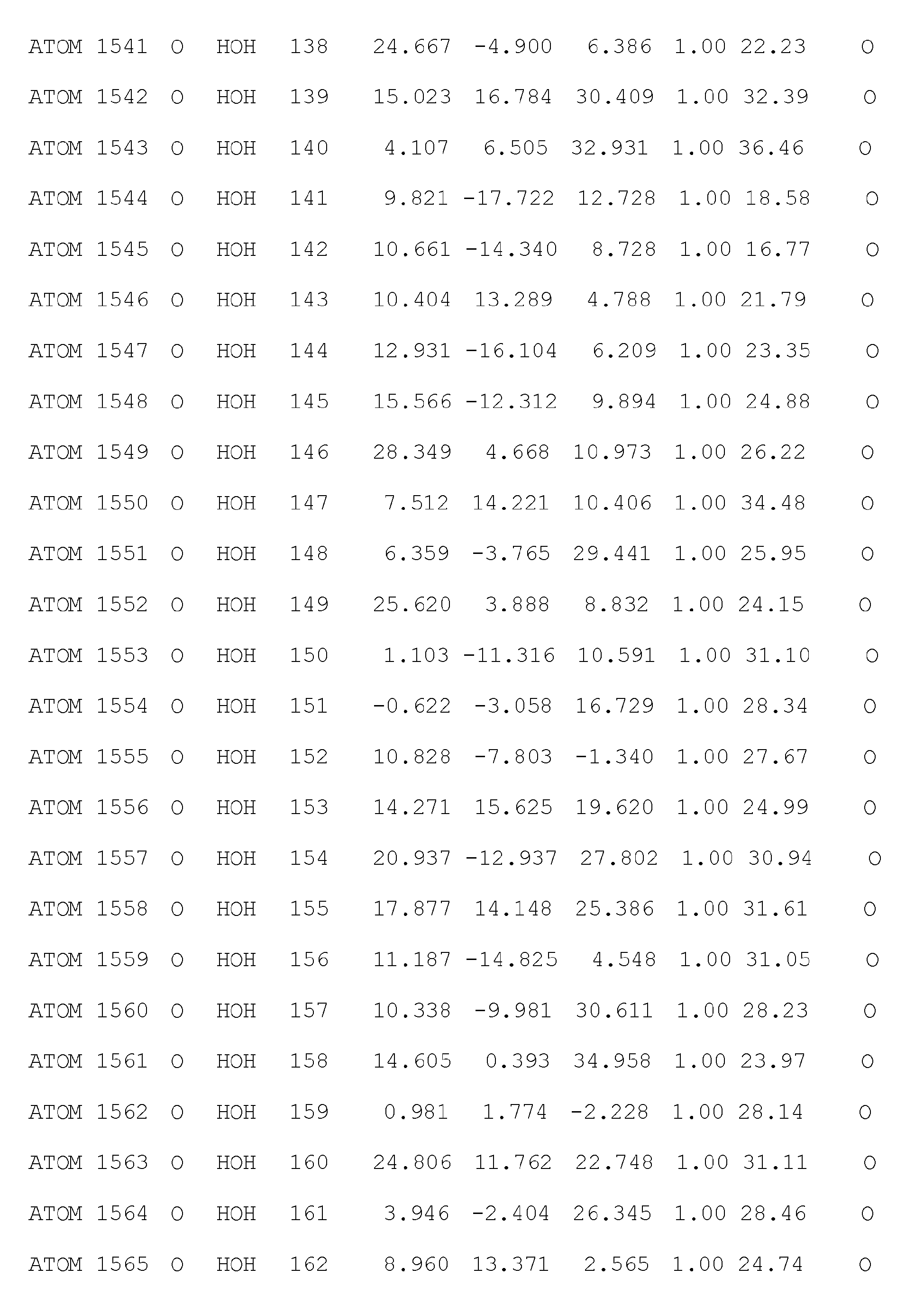

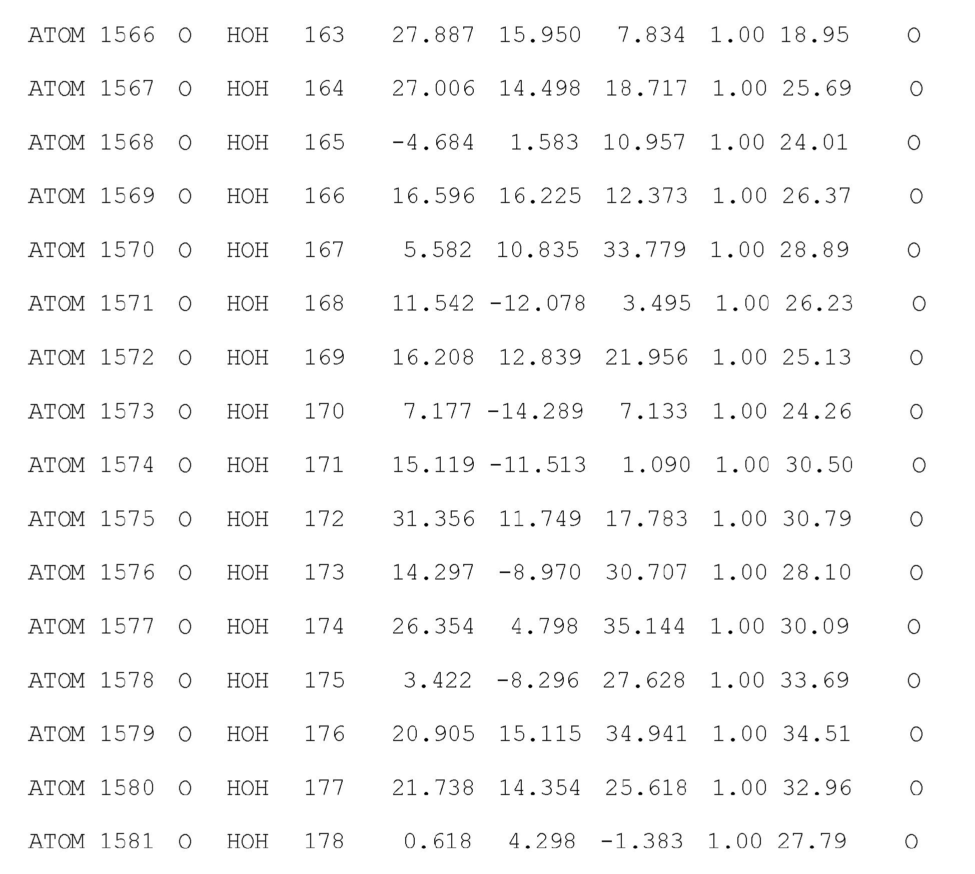

### (Reference Example 1)

M-Ras P40D was analyzed using ³¹P-NMR in the presence or absence of Raf. M-Ras P40D was subjected to mass expression and partial purification using *Escherichia coli* (AG1 strain) and then purified to a high purity (purity of 95% or more on SDS-PAGE) by anion exchange chromatography. GDP bound to the purified protein was hydrolyzed with alkaline phosphatase and replaced by a nonhydrolyzable GTP analog Gpp(NH)p. After that, the replacement efficiency to Gpp(NH)p was confirmed by reverse phase chromatography. When the replacement efficiency was 95% or more, the sample solution was replaced by a buffer for NMR (50 mM Tris-HCl, pH 7.4, 100 to 150 mM NaCl, 5 mM MgCl₂, 1 mM EDTA, 1 mM DTT) and concentrated to a concentration of 1 mM. The resultant sample solution was supplemented with 10% D₂O, then loaded in a 5-mm Shigemi measurement tube, and subjected to ³¹P-NMR measurement using a Bruker AVANCE-500 NMR spectrometer (5 mm probe) at 202 MHz at 5°C for 24 hours.

FIG. 8 shows the results. In M-Ras P40D, state 1 was extremely predominant in the absence of Raf, whereas state 2 was predominant in the presence of Raf.

### Industrial Applicability

As described above, the information about a conformation including a pocket peculiarly found in state 1 of Ras-GTP was able to be obtained by the X-ray crystallographic analysis of the mutant Ras polypeptide of the present invention. Thus, by use of atomic coordinates of the Ras-GTP having a pocket, a novel Ras function inhibitor can be obtained by selecting a low molecular weight organic compound capable of stably binding to a pocket by virtual screening on a computer and verifying an Ras function inhibitory action of the selected candidate compound biochemically and cell biologically. The novel Ras function inhibitor obtained by this method may serve as a candidate for, for example, an anti-cancer agent.

## Claims

1. A co-crystal, comprising:
a mutant Ras polypeptide; and
GTP or a GTP analog,
wherein the mutant Ras polypeptide comprises one or more substitutions of amino acid residues introduced in a site including a vicinity of the switch I region in a partial polypeptide of Ras, and adopts a conformation having a pocket on the molecular surface through the introduced mutation.

2. A co-crystal according to claim 1, wherein the mutant Ras polypeptide comprises the following polypeptide 1) or 2):
1) a polypeptide in which the partial polypeptide comprises a partial polypeptide of H-Ras formed of amino acid residues at positions 1 to 166 set forth in SEQ ID NO: 1, and the mutation comprises a threonine-to-serine substitution at position 35 of an amino acid sequence set forth in SEQ ID NO: 1; or
2) a polypeptide in which the partial polypeptide comprises a partial polypeptide of M-Ras formed of amino acid residues at positions 1 to 178 set forth in SEQ ID NO: 2, and the mutation comprises a proline-to-aspartic acid substitution at position 40 of an amino acid sequence set forth in SEQ ID NO: 2.

3. A co-crystal according to claim 1 or 2, wherein the co-crystal has a space group of R32, I222, or C2 and lattice constants of a=30 to 100 Å, b=60 to 100 Å, c=70 to 125 Å, α=90°, β=90 to 100°, and γ=90 to 120°.

4. A co-crystal according to claim 2 or 3, wherein the mutant Ras polypeptide comprises the polypeptide according the item 1) and the co-crystal has a space group of R32 and lattice constants of a=b=91 . 81 to 95.20 Å, c=116.13 to 121.97 Å, a=β=90°, and γ=120°.

5. A co-crystal according to claim 4, wherein the co-crystal has lattice constants of a=94.20 Å, b=94.20 Å, c=120.97 Å, α=β=90.00°, and γ=120.00°.

6. A co-crystal according to claim 1 or 2, wherein the mutant Ras polypeptide comprises the polypeptide according the item 1) and the co-crystal has a space group of I222 and lattice constants of a=34 .28 to 34.88 Å, b=81.20 to 82.80 Å, c=120.80 to 123.20 Å, and α=β=γ=90°.

7. A co-crystal according to claim 1 or 2, wherein the co-crystal has lattice constants of a=34.58 Å, b=82.00 Å, c=122.00 Å, and α=β=γ=90°.

8. A co-crystal according to claim 1 or 2, wherein the mutant Ras polypeptide comprises the polypeptide according the item 2) and the co-crystal has a space group of C2 and lattice constants of a=33.12 to 34.04 Å, b=64. 69 to 66.33 Å, c=72. 67 to 74.93 Å, a=γ=90°, and β=94.92 to 95.33°.

9. A co-crystal according to claim 8, wherein the co-crystal has lattice constants of a=33.72 Å, b=65.70 Å, c=74.08 Å, α=γ=90.00°, and β=95.02°.

10. A production method for a co-crystal comprising:
a mutant Ras polypeptide; and
GTP or a GTP analog,
wherein the mutant Ras polypeptide comprises one or more substitutions of amino acid residues introduced in a site including a vicinity of a switch I region in a partial polypeptide of Ras, and adopts a conformation having a pocket on the molecular surface through the introduced mutation.

11. A production method according to claim 10, wherein the mutant Ras polypeptide comprises the following polypeptide 1) or 2):
1) a polypeptide in which the partial polypeptide comprises a partial polypeptide of H-Ras formed of amino acid residues at positions 1 to 166 set forth in SEQ ID NO: 1, and the mutation comprises a threonine-to-serine substitution at position 35 of an amino acid sequence set forth in SEQ ID NO: 1; or
2) a polypeptide in which the partial polypeptide comprises a partial polypeptide of M-Ras formed of amino acid residues at positions 1 to 178 set forth in SEQ ID NO: 2, and the mutation comprises a proline-to-aspartic acid substitution at position 40 of an amino acid sequence set forth in SEQ ID NO: 2.

12. A production method according to claim 11, wherein the mutant Ras polypeptide comprises the polypeptide according to the item 1), and the method comprises the step of crystallizing a solution including the Ras protein by a vapor diffusion method using a precipitant solution including ammonium sulfate or polyethylene glycol having a molecular weight of 2,000 to 5,000.

13. A production method according to claim 12, wherein the mutant Ras polypeptide comprises the polypeptide according to the item 2), and the method comprises the step of crystallizing a solution including the Ras protein by a vapor diffusion method using a precipitant solution including polyethylene glycol having an average molecular weight of 1,000 to 2,000.

14. A screening method for a Ras function inhibitor, comprising the following steps of:
(1) designing or selecting a candidate compound capable of binding to a pocket using information about a conformation obtained from the co-crystal according to any one of claims 1 to 9;
(2) synthesizing or acquiring the designed or selected candidate compound; and
(3) bringing the candidate compound into contact with Ras to examine a Ras function-inhibiting activity of the candidate compound.

15. A screening method for a Ras function inhibitor, comprising the following steps of:
(1) producing a co-crystal comprising a mutant Ras polypeptide and GTP or a GTP analog by the method according to any one of claims 10 to 13;
(2) elucidating a structure of the crystallized polypeptide by X-ray crystallographic analysis to obtain information about a conformation of the polypeptide;
(3) designing or selecting a candidate compound capable of binding to a pocket using the obtained information about the conformation;
(4) synthesizing or acquiring the designed or selected candidate compound; and
(5) bringing the candidate compound into contact with Ras to examine an Ras function-inhibiting activity of the candidate compound.
